# EUROPEAN PATENT SPECIFICATION

(11) **EP 2 598 642 B1**
(45) Date of publication and mention of the grant of the patent: **02.09.2015**
(21) Application number: 11812939.4
(22) Date of filing: 14.07.2011
(51) Int. Cl.: C12P 7/10, C12N 9/42, C21C 1/00, A61K 38/47, C12P 19/14

(54) **POLYPEPTIDES FOR USE IN THE DECONSTRUCTION OF CELLULOSE**
POLYPEPTIDE ZUR VERWENDUNG IN DER DEKONSTRUKTION VON ZELLULOSE
POLYPEPTIDES UTILISABLES DANS LA DÉCONSTRUCTION DE LA CELLULOSE

(30) Priority: 30.07.2010 US 369588 P
(43) Date of publication of application: 05.06.2013
(73) Proprietor: The Regents of the University of California, Oakland, CA 94607 (US); University of Maryland, Baltimore, Baltimore, MD 21201 (US)
(72) Inventor: GRAHAM, Joel, Edward, Baltimore MD 21202 (US); CLARK, Melinda, E., Charlottesville, VA 22902 (US); ROBB, Frank, Thomas, Gaithersburg MD 20882 (US); CLARK, Douglas, S., Orinda CA 94563 (US); BLANCH, Harvey, W., San Francisco CA 94158 (US)
(74) Representative: Zacco Sweden AB
(86) International application number: PCT/US2011/044074
(87) International publication number: WO 2012/015605

(56) References cited:
- WO-A1-2007/118935
- WO-A1-2008/025164
- POTTKAEMPER J ET AL: "Applying metagenomics for the identification of bacterial cellulases that are stable in ionic liquids", GREEN CHEMISTRY, ROYAL SOCIETY OF CHEMISTRY, CAMBRIDGE, GB, vol. 11, no. 7, 1 July 2009 (2009-07-01), pages 957-965, XP008122938, ISSN: 1463-9262, DOI: 10.1039/B820157A [retrieved on 2009-04-15]
- DATABASE UNIPROT [Online] 29 May 2007 COPELAND, A. ET AL., XP003032501 Database accession no. A4XMG8
- DATABASE EMBL [Online] 03 June 2003 MUZNY, D.M. ET AL., XP003032502 Database accession no. AC133618
- DATABASE EMBL [Online] 03 June 2010 RUSCH, D.B. ET AL., XP003032503 Database accession no. EK682540
- DATABASE UNIPROT [Online] 23 October 2007 SUDARSANAM, P. ET AL., XP003032504 Database accession no. A7VX72
- DATABASE UNIPROT [Online] 01 December 2001 FINAN, T.M. ET AL., XP003032505 Database accession no. Q92TY9

### SEQUENCE LISTING

<110> GRAHAM, JOEL E. CLARK, MELINDA E. ROBB, FRANK T. CLARK, DOUGLAS S. BLANCH, HARVEY W.
<120> POLYPEPTIDES FOR USE IN THE
   DECONSTRUCTION OF CELLULOSE
<130> 67779-20009.40
<150> 61/369,588
   <151> 2010-07-30
<160> 37
<170> FastSEQ for Windows Version 4.0
<210> 1
   <211> 842
   <212> PRT
   <213> Artificial Sequence
<220>
   <223> EBI5326244 deduced protein sequence
<400> 1
   Leu Lys Lys Val His Ile Ile Ala Ile Val Val Ile Ile Ala Ile Ala
    1               5                  10                  15
   Phe Ala Leu Ile Leu Ala Arg Tyr Tyr Thr Met Gln Arg Gly Tyr Glu
               20                  25                  30
   Thr Val Thr Pro Thr Thr Pro Pro Gln Gln Thr Thr Thr Thr Glu Thr
           35                  40                  45
   Thr Pro Val Pro Thr Glu Ala Gly Thr Thr Thr Pro Ile Thr Glu Ala
       50                  55                  60
   Thr Val Thr Gln Pro Pro Gln Thr Pro Thr Thr Pro Ser Pro Gln Thr
   65                  70                  75                  80
   Pro Thr Thr Pro Thr Ala Leu Pro Thr Pro Ser Pro Thr Pro Thr Ala
                   85                  90                  95
   Pro Ser Ala Thr Val Thr Glu Thr Thr Ser Pro Gln Thr Pro Thr Thr
               100                 105                 110
   Thr Ile Thr Thr Glu Thr Thr Thr Thr Pro Ala Pro Gln Pro Gln Val
           115                 120                 125
   Val Phe Leu Lys Leu Pro Glu Gly Glu Glu Pro Lys Phe Gly Leu Val
       130                 135                 140
   Glu Ile Ala Phe Asn Ile Ser Gly Leu Ser Tyr Ser Asn Pro Phe Asp
   145                 150                 155                 160
   Thr Ser Asp Ile Asp Val Trp Val His Ile Glu Thr Pro Ser Gly Ser
                   165                 170                 175
   Arg Val Ala Val Pro Ala Phe Tyr Phe Gln Asn Tyr Thr Val Lys Arg
               180                 185                 190
   Leu Gly Pro Gly Glu Glu Ile Ile Val Arg Val Gly Arg Pro Tyr Trp
           195                 200                 205
   Leu Ala Arg Phe Ala Pro Val Glu Glu Gly Val His Lys Phe Tyr Val
       210                 215                 220
   Lys Ala Val Asp Gly Arg Gly Ser Ala Val Val Ser Glu Ile Arg Glu
   225                 230                 235                 240
   Phe Met Val Lys Gly Val Ala Gly Arg Gly Phe Val Arg Val Asp Ser
                   245                 250                 255
   Gly Lys Arg Leu Phe Val Phe Asp Ser Gly Glu Ser Met Phe Met Leu
               260                 265                 270
   Gly Ile Asp Val Ala Trp Pro Pro Asp Arg Arg Ser Ser Ile Ser Phe
               275                 280                 285
   Tyr Glu Gln Trp Phe Asp Lys Leu Asn Lys Ser Gly Ile Lys Val Val
           290                 295                 300
   Arg Ile Gly Leu Val Pro Trp Ala Leu Thr Leu Glu Trp Ser Lys Leu
   305                 310                 315                 320
   His Tyr Tyr Ser Leu Asp Asp Ala Ala Arg Ile Asp Glu Ile Val Lys
                   325                 330                 335
   Leu Ala Glu Lys Tyr Asp Ile Tyr Ile Val Phe Val Phe Met Trp His
               340                 345                 350
   Gly Glu Leu Ala Asp Asn Trp Gly Asp Asn Pro Tyr Asn Ala Ala Arg
           355                 360                 365
   Gly Gly Pro Leu Gln Ser Pro Glu Glu Phe Trp Ser Asn Ala Val Ala
       370                 375                 380
   Ile Ser Ile Phe Lys Asp Lys Val Arg Tyr Ile Ile Ala Arg Trp Gly
   385                 390                 395                 400
   Tyr Ser Thr His Ile Leu Ala Trp Glu Leu Ile Asn Glu Ala Asp Leu
                   405                 410                 415
   Thr Thr Asn Phe Phe Ser Ala Arg Ser Ala Phe Val Ser Trp Val Lys
               420                 425                 430
   Glu Ile Ser Ser Tyr Ile Lys Ser Val Asp Pro Tyr Asn Arg Ile Val
           435                 440                 445
   Thr Val Asn Leu Ala Asp Tyr Asn Ser Glu Pro Arg Val Trp Ser Val
       450                 455                 460
   Glu Ser Ile Asp Ile Ile Asn Val His Arg Tyr Gly Pro Glu Gly Phe
   465                 470                 475                 480
   Lys Asp Ile Ala Leu Ala Ile Pro Ser Ile Val Glu Gly Leu Trp Asn
                   485                 490                 495
   Thr Tyr Arg Lys Pro Ile Ile Ile Thr Glu Phe Gly Val Asp Tyr Arg
               500                 505                 510
   Trp Ile Gly Tyr Pro Gly Phe Lys Gly Thr Pro Tyr Trp Ala Tyr Asp
           515                 520                 525
   Lys Ser Gly Val Gly Leu His Glu Gly Leu Trp Ser Ser Ile Phe Ser
       530                 535                 540
   Leu Ser Pro Val Ser Ala Met Ser Trp Trp Trp Asp Thr Gln Ile Asp
   545                 550                 555                 560
   Ser Tyr Asn Leu Trp Tyr His Tyr Lys Ala Leu Tyr Glu Phe Leu Lys
                   565                 570                 575
   Ser Val Asp Pro Val Arg Gly Gly Leu Gly Lys Ala Arg Ala Ser Leu
               580                 585                 590
   Val Ile Thr Asp Val Thr Pro Ser Ser Ile Thr Leu Tyr Pro Leu Ala
           595                 600                 605
   Gly Trp Val Trp Val Ser Pro Val Arg Glu Asn Arg Leu Val Ile Arg
       610                 615                 620
   Pro Asp Gly Ala Ile Glu Gly Arg Val Asp Leu Leu Ser Gly Phe Ile
   625                 630                 635                 640
   Tyr Gly Thr Cys His Ser Gln Arg Thr Leu Asn Pro Val Phe Thr Val
                   645                 650                 655
   Met Phe Ile Asp Arg Gly Arg Val Val Leu His Ile Asn Ser Val Gly
               660                 665                 670
   Arg Gly Ser Ala Lys Leu Val Ile Tyr Val Asn Gly Ser Leu Ala Thr
           675                 680                 685
   Gln Leu Asp Leu Pro Asp Lys Asp Gly Lys Ser Asp Gly Ser Ala Asn
       690                 695                 700
   Glu Tyr Asp Met Asp Val Glu Leu Trp Phe Glu Pro Gly Thr Tyr Glu
   705                 710                 715                 720
   Ile Lys Ile Asp Ser Glu Ala Cys Asp Trp Phe Thr Trp Asp Tyr Ile
                   725                 730                 735
   Val Phe Glu Asn Ala Val Tyr Arg Ala Ala Lys Val Asp Leu Tyr Ala
               740                 745                 750
   Leu Ala Asn Ser Thr Phe Ala Met Leu Trp Val Arg Asn Lys Asp Tyr
           755                 760                 765
   Asn Trp Trp Asn Val Val Val Leu Asn Lys Thr Leu Glu Pro Ala Glu
       770                 775                 780
   Gly Val Glu Val Glu Ile Arg Gly Leu Gln Asp Gly Val Tyr Arg Val
   785                 790                 795                 800
   Glu Phe Trp Asp Thr Cys Arg Gly Val Val Val Lys Ser Met Glu Val
                   805                 810                 815
   Gln Val Ser Asn Gly Val Ala Arg Val Pro Val Gly Ser Val Glu Lys
               820                 825                 830
   Asp Ile Ala Met Lys Ile Thr Arg Ala Gly
           835                 840
<210> 2
   <211> 2532
   <212> DNA
   <213> Artificial Sequence
<220>
   <223> EBI5326244 coding region
<400> 2
   atgttgaaaa aggttcacat tattgctata gtagttataa tagcgatagc ttttgcactt 60
   atactagcac ggtactacac aatgcagaga ggctatgaaa cagtgacacc tacaacacca 120
   cctcagcaaa ctactaccac agaaacaact ccagtgccta cagaggcagg tactacaaca 180
   ccaataactg aggccactgt gactcaaccc cctcaaaccc ctacaacacc ttcaccacaa 240
   acaccaacaa caccaacagc gttgcccacg ccatcaccaa cccccacagc gccctctgcc 300
   acagtaacag agactacatc gcctcaaact cctacaacta caataactac agaaacaaca 360
   actacaccag ccccccaacc ccaggtggtg tttctaaagc taccagaggg agaggagcca 420
   aagtttggct tagttgaaat agcctttaac atatctggtc taagctactc aaaccccttt 480
   gacacaagcg atattgatgt gtgggtgcac atagagacgc caagtggctc tagagtggct 540
   gtaccagctt tctacttcca gaactacact gtgaaaaggc ttggaccagg ggaggagatc 600
   atagtcaggg ttggaaggcc atactggctc gctaggttcg cacctgttga ggagggggtg 660
   cacaagttct acgtaaaggc agttgatggc agggggagtg ctgtggtgag cgagattaga 720
   gagtttatgg ttaagggggt ggctggcagg gggtttgtca gagttgacag tggcaaaagg 780
   ctattcgtat ttgacagcgg tgaatcaatg tttatgctgg ggatagatgt tgcgtggcca 840
   ccagatagga ggagctctat atcgttctat gagcagtggt ttgataaact aaataagagt 900
   gggattaagg ttgtgagaat aggcctagtg ccctgggctc taacactaga gtggagcaag 960
   ctccactact acagcttaga tgacgctgct agaatagatg agattgtgaa gcttgctgaa 1020
   aagtatgaca tatacatagt gtttgtgttt atgtggcatg gagagcttgc ggataactgg 1080
   ggggataacc catacaatgc agcaaggggt ggtcctcttc agagcccaga ggagttctgg 1140
   agcaatgcag tagctatcag tatatttaag gataaggtga ggtacattat agctaggtgg 1200
   gggtactcaa cacacatact cgcatgggag ctgataaacg aggctgacct aacaacaaac 1260
   ttctttagcg ctagaagtgc ttttgtgagc tgggttaagg agataagcag ctacataaag 1320
   tccgtagacc cctacaacag gattgtcact gtgaacctcg ctgactacaa ttctgagcca 1380
   agggtgtgga gcgtagagtc catagacatc ataaatgtgc ataggtatgg gccagagggc 1440
   tttaaggaca tagccttggc tattccaagc atagtagagg ggctttggaa cacctacaga 1500
   aagcctatta taataacaga gtttggtgtt gactatcggt ggattggcta cccaggcttt 1560
   aaaggaaccc cctactgggc atacgacaag agtggggttg ggcttcatga agggctctgg 1620
   agctctattt tcagcctctc cccagtttct gctatgagct ggtggtggga tacacagata 1680
   gactcttata atctgtggta ccactacaaa gccctctacg agtttctaaa gagtgtcgac 1740
   cctgttagag gaggcttggg caaggccaga gcatcgctag tcattacaga tgtgaccccc 1800
   tctagcataa cactataccc cttagcgggc tgggtgtggg tctcgccagt gagagaaaat 1860
   aggcttgtta taaggcctga tggggctatc gaggggaggg ttgatttgct aagtgggttt 1920
   atctatggca cgtgccacag ccagcggaca ctaaacccag tatttactgt gatgttcatt 1980
   gatagaggca gggtggtgct acacataaac tctgttggca ggggctctgc aaagctcgtg 2040
   atatatgtta atggctctct agctacacaa ctggacttgc ctgataagga tggtaagagt 2100
   gatgggagtg caaatgagta cgacatggat gtggagctgt ggtttgagcc tggcacctac 2160
   gaaataaaaa ttgatagtga agcttgcgac tggttcacat gggactacat agtgtttgaa 2220
   aatgctgtgt atagagctgc taaagtagat ctctatgcac ttgcaaacag cacctttgca 2280
   atgctctggg taaggaacaa ggactacaac tggtggaacg tagtggtgct gaacaagact 2340
   ctagagcctg ctgagggagt agaggtagag attagaggac tgcaagatgg ggtgtacaga 2400
   gtagagtttt gggacacatg cagaggggtg gtggtgaaga gcatggaggt tcaagtgtca 2460
   aatggtgtag ccagggttcc ggtgggtagc gtagaaaagg acatagctat gaaaatcact 2520
   agggctggct aa                                                     2532
<210> 3
   <211> 2553
   <212> DNA
   <213> Artificial Sequence
<220>
   <223> EBI5326244 coding region
<400> 3
   atgctgaaga aagtccacat catcgcaatc gtcgtcatca tcgccatcgc attcgcactg 60
   attctggcgc gctactacac catgcagcgt ggctatgaaa cggtcactcc gaccaccccg 120
   cctcaacaaa cgacgaccac cgaaaccacc ccggtgccta ccgaagcggg caccaccact 180
   ccgatcactg aggcgaccgt gacccagccg cctcagacgc caacgacgcc atcgccgcaa 240
   acgccgacga ccccaaccgc cctgccgacc ccgagcccga cgccgaccgc gccaagcgcc 300
   acggtcactg aaacgaccag cccgcagacc ccgaccacca ccatcacgac ggaaaccact 360
   accacgccgg caccgcagcc gcaggttgtg tttctgaaat tgccggaggg tgaagaaccg 420
   aaattcggcc tggttgagat cgctttcaac attagcggcc tgagctacag caaccctttt 480
   gataccagcg atattgacgt ttgggttcat attgaaaccc cgagcggtag ccgcgtggcc 540
   gttccggcat tttactttca aaactacacg gtcaagcgcc tgggtccggg tgaagaaatc 600
   atcgtgcgtg tgggtcgtcc gtattggctg gctcgctttg cgccggtcga agagggcgtt 660
   cacaagttct acgtgaaagc ggttgatggt cgcggctccg cggtggttag cgagattcgt 720
   gagtttatgg taaagggcgt tgccggtcgc ggcttcgttc gtgtggactc tggcaaacgt 780
   ctgtttgtgt tcgatagcgg cgagagcatg tttatgctgg gtattgatgt cgcgtggcca 840
   ccggaccgtc gttctagcat cagcttttac gagcaatggt tcgacaaatt gaacaagtct 900
   ggcattaaag ttgttcgcat tggtctggtc ccgtgggcgc tgaccctgga atggagcaag 960
   ctgcactact atagcctgga tgatgcggct cgcattgacg agatcgtcaa actggcagag 1020
   aagtatgaca tctacattgt ctttgtgttt atgtggcatg gtgagttggc agacaattgg 1080
   ggcgataacc cgtataacgc ggctcgtggt ggtccgctgc agagcccgga agagttttgg 1140
   agcaatgcgg tggcaattag catctttaag gacaaagttc gttatatcat cgcgcgctgg 1200
   ggttatagca ctcacattct ggcgtgggag ctgattaatg aggcagactt gaccacgaac 1260
   ttcttttccg cccgtagcgc cttcgtcagc tgggtcaaag agatcagcag ctacattaag 1320
   agcgtggacc cgtacaaccg catcgtcacc gtcaatctgg ctgattacaa ctctgaaccg 1380
   cgtgtttggt ccgtggagtc catcgacatc atcaatgttc atcgctatgg tccggaaggt 1440
   ttcaaggaca ttgccctggc aattccgtcg attgtcgagg gtctgtggaa cacttatcgt 1500
   aagcctatca tcattaccga gttcggcgtg gattatcgtt ggattggcta tccgggtttc 1560
   aaaggtaccc cgtactgggc gtatgataag agcggtgttg gcttgcacga gggcctgtgg 1620
   agcagcatct tctcgctgtc cccggtaagc gcgatgtctt ggtggtggga cacccaaatt 1680
   gacagctaca acctgtggta tcattacaaa gcgctgtacg agttcctgaa atccgttgac 1740
   ccggttcgtg gtggcctggg taaagcccgt gcgagcttgg ttatcacgga cgtgacgccg 1800
   agcagcatta ccctgtatcc gctggcgggt tgggtgtggg tgtcgccggt ccgtgagaat 1860
   cgtttggtta ttcgtccaga tggcgcaatc gaaggccgcg ttgacctgct gagcggtttc 1920
   atctatggta cgtgtcacag ccagcgtacc ctgaatccgg tttttacggt catgttcatt 1980
   gatcgtggtc gcgtggtgct gcacattaac agcgtgggtc gtggttctgc taagctggtg 2040
   atttacgtca atggcagcct ggcgacgcaa ctggatttgc cggacaaaga cggcaagagc 2100
   gacggtagcg cgaacgagta cgatatggac gtcgagctgt ggttcgagcc gggtacctac 2160
   gagatcaaaa ttgattccga agcttgcgac tggttcacct gggattacat tgttttcgaa 2220
   aatgcggttt atcgtgcggc aaaggttgat ctgtatgcct tggcaaacag cacctttgcc 2280
   atgctgtggg tacgtaacaa ggattacaat tggtggaatg tggtggtgct gaataagacc 2340
   ctggaaccgg cggagggtgt tgaagtggag atccgtggcc tgcaagacgg tgtgtaccgc 2400
   gtcgaattct gggacacgtg ccgcggtgtg gttgttaaaa gcatggaagt ccaggtttct 2460
   aatggtgtgg cgcgtgtccc ggttggtagc gtcgagaaag atattgcaat gaagattacc 2520
   cgtgcaggcc atcatcacca tcaccactaa taa                              2553
<210> 4
   <211> 2529
   <212> DNA
   <213> Artificial Sequence
<220>
   <223> EBI5326244 coding region
<400> 4
   atgctgaaga aagtccacat catcgcaatc gtcgtcatca tcgccatcgc attcgcactg 60
   attctggcgc gctactacac catgcagcgt ggctatgaaa cggtcactcc gaccaccccg 120
   cctcaacaaa cgacgaccac cgaaaccacc ccggtgccta ccgaagcggg caccaccact 180
   ccgatcactg aggcgaccgt gacccagccg cctcagacgc caacgacgcc atcgccgcaa 240
   acgccgacga ccccaaccgc cctgccgacc ccgagcccga cgccgaccgc gccaagcgcc 300
   acggtcactg aaacgaccag cccgcagacc ccgaccacca ccatcacgac ggaaaccact 360
   accacgccgg caccgcagcc gcaggttgtg tttctgaaat tgccggaggg tgaagaaccg 420
   aaattcggcc tggttgagat cgctttcaac attagcggcc tgagctacag caaccctttt 480
   gataccagcg atattgacgt ttgggttcat attgaaaccc cgagcggtag ccgcgtggcc 540
   gttccggcat tttactttca aaactacacg gtcaagcgcc tgggtccggg tgaagaaatc 600
   atcgtgcgtg tgggtcgtcc gtattggctg gctcgctttg cgccggtcga agagggcgtt 660
   cacaagttct acgtgaaagc ggttgatggt cgcggctccg cggtggttag cgagattcgt 720
   gagtttatgg taaagggcgt tgccggtcgc ggcttcgttc gtgtggactc tggcaaacgt 780
   ctgtttgtgt tcgatagcgg cgagagcatg tttatgctgg gtattgatgt cgcgtggcca 840
   ccggaccgtc gttctagcat cagcttttac gagcaatggt tcgacaaatt gaacaagtct 900
   ggcattaaag ttgttcgcat tggtctggtc ccgtgggcgc tgaccctgga atggagcaag 960
   ctgcactact atagcctgga tgatgcggct cgcattgacg agatcgtcaa actggcagag 1020
   aagtatgaca tctacattgt ctttgtgttt atgtggcatg gtgagttggc agacaattgg 1080
   ggcgataacc cgtataacgc ggctcgtggt ggtccgctgc agagcccgga agagttttgg 1140
   agcaatgcgg tggcaattag catctttaag gacaaagttc gttatatcat cgcgcgctgg 1200
   ggttatagca ctcacattct ggcgtgggag ctgattaatg aggcagactt gaccacgaac 1260
   ttcttttccg cccgtagcgc cttcgtcagc tgggtcaaag agatcagcag ctacattaag 1320
   agcgtggacc cgtacaaccg catcgtcacc gtcaatctgg ctgattacaa ctctgaaccg 1380
   cgtgtttggt ccgtggagtc catcgacatc atcaatgttc atcgctatgg tccggaaggt 1440
   ttcaaggaca ttgccctggc aattccgtcg attgtcgagg gtctgtggaa cacttatcgt 1500
   aagcctatca tcattaccga gttcggcgtg gattatcgtt ggattggcta tccgggtttc 1560
   aaaggtaccc cgtactgggc gtatgataag agcggtgttg gcttgcacga gggcctgtgg 1620
   agcagcatct tctcgctgtc cccggtaagc gcgatgtctt ggtggtggga cacccaaatt 1680
   gacagctaca acctgtggta tcattacaaa gcgctgtacg agttcctgaa atccgttgac 1740
   ccggttcgtg gtggcctggg taaagcccgt gcgagcttgg ttatcacgga cgtgacgccg 1800
   agcagcatta ccctgtatcc gctggcgggt tgggtgtggg tgtcgccggt ccgtgagaat 1860
   cgtttggtta ttcgtccaga tggcgcaatc gaaggccgcg ttgacctgct gagcggtttc 1920
   atctatggta cgtgtcacag ccagcgtacc ctgaatccgg tttttacggt catgttcatt 1980
   gatcgtggtc gcgtggtgct gcacattaac agcgtgggtc gtggttctgc taagctggtg 2040
   atttacgtca atggcagcct ggcgacgcaa ctggatttgc cggacaaaga cggcaagagc 2100
   gacggtagcg cgaacgagta cgatatggac gtcgagctgt ggttcgagcc gggtacctac 2160
   gagatcaaaa ttgattccga agcttgcgac tggttcacct gggattacat tgttttcgaa 2220
   aatgcggttt atcgtgcggc aaaggttgat ctgtatgcct tggcaaacag cacctttgcc 2280
   atgctgtggg tacgtaacaa ggattacaat tggtggaatg tggtggtgct gaataagacc 2340
   ctggaaccgg cggagggtgt tgaagtggag atccgtggcc tgcaagacgg tgtgtaccgc 2400
   gtcgaattct gggacacgtg ccgcggtgtg gttgttaaaa gcatggaagt ccaggtttct 2460
   aatggtgtgg cgcgtgtccc ggttggtagc gtcgagaaag atattgcaat gaagattacc 2520
   cgtgcaggc                                                         2529
<210> 5
   <211> 849
   <212> PRT
   <213> Artificial Sequence
<220>
   <223> Synthetic Construct
<400> 5
   Met Leu Lys Lys Val His Ile Ile Ala Ile Val Val Ile Ile Ala Ile
    1               5                  10                  15
   Ala Phe Ala Leu Ile Leu Ala Arg Tyr Tyr Thr Met Gln Arg Gly Tyr
               20                  25                  30
   Glu Thr Val Thr Pro Thr Thr Pro Pro Gln Gln Thr Thr Thr Thr Glu
           35                  40                  45
   Thr Thr Pro Val Pro Thr Glu Ala Gly Thr Thr Thr Pro Ile Thr Glu
       50                  55                  60
   Ala Thr Val Thr Gln Pro Pro Gln Thr Pro Thr Thr Pro Ser Pro Gln
   65                  70                  75                  80
   Thr Pro Thr Thr Pro Thr Ala Leu Pro Thr Pro Ser Pro Thr Pro Thr
                   85                  90                  95
   Ala Pro Ser Ala Thr Val Thr Glu Thr Thr Ser Pro Gln Thr Pro Thr
               100                 105                 110
   Thr Thr Ile Thr Thr Glu Thr Thr Thr Thr Pro Ala Pro Gln Pro Gln
           115                 120                 125
   Val Val Phe Leu Lys Leu Pro Glu Gly Glu Glu Pro Lys Phe Gly Leu
       130                 135                 140
   Val Glu Ile Ala Phe Asn Ile Ser Gly Leu Ser Tyr Ser Asn Pro Phe
   145                 150                 155                 160
   Asp Thr Ser Asp Ile Asp Val Trp Val His Ile Glu Thr Pro Ser Gly
                   165                 170                 175
   Ser Arg Val Ala Val Pro Ala Phe Tyr Phe Gln Asn Tyr Thr Val Lys
               180                 185                 190
   Arg Leu Gly Pro Gly Glu Glu Ile Ile Val Arg Val Gly Arg Pro Tyr
           195                 200                 205
   Trp Leu Ala Arg Phe Ala Pro Val Glu Glu Gly Val His Lys Phe Tyr
       210                 215                 220
   Val Lys Ala Val Asp Gly Arg Gly Ser Ala Val Val Ser Glu Ile Arg
   225                 230                 235                 240
   Glu Phe Met Val Lys Gly Val Ala Gly Arg Gly Phe Val Arg Val Asp
                   245                 250                 255
   Ser Gly Lys Arg Leu Phe Val Phe Asp Ser Gly Glu Ser Met Phe Met
               260                 265                 270
   Leu Gly Ile Asp Val Ala Trp Pro Pro Asp Arg Arg Ser Ser Ile Ser
           275                 280                 285
   Phe Tyr Glu Gln Trp Phe Asp Lys Leu Asn Lys Ser Gly Ile Lys Val
       290                 295                 300
   Val Arg Ile Gly Leu Val Pro Trp Ala Leu Thr Leu Glu Trp Ser Lys
   305                 310                 315                 320
   Leu His Tyr Tyr Ser Leu Asp Asp Ala Ala Arg Ile Asp Glu Ile Val
                   325                 330                 335
   Lys Leu Ala Glu Lys Tyr Asp Ile Tyr Ile Val Phe Val Phe Met Trp
               340                 345                 350
   His Gly Glu Leu Ala Asp Asn Trp Gly Asp Asn Pro Tyr Asn Ala Ala
           355                 360                 365
   Arg Gly Gly Pro Leu Gln Ser Pro Glu Glu Phe Trp Ser Asn Ala Val
       370                 375                 380
   Ala Ile Ser Ile Phe Lys Asp Lys Val Arg Tyr Ile Ile Ala Arg Trp
   385                 390                 395                 400
   Gly Tyr Ser Thr His Ile Leu Ala Trp Glu Leu Ile Asn Glu Ala Asp
                   405                 410                 415
   Leu Thr Thr Asn Phe Phe Ser Ala Arg Ser Ala Phe Val Ser Trp Val
               420                 425                 430
   Lys Glu Ile Ser Ser Tyr Ile Lys Ser Val Asp Pro Tyr Asn Arg Ile
           435                 440                 445
   Val Thr Val Asn Leu Ala Asp Tyr Asn Ser Glu Pro Arg Val Trp Ser
       450                 455                 460
   Val Glu Ser Ile Asp Ile Ile Asn Val His Arg Tyr Gly Pro Glu Gly
   465                 470                 475                 480
   Phe Lys Asp Ile Ala Leu Ala Ile Pro Ser Ile Val Glu Gly Leu Trp
                   485                 490                 495
   Asn Thr Tyr Arg Lys Pro Ile Ile Ile Thr Glu Phe Gly Val Asp Tyr
               500                 505                 510
   Arg Trp Ile Gly Tyr Pro Gly Phe Lys Gly Thr Pro Tyr Trp Ala Tyr
           515                 520                 525
   Asp Lys Ser Gly Val Gly Leu His Glu Gly Leu Trp Ser Ser Ile Phe
       530                 535                 540
   Ser Leu Ser Pro Val Ser Ala Met Ser Trp Trp Trp Asp Thr Gln Ile
   545                 550                 555                 560
   Asp Ser Tyr Asn Leu Trp Tyr His Tyr Lys Ala Leu Tyr Glu Phe Leu
                   565                 570                 575
   Lys Ser Val Asp Pro Val Arg Gly Gly Leu Gly Lys Ala Arg Ala Ser
               580                 585                 590
   Leu Val Ile Thr Asp Val Thr Pro Ser Ser Ile Thr Leu Tyr Pro Leu
           595                 600                 605
   Ala Gly Trp Val Trp Val Ser Pro Val Arg Glu Asn Arg Leu Val Ile
       610                 615                 620
   Arg Pro Asp Gly Ala Ile Glu Gly Arg Val Asp Leu Leu Ser Gly Phe
   625                 630                 635                 640
   Ile Tyr Gly Thr Cys His Ser Gln Arg Thr Leu Asn Pro Val Phe Thr
                   645                 650                 655
   Val Met Phe Ile Asp Arg Gly Arg Val Val Leu His Ile Asn Ser Val
               660                 665                 670
   Gly Arg Gly Ser Ala Lys Leu Val Ile Tyr Val Asn Gly Ser Leu Ala
           675                 680                 685
   Thr Gln Leu Asp Leu Pro Asp Lys Asp Gly Lys Ser Asp Gly Ser Ala
       690                 695                 700
   Asn Glu Tyr Asp Met Asp Val Glu Leu Trp Phe Glu Pro Gly Thr Tyr
   705                 710                 715                 720
   Glu Ile Lys Ile Asp Ser Glu Ala Cys Asp Trp Phe Thr Trp Asp Tyr
                   725                 730                 735
   Ile Val Phe Glu Asn Ala Val Tyr Arg Ala Ala Lys Val Asp Leu Tyr
               740                 745                 750
   Ala Leu Ala Asn Ser Thr Phe Ala Met Leu Trp Val Arg Asn Lys Asp
           755                 760                 765
   Tyr Asn Trp Trp Asn Val Val Val Leu Asn Lys Thr Leu Glu Pro Ala
       770                 775                 780
   Glu Gly Val Glu Val Glu Ile Arg Gly Leu Gln Asp Gly Val Tyr Arg
   785                 790                 795                 800
   Val Glu Phe Trp Asp Thr Cys Arg Gly Val Val Val Lys Ser Met Glu
                   805                 810                 815
   Val Gln Val Ser Asn Gly Val Ala Arg Val Pro Val Gly Ser Val Glu
               820                 825                 830
   Lys Asp Ile Ala Met Lys Ile Thr Arg Ala Gly His His His His His
           835                 840                 845
   His
<210> 6
   <211> 611
   <212> PRT
   <213> Artificial Sequence
<220>
   <223> A4XMG8_CALS8
<400> 6
   Met Arg Lys Lys Ile Thr Ser Leu Ile Ser Tyr Val Ile Ala Phe Leu
    1               5                  10                  15
   Ile Leu Leu Thr Leu Ser Val Thr Gly Phe Gly Ala Pro Ser Asn Ile
               20                  25                  30
   Lys Ile Thr Asp Phe Lys His Leu Thr Ser Val Ala Tyr Lys Tyr Ser
           35                  40                  45
   Lys Phe Glu Ile Ser Phe Lys Thr Pro Ala Phe Lys Gly Asn Cys Phe
       50                  55                  60
   Asp Pro Asp Glu Ile Asp Ile Trp Gly Glu Phe Val Ser Pro Ser Gly
   65                  70                  75                  80
   Lys Lys Tyr Val Met Pro Ala Phe Trp Tyr Gln Asp Tyr Lys Arg Gln
                   85                  90                  95
   Leu Leu Pro Ile Asn Glu Lys Lys Leu Glu Arg Leu Asn Lys Asn Gly
               100                 105                 110
   Ile Gly Gly Thr Ala Ser Asn Asn Pro Asn Glu Pro Gln Gly Lys Glu
           115                 120                 125
   Val Leu Thr Lys Val Gly Gln Pro Glu Trp Arg Ile Arg Phe Cys Pro
       130                 135                 140
   Val Glu Ile Gly Lys Trp Lys Tyr Thr Ile Tyr Val Lys Ala Lys Gly
   145                 150                 155                 160
   Arg Val Gln Asp Phe Lys Lys Gly Glu Phe Ser Val Lys Glu Ala Lys
                   165                 170                 175
   Asn His Gly Phe Ile Arg Val Glu Pro Lys Lys Lys Arg His Phe Val
               180                 185                 190
   Phe Asp Asp Gly Thr Pro Tyr Ile Pro Ile Gly Gln Asn Val Ala Trp
           195                 200                 205
   Trp Thr Ser Pro Thr Arg Gly Ser Tyr Asp Tyr Asn Val Trp Phe Ser
       210                 215                 220
   Lys Met Ala Glu Ser Gly Ala Asn Phe Ala Arg Ile Trp Met Gly Ser
   225                 230                 235                 240
   Trp Ser Phe Gly Leu Tyr Trp Asn Asp Thr Gly Ile Tyr Asp Phe Thr
   245 250 255
   Asn Arg Leu Asp Arg Ala Tyr Gln Leu Asp Lys Val Leu Glu Leu Ala
   260 265 270
   Glu Gln Lys Gly Ile Tyr Ile Met Leu Thr Phe Ile Asn His Gly Gln
   275 280 285
   Phe Ser Thr Lys Val Asn Pro Gln Trp Asn Glu Asn Pro Trp Asn Lys
   290 295 300
   Lys Asn Gly Gly Ile Leu Thr Lys Pro Glu Glu Phe Phe Thr Asn Thr
   305 310 315 320
   Glu Ala Lys Lys Gln Phe Lys Lys Ile Ile Arg Tyr Ile Ile Ala Arg
   325 330 335
   Trp Gly Tyr Ser Thr Asn Ile Met Ser Trp Glu Leu Phe Asn Glu Val
   340 345 350
   Ser Trp Thr Asp Asn Tyr Asp Pro Glu Lys Ser Asn Ala Trp His Lys
   355 360 365
   Glu Met Ala Leu Phe Ile Lys Ser Ile Asp Pro Tyr Lys His Leu Val
   370 375 380
   Ser Ser Ser Ser Ala Val Leu Tyr Asp Pro Leu Glu Lys Val Lys Glu
   385 390 395 400
   Leu Asp Phe Ile Asn Ile His Asp Tyr Gly Ile Thr Asn Phe Cys Lys
   405 410 415
   Asn Ile Pro Ser Lys Gln Arg Asp Ile Ala Asp Met Tyr Asn Lys Pro
   420 425 430
   Ala Phe Phe Cys Glu Met Gly Ile Ala Ser Asp Pro Thr Thr Thr Lys
   435 440 445
   Arg Leu Asp Pro Lys Gly Met His Val His Leu Gly Leu Trp Ala Gly
   450 455 460
   Val Met Gly Gly Gly Ala Gly Thr Gly Met Thr Trp Trp Trp Asp Ser
   465 470 475 480
   Tyr Val His Pro Leu Asn Leu Tyr Thr Tyr Phe Lys Pro Val Ser Leu
   485 490 495
   Tyr Val Lys Lys Ile Pro Trp Asn Asp Pro Phe Leu Lys Tyr Ile Asp
   500 505 510
   Glu Met Gln Leu Asp Ile Ser Asn Phe Asp Val Gly Val His Gly Tyr
   515 520 525
   Ile Lys Gln Asp Ser Ala Tyr Leu Trp Phe Tyr Asp Thr Glu Tyr Ser
   530 535 540
   His Ile Gly Gly Ile Glu Arg Leu Phe Lys Asp Val Thr Val Arg Ile
   545 550 555 560
   Lys Leu Asp Asn Gly Ile Tyr Gln Val Glu Trp Phe Asp Thr Phe Ser
   565 570 575
   Gly Asn Ala Val Lys Lys Glu Asn Val Ala Val Lys Asn Lys Ile Leu
   580 585 590
   Asn Ile Lys Met Pro Asn Trp Lys Ile Asp Ile Ala Phe Ile Ala Lys
   595 600 605
   Lys Val Lys
   610
<210> 7
   <211> 787
   <212> PRT
   <213> Artificial Sequence
<220>
   <223> A7VX72_9CLOT
<400> 7
   Met Thr Gln Asp Lys Glu Leu Cys Phe Leu Trp Asp Asn Thr Ile His
    1               5                   10                  15
   Gly Ala Ile Pro Ala Phe Glu Lys Phe Glu Ala Ala Phe Ala Phe Glu
               20                  25                  30
   Lys Val Phe Glu His Pro Tyr Cys Pro Glu Glu Val Asp Leu Lys Ala
           35                  40                  45
   Tyr Ile Leu Lys Pro Asn Gly Asp Gln Lys Gln Ile Ser Gly Phe Trp
       50                  55                  60
   Tyr Glu Gly Phe Gln Arg Val Leu Arg Asn Gly Arg Glu Ile Leu Ile
   65                  70                  75                  80
   Ser Thr Leu Glu Lys Asp Trp Arg Ile Arg Tyr Ser Ala Gln Val Pro
                   85                  90                  95
   Gly Glu Tyr Arg Tyr Tyr Val Thr Leu Leu Asp Lys Lys Arg His Arg
               100                 105                 110
   Ser Tyr Arg Tyr Pro Glu Lys Gly Glu Leu Ser Phe Thr Val Thr Pro
           115                 120                 125
   Ser Asp Arg Lys Gly Phe Leu Arg Val Ser Ala Arg Asp Pro Ala Tyr
       130                 135                 140
   Leu Glu Phe Ser Asp Gly Ser Pro Tyr Leu Gly Ile Gly His Asn Leu
   145                 150                 155                 160
   Cys Gly Trp Glu Trp Gly Gly Thr Asp Asn Arg Leu Gly Thr Tyr Glu
                   165                 170                 175
   Tyr Asp Arg Trp Leu Ser Ser Met Ala Gln Asn Gly Ala Asn Leu Thr
               180                 185                 190
   Gln Phe Asp Phe Cys Glu Gly Asp Gln Ile Glu Trp Thr Pro Cys Asp
           195                 200                 205
   Asn Glu Leu Pro Phe Ser Glu Asp Trp Lys Gly Leu Asn Glu Tyr Asn
       210                 215                 220
   Gln Gln Asn Ala Trp Lys Met Asp Arg Arg Phe Gln Thr Ala Glu Glu
   225                 230                 235                 240
   Leu Gly Ile Phe Phe Arg Leu Ser Leu Phe His Trp Glu Asp Phe Asp
                   245                 250                 255
   Asp Glu Thr Glu Lys Phe Pro Asp Trp Gly Trp Asn Arg Asn Pro Tyr
               260                 265                 270
   His Asp Gln Asn Gly Gly Pro Ala Lys Asn Val Ser Glu Phe Phe Glu
           275                 280                 285
   Lys Pro Ala Cys Lys Lys Tyr Val Arg Tyr Tyr Leu Lys Tyr Val Ala
       290                 295                 300
   Ala Arg Trp Gly Tyr Ser Pro Asn Leu Met Ala Tyr Glu Leu Trp Asn
   305                 310                 315                 320
   Glu Ile Asp Ala Pro Glu Val Met Trp Arg Ala Gly Glu Asp Tyr Asp
                   325                 330                 335
   Gln Glu Ala Ser Lys Val Ile Gly Trp His Ser Glu Met Gly Ser Tyr
               340                 345                 350
   Leu Lys Gln Leu Asp Ser Lys His Leu Val Thr Ser Ser Phe Ala Asp
           355                 360                 365
   Ser Arg Arg Asp Leu Asn Leu Trp Gln Leu Pro Cys Ile Asp Leu Thr
       370                 375                 380
   Thr Val His Arg Tyr Thr Tyr Phe Asn Glu Glu Tyr Gly Gln Arg Gln
   385                 390                 395                 400
   Tyr Asp Thr Glu Gly Ala Leu Ser Ala Val Leu Lys Glu Arg Phe Ser
                   405                 410                 415
   Gln Val Glu Lys Pro Val Leu Phe Gly Glu Phe Ala Leu Ser Pro Gly
               420                 425                 430
   Gly Asp Ile Gln Lys Asp Tyr Asp Pro Glu Gly Ile Glu Phe His Asn
           435                 440                 445
   Gln Leu Trp Ala Ser Leu Leu Leu Lys Ser Leu Gly Thr Ala Met His
       450                 455                 460
   Trp Thr Trp Gly Ser Tyr Val Asp Lys Asn Arg Leu Tyr Ser Lys Tyr
   465                 470                 475                 480
   Leu Pro Val Ser Arg Phe Phe Ala Gly Glu Asp Leu Arg Arg Thr Val
                   485                 490                 495
   Ser Phe Ser Asn Leu Asp Ala Val Thr Glu Arg Leu Leu Ile Leu Gly
               500                 505                 510
   Leu Arg Lys Thr Asp Arg Ala Cys Leu Trp Ile Lys Lys Arg Asp Trp
           515                 520                 525
   Gly Phe Cys Gln Ala Asn Glu Gly Lys Ser Ser Ser Val Glu Lys Gly
       530                 535                 540
   Arg Thr Ala Glu Val Pro Gly Leu Lys Ala Gly Asp Tyr Gln Val Glu
   545                 550                 555                 560
   Phe Tyr Asp Thr Lys Thr Gly Lys Ile Leu Glu Lys Ser Thr Ile Thr
                   565                 570                 575
   Ala Ala Gly Glu Thr Leu Thr Leu Leu Leu Pro Gly Phe Ser Gly Asp
               580                 585                 590
   Leu Ala Val Lys Leu Lys Pro Lys Glu Lys Asp Thr Leu Trp Lys Ser
           595                 600                 605
   Ile Asp Phe Pro Arg Pro Lys Lys Ser Ser Arg Thr Glu Phe Leu Gln
       610                 615                 620
   Asp Gly Ala Ile Leu Ser Ala Gly Gly Ala Gly Phe Cys Gly Glu Lys
   625                 630                 635                 640
   Glu Glu Tyr Arg Phe Val Tyr Gln Gln Ala Ser Gly Asp Phe Arg Leu
                   645                 650                 655
   Ser Ala Glu Ile Arg Ser Leu Thr Asn Leu Gly Glu Arg Val Ala Ala
               660                 665                 670
   Gly Leu Met Val Arg Asp Ser Leu Glu Pro Glu Ser Gly Tyr Ile Ala
           675                 680                 685
   Val Leu Leu His Pro Tyr Ser Lys Ala Gln Val Ile Ile Arg Arg Asp
       690                 695                 700
   Gly Asn Thr Glu Ile Leu Lys Glu Phe Asp Ala Gly Glu Arg Pro Cys
   705                 710                 715                 720
   Phe Gly Leu Asn Arg Ala Ala Gly Val Leu Thr Val Arg Leu Ala Lys
                   725                 730                 735
   Gln Gly Arg Glu Trp Glu Pro Val Phe Gln Ile Gln Val Ser Lys Glu
               740                 745                 750
   Lys Glu Leu Leu Val Gly Leu Thr Ala Ala Ser Ser His Thr Ile Thr
           755                 760                 765
   Tyr Ile Thr Ala Glu Phe His Gln Leu Arg Leu Ala Lys Ile Glu Glu
       770                 775                 780
   Glu Ile Leu
   785
<210> 8
   <211> 787
   <212> PRT
   <213> Artificial Sequence
<220>
   <223> B1ZN60_OPITP
<400> 8
   Met Thr Gln Asp Lys Glu Leu Cys Phe Leu Trp Asp Asn Thr Ile His
   1 5 10 15
   Gly Ala Ile Pro Ala Phe Glu Lys Phe Glu Ala Ala Phe Ala Phe Glu
   20 25 30
   Lys Val Phe Glu His Pro Tyr Cys Pro Glu Glu Val Asp Leu Lys Ala
   35 40 45
   Tyr Ile Leu Lys Pro Asn Gly Asp Gln Lys Gln Ile Ser Gly Phe Trp
   50 55 60
   Tyr Glu Gly Phe Gln Arg Val Leu Arg Asn Gly Arg Glu Ile Leu Ile
   65 70 75 80
   Ser Thr Leu Glu Lys Asp Trp Arg Ile Arg Tyr Ser Ala Gln Val Pro
   85 90 95
   Gly Glu Tyr Arg Tyr Tyr Val Thr Leu Leu Asp Lys Lys Arg His Arg
   100 105 110
   Ser Tyr Arg Tyr Pro Glu Lys Gly Glu Leu Ser Phe Thr Val Thr Pro
   115 120 125
   Ser Asp Arg Lys Gly Phe Leu Arg Val Ser Ala Arg Asp Pro Ala Tyr
   130 135 140
   Leu Glu Phe Ser Asp Gly Ser Pro Tyr Leu Gly Ile Gly His Asn Leu
   145 150 155 160
   Cys Gly Trp Glu Trp Gly Gly Thr Asp Asn Arg Leu Gly Thr Tyr Glu
   165 170 175
   Tyr Asp Arg Trp Leu Ser Ser Met Ala Gln Asn Gly Ala Asn Leu Thr
   180 185 190
   Gln Phe Asp Phe Cys Glu Gly Asp Gln Ile Glu Trp Thr Pro Cys Asp
                   195                 200                 205
   Asn Glu Leu Pro Phe Ser Glu Asp Trp Lys Gly Leu Asn Glu Tyr Asn
       210                 215                 220
   Gln Gln Asn Ala Trp Lys Met Asp Arg Arg Phe Gln Thr Ala Glu Glu
   225                 230                 235                 240
   Leu Gly Ile Phe Phe Arg Leu Ser Leu Phe His Trp Glu Asp Phe Asp
                   245                 250                 255
   Asp Glu Thr Glu Lys Phe Pro Asp Trp Gly Trp Asn Arg Asn Pro Tyr
               260                 265                 270
   His Asp Gln Asn Gly Gly Pro Ala Lys Asn Val Ser Glu Phe Phe Glu
           275                 280                 285
   Lys Pro Ala Cys Lys Lys Tyr Val Arg Tyr Tyr Leu Lys Tyr Val Ala
       290                 295                 300
   Ala Arg Trp Gly Tyr Ser Pro Asn Leu Met Ala Tyr Glu Leu Trp Asn
   305                 310                 315                 320
   Glu Ile Asp Ala Pro Glu Val Met Trp Arg Ala Gly Glu Asp Tyr Asp
                   325                 330                 335
   Gln Glu Ala Ser Lys Val Ile Gly Trp His Ser Glu Met Gly Ser Tyr
               340                 345                 350
   Leu Lys Gln Leu Asp Ser Lys His Leu Val Thr Ser Ser Phe Ala Asp
           355                 360                 365
   Ser Arg Arg Asp Leu Asn Leu Trp Gln Leu Pro Cys Ile Asp Leu Thr
       370                 375                 380
   Thr Val His Arg Tyr Thr Tyr Phe Asn Glu Glu Tyr Gly Gln Arg Gln
   385                 390                 395                 400
   Tyr Asp Thr Glu Gly Ala Leu Ser Ala Val Leu Lys Glu Arg Phe Ser
                   405                 410                 415
   Gln Val Glu Lys Pro Val Leu Phe Gly Glu Phe Ala Leu Ser Pro Gly
               420                 425                 430
   Gly Asp Ile Gln Lys Asp Tyr Asp Pro Glu Gly Ile Glu Phe His Asn
           435                 440                 445
   Gln Leu Trp Ala Ser Leu Leu Leu Lys Ser Leu Gly Thr Ala Met His
       450                 455                 460
   Trp Thr Trp Gly Ser Tyr Val Asp Lys Asn Arg Leu Tyr Ser Lys Tyr
   465                 470                 475                 480
   Leu Pro Val Ser Arg Phe Phe Ala Gly Glu Asp Leu Arg Arg Thr Val
                   485                 490                 495
   Ser Phe Ser Asn Leu Asp Ala Val Thr Glu Arg Leu Leu Ile Leu Gly
               500                 505                 510
   Leu Arg Lys Thr Asp Arg Ala Cys Leu Trp Ile Lys Lys Arg Asp Trp
           515                 520                 525
   Gly Phe Cys Gln Ala Asn Glu Gly Lys Ser Ser Ser Val Glu Lys Gly
       530                 535                 540
   Arg Thr Ala Glu Val Pro Gly Leu Lys Ala Gly Asp Tyr Gln Val Glu
   545                 550                 555                 560
   Phe Tyr Asp Thr Lys Thr Gly Lys Ile Leu Glu Lys Ser Thr Ile Thr
                   565                 570                 575
   Ala Ala Gly Glu Thr Leu Thr Leu Leu Leu Pro Gly Phe Ser Gly Asp
               580                 585                 590
   Leu Ala Val Lys Leu Lys Pro Lys Glu Lys Asp Thr Leu Trp Lys Ser
           595                 600                 605
   Ile Asp Phe Pro Arg Pro Lys Lys Ser Ser Arg Thr Glu Phe Leu Gln
       610                 615                 620
   Asp Gly Ala Ile Leu Ser Ala Gly Gly Ala Gly Phe Cys Gly Glu Lys
   625                 630                 635                 640
   Glu Glu Tyr Arg Phe Val Tyr Gln Gln Ala Ser Gly Asp Phe Arg Leu
                   645                 650                 655
   Ser Ala Glu Ile Arg Ser Leu Thr Asn Leu Gly Glu Arg Val Ala Ala
               660                 665                 670
   Gly Leu Met Val Arg Asp Ser Leu Glu Pro Glu Ser Gly Tyr Ile Ala
           675                 680                 685
   Val Leu Leu His Pro Tyr Ser Lys Ala Gln Val Ile Ile Arg Arg Asp
       690                 695                 700
   Gly Asn Thr Glu Ile Leu Lys Glu Phe Asp Ala Gly Glu Arg Pro Cys
   705                 710                 715                 720
   Phe Gly Leu Asn Arg Ala Ala Gly Val Leu Thr Val Arg Leu Ala Lys
                   725                 730                 735
   Gln Gly Arg Glu Trp Glu Pro Val Phe Gln Ile Gln Val Ser Lys Glu
               740                 745                 750
   Lys Glu Leu Leu Val Gly Leu Thr Ala Ala Ser Ser His Thr Ile Thr
           755                 760                 765
   Tyr Ile Thr Ala Glu Phe His Gln Leu Arg Leu Ala Lys Ile Glu Glu
       770                 775                 780
   Glu Ile Leu
   785
<210> 9
   <211> 504
   <212> PRT
   <213> Artificial Sequence
<220>
   <223> B0UPR0_METS4
<400> 9
   Met Thr Asp Asp Arg Ala Leu Asp Pro Asp Cys Ser Leu Ala Asp Gly
   1               5                   10                  15
   Pro Ser Pro Ser Pro Arg Pro Asp Pro Ala Pro Ala Arg Thr Ala Gly
               20                  25                  30
   Ala Arg Gly Gly Arg Leu Pro Trp Ile Arg Val Ala Gly Pro Gly Ile
           35                  40                  45
   Pro Tyr Phe Glu Thr Glu Thr Gly Ala Ala Trp Thr Pro Val Gly Gln
       50                  55                  60
   Asn Asp Ala Ile Ser Trp His Glu Leu Glu Gly Leu Phe Gly Arg Arg
   65                  70                  75                  80
   Asp Leu Ala Ala Ala Glu Ala His Leu Arg His Leu Ala Asp His Gly
                   85                  90                  95
   Val Thr Cys Leu Arg Leu Met Leu Glu Tyr Ala Gln Val Arg His Arg
               100                 105                 110
   Tyr Ile Glu Arg Pro Val Gly Arg Phe Val Pro Ala Met Val Arg Leu
           115                 120                 125
   Trp Asp Asp Leu Phe Ala Leu Cys Glu Thr Val Gly Leu Arg Ile Leu
       130                 135                 140
   Leu Thr Pro Phe Asp Thr Phe Trp Met Trp Leu His Trp His Arg His
   145                 150                 155                 160
   Pro Tyr Asn Arg Arg His Gly Gly Pro Leu Ala Glu Pro Ser Arg Phe
                   165                 170                 175
   Leu Leu Asp Pro Gln Val Arg Glu Ala Ile Lys Asn Arg Leu Ala Phe
               180                 185                 190
   Ala Val Ala Arg Trp Gly Gly Ser Gly Ala Leu Phe Ala Trp Asp Leu
           195                 200                 205
   Trp Asn Glu Ile His Pro Ala His Ala Glu Gly Ser Ala Glu Gly Phe
       210                 215                 220
   Ala Pro Phe Ile Ala Asp Leu Ser Arg His Val Arg Ala Leu Glu Thr
   225                 230                 235                 240
   Arg Leu Tyr Gly Arg Ala His Pro Gln Thr Val Ser Leu Phe Gly Pro
                   245                 250                 255
   Glu Leu Gly Trp Arg Pro His Leu Gly Leu Glu Glu Pro Ile Phe Arg
               260                 265                 270
   His Pro Asp Leu Asp Phe Ala Thr Leu His Ile Tyr Ala Glu Gly Thr
           275                 280                 285
   Ile Asp Asp Pro Arg Asn Thr Val Glu Pro Ala Ile Ala Met Gly Arg
       290                 295                 300
   Ile Val Arg Glu Gly Leu Ala Gln Ile Arg Asp Gly Arg Pro Phe Leu
   305                 310                 315                 320
   Asp Ser Glu His Gly Pro Ile His Ser Phe Lys Asp Arg Arg Leu Thr
                   325                 330                 335
   Leu Pro Glu Pro Phe Asp Asp Glu Tyr Phe Arg His Met Gln Trp Ala
               340                 345                 350
   His Leu Ala Ser Gly Gly Ala Gly Gly Gly Met Arg Trp Pro Asn Arg
   355 360 365
   His Pro His Ser Leu Thr Ala Gly Met Arg Ala Ala Gln Arg Gly Leu
   370 375 380
   Ser Gly Phe Leu Pro Leu Ile Asp Trp Arg Arg Phe Arg Arg Arg Asn
   385 390 395 400
   Leu Ser Gly Asp Leu Gly Asp Pro Gly Pro Gly Ala Ala Leu Phe Ala
   405 410 415
   Cys Gly Asp Ala Glu Gln Ala Val Ile Trp Cys Leu Arg Ala Asp Ser
   420 425 430
   Leu Ala Pro Asp Gly Arg Leu Arg Arg Asp Ala Ala Pro Leu Gly Ile
   435 440 445
   Arg Leu Ala Leu Pro Gly Leu Arg Ala Gly Arg Tyr Ala Leu Thr Ala
   450 455 460
   Trp Asp Thr Arg Ala Gly Arg Pro Cys Gly Arg Arg Glu Val Thr Ala
   465 470 475 480
   Arg Asp Gly Val Ala Thr Glu Ile Glu Pro Pro Pro Phe Val Thr Asp
   485 490 495
   Val Ala Leu Ala Val Arg Arg Val
   500
<210> 10
   <211> 777
   <212> PRT
   <213> Artificial Sequence
<220>
   <223> D1N449_9BACT
<400> 10
   Met Arg Arg Ile Ser Ala Leu Phe Pro Thr Ile Leu Ser Leu Ala Phe
    1               5                  10                  15
   Leu Pro Leu Leu Asn Ala Ala Glu Leu Thr Gly Val Trp Lys Ala Asp
               20                  25                  30
   Gly Thr Asn Thr Pro His Ser Ser Pro Glu Ala Pro Gly Glu Ala Ala
           35                  40                  45
   Val Thr Val Arg Phe Pro Gly Ser Ala Gln Leu Tyr Arg Glu Pro Asp
       50                  55                  60
   Arg Ala Thr Phe Arg Pro Ser Arg Glu Ala Phe Glu Ala Ala Glu Phe
   65                  70                  75                  80
   Glu Leu Glu Ala Arg Val Ile Thr Asp Thr Pro Asp Pro Val Arg Ala
                   85                  90                  95
   Trp Leu Phe Phe Lys Asp Lys Asp Gly Arg Trp Tyr Gln Thr Ile Glu
               100                 105                 110
   Glu Tyr Arg Leu Ala Pro Gly Val Trp Gln Lys Leu Ser Ala Arg Leu
           115                 120                 125
   Asp Arg Thr Gly Ala Val Trp Arg Gly Val Gly His Thr Ala Thr Phe
       130                 135                 140
   Asp Ala Met Ala Ala Thr Glu Phe Tyr Ala Gly Gly Ile Ser Val Tyr
   145                 150                 155                 160
   Gly Glu Glu Lys Arg Glu Phe Thr Leu Glu Val Arg Asn Ala Ala Arg
                   165                 170                 175
   Thr Gly Lys Arg Glu Pro Gly Lys Leu Ala Leu Leu Asp Cys His Phe
               180                 185                 190
   Pro Glu Gln Gly Glu Ala Asn Ala Leu Phe Gln Gly Arg Phe Arg Leu
           195                 200                 205
   Leu Arg Glu Phe Phe Asn Pro Phe Asp Pro Asp Glu Val Thr Val Asp
       210                 215                 220
   Phe Glu Ile Lys Ala Pro Asn Gly Lys Leu Thr Arg Leu Pro Ala Phe
   225                 230                 235                 240
   Tyr Ser Arg Asp Tyr Glu Arg Arg Leu His His Thr Arg Glu Thr Ala
                   245                 250                 255
   Thr Pro Ile Gly Gln Gly Phe Trp Glu Phe Arg Phe Thr Pro Pro Val
               260                 265                 270
   Pro Gly Glu Tyr Arg Leu Arg Ala Val Ile Ala Asp Lys Thr Ala Arg
   275 280 285
   Glu Thr Val Thr Gly Ser Trp Lys Ser Phe Thr Ala Leu Pro Ser Arg
   290 295 300
   Arg Pro Gly Leu Val Arg Ala Ser Glu Lys Asp Pro Phe Phe Glu Leu
   305 310 315 320
   Gly Thr Gly Glu Phe Phe Phe Pro Val Gly Leu Asn Ile His Thr Asn
   325 330 335
   Thr Asp Arg Arg Ser Glu Phe Gly Phe Lys Phe Gly Gln Leu Pro Asp
   340 345 350
   Arg Gly Thr Phe Asp Tyr Asp Asp Tyr Leu Glu Ala Cys Gly Arg Gly
   355 360 365
   Gly Ile Asn Ala Val Glu Ile Trp Met Ala Gly Trp Thr Tyr Ala Ile
   370 375 380
   Glu His Asp Ala Thr Arg Ala Gly Asn Tyr Gly Val Gly Arg Tyr Asn
   385 390 395 400
   Leu Glu Ala Ala Trp Lys Leu Asp His Ile Phe Glu Gln Ala Arg Lys
   405 410 415
   Asn Gly Ile Tyr Leu Asn Leu Ile Leu Asp Asn His Gly Arg Leu Ser
   420 425 430
   Asp Arg Ser Asp Pro Glu Trp Gln Asp Asn Pro Ile Asn Ser Thr Thr
   435 440 445
   Pro Tyr Ala Lys Ala Asn Gly Gly Phe Leu Ala Asn Pro Ala Asp Phe
   450 455 460
   Phe Arg Ser Glu Ala Ala Glu Lys Asn Asp Arg Lys Arg Ala Arg Tyr
   465 470 475 480
   Ile Ala Ala Arg Trp Gly Asn Ala Pro Asn Leu Met Ala Val Glu Leu
   485 490 495
   Trp Ser Glu Val Asp Leu Thr Glu Asp Tyr Trp Gly Arg Tyr Asn Asp
   500 505 510
   Gly Ser Ala Ile Arg Trp Ala Glu Lys Ala Ala Ala Phe Leu Gln Ala
   515 520 525
   Asn Ser Arg Pro Asp Leu Pro Val Ser Ile His Phe Cys Ser Asp Tyr
   530 535 540
   Asn Asn Val Arg Arg Phe Ile Lys Leu Phe Asp Asn Pro Ser Ile Thr
   545 550 555 560
   His Leu Ala Gly Asp Ala Tyr Arg Ser Pro Gln Ile His Phe Val Asp
   565 570 575
   His Leu Arg Gly Tyr Glu Gln Asn Met Arg Tyr Asn Lys Pro Gln Leu
   580 585 590
   Ile Thr Glu Phe Gly Gly Asn Pro Gln Gly Ser Ser Glu Arg Gln Val
   595 600 605
   Leu Ala Asp Ile His Ser Gly Leu Trp Ser Ser Leu Phe Val Arg Leu
   610 615 620
   Ala Gly Thr Pro Phe Leu Trp Trp His Asp Phe Val His Leu Arg Asn
   625 630 635 640
   His Tyr Gln His Tyr Leu Gly Phe Ser Arg Tyr Leu Ala Gly Ile Asp
   645 650 655
   Leu Arg Gly Lys Glu Arg Val Tyr Phe Thr Pro Ala Val Ala Val Pro
   660 665 670
   Ala Asn Gln Gln Lys Tyr Glu Ser Leu Gly Leu Ser Leu Pro Ala Ala
   675 680 685
   Ala Tyr Gly Trp Ile Tyr Asn Arg Asn Ala Met Leu Glu Tyr Pro Asp
   690 695 700
   Asp Pro Asn Gln Phe Pro Glu Thr Arg Pro Gly Ser Val Thr Leu Ala
   705 710 715 720
   Gly His Asn Leu Thr Gly Gly Val Tyr Leu Leu Arg Trp Phe Val Pro
   725 730 735
   Leu Thr Gly Glu Cys Leu Pro Gly Glu Leu Lys Leu Asn Val Glu Ala
   740 745 750
   Gly Lys Pro Val Thr Phe Ala Val Pro Ser Phe Arg Leu Asp Leu Ala
   755 760 765
   Phe Lys Leu Glu Lys Thr Glu Ala Lys
   770 775
<210> 11
   <211> 566
   <212> PRT
   <213> Artificial Sequence
<220>
   <223> A7HFC4_ANADF
<400> 11
   Met Ser Gly Val Thr Thr Arg Arg Leu His Cys Thr Gly Pro Arg Ser
   1 5 10 15
   Ala Ala Ala Ala Met Leu Ala Ala Ala Leu Ala Leu Gly Cys Ala Arg
   20 25 30
   Ala Pro Val Arg Pro Gly Ala Asp Ala Ala Gly Gly Arg Glu Ala Ala
   35 40 45
   Val Asn Gly Val Val Glu Leu Arg Leu Ala Val Pro Ala Gly Ala Pro
   50 55 60
   Val Arg Ala Glu Val Leu Ala Pro Ser Gly Ala Arg Ile His Val Pro
   65 70 75 80
   Ala Phe Pro Val Pro Gly Gly Trp Ala Ala Arg Phe Arg Pro Arg Glu
   85 90 95
   Pro Gly Arg His Arg Trp Val Ala Arg Ser Gly Glu Gly Ala Ala Ala
   100 105 110
   Ala Glu Val Ala Arg Gly Glu Val Met Ala Glu Asp Arg Gly Leu Ala
   115 120 125
   Gly Gln Val Ile Val Ser Gly Gly Thr Leu Arg Thr Glu Asp Gly Arg
   130 135 140
   Pro Phe Arg Pro Leu Gly Glu Asn Arg Phe Asn Val Tyr Asp Pro Thr
   145 150 155 160
   Trp Ser Asp Gly Leu Ser Pro Ala Asp Tyr Val Ala Arg Met Ala Ala
   165 170 175
   Asp Gly Met Asn Ala Leu Arg Val Phe Val Phe Thr Ala Cys Gly Arg
   180 185 190
   Ala Gly Thr Met Pro Asn Pro Gly Cys Leu Glu Pro Val Leu Gly Ala
   195 200 205
   Phe Asp Glu Ala Ala Ala Ala Arg Tyr Asp Ala Ile Phe Ala Ala Ala
   210 215 220
   Glu Ala His Gly Val Lys Val Val Leu Ser Val Phe Ala Ile Gly Phe
   225 230 235 240
   Thr Pro Gly Asp Ala Trp Lys Gly Trp Glu Glu Asn Pro Tyr Ser Ala
   245 250 255
   Ala Arg Gly Gly Pro Ala Ala Gly Asn Thr Asp Phe Phe Leu Asp Pro
   260 265 270
   Arg Ala Arg Glu Ala Ala Arg Ala Arg Leu Arg Tyr Val Leu Ala Arg
   275 280 285
   Trp Gly Ala Ser Pro Ala Leu Leu Ala Ile Asp Leu Leu Asn Glu Pro
   290 295 300
   Glu Trp Asp Gly Ala Ile Pro Glu Asp His Trp Ile Pro Trp Ala Glu
   305 310 315 320
   Asp Leu Ala Arg Thr Trp Arg Ala Glu Asp Pro Tyr Gly His Pro Val
   325 330 335
   Thr Ala Gly Pro Val Gly Leu His Trp Asn Val Glu Glu Asp Glu Arg
   340 345 350
   Ala Trp Trp Ala Ser Ala Ala Cys Asp Ile Val Gln Trp His Arg Tyr
   355 360 365
   Gly Pro Asp Val His Asp Val His Asp Leu Ala Glu Ala Leu Val Glu
   370 375 380
   Thr Thr Arg Asp Thr Ala Arg Tyr Gly Lys Pro Val Leu Ile Gly Glu
   385 390 395 400
   Phe Gly Trp Gly Gly Asp Ala Lys Pro Glu His Asp His Thr His Val
   405 410 415
   Gly Ile Trp Ala Ala Thr Phe Ala Gly Ala Gly Val Leu Ser His Ser
   420 425 430
   Ala Pro Pro Phe Thr Glu Asp Ser Asp Glu Pro Met Thr Pro Ala Arg
               435                 440                 445
   Ala Arg His Phe Arg Thr Leu Ala Ala Phe Leu Arg Arg Ala Glu Ala
           450                 455                 460
   Arg Gly Pro Leu Ala Pro Ala Pro Glu Pro Ala Val Arg Arg Ala Pro
   465                 470                 475                 480
   Gly Leu Arg Ala Leu Ala Leu Gly Gly Glu Arg Ala Ala Ala Val Trp
                   485                 490                 495
   Leu Leu Ala Pro Arg Pro Gly Tyr Gly Gly Arg Val Lys Gly Ala Arg
               500                 505                 510
   Leu Thr Leu Ala Gly Ile Ala Pro Gly Arg Trp Arg Val Thr Trp Val
           515                 520                 525
   Glu Asp Val Ser Gly Glu Val Ile Ala Val Glu Glu Arg Asp Ala Ser
       530                 535                 540
   Gly Pro Leu Pro Leu Asp Val Pro Pro Phe Ala Arg His Val Ala Ala
   545                 550                 555                 560
   Leu Val Glu Arg Ile Glu
                   565
<210> 12
   <211> 591
   <212> PRT
   <213> Artificial Sequence
<220>
   <223> A9AYF5_HERA2
<400> 12
   Met Arg Arg Trp Leu Tyr Arg Leu His Leu Trp Leu Val Leu Leu Leu
   1               5                   10                  15
   Leu Ile Ala Ala Cys Thr Gln Val Gly Glu Ser Gly Gly Asn Gln Thr
               20                  25                  30
   Leu Ser Leu Arg Thr Leu Thr Gly Asn Ala Ala Val Phe Gly Thr Ile
           35                  40                  45
   Glu Leu Ala Ile Asp Thr Thr Ile Thr Val Ala Asn Pro Tyr Asp Pro
       50                  55                  60
   Asn Gln Ile Asp Leu Met Val Ser Phe Ile Ser Ala Thr Gly Gln Ile
   65                  70                  75                  80
   Tyr Arg Val Pro Ala Phe Trp Tyr Gln Asp Phe Asp Gln Leu Ser Leu
                   85                  90                  95
   Gln Pro Lys Gly Asn Pro Glu Trp Arg Val Arg Phe Thr Pro Ser Glu
               100                 105                 110
   Pro Gly Ala Trp Gln Val Lys Ala Glu Leu Ala Lys Pro Ala Leu Ser
           115                 120                 125
   Ser Asp Val Ile Thr Ile Glu Val Ser Ala Asn Lys Gln Ser Pro Gly
       130                 135                 140
   Phe Val Arg Ile Asn Thr Ser Asn Pro Arg Tyr Phe Ala Arg Gln Asp
   145                 150                 155                 160
   Gly Thr Phe Phe Met Pro Ile Gly Leu Asn Leu Gly Trp Ser Thr Gln
                   165                 170                 175
   Gln Gly Thr Gly Ile Leu Arg Glu Tyr Glu His Trp Phe Asp Gln Leu
               180                 185                 190
   Ser Lys Asn Gly Gly Asn Ile Ala Arg Ile Trp Met Ala Ser Trp Ser
           195                 200                 205
   Phe Gly Ile Glu Trp Gln Asp Thr Gly Leu Gly Asp Tyr Ser Lys Arg
       210                 215                 220
   Met Gln Gln Ala Trp Met Leu Asp Gln Ile Phe Lys Leu Ala Glu Gln
   225                 230                 235                 240
   Arg Asn Ile Thr Ile Met Leu Thr Leu Ile Asn His Gly Ala Phe Ser
                   245                 250                 255
   Thr Ser Thr Asp Ser Glu Trp Ala Ser Asn Pro Tyr Asn Ala Ala Asn
               260                 265                 270
   Gly Gly Pro Ile Ala Glu Pro Arg Leu Phe Ala Thr Asp Ile Gln Ser
           275                 280                 285
   Arg Glu Val Phe Lys His Arg Val Arg Tyr Ile Ala Ala Arg Trp Ala
       290                 295                 300
   His Ser Pro Ser Leu Phe Ala Trp Glu Trp Trp Asn Glu Ala Asn Trp
   305                 310                 315                 320
   Thr Pro Ile Asn Asp Ala Leu Met Gln Pro Trp Ile Ser Glu Met Thr
                   325                 330                 335
   Arg His Leu Ala Gln Phe Asp Pro Tyr Gln His Leu Val Ser Thr Ser
               340                 345                 350
   Tyr Ala Ser Asn Thr Ser Thr Ser Met Trp Val Gln Pro Glu Ile Asn
           355                 360                 365
   Phe Thr Gln His His Asp Tyr Thr Gly Arg Asp Leu Gly Gln Ala Phe
       370                 375                 380
   Pro Leu Val Ile Arg Glu Leu Asn Ala Ala Ala Pro Gln Lys Pro Ala
   385                 390                 395                 400
   Leu Val Ser Glu Leu Gly Tyr Ala Gly Thr Gly Arg Asp Glu Val Ile
                   405                 410                 415
   Asn Arg Asp Val Trp Gln Phe His Gln Gly Leu Trp Ala Ala Pro Phe
               420                 425                 430
   Ser Gly Phe Ala Gly Ser Gly Met Tyr Trp Trp Trp Asp Thr Leu Val
           435                 440                 445
   Asp Pro Asp Asn Leu Trp Ser Glu Tyr Ser Lys Leu Ala Glu Phe Phe
       450                 455                 460
   Lys Asp Gln Asp Leu Thr Ile Tyr Asn Pro Val Val Ala Gln Ile Ser
   465                 470                 475                 480
   Pro Leu Lys Ala Arg Ala Leu Ala Leu Gln Thr Lys Ser Gln Ala Leu
                   485                 490                 495
   Val Trp Val Arg Ser Asn Glu Tyr Glu Pro Glu Ala Leu Thr Lys Ala
               500                 505                 510
   Tyr Glu Glu Ala Leu Lys Lys Arg Glu Phe Asn Asp Thr Trp Glu Tyr
           515                 520                 525
   Val Pro Pro Thr Tyr Ala Asp Leu Thr Leu Lys Leu Asn Gly Leu Glu
       530                 535                 540
   Ala Gly Asn Tyr Gln Ala Thr Trp Tyr Asp Pro Gln Thr Gly Thr Trp
   545                 550                 555                 560
   Ser Gln Pro Thr Thr Val Thr Leu Glu Ala Asn Gln Ser Ser Ile Ala
                   565                 570                 575
   Val Pro Ser Phe Asn Tyr Asp Leu Ala Leu Lys Leu Val Lys Gln
               580                 585                 590
<210> 13
   <211> 557
   <212> PRT
   <213> Artificial Sequence
<220>
   <223> C7PTR3_CHIPD
<400> 13
   Met Lys Asn Tyr Val Lys Ala Ile Pro Cys Tyr Ala Leu Leu Met Ala
    1               5                  10                  15
   Thr Phe Thr Val Tyr Ala Asn Asp Ser Thr Lys Leu Gln Arg Ile Thr
               20                  25                  30
   Pro Pro Ala Ala Ala Val Asn Leu Tyr Glu Lys Ala Glu Trp Thr Ile
           35                  40                  45
   Asp Leu Thr Ala Asn Tyr Ser Asn Pro Tyr Asp Gln Arg Glu Ile Lys
       50                  55                  60
   Leu Asp Met Cys Leu Val Ser Pro Ser Gly Lys Pro Leu Leu Leu Pro
   65                  70                  75                  80
   Ala Tyr Phe Asp Gln Val Asn His His Trp Gln Ser Arg Phe Ala Pro
                   85                  90                  95
   Gln Glu Thr Gly Gln Tyr Gln Tyr Tyr Phe Glu Leu Ile Ala Gly Lys
               100                 105                 110
   Asp Thr Val Gln Ser Lys Pro Ser Val Phe Thr Val Tyr Lys Ser Thr
           115                 120                 125
   Arg Lys Gly Phe Leu His Lys Asn Asp Leu Trp Thr Phe Arg Phe Asp
       130                 135                 140
   Asn Gly Glu Leu Phe Arg Gly Val Gly Glu Asn Val Ala Trp Glu Ser
   145                 150                 155                 160
   Arg Ser Phe Glu Asp Asp Lys Trp Thr Tyr Asp Tyr Leu Leu Pro Ser
                   165                 170                 175
   Leu Ala His Asn Gly Ala Asn Phe Phe Arg Thr Trp Met Cys Tyr Trp
               180                 185                 190
   Asn Leu Pro Leu Glu Trp Lys Gln Pro Arg Ser Thr Lys Arg Tyr Gln
           195                 200                 205
   Pro Ser Ala Glu Tyr Phe His Pro Gly Ala Ile Arg Arg Met Asp Gln
       210                 215                 220
   Leu Val Asp Met Cys Asp Ser Leu Gly Leu Tyr Phe Met Leu Thr Leu
   225                 230                 235                 240
   Asp Trp His Gly His Leu Met Glu His Gly Gly Trp Lys His Ser Ser
                   245                 250                 255
   Tyr Asn Lys Ala Asn Gly Gly Pro Ala Glu Thr Pro Thr Ala Phe Phe
               260                 265                 270
   Thr Ser Gln Gln Ala Gln Glu Lys Tyr Lys Asn Lys Leu Arg Tyr Ile
           275                 280                 285
   Ile Ala Arg Trp Gly Tyr Ser Ser Ser Ile Ala Val Trp Glu Phe Phe
       290                 295                 300
   Asn Glu Val Asp Asn Ala Ala Phe Thr Gln Gln Asp Ser Ile Leu Ile
   305                 310                 315                 320
   Pro Leu Pro Val Ile Ala Gln Trp His Leu Glu Met Ser Arg Tyr Leu
                   325                 330                 335
   Lys Asp Ile Asp Pro Tyr His His Leu Val Ser Thr Ser Ile Ser His
               340                 345                 350
   Arg Asp Ile Ile Gly Met Asn Ala Ile Pro Tyr Ile Asp Phe Asn Gln
           355                 360                 365
   Lys His Ile Tyr Lys His Thr Glu Lys Ile Pro Gly Ile Tyr Pro Asp
       370                 375                 380
   Tyr Ile Gln Thr Phe Gly Lys Pro Tyr Val Val Gly Glu Phe Gly Tyr
   385                 390                 395                 400
   Arg Trp Glu Asp Gln Asp Pro Lys Tyr Ala Thr Glu Ala Asn Tyr Asp
                   405                 410                 415
   Tyr Arg Arg Gly Leu Trp Tyr Gly Met Phe Ser Pro Thr Pro Val Leu
               420                 425                 430
   Pro Met Ser Trp Trp Trp Glu Leu Phe Asp Asp Gln His Met Thr Pro
           435                 440                 445
   Tyr Leu Gln Ser Val Ser Thr Ile Asn Lys Met Met Leu Gln Ala Gly
       450                 455                 460
   Lys Gly Gln Phe Glu Gln Leu Pro Val Gln Ala Ala Ile Leu Glu Ser
   465                 470                 475                 480
   Tyr Ala Ile Lys Cys Gly Asn Thr Ile Phe Val Tyr Ala Leu Asn Asn
                   485                 490                 495
   Thr Thr Lys Gln Gln Ser Ala Asp Ile Arg Val Asn Ile Pro Ser Gly
               500                 505                 510
   Tyr Thr Leu Gln Cys Phe His Pro Leu Lys Asn Thr Trp Asn Lys Ser
           515                 520                 525
   Ile Tyr Lys Arg Thr Ala Asp Gly Thr Val Gln Ile Ser Asn Thr Val
       530                 535                 540
   Leu Pro Ala Lys Glu Glu Ile Ile Leu Val Phe Lys Pro
   545                 550                 555
<210> 14
   <211> 843
   <212> PRT
   <213> Artificial Sequence
<220>
   <223> EBI5326244 deduced protein sequence
<400> 14
   Met Leu Lys Lys Val His Ile Ile Ala Ile Val Val Ile Ile Ala Ile
   1               5                   10                  15
   Ala Phe Ala Leu Ile Leu Ala Arg Tyr Tyr Thr Met Gln Arg Gly Tyr
               20                  25                  30
   Glu Thr Val Thr Pro Thr Thr Pro Pro Gln Gln Thr Thr Thr Thr Glu
           35                  40                  45
   Thr Thr Pro Val Pro Thr Glu Ala Gly Thr Thr Thr Pro Ile Thr Glu
       50                  55                  60
   Ala Thr Val Thr Gln Pro Pro Gln Thr Pro Thr Thr Pro Ser Pro Gln
   65                  70                  75                  80
   Thr Pro Thr Thr Pro Thr Ala Leu Pro Thr Pro Ser Pro Thr Pro Thr
                   85                  90                  95
   Ala Pro Ser Ala Thr Val Thr Glu Thr Thr Ser Pro Gln Thr Pro Thr
               100                 105                 110
   Thr Thr Ile Thr Thr Glu Thr Thr Thr Thr Pro Ala Pro Gln Pro Gln
           115                 120                 125
   Val Val Phe Leu Lys Leu Pro Glu Gly Glu Glu Pro Lys Phe Gly Leu
       130                 135                 140
   Val Glu Ile Ala Phe Asn Ile Ser Gly Leu Ser Tyr Ser Asn Pro Phe
   145                 150                 155                 160
   Asp Thr Ser Asp Ile Asp Val Trp Val His Ile Glu Thr Pro Ser Gly
                   165                 170                 175
   Ser Arg Val Ala Val Pro Ala Phe Tyr Phe Gln Asn Tyr Thr Val Lys
               180                 185                 190
   Arg Leu Gly Pro Gly Glu Glu Ile Ile Val Arg Val Gly Arg Pro Tyr
           195                 200                 205
   Trp Leu Ala Arg Phe Ala Pro Val Glu Glu Gly Val His Lys Phe Tyr
       210                 215                 220
   Val Lys Ala Val Asp Gly Arg Gly Ser Ala Val Val Ser Glu Ile Arg
   225                 230                 235                 240
   Glu Phe Met Val Lys Gly Val Ala Gly Arg Gly Phe Val Arg Val Asp
                   245                 250                 255
   Ser Gly Lys Arg Leu Phe Val Phe Asp Ser Gly Glu Ser Met Phe Met
               260                 265                 270
   Leu Gly Ile Asp Val Ala Trp Pro Pro Asp Arg Arg Ser Ser Ile Ser
           275                 280                 285
   Phe Tyr Glu Gln Trp Phe Asp Lys Leu Asn Lys Ser Gly Ile Lys Val
       290                 295                 300
   Val Arg Ile Gly Leu Val Pro Trp Ala Leu Thr Leu Glu Trp Ser Lys
   305                 310                 315                 320
   Leu His Tyr Tyr Ser Leu Asp Asp Ala Ala Arg Ile Asp Glu Ile Val
                   325                 330                 335
   Lys Leu Ala Glu Lys Tyr Asp Ile Tyr Ile Val Phe Val Phe Met Trp
               340                 345                 350
   His Gly Glu Leu Ala Asp Asn Trp Gly Asp Asn Pro Tyr Asn Ala Ala
           355                 360                 365
   Arg Gly Gly Pro Leu Gln Ser Pro Glu Glu Phe Trp Ser Asn Ala Val
       370                 375                 380
   Ala Ile Ser Ile Phe Lys Asp Lys Val Arg Tyr Ile Ile Ala Arg Trp
   385                 390                 395                 400
   Gly Tyr Ser Thr His Ile Leu Ala Trp Glu Leu Ile Asn Glu Ala Asp
                   405                 410                 415
   Leu Thr Thr Asn Phe Phe Ser Ala Arg Ser Ala Phe Val Ser Trp Val
               420                 425                 430
   Lys Glu Ile Ser Ser Tyr Ile Lys Ser Val Asp Pro Tyr Asn Arg Ile
           435                 440                 445
   Val Thr Val Asn Leu Ala Asp Tyr Asn Ser Glu Pro Arg Val Trp Ser
       450                 455                 460
   Val Glu Ser Ile Asp Ile Ile Asn Val His Arg Tyr Gly Pro Glu Gly
   465                 470                 475                 480
   Phe Lys Asp Ile Ala Leu Ala Ile Pro Ser Ile Val Glu Gly Leu Trp
                   485                 490                 495
   Asn Thr Tyr Arg Lys Pro Ile Ile Ile Thr Glu Phe Gly Val Asp Tyr
               500                 505                 510
   Arg Trp Ile Gly Tyr Pro Gly Phe Lys Gly Thr Pro Tyr Trp Ala Tyr
           515                 520                 525
   Asp Lys Ser Gly Val Gly Leu His Glu Gly Leu Trp Ser Ser Ile Phe
       530                 535                 540
   Ser Leu Ser Pro Val Ser Ala Met Ser Trp Trp Trp Asp Thr Gln Ile
   545                 550                 555                 560
   Asp Ser Tyr Asn Leu Trp Tyr His Tyr Lys Ala Leu Tyr Glu Phe Leu
                   565                 570                 575
   Lys Ser Val Asp Pro Val Arg Gly Gly Leu Gly Lys Ala Arg Ala Ser
               580                 585                 590
   Leu Val Ile Thr Asp Val Thr Pro Ser Ser Ile Thr Leu Tyr Pro Leu
           595                 600                 605
   Ala Gly Trp Val Trp Val Ser Pro Val Arg Glu Asn Arg Leu Val Ile
       610                 615                 620
   Arg Pro Asp Gly Ala Ile Glu Gly Arg Val Asp Leu Leu Ser Gly Phe
   625                 630                 635                 640
   Ile Tyr Gly Thr Cys His Ser Gln Arg Thr Leu Asn Pro Val Phe Thr
                   645                 650                 655
   Val Met Phe Ile Asp Arg Gly Arg Val Val Leu His Ile Asn Ser Val
               660                 665                 670
   Gly Arg Gly Ser Ala Lys Leu Val Ile Tyr Val Asn Gly Ser Leu Ala
           675                 680                 685
   Thr Gln Leu Asp Leu Pro Asp Lys Asp Gly Lys Ser Asp Gly Ser Ala
       690                 695                 700
   Asn Glu Tyr Asp Met Asp Val Glu Leu Trp Phe Glu Pro Gly Thr Tyr
   705                 710                 715                 720
   Glu Ile Lys Ile Asp Ser Glu Ala Cys Asp Trp Phe Thr Trp Asp Tyr
                   725                 730                 735
   Ile Val Phe Glu Asn Ala Val Tyr Arg Ala Ala Lys Val Asp Leu Tyr
               740                 745                 750
   Ala Leu Ala Asn Ser Thr Phe Ala Met Leu Trp Val Arg Asn Lys Asp
           755                 760                 765
   Tyr Asn Trp Trp Asn Val Val Val Leu Asn Lys Thr Leu Glu Pro Ala
       770                 775                 780
   Glu Gly Val Glu Val Glu Ile Arg Gly Leu Gln Asp Gly Val Tyr Arg
   785                 790                 795                 800
   Val Glu Phe Trp Asp Thr Cys Arg Gly Val Val Val Lys Ser Met Glu
                   805                 810                 815
   Val Gln Val Ser Asn Gly Val Ala Arg Val Pro Val Gly Ser Val Glu
               820                 825                 830
   Lys Asp Ile Ala Met Lys Ile Thr Arg Ala Gly
           835                 840
<210> 15
   <211> 2516
   <212> DNA
   <213> Artificial Sequence
<220>
   <223> EBI5326244-OA
<400> 15
   atgaaaaaga cagctatcgc gattgcagtg gcactggctg gtttcgctac cgttgcgcaa 60
   gctgctatgc agagaggcta tgaaacagtg acacctacaa caccacctca gcaaactact 120
   accacagaaa caactccagt gcctacagag gcaggtacta caacaccaat aactgaggcc 180
   actgtgactc aaccccctca aacccctaca acaccttcac cacaaacacc aacaacacca 240
   acagcgttgc ccacgccatc accaaccccc acagcgccct ctgccacagt aacagagact 300
   acatcgcctc aaactcctac aactacaata actacagaaa caacaactac accagccccc 360
   caaccccagg tggtgtttct aaagctacca gagggagagg agccaaagtt tggcttagtt 420
   gaaatagcct ttaacatatc tggtctaagc tactcaaacc cctttgacac aagcgatatt 480
   gatgtgtggg tgcacataga gacgccaagt ggctctagag tggctgtacc agctttctac 540
   ttccagaact acactgtgaa aaggcttgga ccaggggagg agatcatagt cagggttgga 600
   aggccatact ggctcgctag gttcgcacct gttgaggagg gggtgcacaa gttctacgta 660
   aaggcagttg atggcagggg gagtgctgtg gtgagcgaga ttagagagtt tatggttaag 720
   ggggtggctg gcagggggtt tgtcagagtt gacagtggca aaaggctatt cgtatttgac 780
   agcggtgaat caatgtttat gctggggata gatgttgcgt ggccaccaga taggaggagc 840
   tctatatcgt tctatgagca gtggtttgat aaactaaata agagtgggat taaggttgtg 900
   agaataggcc tagtgccctg ggctctaaca ctagagtgga gcaagctcca ctactacagc 960
   ttagatgacg ctgctagaat agatgagatt gtgaagcttg ctgaaaagta tgacatatac 1020
   atagtgtttg tgtttatgtg gcatggagag cttgcggata actgggggga taacccatac 1080
   aatgcagcaa ggggtggtcc tcttcagagc ccagaggagt tctggagcaa tgcagtagct 1140
   atcagtatat ttaaggataa ggtgaggtac attatagcta ggtgggggta ctcaacacac 1200
   atactcgcat gggagctgat aaacgaggct gacctaacaa caaacttctt tagcgctaga 1260
   agtgcttttg tgagctgggt taaggagata agcagctaca taaagtccgt agacccctac 1320
   aacaggattg tcactgtgaa cctcgctgac tacaattctg agccaagggt gtggagcgta 1380
   gagtccatag acatcataaa tgtgcatagg tatgggccag agggctttaa ggacatagcc 1440
   ttggctattc caagcatagt agaggggctt tggaacacct acagaaagcc tattataata 1500
   acagagtttg gtgttgacta tcggtggatt ggctacccag gctttaaagg aaccccctac 1560
   tgggcatacg acaagagtgg ggttgggctt catgaagggc tctggagctc tattttcagc 1620
   ctctccccag tttctgctat gagctggtgg tgggatacac agatagactc ttataatctg 1680
   tggtaccact acaaagccct ctacgagttt ctaaagagtg tcgaccctgt tagaggaggc 1740
   ttgggcaagg ccagagcatc gctagtcatt acagatgtga ccccctctag cataacacta 1800
   taccccttag cgggctgggt gtgggtctcg ccagtgagag aaaataggct tgttataagg 1860
   cctgatgggg ctatcgaggg gagggttgat ttgctaagtg ggtttatcta tggcacgtgc 1920
   cacagccagc ggacactaaa cccagtattt actgtgatgt tcattgatag aggcagggtg 1980
   gtgctacaca taaactctgt tggcaggggc tctgcaaagc tcgtgatata tgttaatggc 2040
   tctctagcta cacaactgga cttgcctgat aaggatggta agagtgatgg gagtgcaaat 2100
   gagtacgaca tggatgtgga gctgtggttt gagcctggca cctacgaaat aaaaattgat 2160
   agtgaagctt gcgactggtt cacatgggac tacatagtgt ttgaaaatgc tgtgtataga 2220
   gctgctaaag tagatctcta tgcacttgca aacagcacct ttgcaatgct ctgggtaagg 2280
   aacaaggact acaactggtg gaacgtagtg gtgctgaaca agactctaga gcctgctgag 2340
   ggagtagagg tagagattag aggactgcaa gatggggtgt acagagtaga gttttgggac 2400
   acatgcagag gggtggtggt gaagagcatg gaggttcaag tgtcaaatgg tgtagccagg 2460
   gttccggtgg gtagcgtaga aaaggacata gctatgaaaa tcactagggc tggcta 2516
<210> 16
   <211> 838
   <212> PRT
   <213> Artificial Sequence
<220>
   <223> EBI5326244-OA
<400> 16
   Met Lys Lys Thr Ala Ile Ala Ile Ala Val Ala Leu Ala Gly Phe Ala
    1               5                  10                  15
   Thr Val Ala Gln Ala Ala Met Gln Arg Gly Tyr Glu Thr Val Thr Pro
               20                  25                  30
   Thr Thr Pro Pro Gln Gln Thr Thr Thr Thr Glu Thr Thr Pro Val Pro
           35                  40                  45
   Thr Glu Ala Gly Thr Thr Thr Pro Ile Thr Glu Ala Thr Val Thr Gln
       50                  55                  60
   Pro Pro Gln Thr Pro Thr Thr Pro Ser Pro Gln Thr Pro Thr Thr Pro
   65                  70                  75                  80
   Thr Ala Leu Pro Thr Pro Ser Pro Thr Pro Thr Ala Pro Ser Ala Thr
                   85                  90                  95
   Val Thr Glu Thr Thr Ser Pro Gln Thr Pro Thr Thr Thr Ile Thr Thr
               100                 105                 110
   Glu Thr Thr Thr Thr Pro Ala Pro Gln Pro Gln Val Val Phe Leu Lys
           115                 120                 125
   Leu Pro Glu Gly Glu Glu Pro Lys Phe Gly Leu Val Glu Ile Ala Phe
       130                 135                 140
   Asn Ile Ser Gly Leu Ser Tyr Ser Asn Pro Phe Asp Thr Ser Asp Ile
   145                 150                 155                 160
   Asp Val Trp Val His Ile Glu Thr Pro Ser Gly Ser Arg Val Ala Val
                   165                 170                 175
   Pro Ala Phe Tyr Phe Gln Asn Tyr Thr Val Lys Arg Leu Gly Pro Gly
               180                 185                 190
   Glu Glu Ile Ile Val Arg Val Gly Arg Pro Tyr Trp Leu Ala Arg Phe
           195                 200                 205
   Ala Pro Val Glu Glu Gly Val His Lys Phe Tyr Val Lys Ala Val Asp
       210                 215                 220
   Gly Arg Gly Ser Ala Val Val Ser Glu Ile Arg Glu Phe Met Val Lys
   225                 230                 235                 240
   Gly Val Ala Gly Arg Gly Phe Val Arg Val Asp Ser Gly Lys Arg Leu
                   245                 250                 255
   Phe Val Phe Asp Ser Gly Glu Ser Met Phe Met Leu Gly Ile Asp Val
               260                 265                 270
   Ala Trp Pro Pro Asp Arg Arg Ser Ser Ile Ser Phe Tyr Glu Gln Trp
           275                 280                 285
   Phe Asp Lys Leu Asn Lys Ser Gly Ile Lys Val Val Arg Ile Gly Leu
       290                 295                 300
   Val Pro Trp Ala Leu Thr Leu Glu Trp Ser Lys Leu His Tyr Tyr Ser
   305                 310                 315                 320
   Leu Asp Asp Ala Ala Arg Ile Asp Glu Ile Val Lys Leu Ala Glu Lys
                   325                 330                 335
   Tyr Asp Ile Tyr Ile Val Phe Val Phe Met Trp His Gly Glu Leu Ala
               340                 345                 350
   Asp Asn Trp Gly Asp Asn Pro Tyr Asn Ala Ala Arg Gly Gly Pro Leu
           355                 360                 365
   Gln Ser Pro Glu Glu Phe Trp Ser Asn Ala Val Ala Ile Ser Ile Phe
       370                 375                 380
   Lys Asp Lys Val Arg Tyr Ile Ile Ala Arg Trp Gly Tyr Ser Thr His
   385                 390                 395                 400
   Ile Leu Ala Trp Glu Leu Ile Asn Glu Ala Asp Leu Thr Thr Asn Phe
                   405                 410                 415
   Phe Ser Ala Arg Ser Ala Phe Val Ser Trp Val Lys Glu Ile Ser Ser
               420                 425                 430
   Tyr Ile Lys Ser Val Asp Pro Tyr Asn Arg Ile Val Thr Val Asn Leu
           435                 440                 445
   Ala Asp Tyr Asn Ser Glu Pro Arg Val Trp Ser Val Glu Ser Ile Asp
       450                 455                 460
   Ile Ile Asn Val His Arg Tyr Gly Pro Glu Gly Phe Lys Asp Ile Ala
   465                 470                 475                 480
   Leu Ala Ile Pro Ser Ile Val Glu Gly Leu Trp Asn Thr Tyr Arg Lys
                   485                 490                 495
   Pro Ile Ile Ile Thr Glu Phe Gly Val Asp Tyr Arg Trp Ile Gly Tyr
               500                 505                 510
   Pro Gly Phe Lys Gly Thr Pro Tyr Trp Ala Tyr Asp Lys Ser Gly Val
           515                 520                 525
   Gly Leu His Glu Gly Leu Trp Ser Ser Ile Phe Ser Leu Ser Pro Val
       530                 535                 540
   Ser Ala Met Ser Trp Trp Trp Asp Thr Gln Ile Asp Ser Tyr Asn Leu
   545                 550                 555                 560
   Trp Tyr His Tyr Lys Ala Leu Tyr Glu Phe Leu Lys Ser Val Asp Pro
                   565                 570                 575
   Val Arg Gly Gly Leu Gly Lys Ala Arg Ala Ser Leu Val Ile Thr Asp
               580                 585                 590
   Val Thr Pro Ser Ser Ile Thr Leu Tyr Pro Leu Ala Gly Trp Val Trp
           595                 600                 605
   Val Ser Pro Val Arg Glu Asn Arg Leu Val Ile Arg Pro Asp Gly Ala
       610                 615                 620
   Ile Glu Gly Arg Val Asp Leu Leu Ser Gly Phe Ile Tyr Gly Thr Cys
   625                 630                 635                 640
   His Ser Gln Arg Thr Leu Asn Pro Val Phe Thr Val Met Phe Ile Asp
                   645                 650                 655
   Arg Gly Arg Val Val Leu His Ile Asn Ser Val Gly Arg Gly Ser Ala
               660                 665                 670
   Lys Leu Val Ile Tyr Val Asn Gly Ser Leu Ala Thr Gln Leu Asp Leu
           675                 680                 685
   Pro Asp Lys Asp Gly Lys Ser Asp Gly Ser Ala Asn Glu Tyr Asp Met
       690                 695                 700
   Asp Val Glu Leu Trp Phe Glu Pro Gly Thr Tyr Glu Ile Lys Ile Asp
   705                 710                 715                 720
   Ser Glu Ala Cys Asp Trp Phe Thr Trp Asp Tyr Ile Val Phe Glu Asn
                   725                 730                 735
   Ala Val Tyr Arg Ala Ala Lys Val Asp Leu Tyr Ala Leu Ala Asn Ser
   740 745 750
   Thr Phe Ala Met Leu Trp Val Arg Asn Lys Asp Tyr Asn Trp Trp Asn
   755 760 765
   Val Val Val Leu Asn Lys Thr Leu Glu Pro Ala Glu Gly Val Glu Val
   770 775 780
   Glu Ile Arg Gly Leu Gln Asp Gly Val Tyr Arg Val Glu Phe Trp Asp
   785 790 795 800
   Thr Cys Arg Gly Val Val Val Lys Ser Met Glu Val Gln Val Ser Asn
   805 810 815
   Gly Val Ala Arg Val Pro Val Gly Ser Val Glu Lys Asp Ile Ala Met
   820 825 830
   Lys Ile Thr Arg Ala Gly
   835
<210> 17
   <211> 60
   <212> PRT
   <213> Artificial Sequence
<220>
   <223> EBI5324142
<400> 17
   Met Ala Met Arg Thr Gly Leu Ala Leu Gly Ile Val Ala Leu Ile Ala
   1 5 10 15
   Val Ile Leu Ile Ala Val Leu Leu Ala Thr Gln Gln Gln Pro Thr Pro
   20 25 30
   Thr Pro Ser Pro Thr Pro Thr Pro Ser Pro Thr Pro Thr Pro Thr Pro
   35 40 45
   Thr Pro Thr Pro Thr Pro Thr Pro Thr Pro Thr Pro
   50 55 60
<210> 18
   <211> 41
   <212> PRT
   <213> Artificial Sequence
<220>
   <223> EBI5327647
<400> 18
   Leu Asn Lys Thr Val Ile Ala Ile Ala Val Leu Leu Val Val Val Ile
   1 5 10 15
   Ala Ala Ala Leu Ile Tyr Val Ile Tyr Tyr Pro Thr Thr Pro Thr Thr
   20 25 30
   Thr Thr Pro Thr Val Thr Thr Pro Val
   35 40
<210> 19
   <211> 1258
   <212> PRT
   <213> Artificial Sequence
<220>
   <223> YP_003548440.1
<400> 19
   Met His Arg Cys Arg Tyr Ser Ile Ser Leu Val Trp Lys Gln Leu Ser
   1               5                   10                  15
   Gly Arg Lys Leu Ala Leu Thr Lys Ser Lys Ala Ser Ala Ser Arg Leu
               20                  25                  30
   Arg Thr Arg Gln Ser Leu Trp Ala Leu Leu Ser Ser Val Leu Leu Gly
           35                  40                  45
   Phe Val Cys Ala Gly Ala Leu Gln Ala Gln Val Leu Glu Asp Ala Tyr
       50                  55                  60
   Leu Glu Ser Gly Gly Ala Val Val Phe Glu Val Glu Ser Glu Ala Ala
   65                  70                  75                  80
   Val Ser Pro Trp Met Leu Asp Asn Ser Val Ala Gly Tyr Lys Gly Thr
                   85                  90                  95
   Gly Tyr Phe Glu Gly Thr Ala Asp Tyr Phe Ser Thr Pro Gly Gln Gly
               100                 105                 110
   Val Val Arg Tyr Pro Ile Lys Ile Thr Thr Ser Gly Arg Tyr Gln Leu
           115                 120                 125
   Gln Trp Arg Ser Arg Ile Asn Phe Gly Thr Glu Thr Ser Glu His Asn
       130                 135                 140
   Asp Ser Trp Ala Arg Leu Thr Asp Ala Asn Gly Asn Pro Val Ser Pro
   145                 150                 155                 160
   Ala Ser Asn Ser Asn Val Ala Asn Ser Gln Trp Tyr Lys Val Tyr Val
                   165                 170                 175
   Gly Trp Thr Gly Trp Gln Trp Gly Ser Ser Asn Lys Asp Asn Asp Pro
               180                 185                 190
   Arg Ser Leu Ser Trp Asn Leu Thr Ala Gly Asp Tyr Tyr Tyr Val Glu
           195                 200                 205
   Ile Ser Val Arg Ser His Tyr His Ala Leu Asp Arg Ile Val Leu Trp
       210                 215                 220
   Asp His Asn Arg Leu Ala Leu Ala Asn Thr Thr Thr Gly Lys Gly Ala
   225                 230                 235                 240
   Asn Asn Ser Ala Leu Asp Ala Leu Pro Val Ser Ala Ile Glu Val Gln
                   245                 250                 255
   Glu Gly Pro Asp Val Glu Ile Thr Asp Pro Val His Gly Thr Thr Ile
               260                 265                 270
   Val Pro Gly Gly Thr Val Thr Phe Thr Ala Ser Ala Ser Asp Ala Gln
           275                 280                 285
   Gly Ser Val Val Ser Val Glu Phe Phe Ala Gly Thr Thr Ser Leu Gly
       290                 295                 300
   Ile Asp Thr Ser Ala Pro Phe Ser Gln Ala Trp Ser Ser Ala Ala Glu
   305                 310                 315                 320
   Gly Val Tyr Glu Ile Thr Ala Leu Ala Thr Asp Asn Glu Gly Tyr Thr
                   325                 330                 335
   Thr Thr Ser Ala Pro Ile Thr Leu His Val Ala Pro Ser Met Gly Ala
               340                 345                 350
   Asn Gly Thr Val Ser Gly Glu Leu Met Gln Trp His Lys Val Met Leu
           355                 360                 365
   Thr Phe Asp Gly Pro Gly Thr Ser Glu Thr Ala Thr Pro Asn Pro Phe
       370                 375                 380
   Arg Asp Tyr Arg Met Asp Val Thr Phe Thr Gly Pro Ser Ser Gln Ser
   385                 390                 395                 400
   Tyr Val Val Pro Gly Tyr Tyr Ala Ala Asp Gly Asn Ser Gly Glu Thr
                   405                 410                 415
   Ser Leu Gly Ser Gly Asn Arg Trp Arg Val Ala Phe Ala Pro Asp Glu
               420                 425                 430
   Ala Gly Thr Trp Asn Tyr Ser Val Ser Phe Val Thr Gly Thr Asp Ile
           435                 440                 445
   Ala Ala Asp Leu Ser Gly Gly Ala Ser Ala Gly Phe Phe Asp Gly Ala
       450                 455                 460
   Thr Gly Thr Phe Ser Val Ala Ala Ser Asp Lys Ser Gly Ala Asp Leu
   465                 470                 475                 480
   Arg Ala Lys Gly Lys Leu Glu Tyr Val Gly Asp His Tyr Leu Gln Phe
                   485                 490                 495
   Arg Asn Gly Glu Tyr Phe Ile Lys Gly Gly Ala Asn Ser Pro Glu Val
               500                 505                 510
   Leu Leu Glu Tyr Ser Gly Phe Asp Asn Thr Asp Ser Thr Arg Thr Tyr
           515                 520                 525
   Ser Ala His Thr Ile Asn Trp Gln Leu Gly Asp Pro Thr Trp Lys Gly
       530                 535                 540
   Gly Glu Gly Lys Gly Leu Val Gly Val Ile Asn Tyr Leu Ala Asp Leu
   545 550 555 560
   Gly Leu Asn Ser His Tyr Phe Leu Leu Met Asn Ser Tyr Gly Asp Gly
   565 570 575
   Lys Lys Ala Phe Pro Phe Leu Gly Glu Asp Asp Ile Trp Arg Tyr Asp
   580 585 590
   Cys Ser Lys Leu Glu Gln Trp Asp Val Leu Phe Glu His Phe Asp Arg
   595 600 605
   Lys Gly Met Met Met His Phe Val Met Thr Glu Gln Glu Asn Gln Gln
   610 615 620
   Leu Phe Glu Val Ala Asp Pro Ala Thr Val Glu Gly Gly Phe Ser Asp
   625 630 635 640
   Ser Arg Arg Ile Tyr Phe Arg Glu Met Val Ala Arg Phe Gly His His
   645 650 655
   Met Ala Ile Thr Trp Asn Ile Gly Glu Glu Asn Gly Trp Glu Lys Gln
   660 665 670
   Thr Arg Pro Thr Ile Tyr Ala Gly Ala Cys Ser Asp Thr Gln Arg Lys
   675 680 685
   Asp Phe Ser Asp His Leu Arg Ala Leu Leu Pro Tyr Glu Asp His Ile
   690 695 700
   Ser Ile His Asn Gly Pro Ser Ser Thr Asp Ala Ile Phe Asn Ala Leu
   705 710 715 720
   Val Gly His Thr Ser Phe Thr Gly Pro Ala Phe Gln Trp Asn Ile Asn
   725 730 735
   Thr Asn Ile Ala Ala Lys Thr Lys Gln Trp Arg Asp Ala Ser Ile Ala
   740 745 750
   Ser Gly His Lys Trp Val Phe Cys Met Asp Glu Pro Tyr Leu Gly Gly
   755 760 765
   Asn Pro Asn Asp Ala His Asp Thr Asn Arg Lys Gln Thr Leu Trp Pro
   770 775 780
   Ala Tyr Met Ala Gly Ala Ala Gly Val Glu Trp Tyr Ile Gly Gly Gly
   785 790 795 800
   Gln Asp Leu Gln Val Gln Asp Tyr Thr Leu Tyr Glu Pro Leu Trp Thr
   805 810 815
   Glu Met Gly Tyr Ala Val Asp Leu Leu Glu Ile Ile Pro Phe His Ala
   820 825 830
   Met Glu Pro Asn Asp Ala Leu Leu Thr Gly Glu Thr Gly Gly Ala Gly
   835 840 845
   Gln Val Leu Ala Asp Leu Gly Ala Ser Tyr Leu Ala Tyr Leu Pro Asn
   850 855 860
   Ala Thr Ala Ser Ala Ser Leu Asn Leu Ser Gly Gln Ser Gly Asn Phe
   865 870 875 880
   Asp Val Met Trp Tyr Asp Pro Arg Asn Gly Gly Asp Leu Gln Met Gly
   885 890 895
   Ser Val Ser Thr Val Thr Gly Gly Gly Ile Arg Ser Leu Gly Ala Ala
   900 905 910
   Pro Ser Ala Ser Ala Glu Asp Trp Leu Val Leu Val Phe Ala Glu Gly
   915 920 925
   Thr Met Pro Val Met Pro Gly Asp Pro Val Pro Pro Ala Leu Ser Tyr
   930 935 940
   Leu Glu Ile Trp Asn Glu Gly Phe Glu Asn Ala Asn Leu Gly Ala Thr
   945 950 955 960
   Ser Ala Ser Asn Ala Asp Leu Pro Gly Ala Val Phe Tyr Gly Arg Asn
   965 970 975
   Gly Leu Thr Ala Glu Val Val Asn Ala Pro Ala Gly Phe Ser Ser Ala
   980 985 990
   Ser Gly Gln Val Ile Ala Leu Ser Thr Thr Thr Asn Ala Tyr Ala Ala
   995 1000 1005
   Ala Lys Arg Gln Glu Ser Ala Ile Asp Leu Ser Ala Leu Ser Leu Lys
   1010 1015 1020
   Ala Gly Asp Thr Tyr Arg Leu Ser Phe Asp Met Tyr Ile Pro Ser Pro
   1025 1030 1035 1040
   Leu Ser Thr Ala Val Gly Ala Ile Ser Phe Arg Trp Arg Thr Ala Thr
   1045 1050 1055
   Ala Thr Gly Asn Gly Pro Thr Asp Ser Ser Gln Ala Thr Leu Ser Ala
   1060 1065 1070
   Gly Val His Arg Ile Glu Tyr Thr Gly Thr Phe Pro Val Ile Asn Gly
           1075                1080                1085
   Ser Glu Ile Leu Pro Thr Ser Val Glu Pro Phe Ile Met Phe His Gln
       1090                1095                1100
   Asn Gly Val Ala Ala Ser Gln His Val Tyr Leu Asp Asn Ile Leu Phe
   1105                1110                1115                1120
   Glu Ile Glu Ser Pro Gln Leu Ser Gly Phe Glu Lys Phe Ala Asp Asp
                   1125                1130                1135
   Tyr Ala Leu Ile Gly Gly Lys Thr Asp Asp Asp Asp Leu Asp Gly Gln
               1140                1145                1150
   Thr Asn Phe Met Glu Phe Ala Thr Gly Gly Asn Pro Thr Asp Pro Ser
           1155                1160                1165
   Asp Ile Gly Leu Ile Arg Val Ser Phe Asp Gly Asp Gly Asn Ala Arg
       1170                1175                1180
   Val Ser Val Pro Gln Arg Ile Asp Gly Asn Glu Leu Gly Leu Ser Tyr
   1185                1190                1195                1200
   Thr Val Tyr Asn Arg Thr Ser Leu Thr Glu Gly Ser Trp Ala Glu Leu
                   1205                1210                1215
   Ser Thr Asn Ala Ile Phe Thr Ser Ser Ile Glu Gly Thr Ala Glu Tyr
               1220                1225                1230
   Glu Thr Tyr Gly Tyr Arg Phe Trp Val Gly Thr Gly Ser Phe Ser Asp
           1235                1240                1245
   Arg Phe Phe Arg Val Glu Ile Ser Asp Asn
       1250                1255
<210> 20
   <211> 859
   <212> PRT
   <213> Artificial Sequence
<220>
   <223> YP_001820771.1
<400> 20
   Met Met Leu Leu Arg Thr Val Val Trp Ala Gly Ala Leu Val Leu Gly
    1               5                  10                  15
   Ser Val Phe Cys Val Ser Ala Ser Gly Ala Thr Arg Asn Leu Gly Ser
               20                  25                  30
   Lys Thr Thr Phe Ser Gly Glu Gln Lys Gln Trp His Lys Val Ser Leu
           35                  40                  45
   Thr Phe Ala Gly Pro Ser Thr Ser Glu Thr Asn Ser Val Asn Pro Phe
       50                  55                  60
   Thr Asn Tyr Arg Leu Asn Val Thr Phe Lys His Ser Ala Ser Asn Arg
   65                  70                  75                  80
   Thr Leu Ile Val Pro Gly Tyr Phe Ala Ala Asp Gly Asn Ala Ala Asn
                   85                  90                  95
   Thr Gly Ala Val Ser Gly Asp Lys Trp Arg Val Asp Phe Thr Pro Asp
               100                 105                 110
   Ala Thr Gly Thr Trp Thr Tyr Val Ala Ser Phe Arg Thr Gly Ser Asn
           115                 120                 125
   Val Ala Ala Ser Thr Ser Ala Thr Ala Gly Thr Ala Thr Ser Phe Asn
       130                 135                 140
   Gly Glu Ser Gly Ser Phe Thr Ile Asp Pro Thr Asp Lys Thr Gly Ala
   145                 150                 155                 160
   Asp Phe Arg Ala Lys Gly Arg Leu Arg Glu Val Gly Gln His Tyr Leu
                   165                 170                 175
   Gln His Ala Gly Ser Lys Glu Tyr Phe Ile Lys Ser Gly Ala Gly Ser
               180                 185                 190
   Pro Glu Asn Phe Leu Ala Phe Ala Asp Phe Asp Asn Thr Ser Ala Gly
           195                 200                 205
   Lys Lys Ile Leu His His Tyr Thr Ala His Leu Ser Ala Tyr Arg Ser
       210                 215                 220
   Gly Asp Pro Thr Trp Lys Ser Gly Lys Gly Lys Ala Ile Ile Gly Ala
   225                 230                 235                 240
   Leu Asn Tyr Leu Ala Ser Lys Lys Val Asn Ser Val Tyr Phe Leu Thr
   245 250 255
   Met Asn Ile Gly Gly Asp Gly Asp Asp Val Phe Pro Phe Val Ser Lys
   260 265 270
   Thr Asp Arg Thr Arg Phe Asp Val Ser Lys Leu Ala Gln Trp Glu Ile
   275 280 285
   Val Phe Ser His Met Asp Lys Leu Gly Ile Met Leu Asn Val Val Thr
   290 295 300
   Gln Glu Gln Glu Cys Asp Gln Leu Leu Asp Gly Gly Ser Leu Gly Asn
   305 310 315 320
   Thr Arg Lys Ile Tyr Tyr Arg Glu Leu Val Ala Arg Phe Gly His His
   325 330 335
   Leu Gly Val Thr Trp Asn Leu Gly Glu Glu Asn Thr Asn Thr Asp Ala
   340 345 350
   Gln Arg Glu Ala Phe Ala Asp Tyr Leu Asn Ala Leu Asp Pro Tyr Phe
   355 360 365
   Ser Leu Ile Ala Val His Thr Tyr Pro Ser Gln Arg Asp Thr Ile Tyr
   370 375 380
   Thr Gly His Leu Gly Ser Glu Leu Ile Ser Gly Ala Ser Leu Gln Leu
   385 390 395 400
   Glu Ser Pro Ser Ile Val His Glu Gln Thr Leu Lys Trp Val Lys Lys
   405 410 415
   Ser Ala Ala Ala Gly Ser Lys Trp Val Val Ser Val Asp Glu Leu Gly
   420 425 430
   Pro Ser Ser Ala Gly Val Val Pro Asp Ala Asn Asp Pro Ala His Glu
   435 440 445
   Thr Ile Val His Arg Val Leu Trp Gly Ser Leu Leu Ala Gly Gly Ala
   450 455 460
   Gly Val Glu Trp Tyr Phe Gly Tyr Asn Tyr Pro Gln Thr Asp Leu Thr
   465 470 475 480
   Leu Glu Asp Trp Lys Ser Arg Asp Lys Met Trp Thr Leu Thr Gln His
   485 490 495
   Ala Ala Gln Phe Met Arg Asp Tyr Met Pro Leu Pro Leu Val Ala Asn
   500 505 510
   Tyr Asp Ser Ile Thr Ser Ser Thr Ser Asp Tyr Cys Phe Gly Lys Pro
   515 520 525
   Gly Val Ala Tyr Ala Ile Tyr Leu Pro Gln Gly Ala Ile Thr Asn Ile
   530 535 540
   Thr Val Pro Ser Gly Glu Gly Tyr Thr Val His Trp Tyr Asn Pro Arg
   545 550 555 560
   Ala Gly Gly Ser Leu Gln Thr Gly Thr Val Lys Ser Ile Ala Gly Gly
   565 570 575
   Thr Ala Ala Ile Gly Arg Pro Pro Thr Gln Gln Ser Glu Asp Trp Val
   580 585 590
   Ala Leu Leu Arg Arg Thr Ser Gly Thr Thr Thr Gly Ala Pro Ala Pro
   595 600 605
   Ala Pro Thr Glu Pro Thr Ser Thr Thr Ala Val Thr Gln Leu Thr Leu
   610 615 620
   Val Asn Ala Ser Thr Glu Lys Asp Leu Arg Ala Leu Thr Asn Gly Ser
   625 630 635 640
   Thr Ile Thr Phe Gly Thr Asp Gly Lys Ala Leu Asn Val Arg Ala Thr
   645 650 655
   Thr Ser Gly Thr Val Gly Ser Val Ala Phe Ile Leu Asp Gly Gln Thr
   660 665 670
   Ile Gln Thr Glu Asn Met Ala Pro Tyr Thr Leu Ala Gly Asp Ser Asn
   675 680 685
   Gly Asp Tyr Ala Ser Trp Thr Pro Ser Val Gly Thr His Val Leu Lys
   690 695 700
   Val Val Pro Tyr Ser Gly Arg Asp Arg Thr Gly Asn Ala Gly Thr Ala
   705 710 715 720
   Leu Gln Val Ser Phe Thr Val Gln Ser Thr Ala Thr Glu Asp Ser Ser
   725 730 735
   Ser Ala Pro Val Val Ser Glu Pro Ala Ser Gly Ala Ser Val Thr Lys
   740 745 750
   Leu Thr Leu Ile Asn Ala Ser Thr Glu Lys Asp Leu Arg Ala Leu Thr
                   755                 760                 765
   Asn Gly Ser Thr Ile Thr Phe Gly Thr Asp Gly Lys Ala Leu Asn Val
       770                 775                 780
   Arg Ala Glu Thr Ser Gly Thr Val Gly Ser Val Ala Phe Ile Leu Asp
   785                 790                 795                 800
   Gly Lys Thr Leu Arg Thr Glu Asn Val Ala Pro Tyr Thr Leu Ala Gly
                   805                 810                 815
   Asp Gly Thr Gly Asn Tyr Tyr Ser Trp Thr Pro Ser Val Gly Ser His
               820                 825                 830
   Thr Leu Lys Val Val Pro Tyr Ser Gly Lys Asp Arg Thr Gly Thr Ala
           835                 840                 845
   Gly Thr Ser Leu Gln Val Gly Phe Thr Val Lys
       850                 855
<210> 21
   <211> 606
   <212> PRT
   <213> Artificial Sequence
<220>
   <223> YP_003547883.1
<400> 21
   Met Lys Asn Thr Ile Arg Leu Thr Thr Leu Ala Leu Leu Ala Ala Ala
    1               5                  10                  15
   Gln Ala Ala His Ala Glu Ser Ile Ala Glu Val Ser Gly Ser Leu Arg
               20                  25                  30
   Thr Trp His Lys Val Thr Leu Ser Trp Asn Gly Pro Gln Thr Asn Glu
           35                  40                  45
   Leu Ala Thr Pro Asn Pro Phe Thr Asp Tyr Arg Leu Asp Val Arg Phe
       50                  55                  60
   Thr His Gln Gln Ser Gly Thr Ser Tyr Leu Val Pro Gly Tyr Tyr Ala
   65                  70                  75                  80
   Ala Asp Gly Asp Ala Ala Asn Thr Gly Ala Asp Ser Gly Ser Val Trp
                   85                  90                  95
   Arg Val His Phe Ala Pro Asp Ala Ile Gly Asn Trp Asp Tyr Ala Val
               100                 105                 110
   Ser Phe Arg Thr Gly Glu Ala Val Ala Met Ala Gln His Pro Gln Val
           115                 120                 125
   Gly Asp Gly Thr His Phe Asp Gly Asp Ser Gly Thr Leu Asn Ile Arg
       130                 135                 140
   Pro Ser Ala Gln Lys Ala Pro Asp Leu Arg Ala Lys Gly Arg Leu Gln
   145                 150                 155                 160
   Tyr Val Gly Glu His Tyr Leu Lys Phe Ala Ala Ser Gly Glu Tyr Phe
                   165                 170                 175
   Leu Lys Gln Gly Ala Asp Ala Pro Glu Asn Phe Leu Ser Tyr Lys Gly
               180                 185                 190
   Phe Asp Gly Asp Phe Lys Ser Asp Gly Ile Asn Asp His Leu Val Lys
           195                 200                 205
   Asp Trp Glu Pro His Val Gln Asp Trp Lys Asp Gly Asp Pro Ser Trp
       210                 215                 220
   Ala Asp Gly Gln Gly Lys Gly Ile Ile Gly Ala Val Asn Tyr Leu Ala
   225                 230                 235                 240
   Ser Glu Gly Leu Asn Ala Phe Ser Phe Leu Thr Met Asn Ile Glu Gly
                   245                 250                 255
   Asp Asp Arg Asn Val Phe Pro Tyr Thr Thr Tyr Lys Glu Arg Tyr Arg
               260                 265                 270
   Met Asp Cys Ser Lys Leu Ala Gln Trp Glu Val Val Phe Glu His Ala
           275                 280                 285
   Asp Ser Lys Gly Met Phe Leu His Phe Lys Thr Gln Glu Thr Glu Asn
       290                 295                 300
   Glu Cys Leu Leu Asp Asn Gly Asp Thr Gly Pro Met Arg Arg Leu Tyr
   305                 310                 315                 320
   Tyr Arg Glu Leu Val Ala Arg Phe Gly His His Leu Ala Leu Asn Trp
                           325                 330                 335
   Asn Leu Gly Glu Glu Asn Gly Lys Trp Asp Trp Pro Gly His Val Lys
               340                 345                 350
   Glu His Phe Gln Ser Thr Glu Gln Arg Gln Ala Met Ala Gln Trp Phe
           355                 360                 365
   Tyr Asp Asn Asp Pro Tyr Lys His His Leu Val Ile His Asn Gly Gln
       370                 375                 380
   Ser Pro Asn Asp Leu Leu Gly Asp Ala Ser Lys Leu Thr Gly Phe Ser
   385                 390                 395                 400
   Leu Gln Thr Asn Leu Glu Asp Phe Ala Asn Val Pro Gly Thr Val Ala
                   405                 410                 415
   Ser Trp Ile Arg Lys Ser Ala Glu Ala Gly Lys Pro Trp Ala Val Ala
               420                 425                 430
   Cys Asp Glu Pro Gly Asp Ala Ser His Ala Ile Arg Pro Asp Asp Asn
           435                 440                 445
   Ala Gly Ser Ser His Glu Asn Gly Arg Arg Asn Ala Leu Trp Gly Cys
       450                 455                 460
   Leu Met Asn Gln Gly Tyr Gly Ser Glu Tyr Tyr Phe Gly Tyr Lys Asn
   465                 470                 475                 480
   Ala His Ser Asp Leu Thr Cys Asn Asp Tyr Arg Ser Arg Asp Lys Trp
                   485                 490                 495
   Trp Asp Tyr Cys Arg Tyr Ala Leu Glu Phe Phe His Asn His Lys Val
               500                 505                 510
   Ala Ile Trp Glu Leu Ala Pro Ala His Lys Leu Ser Ser Asn Ser Glu
           515                 520                 525
   Ser Trp Cys Leu Ala Lys Thr Gly Glu Thr Tyr Leu Ile Tyr Ile Lys
       530                 535                 540
   Asp Gly Ala Thr Thr Asn Leu Asp Leu Ser Gly Asp Ser Gly Lys Phe
   545                 550                 555                 560
   Gln Val Gln Trp Tyr Asp Thr Arg Lys Gly Gly Ser Leu Leu Ala Gly
                   565                 570                 575
   Ser Glu Lys Lys Ile Lys Ala Gly Glu Ala Val Ser Ile Gly Lys Pro
               580                 585                 590
   Pro Tyr Asp Pro Asp Arg Asp Trp Leu Val Leu Val Ser Lys
           595                 600                 605
<210> 22
   <211> 1853
   <212> PRT
   <213> Artificial Sequence
<220>
   <223> YP_003547687.1
<400> 22
   Met Met Lys Leu Leu Gln Leu Phe Thr Leu Cys Leu Leu Ser Met Ala
    1               5                  10                  15
   Thr Phe Ala Gln Thr Ala Leu Gly Gln Asp Thr Val Asp Leu Ser Gln
               20                  25                  30
   Leu Pro Thr Ser Leu Thr Pro Gln Thr Ser Tyr Thr Val Ser Val Pro
           35                  40                  45
   Tyr Thr Ala Ser Val Asp Arg Asp Ile Ala Val Glu Phe Trp Lys Gly
       50                  55                  60
   Gly Ala Trp Val Thr Ala Lys Thr Thr Thr Val Thr Ala Gly Ser Gly
   65                  70                  75                  80
   Thr Ala Ser Val Thr Leu Thr Leu Ala Thr Ala Pro Val Glu Gly Thr
                   85                  90                  95
   Asp Tyr Leu Trp Lys Ala Asn Ile Arg Pro Val Gly Thr Asp Trp Thr
               100                 105                 110
   Gln Asn Leu Asn Gly Gly Val Val Glu Asn Val Val Val Ser Leu Pro
           115                 120                 125
   Val Thr Glu Asp Thr Ile Asp Leu Thr Glu Leu Pro Thr Ser Met Pro
       130                 135                 140
   Pro Gln Ser Ser Tyr Thr Val Thr Val Pro Tyr Thr Ala Leu Glu Ser
   145                 150                 155                 160
   Arg Asp Ile Ala Leu Ser Leu Tyr Lys Gly Gly Ile Trp Gln Thr Gly
                   165                 170                 175
   Leu Thr Gln Thr Val Ala Ala Gly Arg His Thr Ala Ser Phe Thr Leu
               180                 185                 190
   Asn Leu Gly Ser Gln Ala Ala Glu Asp Thr Asp Tyr Glu Trp Arg Cys
           195                 200                 205
   Gly Ile Arg Pro Val Gly Ala Asp Trp Thr Gln Asn Leu Asp Ala Gly
       210                 215                 220
   Thr Ile Asp Asn Val Val Val Ser Ser Gly Ser Ser Gly Gly Gly Ser
   225                 230                 235                 240
   Gly Asn Gly Ala Trp Ile Glu Ser Gly Gly Met Val Val Ile Glu Ala
                   245                 250                 255
   Glu Asn Val Asp Leu Thr Ser Asp Trp Val Ala Arg Pro Ser Thr His
               260                 265                 270
   Gly Ala Ala Asn Ala Met Gly Gly Ser Leu Gly Asp Gly Trp Leu Glu
           275                 280                 285
   Trp Thr Gly Ala Gln Tyr Tyr Gly Asn Thr Gln Thr Glu Ala Gln Ala
       290                 295                 300
   Val Ala Ile Leu Thr Phe Glu Phe Glu Ile Thr Asn Pro Gly Asp Tyr
   305                 310                 315                 320
   Tyr Phe Arg Trp Arg Ser Lys Gln Tyr Asn Asn Val Gly Ser Gly Asp
                   325                 330                 335
   Ala Gly Asn Asp Ser Tyr Val Ser Leu Thr Ser Gly Thr Pro Val Ala
               340                 345                 350
   Gly Tyr Gln Asp Phe Gly Gln Phe His Lys Val Trp Val Gln Ser Gln
           355                 360                 365
   Gln Ala Trp Ser Trp Gln Thr Thr Phe Glu Pro His His Gly Glu His
       370                 375                 380
   Tyr Ala Asn Asn Leu Val Arg Arg His Tyr Glu Ala Gly Thr His Thr
   385                 390                 395                 400
   Ile Arg Leu Ala Ala Arg Ser Pro Gly His Ala Ile Asp Arg Ile Val
                   405                 410                 415
   Leu His Arg Thr Asp Val Pro Phe Asn Gln Ala Thr Phe Glu Ser Ala
               420                 425                 430
   Ala Glu Ser Glu Arg Ala Ala Gly Ile Gly Asp Thr Ile Thr Tyr Arg
           435                 440                 445
   Ala Thr Glu Asp Phe Pro Thr Leu Asn Ile Tyr Gly Thr Glu Ala Arg
       450                 455                 460
   Gly Thr Val Gln Val Asn Pro Gly Ala Gly Ala Val Asn Tyr Asp Asp
   465                 470                 475                 480
   Thr Val Phe Ala Ser Ala Thr Arg Thr Phe Asp Gly Pro Thr Gly Thr
                   485                 490                 495
   Tyr Asp Ile Asp Leu Thr Thr Trp Val Glu Tyr Asp Gly Glu Ser Thr
               500                 505                 510
   Tyr Arg Leu Leu Val Asn Gly Ser Gln Val Ala Ser Tyr Gln Asn Pro
           515                 520                 525
   Gln Val Thr Glu Ala Thr Asp Leu Thr Pro Asn Thr His Thr Trp Ser
       530                 535                 540
   Asn Ile Val Leu Thr Gln Gly Asp Ser Ile Thr Val Gln Ser Asn Ala
   545                 550                 555                 560
   His Ser Asn Asn Ile Ile Pro Glu Ala Gly Pro Pro Asn Gly Phe Ala
                   565                 570                 575
   Trp Ala Arg Gly Arg Trp Glu Gln Ile Glu Leu Thr Phe Val Ser Val
               580                 585                 590
   Asn Val Gly Ile Pro Thr Val Asp Ala Gly Pro Asp Gln Ser Val Ser
           595                 600                 605
   Thr Thr Gln Gly Ser Ala Thr Leu Asn Gly Thr Ala Ser Asp Asn Gly
       610                 615                 620
   Ser Ile Thr Asn Tyr Ala Trp Thr Gln Val Ser Gly Pro Asn Thr Ala
   625                 630                 635                 640
   Thr Leu Ser Gly Gln Ser Thr Val Asp Leu Thr Ala Ser Asn Leu Ile
                   645                 650                 655
   Ser Gly Thr Tyr Thr Phe Arg Leu Thr Val Thr Asp Asn Glu Ser Asn
               660                 665                 670
   Thr Ala Ser Asp Asp Ala Ile Val His Val Val Ser Thr Gly Asn Gly
   675 680 685
   Ala Val Ala Ile Thr Gly Asp Leu Met Gln Trp His Asn Val Ile Leu
   690 695 700
   Thr Met Asn Gly Pro Asn Ser Ser Glu Ser Ala Thr Pro Asn Pro Phe
   705 710 715 720
   Lys Asp Tyr Arg Met Asn Val Thr Phe Thr His Pro Asn Ser Gly Leu
   725 730 735
   Ser Tyr Thr Val Pro Gly Tyr Phe Ala Ala Asp Gly Asn Ala Gly Gln
   740 745 750
   Thr Gly Ala Thr Ser Gly Gly Lys Trp Arg Ala His Leu Cys Pro Asp
   755 760 765
   His Ala Gly Gln Trp Thr Tyr Ser Val Ser Phe Arg Ser Gly Thr Asp
   770 775 780
   Val Ala Val Asn Asn Ser Leu Ser Ala Gly Thr Ala Phe Ala Gly Leu
   785 790 795 800
   Asp Gly Lys Thr Gly Ser Phe Thr Val Val Ala Thr Asn Lys Thr Gly
   805 810 815
   Arg Asp His Arg Gly Lys Gly Arg Leu Gln Tyr Asp Gly Thr Arg Tyr
   820 825 830
   Leu Lys Phe Ala Gly Ser Gly Glu Ala Phe Leu Lys Thr Gly Ala Asp
   835 840 845
   Ala Pro Glu Asn Phe Leu Asn Tyr Thr Glu Phe Asp Asn Thr Tyr Thr
   850 855 860
   His Gly Ala Asn Tyr Leu Lys Asp Trp Ser Ala His Val Gly Asp Trp
   865 870 875 880
   Asn Ala Gly Asp Pro Thr Trp His Gly Thr Lys Gly Lys Gly Ile Ile
   885 890 895
   Gly Ala Ile Asn Tyr Leu Ala Ser Glu Gly Gln Asn Val Phe Ser Phe
   900 905 910
   Leu Thr Tyr Asn Ala Gly Gly Asp Ser Lys Asp Val Trp Pro Tyr Val
   915 920 925
   Ser His Thr Asn Pro Leu Gln Phe Asp Cys Ser Lys Leu Asp Gln Trp
   930 935 940
   Asp Ile Val Phe Ser His Gly Asp Lys Met Gly Met Tyr Leu His Phe
   945 950 955 960
   Lys Thr Gln Glu Arg Glu Asn Asp Asp Leu Asp Gly Pro Gly Ser Ala
   965 970 975
   Tyr Ala Leu Asp Gly Gly Asn Val Gly Thr Glu Arg Lys Leu Tyr Tyr
   980 985 990
   Arg Glu Leu Ile Ala Arg Phe Gly His His Leu Ala Leu Asn Trp Asn
   995 1000 1005
   Leu Gly Glu Glu Asn Thr Gln Ser Thr Ser Gln Arg Gln Ala Met Ala
   1010 1015 1020
   Gln Tyr Phe Arg Asp Thr Asp Pro Tyr Gly His Asn Ile Val Leu His
   1025 1030 1035 1040
   Thr Tyr Pro Gly Glu Trp Glu Gln Val Tyr Arg Pro Leu Leu Gly Ser
   1045 1050 1055
   Ala Ser Glu Leu Thr Gly Ala Ser Ile Gln Thr Asn Tyr Asn Thr Val
   1060 1065 1070
   His Ser Arg Thr Leu Gln Trp Leu Asn Glu Ser Thr Ala Ala Gly Lys
   1075 1080 1085
   Val Trp Val Val Ala Asn Asp Glu Gln Gly Pro Ala Ser His Ala Asn
   1090 1095 1100
   Pro Pro Asp Asn Gly Trp Pro Gly Tyr Thr Gly Ser Thr Thr Pro Ser
   1105 1110 1115 1120
   Gln Lys Gln Met Arg Trp Gln Thr Val Trp Gly Asn Tyr Met Ala Gly
   1125 1130 1135
   Gly Ala Gly Ile Glu Leu Tyr Ala Gly Tyr Gln Asn Pro Gln Ser Asp
   1140 1145 1150
   Leu Thr Leu Asp Asp Phe Arg Ser Arg Asp Arg Met Trp Asp Tyr Cys
   1155 1160 1165
   Arg His Ala Asn Thr Phe Phe Thr Glu His Leu Pro Phe Trp Glu Met
   1170 1175 1180
   Ala Asn Ala Asn Ser Leu Ile Gly Asn Thr Ser Asn Asn Asn Asp Lys
   1185 1190 1195 1200
   Tyr Cys Phe Ala Lys Thr Gly Glu Tyr Tyr Ala Ile Tyr Leu Pro Asn
   1205 1210 1215
   Gly Gly Thr Thr Asn Leu Asn Leu Ser Gly Ala Thr Gly Thr Phe Asp
   1220 1225 1230
   Ile Leu Trp Tyr Asp Pro Arg Asn Gly Gly Ala Leu Gln Ala Gly Thr
   1235 1240 1245
   Val Ser Ser Val Ile Gly Gly Ser Asn Val Ser Val Gly Asn Ala Pro
   1250 1255 1260
   Ser Ser Thr Thr Asp Asp Trp Ala Ile Leu Val Val Lys Gln Gly Leu
   1265 1270 1275 1280
   Gly Thr Gly Leu Leu Val Asp Ala Gly Ala Ala Lys Thr Ile Ile Leu
   1285 1290 1295
   Pro Thr Asn Gln Val Thr Leu Asn Gly Ser Ser Ser Asp Asp Gly Thr
   1300 1305 1310
   Ile Thr Ser Arg Leu Trp Thr Gln Ile Ser Gly Pro Asn Thr Ala Ala
   1315 1320 1325
   Leu Ser Gly Gln Thr Ser Asn Thr Leu Gln Ala Ser Ser Leu Ile Ala
   1330 1335 1340
   Gly Ser Tyr Val Phe Arg Leu Thr Val Thr Asp Asn Asp Ser Asn Thr
   1345 1350 1355 1360
   Ala Tyr Asp Gln Thr Thr Val Thr Val Glu Val Asp Ser Ala Pro Ser
   1365 1370 1375
   Ile Thr Thr Ser Ser Leu Pro Asp Gly Thr Val Ser Ala Ser Tyr Ser
   1380 1385 1390
   Gln Thr Leu Ala Ala Ser Gly Gly Asn Pro Gln Leu Ala Trp Ser Ile
   1395 1400 1405
   Ile Glu Gly Ser Leu Pro Thr Gly Leu Ser Ile Asn Ser Ser Gly Val
   1410 1415 1420
   Ile Ser Gly Thr Pro Thr Ala Thr Gly Leu Ser Val Phe Lys Val Gln
   1425 1430 1435 1440
   Thr Gln Asp Ala Asn Gly Asp Thr Asp Asp Ala Val Phe Ser Ile Lys
   1445 1450 1455
   Val Val Glu Val Thr Thr Ser Thr Lys Thr Phe Asn Pro Thr Asp Asp
   1460 1465 1470
   Ala Phe Ile Glu Trp Ser Thr Pro Tyr Asn Thr Thr Gln Leu Lys Ile
   1475 1480 1485
   Glu Asn Gly Ser Arg Val Gly Tyr Met Lys Phe Asn Ile Thr Gly Ile
   1490 1495 1500
   Thr Thr Gln Val Glu Ser Ala Val Leu Ser Met Arg Val Ala Gly Asp
   1505 1510 1515 1520
   Ser Gly Asn Gly Thr Ile Arg Phe Tyr Leu Gly Ser His Asn Asn Trp
   1525 1530 1535
   Thr Glu Ala Thr Ile Thr Thr Ala Asn Arg Pro Ala Lys Gly Ala Gln
   1540 1545 1550
   Val Gly Ser Met Thr Gly Ser Phe Ser Asn Asn Thr Thr Tyr Gln Ala
   1555 1560 1565
   Asp Ile Thr Ser Met Leu Asn Gly Ser Gly Asp Gly Val Tyr Thr Leu
   1570 1575 1580
   Val Ile Glu Met Asp Ser Gly Gly Asn Asp Ala Trp Phe Ser Ser Thr
   1585 1590 1595 1600
   Glu Gly Ala Asn Pro Pro Ser Leu Val Val Asn Tyr Ser Asp Gly Ser
   1605 1610 1615
   Thr Asp Glu Ile Pro Val Ala Asn Ala Gly Ala Asp Lys Ala Ile Thr
   1620 1625 1630
   Leu Pro Thr Asn Gln Val Leu Ile Asn Gly Ser Gly Thr Asp Asp Gly
   1635 1640 1645
   Ser Ile Ser Ser Tyr Ala Trp Ser Gln Val Met Gly Pro Asn Thr Ala
   1650 1655 1660
   Ser Leu Ser Gly Ala Phe Ser Ala Lys Leu Ile Ala Thr Gly Leu Ile
   1665 1670 1675 1680
   Ala Gly Glu Tyr Ala Phe Val Leu Thr Val Thr Asp Asn Thr Ala Asn
   1685 1690 1695
   Glu Asp Ser Asp Met Val Ile Val Val Val Asn Pro Ala Val Gly Ser
   1700 1705 1710
   Gly Ser Ala Tyr Thr Asn Trp Ala Ser Asn Gln Phe Ala Gly Leu Ser
   1715 1720 1725
   Gly Gly Ala Thr Asn Pro Leu Ala Ala Phe Asp Ala Ser Tyr Met Gly
   1730 1735 1740
   Asn Gly Leu Pro Asn Gly Leu Ile Tyr Ala Met Gly Gly Asn Pro His
   1745 1750 1755 1760
   Glu Ala Asn Asn Asp Ile Arg Ala Met Leu Pro Glu Ala Arg Gly Asp
   1765 1770 1775
   Arg Val Glu Phe Thr Leu Pro Asp Ser Ile Pro Ala Gly Val Ser Val
   1780 1785 1790
   Arg Leu Tyr Gln Ala Ser Asp Leu Thr Ala Val Ser Pro Trp Ser Glu
   1795 1800 1805
   Thr His Val Arg Asn Ser Asn Gly Thr Trp Thr Pro Ser Leu Ser Ser
   1810 1815 1820
   Ser Ala Asn Gly Asp Gly Thr Ser Thr Phe Thr Leu Pro Leu Gly Gly
   1825 1830 1835 1840
   Gly Ser Thr Gly Phe Tyr Leu Leu Asp Phe Ser Ala Glu
   1845 1850
<210> 23
   <211> 648
   <212> PRT
   <213> Artificial Sequence
<220>
   <223> YP_001818722.1
<400> 23
   Met Arg Ile Arg His Ser Ser Ile Cys Ala Leu Ala Ser Ala Ala Ile
    1               5                  10                  15
   Tyr Ala Val Phe Thr Pro Ala Ala Ala Gly Ala Ala Ala Leu Val Ala
               20                  25                  30
   Gly Lys Leu Glu Gln Trp His Lys Ile Thr Leu Ser Ile Asp Gly Pro
           35                  40                  45
   Glu Ala Arg Glu Thr Asp Thr Ser Pro Asn Pro Phe Leu Asp Tyr Arg
       50                  55                  60
   Met Asp Val Thr Phe Thr His Glu Ser Gly Ala Pro Ser Tyr Arg Val
   65                  70                  75                  80
   Pro Gly Tyr Phe Ala Val Asp Gly Asn Ala Ala Glu Thr Ser Ala Phe
                   85                  90                  95
   Ala Gly Arg Ile Trp Arg Ala His Leu Ala Pro Asp Lys Pro Gly Met
               100                 105                 110
   Trp Arg Tyr Ala Val Ser Phe Arg Arg Gly Pro Glu Val Ala Val Ser
           115                 120                 125
   Thr Leu Glu Ala Gly Ala Pro Val Asp Gly Cys Asp Gly Ile Ser Gly
       130                 135                 140
   Glu Phe Thr Val Val Pro Thr Asp Lys Thr Gly Arg Asp Phe Arg Ala
   145                 150                 155                 160
   His Gly Arg Leu Asp Tyr Val Gly Gly Arg Tyr Leu Arg Phe Ala Gly
                   165                 170                 175
   Ser Gly Glu Tyr Phe Leu Lys Val Gly Ala Asp Ser Pro Glu Asn Leu
               180                 185                 190
   Leu Gly Tyr Ser Asp Phe Asp Gly Thr Arg Ser Asn Lys Pro Gly Thr
           195                 200                 205
   Pro Ala Arg Pro Asp Glu Ala Ala Pro Pro Ser Leu Leu Lys Thr Trp
       210                 215                 220
   Gln Pro His Val Arg Asp Trp Arg Glu Gly Asp Pro Thr Trp Gln His
   225                 230                 235                 240
   Arg Lys Gly Lys Gly Leu Ile Gly Ala Leu Asn Tyr Leu Ala Ser Thr
                   245                 250                 255
   Gly Cys Asn Ala Phe Ser Phe Leu Thr Tyr Asn Ala Gly Gly Asp Gly
               260                 265                 270
   Asp Asp Val Trp Pro Phe Val Glu Arg Asp Asp Pro Leu His Phe Asp
           275                 280                 285
   Cys Ser Lys Leu Asp Gln Trp Gln Ile Ile Phe Asp His Ala Thr Ala
       290                 295                 300
   Leu Gly Leu His Leu His Phe Lys Leu Glu Glu Thr Glu Asn Asp Asp
   305                 310                 315                 320
   Asn Arg Pro Gly Gly Asp Gly Gln Ile Gly Asp Val Pro Thr Ala Leu
                   325                 330                 335
   Asp Arg Gly Lys Thr Gly Val Glu Arg Lys Leu Tyr Leu Arg Glu Leu
               340                 345                 350
   Ile Ala Arg Phe Ala His Glu Leu Ala Leu Asn Trp Asn Leu Gly Glu
           355                 360                 365
   Glu Asn Thr Leu Ser Thr Glu Gln Gln Gln Ala Met Ala Ala Phe Ile
       370                 375                 380
   Arg Asp Thr Asp Pro Tyr His His Pro Ile Val Leu His Thr Phe Pro
   385                 390                 395                 400
   Asp Trp Gln Glu Arg Val Tyr Arg Pro Leu Leu Gly Asp Arg Ser Ala
                   405                 410                 415
   Leu Thr Gly Val Ser Leu Gln Thr Gly Trp Glu Gln Ser His Arg Arg
               420                 425                 430
   Val Leu Gln Trp Ile Glu Glu Ser Ala Ala Ala Gly Lys Gln Trp Val
           435                 440                 445
   Val Ala His Asp Glu Gln Asn Pro His Tyr Thr Gly Val Pro Pro Asp
       450                 455                 460
   Thr Gly Trp Glu Gly Phe Asp Gly Thr Ala Arg Pro Glu Lys Tyr Ser
   465                 470                 475                 480
   Arg Pro Tyr Thr Ala Asp Asp Val Arg Lys His Thr Leu Trp Gly Ser
                   485                 490                 495
   Leu Leu Ala Gly Gly Ala Gly Val Glu Tyr Tyr Phe Gly Tyr Thr Leu
               500                 505                 510
   Pro Gln Asn Asp Leu Gly Ala Gln Asp Trp Arg Ser Arg Ala Gln Ser
           515                 520                 525
   Trp Lys Trp Cys Asp Leu Ala Leu Arg Phe Phe Arg Glu Asn Ala Ile
       530                 535                 540
   Pro Phe Trp Asn Met His Asn Ala Asp Glu Leu Val Gly Asn Pro Ser
   545                 550                 555                 560
   His Asp Asn Ser Arg Tyr Cys Phe Ala Gln Pro Gly Glu Ile Tyr Val
                   565                 570                 575
   Val Tyr Leu Pro Asn Gly Gly Ser Ala Glu Leu Asp Leu Gly Arg Gly
               580                 585                 590
   Ala Asp Gly Ala Thr Phe Gln Val Arg Trp Phe Asn Pro Arg Glu Gly
           595                 600                 605
   Gly Pro Leu Gln Ser Gly Asn Val Ser Glu Val Arg Gly Ser Gly Arg
       610                 615                 620
   Val Ser Leu Gly Glu Pro Pro Ala Asp Ala Ala Ala Asp Trp Val Val
   625                 630                 635                 640
   Leu Val Ala Arg Ala Pro Arg Pro
                   645
<210> 24
   <211> 1043
   <212> PRT
   <213> Artificial Sequence
<220>
   <223> NP_869354.1
<400> 24
   Met Val Ala Pro Ile Thr Pro Ser Arg Ser Pro Asn Thr Met Gln Val
    1               5                  10                  15
   Cys Arg Leu Arg Lys Phe Leu Thr Leu Gln Thr Val Phe Ala Leu Ala
               20                  25                  30
   Val Thr Ala Thr Trp Cys Val Ser Val Ala Ala Gln Lys Pro Asp Ala
           35                  40                  45
   Val Phe Thr Glu Ala Asn Gly Phe Leu Lys Val Glu Ala Glu Asp Phe
       50                  55                  60
   Ala Ser Gln Thr Asn Thr Asp Lys Arg Ala Phe Tyr Leu Thr Thr Ala
   65 70 75 80
   Glu Ser Ala Pro Ser Val Gln Pro Asp Gly Asp Pro Ser His Ala Ser
   85 90 95
   Asp Ala Ser Gly Gly Ala Tyr Leu Glu Ile Leu Pro Asp Thr Arg Arg
   100 105 110
   Thr His Ala Asp Lys Leu Ile His Gly Thr Asn Phe Ser Pro Gln Pro
   115 120 125
   Gly Lys Met Ala Val Leu Thr Tyr Arg Val Asn Val Gln Thr Pro Gly
   130 135 140
   Arg Tyr Tyr Val Trp Val Arg Ala Tyr Ser Thr Gly Ser Glu Asp Asn
   145 150 155 160
   Gly Leu His Val Gly Ile Asp Gly Thr Trp Pro Glu Ser Gly Gln Arg
   165 170 175
   Leu Gln Trp Cys Gln Gly Lys His Ser Trp Tyr Trp Asp Ser Lys Gln
   180 185 190
   Arg Thr Glu Ala Gln His Cys Gly Glu Pro Gly Lys Ile Phe Leu Asp
   195 200 205
   Ile His Glu Pro Gly Glu His Lys Ile His Phe Ser Met Arg Glu Asp
   210 215 220
   Gly Phe Glu Phe Asp Gln Trp Leu Met Thr Thr Asp Ser Ser Phe Gln
   225 230 235 240
   Arg Pro Pro Ala Gly Lys Ser Asn Lys Pro Lys Glu Asn Ala Thr Thr
   245 250 255
   Gln Val Leu Ser Leu Pro Ala Lys Glu Phe Glu Phe Lys Ser Gly Gly
   260 265 270
   Tyr Tyr Leu Asp Gln Gly Lys Trp Leu Ala Ile Asn Pro Asp Arg Asn
   275 280 285
   Gln Ser Ala Ala Ala Lys Lys Val Phe Pro Phe Pro Ser Gly Arg Tyr
   290 295 300
   Asp Val Thr Leu Lys Ala Val Gly Glu Asn Asp Gly Gln Ser Thr Tyr
   305 310 315 320
   Ser Val Ser Ala Asp Lys Glu Ser Ile Gly Ser Phe Thr Cys Pro Met
   325 330 335
   Ala Asp Gln Thr Phe Ala Glu Gly Lys Gln Phe His Gln Thr Phe Ala
   340 345 350
   Asn Val Gln Ile Thr Glu Gly Ala Glu Leu Glu Val Ala Ser Lys Ile
   355 360 365
   Ala Ser Ala Asp Gly Ala Glu Tyr Ser Arg Ala Arg Trp Ser Glu Leu
   370 375 380
   Thr Phe Thr Pro Ala Asn Glu Ala Thr Ala Lys Ala Ala Ala Asn Phe
   385 390 395 400
   Ala Lys Glu Asn His Leu Val Ala Ala Lys Thr Ser Ala Glu Ser Asn
   405 410 415
   Asp Arg Thr Gly Ser Pro Thr Lys Pro Val Ser Asp Gln Pro Leu Gln
   420 425 430
   Met Pro Arg Glu Lys Asp Gly Asp Gly Ser Val Gln Val Thr Gly Glu
   435 440 445
   Lys Arg Met Trp His Lys Val Thr Val Thr Leu Asn Gly Pro Tyr Ala
   450 455 460
   His Glu Gln Asp Asn Thr Pro Asn Pro Tyr Leu Asp His Arg Met Glu
   465 470 475 480
   Val Glu Phe Lys His Glu Ser Gly Lys Gln Tyr Leu Val Pro Gly Tyr
   485 490 495
   Phe Ala Ala Asp Gly Asn Ala Ala Asn Thr Ser Ala Glu Ser Gly Thr
   500 505 510
   Gln Trp Arg Ala His Phe Ala Pro Asp Glu Thr Gly Glu Trp Thr Tyr
   515 520 525
   Thr Val His Phe Ala Thr Gly Lys Asp Ala Ala Ile Asp Arg Asp Ala
   530 535 540
   Ser Ala Lys Thr Val Ala Ala Phe Asn Gly Lys Thr Gly Thr Phe Asn
   545 550 555 560
   Val Ala Lys Thr Asn Lys Ser Gly Arg Asp Phe Arg Ala His Gly Arg
   565 570 575
   Leu Arg Tyr Val Asn Gln Ser His Leu Gln Phe Ala Gly Thr Gly Gln
               580                 585                 590
   Tyr Phe Leu Lys Ala Gly Ala Asp Ala Pro Glu Thr Leu Leu Gly Tyr
           595                 600                 605
   Ala Glu Phe Asp Gly Thr Val Ala Gly Lys Pro Gly Lys Val Pro Leu
       610                 615                 620
   Lys Lys Tyr Glu Pro His Leu Gly Asp Trp Arg Arg Gly Asp Pro Thr
   625                 630                 635                 640
   Trp Lys Asp Gly Gln Gly Lys Gly Leu Ile Gly Ala Val Asn Tyr Leu
                   645                 650                 655
   Ser Ser Lys Gly Cys Asn Ala Phe Ser Phe Leu Thr Tyr Asn Ala Gly
               660                 665                 670
   Gly Asp Gly Asp Asn Val Trp Pro Phe Ile Gln Arg Asp Asp Lys Leu
           675                 680                 685
   His Tyr Asp Cys Ser Lys Leu Asp Gln Trp Gly Ile Val Phe Asp His
       690                 695                 700
   Gly Thr Glu Asn Gly Met Tyr Leu His Phe Lys Leu Gln Glu Thr Glu
   705                 710                 715                 720
   Asn Asp Asp His Arg Gln Gly Gln Lys Ala Lys Gly Phe Lys Pro Glu
                   725                 730                 735
   Ser Leu Asp Gly Gly Lys Leu Gly Ser Gln Arg Lys Leu Tyr Leu Arg
               740                 745                 750
   Glu Ile Ile Ala Arg Phe Gly His Asn Leu Ala Leu Asn Trp Asn Leu
           755                 760                 765
   Ala Glu Glu Thr Thr Gln Thr Thr Asp Glu His Leu Ala Met Leu Asn
       770                 775                 780
   Tyr Ile Glu Glu Met Asp Pro Tyr Gly His His Arg Val Leu His Thr
   785                 790                 795                 800
   Tyr Pro Gly Glu Gln Asp Lys Lys Tyr Asp Pro Leu Leu Gly Asp Lys
                   805                 810                 815
   Ser Asn Leu Thr Gly Val Ser Leu Gln Asn Ser His Ile Lys Asp Thr
               820                 825                 830
   His Trp Gln Thr Val Lys Trp Ser Glu Lys Ala Arg Glu Ala Gly Lys
           835                 840                 845
   Pro Trp Val Val Ala Phe Asp Glu Ser Gly Ser Ala Ala His Gly Gln
       850                 855                 860
   Cys Pro Asp Leu Gly Tyr Arg Gly Tyr Asp Gly Arg Asp Lys Thr Gly
   865                 870                 875                 880
   Lys Met Thr Tyr Thr Gln His Glu Val Arg Lys Gln Thr Leu Trp Gly
                   885                 890                 895
   Asn Phe Met Gly Gly Gly Gly Gly Val Glu Tyr Tyr Phe Gly Tyr Gln
               900                 905                 910
   Tyr Asp Glu Asn Asp Leu Gly Cys Glu Asp Trp Arg Ser Arg Asp Gln
           915                 920                 925
   Ser Trp Asp Ala Cys Arg Val Ala Ile Glu Phe Phe Gln Asn Asn Ala
       930                 935                 940
   Val Pro Phe Trp Glu Met Val Asn Ala Asp Glu Leu Val Gly Asn Glu
   945                 950                 955                 960
   Lys His Asp Asn Ser Lys Tyr Cys Leu Ala Lys Ala Gly Glu Ala Tyr
                   965                 970                 975
   Val Val Tyr Leu Pro Asn Gly Gly Thr Thr Ser Ile Asp Leu Ser Asp
               980                 985                 990
   Ala Asp Gly Glu Phe Gln Val His Trp Tyr Asn Ala Arg Ile Gly Gly
           995                 1000                1005
   Asp Leu Gln Ser Gly Ser Val Lys Thr Val Ser Gly Gly Gly Ser Val
       1010                1015                1020
   Glu Ile Gly Gln Pro Pro Ala Asp Ala Asp Gln Asp Trp Ala Val Leu
   1025                1030                1035                1040
   Leu Arg Lys
<210> 25
   <211> 1160
   <212> PRT
   <213> Artificial Sequence
<220>
   <223> YP_003195709.1
<400> 25
   Met Ser Pro Arg Asn Leu Leu Leu Ser Leu Thr Leu Phe Val Phe Ala
    1               5                  10                  15
   Thr Ala Gly Leu Arg Ala Gln Gly Gln Val Thr Gly Glu Leu Gln Lys
               20                  25                  30
   Trp His Arg Ile Gln Ile Leu Phe Asp Gly Pro Gln Thr Ser Glu Ser
           35                  40                  45
   Ala Ser Gln Asn Pro Phe Leu Asn Tyr Arg Leu Asn Val Leu Phe Thr
       50                  55                  60
   Ala Pro Asp Gly Arg Glu Phe Thr Val Pro Gly Phe Phe Ala Ala Asp
   65                  70                  75                  80
   Gly Asn Ala Ala Glu Ser Ser Ala Thr Ser Gly Asn Lys Trp Ala Val
                   85                  90                  95
   Arg Phe Ser Pro Asp Gln Val Gly Thr Trp Thr Tyr Thr Ala Ser Phe
               100                 105                 110
   Arg Thr Gly Asp Glu Val Ala Ile Ser Leu Asp Pro Asn Ala Gly Thr
           115                 120                 125
   Ala Thr Gly Phe Asp Gly Ala Ser Gly Ser Phe Gln Ile Gly Leu Ser
       130                 135                 140
   Thr Lys Ser Ala Pro Asp Asn Arg Ser Lys Gly Arg Leu Glu Tyr Val
   145                 150                 155                 160
   Gly Glu Arg Tyr Leu Arg Phe Arg Glu Asn Gly Thr Tyr Phe Leu Lys
                   165                 170                 175
   Ala Gly Ala Asp Ser Pro Glu Asn Leu Leu Ala Tyr Ala Asp Phe Asp
               180                 185                 190
   Asn Thr Val Ala Ser Lys Thr Trp Ser Pro His Leu Gly Asp Trp Gln
           195                 200                 205
   Gln Gly Asp Ala Glu Trp Lys Asn Gly Lys Gly Arg Ala Leu Ile Gly
       210                 215                 220
   Ala Val Asn Tyr Leu Ala Ser Lys Gly Met Asn Ala Phe Ser Phe Leu
   225                 230                 235                 240
   Thr Met Ser Val Ile Gly Asp Gly Lys Asp Val Trp Pro Trp Val Ser
                   245                 250                 255
   Thr Thr His Ser Gly Leu Asp Glu Pro Gly Gly Gln Asp Ala Ala Asn
               260                 265                 270
   Arg Leu Arg Tyr Asp Val Ser Lys Leu Glu Gln Trp Glu Ile Leu Phe
           275                 280                 285
   Gln His Ala Asp Ser Lys Gly Met Phe Leu His Phe Lys Thr Gln Glu
       290                 295                 300
   Glu Glu Asn Asp Arg Leu Leu Asp Gly Gly Glu Leu Gly Val Gln Arg
   305                 310                 315                 320
   Lys Leu Tyr Tyr Arg Glu Leu Val Ala Arg Phe Gly His His Leu Ala
                   325                 330                 335
   Leu Asn Trp Asn Leu Gly Glu Glu Asn Asp Leu Tyr Asp Glu Leu Gly
               340                 345                 350
   Asp Thr Asn Asn Thr Arg Val Arg Ala Tyr Ala Ser Tyr Ile Lys Ser
           355                 360                 365
   Leu Asp Pro Tyr Asn His His Ile Val Ile His Ser Tyr Pro Asn Ser
       370                 375                 380
   Gln Ser Glu Leu Tyr Glu Pro Leu Leu Gly Asp Ser Asp Leu Thr Gly
   385                 390                 395                 400
   Pro Ser Leu Gln Ile Gln Ile Asn Asn Ile His Arg Asp Val Lys Arg
                   405                 410                 415
   Trp Ile Asn Asp Ser Lys Ala Ser Gly Lys Gln Trp Val Val Thr Asn
               420                 425                 430
   Asp Glu Gln Gly Asp His Thr Thr Gly Val Ala Ala Asp Ala Ser Tyr
           435                 440                 445
   Gly Gly Asp Lys Gly Ser Arg Gly Asp Asn Arg Ser Asp Val Arg His
       450                 455                 460
   Lys Thr Leu Trp Gly Thr Leu Met Ala Gly Gly Ala Gly Val Glu Tyr
   465                 470                 475                 480
   Tyr Phe Gly Tyr Gln Thr Gly Val Thr Asp Leu Thr Ala Glu Asp Trp
   485 490 495
   Arg Ser Arg Lys Thr Lys Trp Glu Asp Ala Lys Leu Ala Leu Asp Phe
   500 505 510
   Phe Asn Asp Tyr Leu Pro Phe Trp Ala Met Glu Ser Arg Asp Glu Leu
   515 520 525
   Ile Ser Lys Ser Gly Ser Tyr Cys Phe Ala Lys Thr Gly Glu Ile Tyr
   530 535 540
   Val Val Tyr Ile Pro Ser Ser Gly Thr Glu Ser Leu Asn Leu Ser Gly
   545 550 555 560
   Val Ser Gly Thr Tyr Ser Val Arg Trp Tyr Asn Pro Arg Ser Gly Gly
   565 570 575
   Ser Leu Lys Gln Gly Ser Val Ala Thr Ile Asn Gly Gly Gly Val Arg
   580 585 590
   Asn Leu Gly Thr Ala Pro Thr Asp Thr Gly Ala Asp Trp Val Ala Leu
   595 600 605
   Val Glu Lys Thr Ser Asp Ser Gly Gly Asp Gly Gly Gly Thr Gly Asn
   610 615 620
   Cys Glu Ala Asp Phe Glu Glu Gln Asn Gly Arg Val Ile Ile Glu Ala
   625 630 635 640
   Glu Asn Leu Asp Leu Ala Gln Gly Trp Asn Thr Gly Asn Ser Phe Ala
   645 650 655
   Asp Ala Thr Gly Ser Gly Tyr Ile Val Trp Lys Gly Gly Asn Ser Phe
   660 665 670
   Ser Ser Pro Gly Asn Gly Thr Ile Ser Thr Ser Ile Val Ile His Thr
   675 680 685
   Pro Gly Thr Tyr Arg Phe Glu Trp Arg Asn Lys Val Gly His Gly Thr
   690 695 700
   Asn Ser Thr Glu Ala Asn Asp Ser Trp Val Arg Phe Pro Asp Ala Asp
   705 710 715 720
   Asp Phe Tyr Gly Glu Lys Asn Gly Ser Arg Val Tyr Pro Lys Gly Ser
   725 730 735
   Gly Lys Thr Pro Asn Pro Ala Gly Ala Ser Ala Asp Gly Trp Phe Lys
   740 745 750
   Val Tyr Leu Ser Gly Thr Thr Asp Trp Thr Trp Ser Thr Asn Thr Ser
   755 760 765
   Asp His Asp Ala His Gln Ile Tyr Ala Glu Phe Asp Thr Pro Gly Val
   770 775 780
   Tyr Thr Leu Gln Ile Ser Gly Arg Ser Asn Asp His Leu Ile Asp Arg
   785 790 795 800
   Ile Thr Leu Ala Leu Ala Gly Gln Ser Ala Thr Asp Leu Ser Leu Gly
   805 810 815
   Glu Thr Leu Cys Glu Gly Gly Ser Glu Thr Val Ala Val Thr Gly Val
   820 825 830
   Thr Val Thr Pro Gly Asp Ala Thr Leu Leu Ile Gly Glu Thr Leu Gln
   835 840 845
   Phe Thr Ala Ala Val Leu Pro Ala Asp Ala Thr Asn Lys Ser Val Ser
   850 855 860
   Trp Ser Ser Ser Asp Pro Ser Val Ala Ile Val Ser Gly Asn Gly Thr
   865 870 875 880
   Val Gln Ala Leu Ser Glu Gly Gln Val Glu Ile Thr Ala Thr Thr Ala
   885 890 895
   Asp Gly Asn Phe Thr His Ser Ala Leu Leu Thr Val Glu Ala Ala Asp
   900 905 910
   Pro Pro Gly Gly Asp Thr Gly Asp Gly Gly Ser Gly Glu Asp Pro Gly
   915 920 925
   Asn Asp Gly Gly Ser Gly Glu Asp Pro Gly Asn Asp Gly Gly Ser Gly
   930 935 940
   Glu Asp Pro Gly Asn Asp Gly Gly Ser Gly Glu Asp Pro Asp Gly Asp
   945 950 955 960
   Gly Ser Gly Glu Glu Pro Gly Asp Gly Ala Ala Gln Ala Ala Ile Gln
   965 970 975
   Ala Val Asp Val Arg Gln Thr Glu Gly Arg Pro Leu Ile Phe Glu Phe
   980 985 990
   Ala Leu Ser Gln Pro Val Ser Glu Arg Ile Val Leu Glu Leu Glu Phe
           995                 1000                1005
   Val Asp Ile Thr Thr Glu Gln Ser Asp Tyr Val Val Ser Glu Thr Glu
       1010                1015                1020
   Leu Val Phe Glu Pro Gly Ser Gln Gln Ala Phe Leu Glu Val Arg Thr
   1025                1030                1035                1040
   Ile Ser Asp Leu Lys Thr Glu Glu Asp Glu Ser Phe Gln Ile Lys Val
                   1045                1050                1055
   Val Arg Val Val Ser Gly Gln Val Thr Val Pro Asp Ile Leu Ala Thr
               1060                1065                1070
   Gly Thr Ile Leu Asp Asp Asp Arg Asp Met Lys Val Ser Pro Asn Pro
           1075                1080                1085
   Ala Thr Ser Tyr Ser Leu Val Gln Met Ser Asn Val Gln Glu Gly Thr
       1090                1095                1100
   Tyr Glu Leu Glu Ile Phe Ala Ala Ser Gly His Leu Met Gln Arg Glu
   1105                1110                1115                1120
   Thr Val Thr Ala Asp Gly Ser Gly Ile Ala Ser Val Thr Leu Ala Gly
                   1125                1130                1135
   Met Ala Lys Gly Leu Tyr Ile Val Lys Leu Thr Gly Ile Asp Tyr Ala
               1140                1145                1150
   Tyr Thr Ala Lys Met Leu Val Lys
           1155                1160
<210> 26
   <211> 528
   <212> PRT
   <213> Artificial Sequence
<220>
   <223> YP_003323724.1
<400> 26
   Met Ser Ile Arg Ser Leu Pro Arg Arg Thr Val Gly Glu Trp Glu Val
   1               5                   10                  15
   Thr Ser Thr Arg Glu Tyr Glu Asn Pro Phe Val Asp Val Glu Val Ile
               20                  25                  30
   Gly Arg Phe Ile Ser Pro Ser Gly Arg Glu Trp Arg Val Pro Gly Phe
           35                  40                  45
   Tyr Asp Gly Asp Gly Val Trp Lys Val Arg Phe Asn Pro Gly Glu Glu
       50                  55                  60
   Gly Arg Trp Ala Tyr Arg Leu Glu Ser Tyr Pro Glu Asp Pro Glu Leu
   65                  70                  75                  80
   Arg Ala Glu Gly Thr Phe Glu Val Leu Pro Arg Glu Ala Arg Gly Phe
                   85                  90                  95
   Leu Arg Ser Val Pro Gly Gln Ala Trp Gly Phe Ile Tyr Glu Ser Gly
               100                 105                 110
   Glu Pro Val Phe Ile Leu Gly Asp Thr Val Tyr Asn Leu Phe Gly Met
           115                 120                 125
   Ala His Cys Gly Ala Asp Val Glu Ala Phe Leu Glu Arg Arg Ala Ser
       130                 135                 140
   Gln Gly Phe Asn Leu Leu Arg Val Arg Val Pro Val Ser Pro Phe His
   145                 150                 155                 160
   Pro Pro Lys Gly Tyr Ser Glu Trp Gln Thr Arg Arg Thr Trp Pro Trp
                   165                 170                 175
   Glu Gly Ser Glu Gln Ala Pro Val Phe Asp Arg Phe Asn Leu Glu Tyr
               180                 185                 190
   Phe Ala Thr Val Asp Arg Val Val Arg Lys Val Glu Glu Leu Gly Leu
           195                 200                 205
   Gly Leu Glu Val Ile Met Glu Ala Trp Gly Phe Glu Phe Pro Phe Asn
       210                 215                 220
   Ser Arg His Ile Phe Val Ala Glu Trp Glu Glu Leu Trp Met Arg Tyr
   225                 230                 235                 240
   Leu Val Ala Arg Tyr Asp Ala Tyr Ser Cys Val Tyr Phe Trp Thr Pro
                   245                 250                 255
   Met Asn Glu Tyr Glu Phe Tyr Pro Asn Gly Asp Trp His Tyr Lys Pro
                       260                     265                     270
   Thr Ala Asp Arg Trp Ala Ile Arg Ile Ala Arg Trp Leu Arg Ala Asn
           275                     280                     285
   Ala Pro His Gly His Ile Val Ser Leu His Asn Gly Pro Trp Asp Pro
       290                     295                     300
   Pro Phe Ala His Arg Phe Arg Ser Asp Pro Lys Ala Ile Asp Thr Ile
   305                     310                     315                     320
   Met Phe Gln Phe Trp Gly Thr Thr Gly Arg Asp Asp Ala Trp Leu Ala
                   325                     330                     335
   Ala Gly Ile Glu Asp Arg Ile Ala Tyr Ser Leu Gly Gly Trp Tyr Gly
               340                     345                     350
   Thr Ala Val Phe Ala Glu Tyr Gly Tyr Glu Arg Asn Pro Ala Leu Pro
           355                     360                     365
   Leu Asn Ile Pro Gly His Glu Phe Cys Asp Pro Glu His Thr Arg Arg
       370                     375                     380
   Gly Ala Trp Arg Gly Ala Phe Cys Gly Leu Gly Val Ile His Gly Phe
   385                     390                     395                     400
   Glu Asn Ser Trp Gly Pro Phe Met Val Leu Glu Glu Asp Gln Pro Gly
                   405                     410                     415
   Leu Glu Tyr Leu Leu His Leu Arg Arg Phe Phe Thr Glu Val Val Pro
               420                     425                     430
   Phe His Arg Leu Leu Pro Asp Ala Ser Leu Val Val Ser Asp Ile Ser
           435                     440                     445
   Glu Gln Gly Gly Lys Pro Leu Ala Leu Ser Ser Pro Glu Arg Asp Val
       450                     455                     460
   Leu Ala Val Tyr Leu Pro Arg Gly Gly Glu Phe Lys Leu Ser Val Asn
   465                     470                     475                     480
   Pro Pro Ala Asp Pro Cys Trp Tyr Asp Pro Arg Thr Gly Glu Val Leu
                   485                     490                     495
   Ala Ala Glu Ala Ser Pro Ser Gly Gly Trp Val Ala Pro Gln Ser Gly
               500                     505                     510
   Pro Ala Asp Arg Pro His Asp Trp Val Trp Phe Ser Thr Ser Gly Arg
           515                     520                     525
<210> 27
   <211> 529
   <212> PRT
   <213> Artificial Sequence
<220>
   <223> ZP_02918195.1
<400> 27
   Met Ala Glu Tyr Lys Thr Gln Val Glu Gln His Arg Leu Phe Glu Ile
    1               5                  10                  15
   Asn Leu Thr Gly Thr Thr Glu Gly Asn Pro Tyr Gln Asp Val Thr Leu
               20                  25                  30
   Ser Ala Asp Phe Thr Asn Ala Glu Thr Gly Gln Ile Val Val Val Gly
           35                  40                  45
   Gly Phe Tyr Arg Gly Asn Gly Asn Tyr Ser Val Arg Phe Met Ala Ser
       50                  55                  60
   Ser Ala Gly Arg Trp Ala Phe Thr Thr Arg Ser Thr Asp Pro Ala Leu
   65                  70                  75                  80
   Asp Gly Gln Thr Gly Val Phe Thr Val Thr Pro Ala Thr Gln Asp Asn
                   85                  90                  95
   His Gly Arg Val Leu Thr Ala Thr Glu Ala Leu Ser Gly Lys Ala Arg
               100                 105                 110
   Glu Ala Tyr Gly Ser Glu Leu Lys Tyr Arg Phe Thr Tyr Glu Asp Gly
           115                 120                 125
   Thr Pro Tyr Gln Pro Tyr Gly Thr Thr Cys Tyr Ala Trp Val Ser Gln
       130                 135                 140
   Pro Thr Asp Val Gln Asp Arg Thr Val Ala Thr Leu Ala Lys Ala Pro
   145                 150                 155                 160
   Phe Asn Lys Ile Arg Met Cys Val Phe Pro Lys Phe Tyr Asp Phe Asn
                   165                 170                 175
   Thr Ala Asp Pro Trp Ala Tyr Ala Tyr Pro Gly Thr Arg Glu Thr Gly
               180                 185                 190
   Phe Asp His Thr Cys Phe Asp Glu Glu Phe Phe Ala Asn Leu Asp Cys
           195                 200                 205
   Arg Ile Ala Gln Leu Asp Glu Leu Gly Ile Glu Ala Asp Ile Ile Leu
       210                 215                 220
   Leu His Pro Tyr Asp Lys Pro Asp Trp Gly Phe Ser Lys Met Thr Lys
   225                 230                 235                 240
   Val Glu Asp Glu Met Tyr Leu Ala Tyr Met Ala Arg Arg Tyr Gly Ala
                   245                 250                 255
   Tyr Lys Asn Val Trp Trp Ser Leu Ala Asn Glu Tyr Asp Leu Met Pro
               260                 265                 270
   Gln Lys Ser Leu Glu Asp Trp Arg Glu Tyr Ala Arg Val Val Met Ala
           275                 280                 285
   Asn Asp Ala Phe Gly His Leu Arg Ser Ile His Asn Cys Ile Pro Ile
       290                 295                 300
   Tyr Asp Tyr Asn Glu Pro Trp Cys Thr His Cys Ser Ile Gln Arg Val
   305                 310                 315                 320
   Asp Val Thr Arg Thr Thr Glu Cys Ile Ala Asp Trp Arg Lys Ala Tyr
                   325                 330                 335
   Gly Lys Pro Val Val Cys Asp Glu Pro Gly Tyr Glu Gly Asn Ile Tyr
               340                 345                 350
   Trp Gly Trp Gly Asn Leu Thr Gly Glu Glu Leu Met Arg Arg Phe Trp
           355                 360                 365
   Glu Gly Ala Met Arg Gly Gly Tyr Val Thr His Gly Glu Thr Phe Ile
       370                 375                 380
   Asp Glu Asp Glu Gln Ile Trp Trp Ala His Gly Gly Glu Leu His Gly
   385                 390                 395                 400
   Asp Ala Pro Ala Arg Ile Ala Phe Met Arg Ser Ile Phe Asp Asp Val
                   405                 410                 415
   Pro Leu Asp Ala Thr Pro Leu Asp Gly Asp Leu Asp Ala Asp Arg Ala
               420                 425                 430
   Trp Pro Ile Ala Asn Pro Ile Asn Gly Ser Val Asn Val Ala Pro Ala
           435                 440                 445
   Val Glu Lys Ala Ala Gln Tyr Trp Asp Val Pro Val Leu Arg Gly Gly
       450                 455                 460
   Asp Asp Tyr Gln Leu Ile Tyr Phe Gly Trp Phe Arg Pro Lys Tyr Arg
   465                 470                 475                 480
   Glu Ile Pro Leu Pro Ala Gly Ser Ala Tyr Val Val Asp Val Ile Asp
                   485                 490                 495
   Thr Trp Asn Met Thr Val Glu Thr Leu Pro Asp Ala Tyr Glu Asn Ser
               500                 505                 510
   Val Arg Val Asp Leu Gly Arg Gln Tyr Met Ala Val Arg Ile Arg Lys
           515                 520                 525
   Ala
<210> 28
   <211> 536
   <212> PRT
   <213> Artificial Sequence
<220>
   <223> YP_001819159.1
<400> 28
   Met Asn Leu Arg Thr Phe Glu Val Ala Pro Trp Leu Ala Leu Leu Leu
    1               5                  10                  15
   Ala Leu Ala Ala Pro Met Glu Gly Gly Thr Val Glu Glu Ser Pro Ala
               20                  25                  30
   Val Glu Arg Trp Asn Thr Phe Glu Leu Thr Leu His Gly Pro Ala Thr
           35                  40                  45
   Gly Asn Pro Phe Val Asp Val Arg Leu Gly Ala Arg Phe Thr Asp Gly
   50 55 60
   Thr Arg Thr Ile Glu Val Pro Gly Phe Tyr Asp Gly Asp Gly Val Tyr
   65 70 75 80
   Arg Val Arg Phe Met Pro Asp Ala Thr Gly Ala Trp His Tyr Glu Thr
   85 90 95
   Ser Ser Asn Asp Pro Leu Leu Ala Gly Gln Arg Gly Glu Phe Thr Val
   100 105 110
   Thr Pro Ala Arg Arg Gly Asn His Gly Pro Val Arg Val Arg Asn Thr
   115 120 125
   Tyr His Phe Ala Tyr Ala Asp Gly Thr Pro Phe Lys Pro Ile Gly Thr
   130 135 140
   Thr Ile Tyr Asn Trp Thr Asp Ala Pro Glu Glu Val Gln Glu Gln Thr
   145 150 155 160
   Leu Arg Thr Leu Ala Thr Ala Pro Phe Asn Lys Ala Arg Met Leu Leu
   165 170 175
   Thr Pro Gln Ser Val Ala Tyr Arg Arg Gln Phe Ala Pro Pro Arg Trp
   180 185 190
   Pro Phe Ala Gly Gln Pro Pro Arg Asp Trp Asp Phe Ala Arg Phe Asn
   195 200 205
   Pro Glu Tyr Phe Arg Ala Phe Glu Lys Arg Val Ala Gln Leu Arg Glu
   210 215 220
   Leu Gly Ile Glu Ala Asp Leu Ile Leu Phe His Pro Tyr Gly Lys Ala
   225 230 235 240
   Trp Gly Phe Glu Ala Met Asp Ala Ala Ala Asp Glu Arg Tyr Val Arg
   245 250 255
   Tyr Val Val Ala Arg Phe Ala Ala Phe Arg Asn Val Trp Trp Ser Leu
   260 265 270
   Ala Asn Glu Tyr Asp Leu Val Arg Thr Lys Thr Glu Glu His Trp Asp
   275 280 285
   Gln Leu Gly Arg Leu Val Gln Ala Thr Asp Pro Phe Asp His Leu Arg
   290 295 300
   Ser Ile His His Ser Gln Leu Leu Phe Asp Asn Arg Gln Pro Trp Val
   305 310 315 320
   Thr His Ala Ser Ile Gln Asn Gly Ser Ala Val Glu Glu Pro Gly Arg
   325 330 335
   Ala Glu Leu Tyr Arg Asp Val Trp Arg Lys Pro Val Val Tyr Asp Glu
   340 345 350
   Val Lys Tyr Glu Gly Asn Ala Arg Arg Arg Trp Gly Gln Leu Ser Gly
   355 360 365
   Pro Glu Met Val His Arg Phe Trp Cys Gly Thr Val Ala Gly Thr Tyr
   370 375 380
   Val Gly His Gly Asp Phe Phe Gly Val Glu Asp Ala Pro Asp Thr Trp
   385 390 395 400
   Thr Ser Phe Gly Gly Val Leu Arg Gly Glu Ser Ala Pro Arg Leu Ala
   405 410 415
   Phe Leu Arg Thr Ile Leu Glu Ser Gly Pro Ala Asp Gly Leu Asp Pro
   420 425 430
   Val Asp Lys Trp Gln Asn Val Thr Val Ala Gly Val Pro Gly Glu Tyr
   435 440 445
   Tyr Leu Val Tyr Leu Gly Arg Glu Ser Ala Thr Ala Trp Pro Phe Arg
   450 455 460
   Leu Phe Arg Ser Gly Leu Lys Asp Gly Met Arg Phe Lys Val Glu Val
   465 470 475 480
   Ile Asp Thr Trp Ala Met Thr Ile Thr Pro Val Glu Arg Glu Phe Val
   485 490 495
   Ile Thr Lys Ser Asp Asp Tyr Thr Phe Val Ala Val Asp Gly Gly Ser
   500 505 510
   Ile Ala Leu Pro Gly Lys Pro Gly Ile Ala Leu Arg Ile Gln Arg Thr
   515 520 525
   Ala Pro Ala Lys Phe Leu Lys Glu
   530 535
<210> 29
   <211> 534
   <212> PRT
   <213> Artificial Sequence
<220>
   <223> ZP_06742086.1
<400> 29
   Met Ser Thr Lys Phe Tyr Val Ile Ala Val Trp Thr Leu Leu Ser Phe
   1               5                   10                  15
   Val Val Lys Val Asn Ala Gln Asp Lys Val Glu Cys Trp Asp Arg Phe
               20                  25                  30
   Glu Leu Ser Phe Lys Gln Val Thr Lys Gly Asn Pro Phe Asp Ile Arg
           35                  40                  45
   Leu Ser Ala Thr Phe Val Cys Gly Lys Glu Lys Lys Thr Val Glu Gly
       50                  55                  60
   Phe Tyr Asp Gly Glu Asn Thr Tyr Arg Ile Arg Phe Met Pro Ala Val
   65                  70                  75                  80
   Ala Gly Glu Trp Arg Tyr Val Thr Ser Ser Ser Ile Gly Ala Met Asn
                   85                  90                  95
   Gly Arg Lys Gly Thr Phe Thr Val Ile Pro Ala Gly Lys Asp Asn His
               100                 105                 110
   Gly Met Val Leu Val Asp Gly Glu His Asn Phe Lys Tyr Ala Asp Gly
           115                 120                 125
   Thr Arg Tyr Tyr Pro Met Gly Thr Thr Ala Tyr Ala Trp Thr His Met
       130                 135                 140
   Lys Glu Thr Thr Gln Glu Ala Thr Leu Lys Ser Phe Gly Glu Ala Gly
   145                 150                 155                 160
   Phe Asn Lys Val Arg Met Cys Val Phe Pro Lys Asn Tyr Ser Leu Val
                   165                 170                 175
   Lys Asp Glu Pro Ala Leu Tyr Pro Phe Glu Ile Arg Lys Thr Ile Lys
               180                 185                 190
   Asp Lys Glu Gly Asn Glu Arg Lys Glu Trp Asp Phe Asp Arg Phe Asp
           195                 200                 205
   Pro Ala Phe Phe Gln His Leu Glu Lys Arg Ile Asp Gln Leu Asn Arg
       210                 215                 220
   Leu Gly Ile Glu Ala Asp Leu Ile Leu Phe His Pro Tyr Asp Lys Gly
   225                 230                 235                 240
   Arg Trp Gly Phe Asp Ala Met Ser Asn Glu Val Asn Val Arg Tyr Ile
                   245                 250                 255
   Lys Tyr Ile Thr Ala Arg Leu Ala Ser Phe Arg Asn Val Trp Trp Ser
               260                 265                 270
   Met Ala Asn Glu Trp Asp Tyr Val Lys Ala Lys Thr Val Asp Asp Trp
           275                 280                 285
   Lys Leu Leu Thr Lys Thr Val Val Glu Asn Asp Pro Tyr Arg His Leu
       290                 295                 300
   Cys Ser Ile His Gly Ala Thr Ala Thr Tyr Phe Asp Tyr Trp Met Pro
   305                 310                 315                 320
   Glu Phe Thr His Val Ser Ile Gln Asp Glu Ala Pro Val Leu Ser Ser
                   325                 330                 335
   Thr Ala Ser Ala Thr Leu Arg Lys Ile Tyr Arg Lys Pro Val Ile Cys
               340                 345                 350
   Asp Glu Val Gly Tyr Glu Gly Asn Leu Pro Tyr Arg Trp Gly Arg Leu
           355                 360                 365
   Ser Pro Gln Gln Met Thr Cys Phe Ile Leu Asn Gly Leu Leu Gly Gly
       370                 375                 380
   Ile Tyr Val Thr His Gly Glu Cys Tyr Gln Gln Gly Asn Glu Pro Ile
   385                 390                 395                 400
   Phe Trp Ala Gln Gly Gly Ser Leu Lys Gly Glu Ser Trp Lys Arg Val
                   405                 410                 415
   Lys Phe Leu Arg Thr Ile Ile Glu Ala Ala Pro His Pro Leu Glu Met
               420                 425                 430
   Ala Asp Ile Ser Arg Asp Leu Val Thr Ser Thr Ala Gly Pro Asp Tyr
           435                 440                 445
   Tyr Leu Val Asn Met Gly Lys Asp Val Lys Gly Phe Trp Thr Phe Asn
       450                 455                 460
   Leu Pro Val Lys Asn Ala Asp Tyr Asn Lys Leu Gln Lys Asn Lys Arg
   465                 470                 475                 480
   Phe Lys Val Glu Ile Ile Asp Val Trp Ala Met Thr Val Thr Glu Tyr
                   485                 490                 495
   Pro Val Ile Phe Glu Thr Thr Glu Glu Leu Asp Tyr Arg Val Phe Asp
               500                 505                 510
   Ile His His Arg Gly Val Arg Ile Pro Asp Ala Pro Tyr Ile Val Leu
           515                 520                 525
   Arg Ile Thr Glu Val Lys
       530
<210> 30
   <211> 534
   <212> PRT
   <213> Artificial Sequence
<220>
   <223> YP_003387974.1
<400> 30
   Met Asn Tyr Arg Leu Phe Ser Leu Leu Val Leu Gly Leu Leu Phe Ser
   1               5                   10                  15
   Lys Ser Leu Ile Ala Gln Glu Val Glu Gln Trp Gly Arg Phe Glu Lys
               20                  25                  30
   Thr Phe Thr Ala Lys Ala Thr Gly Asn Pro Phe Thr Glu Val Thr Leu
           35                  40                  45
   Thr Ala Glu Leu Ser Asn Gly Tyr Thr Thr Tyr Ser Val Ala Gly Phe
       50                  55                  60
   Tyr Asp Gly Lys Asp Thr Phe Lys Ile Arg Phe Met Pro Pro Lys Thr
   65                  70                  75                  80
   Gly Lys Trp Arg Tyr Lys Thr Ser Ser Asn Val Ala Gly Leu Thr Asn
                   85                  90                  95
   Lys Lys Gly Glu Phe Thr Cys Val Ala Ala Ala Arg Gly Asn Ser Gly
               100                 105                 110
   Pro Val Arg Val Ser Asp Met Tyr Ala Phe Lys Tyr Ala Asp Gly Thr
           115                 120                 125
   Asn Tyr Tyr Pro Phe Gly Thr Thr Ala Tyr Ala Trp Thr His Met Gly
       130                 135                 140
   Glu Glu Thr Gln Glu Ile Thr Leu Asn Thr Leu Lys Asn Ser Gly Phe
   145                 150                 155                 160
   Asn Lys Leu Arg Met Cys Val Phe Pro Lys Asn Tyr Glu Leu Val Lys
                   165                 170                 175
   Glu Ala Pro Asp Leu Phe Pro Phe Glu Ile Lys Glu Gln Lys Lys Asp
               180                 185                 190
   Ala Asp Gly Lys Pro Tyr Thr Val Trp Asn Phe Asp Lys Phe Asn Pro
           195                 200                 205
   Ala Phe Phe Gln His Leu Glu Lys Arg Ile Asp Asp Leu Asn Lys Leu
       210                 215                 220
   Gly Ile Glu Ala Asp Leu Ile Leu Phe His Pro Tyr Asp Lys Gly Arg
   225                 230                 235                 240
   Trp Gly Phe Asp Ala Met Pro Asn Glu Val Asn Ile Arg Tyr Leu Lys
                   245                 250                 255
   Tyr Leu Leu Ala Arg Leu Ser Ser Tyr Lys Asn Val Trp Trp Ser Leu
               260                 265                 270
   Ala Asn Glu Trp Asp Tyr Val Lys Ser Lys Thr Val Ser Asp Trp Asp
           275                 280                 285
   Leu Leu Ala Lys Thr Val Ala Gln Asn Asp Pro Tyr Lys His Leu Cys
       290                 295                 300
   Ser Ile His Gly Ala Thr Ala Thr Tyr Tyr Asp Tyr Arg Lys Pro Glu
   305                 310                 315                 320
   Phe Thr His Val Ser Ile Gln Asp Glu Thr Pro Val Gln Ser Pro Asn
                   325                 330                 335
   Ala Ala Ala Met Leu Arg His Ile Tyr His Lys Pro Val Ile Ala Asp
               340                 345                 350
   Glu Val Gly Tyr Glu Gly Asn Leu Lys Ser Arg Trp Gly Arg Tyr Ser
   355 360 365
   Pro Glu Glu Met Thr Tyr Leu Val Trp Asn Gly Val Leu Gly Gly Thr
   370 375 380
   Tyr Val Thr His Gly Glu Cys Tyr Met Ala Ala Pro Thr Asp Thr Ile
   385 390 395 400
   Phe Trp Ala Lys Gly Gly Met Phe Lys Gly Thr Ser Trp Lys Arg Ile
   405 410 415
   Ala Phe Leu Arg Gln Ile Val Glu Ala Phe Pro Asn Pro Leu Ser Leu
   420 425 430
   Ser Asp Val Ser Arg Asp Asn Val Thr Ala Thr Ala Gly Asp Gly Gln
   435 440 445
   Tyr Leu Val Tyr Phe Gly Lys Gln Thr Asn Asp Ser Trp Leu Phe Asn
   450 455 460
   Leu Pro Ala Lys Asn Ser Ser Phe Gln Lys Leu Thr Ala Gly Gln Arg
   465 470 475 480
   Phe Lys Val Glu Val Ile Asp Thr Trp Asp Met Thr Ile Gln Pro Asp
   485 490 495
   Pro Gln Ile Phe Glu Thr Ala Ser Glu Asn Asp Tyr Arg Leu Tyr Asp
   500 505 510
   Lys Thr Leu Lys Lys Val Arg Leu Pro Leu Lys Pro Tyr Val Ala Leu
   515 520 525
   Arg Ile Thr Glu Ile Lys
   530
<210> 31
   <211> 481
   <212> PRT
   <213> Artificial Sequence
<220>
   <223> YP_003243090.1
<400> 31
   Met Glu Gly Ser Ala Ser Ala Leu Met Gly Met Gln Glu Val Glu Arg
    1               5                  10                  15
   Trp Gly Ile Phe Glu Val Ala Leu Ser Gly Pro Ser Ala Gly Asn Pro
               20                  25                  30
   Phe Glu Glu Val Ala Val Ser Ala Thr Phe Glu Tyr Arg Asn Asp Arg
           35                  40                  45
   Val Glu Val Glu Gly Phe Tyr Asp Gly Asp Gly Ile Tyr Arg Val Arg
       50                  55                  60
   Phe Met Pro Asp Arg Glu Gly Thr Trp Arg Tyr Val Thr Lys Ser Gly
   65                  70                  75                  80
   Thr Ser Glu Leu Asp Gly Ala Thr Gly Ser Phe Leu Cys Thr Ser Pro
                   85                  90                  95
   Ser Lys Gly Asn His Gly Pro Val Arg Val Lys Asp Gly Tyr Leu Phe
               100                 105                 110
   Ala Phe Glu Asp Gly Thr Pro Tyr Met Pro Phe Gly Thr Thr Cys Tyr
           115                 120                 125
   His Trp Thr His Thr Gly Ser Lys Glu His Glu Leu Gln Thr Leu Glu
       130                 135                 140
   Glu Leu Gly Gln Ser Pro Phe Asn Lys Val Arg Met Cys Leu Leu Pro
   145                 150                 155                 160
   Thr Arg Asp Met Arg Pro Glu Met Val Ala Phe Ala Gly Thr Cys Pro
                   165                 170                 175
   Glu Asp Ala Asp Thr Thr Arg Phe Asn Pro Ala Phe Phe Ala His Leu
               180                 185                 190
   Glu Glu Arg Ile Gly Asp Leu Met Lys Leu Gly Val Glu Ala Asp Leu
           195                 200                 205
   Ile Leu Phe His Pro Tyr Asp Lys Gln Gly Gly Trp Gly Phe Asn Ser
       210                 215                 220
   Met Ser Arg Glu Gln Asp Phe Tyr Tyr Leu Arg Tyr Val Ile Ala Arg
   225                 230                 235                 240
   Leu Gly Ala Tyr Arg Asn Val Trp Trp Ser Leu Ser Asn Glu Tyr Asp
                   245                 250                 255
   Phe Asn Lys Met Lys Thr Ile Glu Asp Trp Asp Arg Leu Leu Gln Tyr
               260                 265                 270
   Val Gln Arg Lys Asp Pro Tyr Gln Arg Leu Arg Ser Ile His Asn Gly
           275                 280                 285
   Thr Lys Met Tyr Glu Gln Ser Ser Leu Tyr Asp Phe Ser Lys Ser Trp
       290                 295                 300
   Leu Thr His Gln Ser Ile Gln His Trp Asp Thr Ser Pro Thr Thr Glu
   305                 310                 315                 320
   Trp Arg Glu Ala Val Arg Lys Pro Ile Val Val Asp Glu Ile Ser Tyr
                   325                 330                 335
   Glu Gly Asn Val Ala Lys Arg Trp Gly Asn Ile Thr Gly Leu Glu Leu
               340                 345                 350
   Ile His Arg Tyr Trp Glu Gly Leu Thr Lys Gly Gly Phe Val Ala His
           355                 360                 365
   Gly Glu Cys Phe Glu Asn Lys Pro Thr Arg Ala Trp Ile Ser Ser Gly
       370                 375                 380
   Gly Lys Met Tyr Gly Glu Ser Pro Glu Arg Ile Arg Phe Leu Arg Gln
   385                 390                 395                 400
   Val Met Glu Glu Gly Pro Glu Asp Trp Met Gln Ala Arg Glu Glu Gly
                   405                 410                 415
   Gly Tyr Ala Leu Leu Tyr Leu Gly Lys Tyr Arg Leu Ser Ser Tyr Lys
               420                 425                 430
   Leu Pro Leu Ser Gln Asp Arg Glu Tyr Arg Ile Glu Leu Ile Asp Ile
           435                 440                 445
   Trp Lys Met Thr Ile Thr Pro Leu Asp Gly Thr Phe Thr Gly Gln Ser
       450                 455                 460
   Asp Leu Ala Leu Pro Ala Arg Pro Tyr Ile Ala Leu Arg Ala Gln Ala
   465                 470                 475                 480
   Val
<210> 32
   <211> 509
   <212> PRT
   <213> Artificial Sequence
<220>
   <223> NP_228758.1
<400> 32
   Met Ile Ser Met Thr Gln Asn Asn Val Glu Lys Trp Gly Thr Tyr Glu
    1              5                   10                  15
   Ile Thr Leu Lys Gly Lys Ala Glu Gly Asn Pro Phe Thr Asp Val Glu
               20                  25                  30
   Ile Lys Ala Val Phe Thr Ser Glu Ala Gly Asp Ser Phe Glu Val Glu
           35                  40                  45
   Gly Phe Tyr Asp Gly Glu Asp Thr Phe Lys Ile Arg Phe Met Pro Asn
       50                  55                  60
   Arg Thr Gly Leu Trp Lys Tyr Glu Val Arg Ser Asp Ile Lys Asp Leu
   65                  70                  75                  80
   Asp Gly Met Lys Gly Glu Phe Leu Cys Val Asp Pro Ser Glu Gly Asn
                   85                  90                  95
   His Gly Pro Val Arg Val Cys Asn Thr Tyr His Phe Cys Tyr Glu Asp
               100                 105                 110
   Gly Thr Pro Tyr His Pro Phe Gly Thr Thr Leu Tyr Ala Trp Val His
           115                 120                 125
   Gln Arg Glu Glu Leu Ile Gln Gln Thr Leu Glu Ser Leu Arg Asn Ser
       130                 135                 140
   Pro Phe Asn Lys Val Arg Met Cys Val Phe Pro Lys Tyr Tyr Ala Tyr
   145                 150                 155                 160
   Asn Arg Glu Glu Pro Pro Met Leu Pro Met Ser Trp Arg Glu Glu Asp
                   165                 170                 175
   Gly Arg Tyr Gln Val Glu Phe Asn Val Lys Phe Phe Gln His Phe Glu
               180                 185                 190
   Arg Leu Val Lys Glu Leu Met Asp Met Gly Ile Glu Ala Asp Val Ile
           195                 200                 205
   Leu Phe His Pro Tyr Asp Lys Trp Gly Phe Cys Ser Met Pro Glu Glu
       210                 215                 220
   Val Asp Lys Ala Tyr Leu Arg Tyr Leu Ile Ala Arg Ile Ser Ser Tyr
   225                 230                 235                 240
   Arg Asn Val Trp Trp Ser Met Ala Asn Glu Tyr Asp Leu Ile Lys Pro
                   245                 250                 255
   Gln Lys Asp Trp Asp Gly Tyr Phe His Phe Ile Val Glu Lys Asp Pro
               260                 265                 270
   Tyr Asn His Leu Arg Ser Val His Gln Cys Phe Lys Phe Tyr Asp His
           275                 280                 285
   Thr Lys Pro Trp Ile Thr His Ala Ser Ile Gln Trp Gln Gly Ser Leu
       290                 295                 300
   Arg Ser Trp Ser Gly Glu Pro Glu Ile Asp Ile Gly Ile Asn Leu Ile
   305                 310                 315                 320
   Pro Lys Trp Arg Glu Met Tyr Lys Lys Pro Val Ile Ile Asp Glu Cys
                   325                 330                 335
   Gly Tyr Glu Gly Asn Ile Glu Tyr Gly Trp Gly Asn Leu Pro Pro Gln
               340                 345                 350
   Glu Met Met Asn Arg Phe Trp Glu Gly Val Thr Ser Gly Gly Tyr Val
           355                 360                 365
   Thr His Gly Glu Thr Tyr Tyr Ser Glu Asp Glu Val Leu Trp Trp Ser
       370                 375                 380
   Lys Gly Gly Arg Leu Lys Gly Glu Ser Pro Lys Arg Ile Ala Phe Leu
   385                 390                 395                 400
   Arg Thr Ile Ile Glu Glu Ala Pro Pro Phe Leu Lys Pro Ile Lys Leu
                   405                 410                 415
   Asp Pro Met Met Asp Trp Asp Val His Cys Ile Gly Lys Glu Gly Glu
               420                 425                 430
   Tyr Tyr Leu Ile Tyr Phe Asp Ile Asn Arg Pro Val Lys Arg Thr Leu
           435                 440                 445
   Lys Leu Pro Glu Gly Lys Tyr Arg Val Asp Leu Val Asp Cys Trp Glu
       450                 455                 460
   Thr Glu Ile His Ser Leu Gly Ile Phe Gln Gly Gln Val Val Ile Arg
   465                 470                 475                 480
   Leu Pro Gly Lys Ser Tyr Val Ala Leu Arg Ile Gln Lys Met Glu Asn
                   485                 490                 495
   Asn Asp Asp Trp Ile Ile Leu Arg Glu Asp Ala Lys Leu
               500                 505
<210> 33
   <211> 725
   <212> PRT
   <213> Artificial Sequence
<220>
   <223> ZP_03628309.1
<400> 33
   Met Ala Tyr Leu Lys Met Pro Ile Ala Leu Leu Ala Leu Phe Ile Cys
    1               5                  10                  15
   Leu Glu Gly Thr Ala Phe Ala Leu Asn Ala Asp Gly Pro Gly Ser Ala
               20                  25                  30
   Leu Ala Asn Gln Met Val Glu Trp Thr Phe Thr Ser Thr Arg Pro Tyr
           35                  40                  45
   Lys Asp Pro Phe Asn Gln Ile Glu Leu Asn Val Thr Phe Thr Thr Pro
       50                  55                  60
   Ser Gly Lys Lys Leu Leu Val Pro Ala Phe Trp Asp Gly Gly Asp Ile
   65                  70                  75                  80
   Trp His Val Arg Tyr Ser Ser Thr Glu Thr Gly Ile His His Phe Ser
                   85                  90                  95
   Thr Val Cys Ser Asp Ser Arg Asn Thr Ser Leu His Ala Val Ala Gly
               100                 105                 110
   Lys Val Glu Ile Lys Pro Tyr Val Gly Thr Asn Ala Phe Tyr Leu His
           115                 120                 125
   Gly Pro Ile Gln Val Ala Lys Asp Lys Asn His Phe Glu His Ala Asp
       130                 135                 140
   Gly Thr Pro Phe Phe Trp Leu Ser Asp Ser Trp Tyr Met Ser Leu Cys
   145                 150                 155                 160
   Lys Arg Leu Lys Trp Ser Glu Glu Phe Asn Thr Leu Thr Gln Asp Arg
                   165                 170                 175
   Val Ala Lys Gly Phe Asn Val Val Gln Val Val Ala Gly Leu Tyr Pro
               180                 185                 190
   Asp Met Gly Ala Phe Asp Glu Arg Ser Ala Asn Glu Gly Gly Phe Pro
           195                 200                 205
   Trp Glu Thr Asn Phe Thr Arg Ile Asn Pro Lys Tyr Phe Gln Ala Ala
       210                 215                 220
   Asp Gln Arg Ile Ala Phe Leu Val Asp Ser Gly Leu Ala Pro Cys Leu
   225                 230                 235                 240
   Phe Gly Ala Trp Gly Tyr Tyr Leu Pro Trp Met Gly Glu Lys Lys Met
                   245                 250                 255
   Lys Gln His Trp Arg Tyr Leu Val Ala Arg Tyr Gly Ala Tyr Pro Met
               260                 265                 270
   Phe Trp Cys Ile Ala Gly Glu Ala Thr Arg Pro Trp Tyr Leu Ser Asn
           275                 280                 285
   Thr Arg Ala Lys Asp Gln Ala Gln Leu Leu Lys Ser Trp Thr Lys Val
       290                 295                 300
   Thr Arg Tyr Val Arg Gln Val Asp Pro Tyr His Arg Pro Leu Ser Ile
   305                 310                 315                 320
   His Pro Pro Ile Glu Leu Gly Arg Gln Gln Val Ser Asp Pro Asn Leu
                   325                 330                 335
   Leu Asp Phe Glu Met Leu Gln Ser Gly His Asp Asp Arg Ala Ser Val
               340                 345                 350
   Pro Tyr Thr Ile Ser Leu Val Arg Arg Ser Arg Ser Ser Ser Pro Arg
           355                 360                 365
   Met Pro Thr Ile Asp Ala Glu Val Cys Tyr Glu Gly Ile Leu Gly Asn
       370                 375                 380
   Cys Glu Ala Asp Val Gln Arg Tyr Met Glu Trp Arg Cys Leu Leu Gly
   385                 390                 395                 400
   Gly Thr Ala Gly His Ser Tyr Gly Ala Asn Gly Val Trp Gln Ile Asn
                   405                 410                 415
   Gln Arg Asn Gln Pro Phe Gly Thr Ser Pro Gly Asn Asn Asn Trp Gly
               420                 425                 430
   Asn Thr Cys Trp Gln Glu Ala Met Arg Leu Pro Gly Ser Gly Gln Met
           435                 440                 445
   Gly Leu Gly Arg His Ile Leu Glu Lys Tyr Lys Trp Trp Glu Phe Asp
       450                 455                 460
   Cys His Pro Glu Trp Val Ser Gln Asp Ser Ser Glu Gly Ser Phe Lys
   465                 470                 475                 480
   Trp Gly Asn Trp Ile Trp Ser Pro Asp Ala Glu Ser Ala Phe Ala Ala
                   485                 490                 495
   Pro Ser Gly Arg Arg Cys Phe Arg Lys Ser Phe Thr Leu Thr Ser Thr
               500                 505                 510
   Ser Gln Ile Thr Gln Ala Leu Leu His Leu Ala Val Asp Asp Asn Ala
           515                 520                 525
   Glu Val Phe Leu Asn Gly Thr Arg Leu Gly Gly Leu Val Gly Trp Asn
       530                 535                 540
   Pro Tyr Arg Glu Leu Glu Val Thr Ser Leu Leu Lys Ser Gly Pro Asn
   545                 550                 555                 560
   Ile Leu Ala Ile His Ala Val Asn Ile Gln Ser Gly Thr Thr Gln Lys
                   565                 570                 575
   Asn Pro Ala Gly Leu Leu Val Asn Leu Asp Ile His Phe Asn Asp Asp
               580                 585                 590
   Ser Arg Lys Gln Ile Val Ser Asp Ala Ser Trp Phe Cys Ser Glu Gln
           595                 600                 605
   Glu Ser Ser Gly Trp Gln Asp Ala Asn Phe Asp Asp Arg Asn Trp Thr
       610                 615                 620
   Ser Ala Lys Ser Leu Ala Glu Pro Gly Gln Gly Pro Trp Lys Ile Leu
   625                 630                 635                 640
   Thr Ser Pro Asn Tyr His Leu Asn Pro Gly Ala Ala Gly Ile Val Lys
                   645                 650                 655
   Lys Ile Arg Leu Ile Tyr Leu Ala Thr Lys Ala Pro Ala Lys Val Glu
               660                 665                 670
   Lys Leu Glu Ala Gly Ile Asn Tyr Arg Ala Thr Phe Ile Asn Pro Gln
           675                 680                 685
   Asn Gly Asn Ser Ala Ser Ala Gly Ile Ala Lys Ala Asp Ala Asp Gln
       690                 695                 700
   Lys Trp Thr Ile Pro Thr Arg Pro Pro Gln Ser Lys Asp Trp Leu Leu
   705                 710                 715                 720
   Leu Leu Glu Ala Glu
                   725
<210> 34
   <211> 1596
   <212> PRT
   <213> Artificial Sequence
<220>
   <223> ZP_03626656.1
<400> 34
   Met Asp Asp Ser Ser His Tyr Pro Leu Lys Pro Met Ser Lys Ile Asn
    1               5                  10                  15
   Pro Asn Lys Thr Trp Trp Ser Gln Leu Val Leu Val Trp Phe Leu Gly
               20                  25                  30
   Phe Gly Leu Pro Phe Trp Thr Met Ala Ala Asp Ala Gly Leu Thr Thr
           35                  40                  45
   Ser Ala Asn Val Met Val Glu Val Ser Phe Thr Ala Asn Arg Thr Tyr
       50                  55                  60
   Ser Asp Pro Phe Asn Glu Val Thr Leu Asp Val Thr Phe Ile Asp Pro
   65                  70                  75                  80
   Lys Gly Gln Glu Leu Arg Val Pro Ala Phe Trp Ala Gly Lys Asn Val
                   85                  90                  95
   Trp Lys Val Arg Tyr Ala Ser Pro Val Val Gly Thr His Ser Phe Gln
               100                 105                 110
   Ser Glu Cys Ser Glu Ala Gly Asp Lys Gly Leu His Gly Ile Arg Gly
           115                 120                 125
   Lys Val Glu Val Lys Pro Tyr Ser Gly Gln Asn Leu Leu Tyr Val His
       130                 135                 140
   Gly Pro Ile Arg Val Ala Ala Asn His Arg Phe Phe Glu His Ala Asp
   145                 150                 155                 160
   Gly Thr Pro Phe Phe Trp Leu Gly Asp Thr Trp Trp Met Gly Leu Ser
                   165                 170                 175
   Gly Arg Leu His Trp Pro Glu Asp Phe Gln Lys Leu Thr Ala Asp Arg
               180                 185                 190
   Lys Ala Lys Gly Phe Asn Val Ile Gln Leu Val Ala Gly Leu Phe Pro
           195                 200                 205
   Asp Met His Pro Phe Asp Pro Arg Gly Ala Asn Glu Ala Gly Tyr Pro
       210                 215                 220
   Trp Glu Thr Asn Tyr Thr Arg Ile Arg Pro Glu Tyr Phe Asp Ala Ala
   225                 230                 235                 240
   Asp Lys Arg Met Met His Leu Val Asp Gln Gly Phe Thr Pro Cys Ile
                   245                 250                 255
   Val Gly Ala Trp Gly Tyr Phe Leu Pro Leu Met Gly Val Asp Lys Ala
               260                 265                 270
   Arg Ala His Trp Arg Tyr Leu Ile Ala Arg Tyr Gly Ala Leu Pro Val
           275                 280                 285
   Val Trp Cys Thr Ala Gly Glu Ala Asn Leu Pro Trp Tyr Leu Ala Lys
       290                 295                 300
   Gly Phe Pro Tyr Asp Asp Arg Lys Gln Val Lys Gly Trp Thr Glu Val
   305                 310                 315                 320
   Thr Arg Tyr Met Arg Ala Thr Asp Pro Phe His Arg Pro Ile Thr Ile
                   325                 330                 335
   His Pro Thr Gly Ile Gly Arg Leu Ser Ala Arg His Ala Met Asp Asp
               340                 345                 350
   Leu Ser Leu Ile Asp Ile Asp Met Leu Gln Thr Pro His Gly Gln Arg
           355                 360                 365
   Asp Ala Val Ala Pro Thr Val His Thr Met Arg Glu Ser Tyr Ala Asp
       370                 375                 380
   Lys Pro Val Met Pro Val Ile Asn Gly Glu Ala Ser Phe Glu Met Leu
   385                 390                 395                 400
   Ser Asp Ser Leu Pro Thr Glu Trp Thr Arg Arg Met Phe Trp Leu Cys
                   405                 410                 415
   Leu Met Asn Gly Ala Ala Gly His Thr Tyr Gly Ala Asn Gly Ile Trp
               420                 425                 430
   Gln Val Asn Arg Arg Gly Asp Pro His Gly Pro Ser Pro His His Asn
           435                 440                 445
   Gly Gly Asn Gly Tyr Gly Val Ile Pro Trp Asp Asp Ala Met Asn Leu
       450                 455                 460
   Pro Gly Ser Gly Gln Val Ala Phe Gly Lys Lys Leu Leu Glu Gln Tyr
   465                 470                 475                 480
   Pro Trp Gln Gln Phe Arg Pro His Pro Glu Trp Ala Ala Phe Ala Asn
                   485                 490                 495
   Lys Ala Ser Leu Ser Phe Glu Gly Cys Arg Trp Ile Trp Phe Pro Glu
               500                 505                 510
   Gly Asn Pro Ala Gln Asp Ala Pro Ala Glu Lys Arg Phe Phe Arg Lys
           515                 520                 525
   Ser Phe Val Leu Pro Glu Gly Lys Val Val Lys Ser Ala Gln Leu Arg
       530                 535                 540
   Val Ser Ala Asp Asp Gln Phe Ile Ala Arg Leu Asn Gly Lys Glu Val
   545                 550                 555                 560
   Gly Ala Ser Asn Ser Gly Ala Glu Thr Trp Arg Val Gly Lys Gln Phe
                   565                 570                 575
   Asn Asp Phe Ser Asn Trp Leu Lys Thr Gly Thr Asn Val Leu Ala Ile
               580                 585                 590
   Met Ala Gln Asn Met Pro Ala Thr Gly Ala Asn Pro Ala Gly Leu Ile
           595                 600                 605
   Gly Arg Leu Glu Ile Arg Phe Thr Asp Gly Glu Leu Leu Thr Val Val
       610                 615                 620
   Ser Asp Asp Thr Trp His Ser Met Lys Ser Glu Val Ala Gly Trp Asp
   625                 630                 635                 640
   Ala Thr Gly Phe Asp Asp Arg Met Cys Glu Arg Ala Lys Val Val Ala
                   645                 650                 655
   Lys Tyr Gly Asp Ser Pro Trp Gly Asn Leu Asp Pro Leu Asn Asn Asp
               660                 665                 670
   Asp Val Phe Gly Pro Gln Ser Ala Gly Val Pro Gly Val Val Arg Ile
           675                 680                 685
   Ile Tyr Val Pro Asp Cys Glu Ser Ile Val Val Lys Asn Leu Gly Arg
       690                 695                 700
   Asp Ala Glu Tyr Ala Ala Thr Tyr Phe Asp Pro Val Asp Gly Thr Lys
   705                 710                 715                 720
   Thr Gln Ala Thr Pro Val Arg Ala Asp Asn Thr Gly Ser Trp Ile Cys
                   725                 730                 735
   Pro Pro Pro Gly Gly Val Ala His Asp Trp Val Leu Ile Leu Glu Gly
               740                 745                 750
   Lys Ser Lys Gly Asp Ala Ser Tyr Ser Lys Gln Lys Ala Gly Ile Ser
           755                 760                 765
   Asn Thr Arg Gln Leu Thr Leu Ala Asn Asp Gln Leu Ala Trp Tyr Phe
       770                 775                 780
   Asp Trp Asn Asp Gly His Leu Ser Ser Arg Tyr Phe Glu Asn Lys Leu
   785                 790                 795                 800
   Ser His His Arg Phe Gly Leu Ser Gly Val Gln Glu Leu Ser Leu Asn
                   805                 810                 815
   Phe Ser Ala Ser Leu Asp Ser Val Ala Gln Pro Phe Val Arg Leu Thr
               820                 825                 830
   Asp Phe Gln Val Glu Gly Ala Gln Leu Ala Asp Ser His His Ala Ile
           835                 840                 845
   Phe Lys Leu Arg Ser Pro Ser Leu Ala Val Glu Ala Asp Val His Phe
       850                 855                 860
   Glu Leu Asp Gly Pro Thr Arg Arg Lys Trp Val Glu Val Thr Asn Lys
   865                 870                 875                 880
   Thr Gly Lys Glu Leu Leu Leu Leu Asp Val Glu Leu Asp Asp Phe Thr
                   885                 890                 895
   Thr Asp Gly Ile Ala Ser Gly Gly Gly Glu Gly Ala Pro Val Phe Val
               900                 905                 910
   Glu Gly Glu Phe Phe Ala Ala Ile Glu His Pro Ala Gly Val Asn Gln
           915                 920                 925
   Gly Asp Lys Gly Arg Val Gln Leu Ala His Tyr Pro Gly Arg Arg Leu
       930                 935                 940
   Ala Pro Asp Ala Ser Phe Arg Ser His Val Ala Leu Val Ser Val Ala
   945                 950                 955                 960
   Lys Ala Gly Gln Ala Glu Glu His Phe Val Ser Tyr Ile Gln Ala Lys
                   965                 970                 975
   Ser Leu Arg Pro Lys Lys Ala Ile Ser Val Tyr Thr Pro Phe Gly Ile
               980                 985                 990
   Asn Asn Leu Trp Gly Gly Cys Pro Ala Leu Asp Asp Glu Gln Thr Leu
           995                 1000                1005
   Asp Val Leu Ser Val Leu Glu Lys Trp Gln Lys Lys Gly Met Arg Phe
       1010                1015                1020
   Asp Tyr Phe Thr Leu Asp Thr Gly Trp Val Asp Pro Thr Ser Asp Leu
   1025                1030                1035                1040
   Thr Arg Phe Arg Pro Thr Cys Tyr Pro Asn Gly Pro Gly Lys Ile Val
                   1045                1050                1055
   Asp Arg Val Thr Gly Leu Asn Met Lys Phe Gly Leu Trp Phe Ala Thr
               1060                1065                1070
   Ser Trp Gly Ala Gln Ser Ala Trp Asp Tyr Pro Pro Ala Phe Pro Asp
           1075                1080                1085
   Gly His Pro Pro Gly Leu Pro Trp Arg Glu Gly Tyr Pro Ile Thr Arg
       1090                1095                1100
   Glu Gly Ile Thr Phe Cys Leu Gly Thr Glu Gln Tyr His Gly Met Leu
   1105                1110                1115                1120
   Lys Lys Ala Val Leu Tyr His Ile Lys Glu Asn His Leu Arg Leu Leu
                   1125                1130                1135
   Lys Phe Asp Gly Gly Asp Tyr Phe Cys Asp His Ala Glu His Gly His
               1140                1145                1150
   Leu Pro Gly Lys Tyr Ser Val Glu Pro Arg Phe Ala Asn Leu Ile Asp
           1155                1160                1165
   Ile Ala Asn Cys Ala Arg Glu Ala Ala Pro Asp Val Phe Ile Met Trp
       1170                1175                1180
   Tyr Trp Gly Leu Arg Ser Pro Phe Trp Ala Leu Tyr Gly Asp Met Leu
   1185                1190                1195                1200
   Phe Glu Ser Gly Leu His Met Glu Gly Ser Ala Thr Ser Ser Phe Pro
                   1205                1210                1215
   Thr Leu Tyr Tyr Arg Asp Ser Val Ser Leu Ala Gln Asp Gln Asn Ala
               1220                1225                1230
   Gln Tyr Ala Lys Thr Ile Pro Pro Ile Val Lys Asp Ser Leu Gly Val
           1235                1240                1245
   Trp Leu Ala Asp Asp Arg Trp Gly Asn Phe Met Gly Lys Glu Arg Trp
       1250                1255                1260
   Arg Glu Ala Leu Val Met Asp Leu Gly Arg Gly Asn Leu Phe Leu Pro
   1265                1270                1275                1280
   Asn Leu Trp Gly Asp Leu Tyr Leu Leu Asn Asp Glu Asp Ile Ser Phe
                   1285                1290                1295
   Met Ala Arg Ile Thr Ala Leu Ala Lys Lys His Glu Ser Met Met Leu
               1300                1305                1310
   His Arg Arg Asn Ile Leu Gly Asp Pro Phe Arg Asn Glu Val Tyr Gly
           1315                1320                1325
   Tyr Ala Tyr Gly Gln Gly Ala His Gly Leu Leu Phe Leu Asn Asn Asp
       1330                1335                1340
   His Phe Ala Ser Arg Arg Ala Glu Leu Arg Leu Asp Ala Ser Ile Gly
   1345 1350 1355 1360
   Ile Glu Ala Lys Pro Gly Thr Thr Leu His Val Val Ser His Phe Pro
   1365 1370 1375
   Asn Gln Thr Arg Leu Leu Arg Pro Asp Gly Lys Pro Phe Lys Val Gly
   1380 1385 1390
   Asp Ser Leu Gly Met Trp Leu Arg Pro Phe Glu Phe Leu Met Leu Glu
   1395 1400 1405
   Val Thr Pro Asp Gly Lys Glu Gly Lys Gly Leu Pro Thr Arg Ser Ile
   1410 1415 1420
   Thr Arg Gln Gln Ala Ala Asp Leu Gly Val Leu Leu Thr Leu Lys Pro
   1425 1430 1435 1440
   Gln Pro Leu Asp Ala Arg Met Asp Ile Arg Phe Ala Asp Ala Asn Gln
   1445 1450 1455
   Phe Glu Gly Gln Lys Phe Lys Lys Lys Val Tyr Ala Phe Glu Thr Thr
   1460 1465 1470
   Leu Pro Ala Leu Glu Gly Asp Gln Pro Ile Leu Ala Val Ala Ile Arg
   1475 1480 1485
   Leu Arg Lys Gly Gly Ala Glu Trp Arg Tyr Ala Pro Thr Val Val Arg
   1490 1495 1500
   Val Val Gln Ala Leu Ala Arg Ile Gly Asp Gln Asn Val Gln Met Val
   1505 1510 1515 1520
   Pro Val Pro Asp Ser Arg Gln Tyr Gly Asn Thr Gln Ala Glu Gly Cys
   1525 1530 1535
   Ser Trp Val Val Tyr Lys Val Arg Leu Ser Ser Lys Trp Ser His Thr
   1540 1545 1550
   Gln Leu Lys Ile Ala Val Gln Ala Tyr Leu Pro Asp Gly Val Glu Ala
   1555 1560 1565
   Glu Ile Glu Ser Trp Val Val Lys Arg Trp Trp Gln Glu Asp Ala Arg
   1570 1575 1580
   Pro Ala Ser Asp Gly Tyr Phe Thr Asp Glu Pro Ser
   1585 1590 1595
<210> 35
   <211> 542
   <212> PRT
   <213> Artificial Sequence
<220>
   <223> ZP_01717989.1
<400> 35
   Met Lys Val Leu Gln Ile Ala Phe Phe Phe Leu Ile Val Leu Ser Ser
    1               5                  10                  15
   His Phe Ser Phe Cys Gln Val Lys Lys Tyr Asp Arg Phe Glu Glu Glu
               20                  25                  30
   Phe Gln Ser Asp Lys Thr Tyr Glu Asn Pro Leu Tyr Asp Val Ser Glu
           35                  40                  45
   Phe Asp Val Lys Phe Ile Ser Pro Ser Gly Arg Glu Gln Val Val Arg
       50                  55                  60
   Gly Phe Trp Asp Gly Gly Ser Thr Trp Arg Val Arg Phe Met Pro Asp
   65                  70                  75                  80
   Glu Ile Gly Thr Trp Gln Phe Ser Thr Ser Ala Ser Asp Lys Asn Asn
                   85                  90                  95
   Thr Gly Leu Asn Gly Val Lys Gly Ser Phe Asn Cys Ile Glu Asn Asp
               100                 105                 110
   Ser Pro Leu Asn Leu Phe Gln Lys Gly Asn Ile Ile His Thr Lys Gly
           115                 120                 125
   Thr Tyr His Leu Ser Tyr Ala Asp Gly Thr Pro Phe Phe Trp Thr Ala
       130                 135                 140
   Cys Thr Ala Trp Asn Gly Ala Leu Lys Ser Thr Asp Glu Glu Trp Asp
   145                 150                 155                 160
   Tyr Tyr Leu Asn His Arg Val Glu Asn Asn Tyr Asn Thr Ile Gln Leu
                   165                 170                 175
   Val Thr Thr Gln Trp Arg Gly Gly Thr Lys Asn Ser Glu Gly Glu Val
                   180                 185                 190
   Ala Phe Thr Gly Ser Gly Lys Ile Ser Leu Asn Pro Glu Phe Phe Asp
               195                 200                 205
   Arg Ile Asp Gln Lys Ile Glu Glu Ala Asn Ala Lys Gly Leu Leu Val
       210                 215                 220
   Ser Pro Val Val Leu Trp Ala Leu Pro Phe Gly Gln Gly Thr Glu Tyr
   225                 230                 235                 240
   Ser Pro Gly Tyr Tyr Leu Pro Ile Arg Glu Ala Val Ile Leu Ala Lys
                   245                 250                 255
   Tyr Ile Val Ala Arg Tyr Gln Gly Asn His Val Leu Trp Thr Leu Gly
               260                 265                 270
   Gly Asp Gly Lys Tyr Tyr Gly Asp Leu Glu Asp Arg Trp Lys Ser Ile
           275                 280                 285
   Gly Ser Gln Val Phe Gly Asp Gly Lys His Gln Gly Leu Val Thr Leu
       290                 295                 300
   His Pro His Gly Leu Ser Trp Ile Gly Asp Ile Tyr Glu Asn Gln Asp
   305                 310                 315                 320
   Trp Tyr Asp Val Ile Thr Tyr Gln Ser Ser His Ser Asn Ser Glu Asn
                   325                 330                 335
   Thr Val Asn Trp Ile Asn Lys Gly Pro Ile Ala Lys Gln Trp Asp Asn
               340                 345                 350
   Leu Arg Pro Met Pro Leu Ile Asn Thr Glu Pro Asn Tyr Glu Glu Ile
           355                 360                 365
   Phe Phe Arg Ile Thr Ala Lys Asp Val Arg Asn Ala Ser Tyr Trp Ser
       370                 375                 380
   Val Phe Ala Ala Pro Pro Ser Gly Ile Thr Tyr Gly Ala Asn Gly Ile
   385                 390                 395                 400
   Trp Pro Trp Leu Arg Glu Gly Glu Asp Ile Glu Asn His Glu Ser Ala
                   405                 410                 415
   Ile Gly Thr His Ser Trp Lys Gln Ser Leu Asp Phe Glu Gly Ser Lys
               420                 425                 430
   Gln Ile Gly Tyr Leu Asn Glu Phe Ile Asn Gln Phe Asp Trp Trp Asn
           435                 440                 445
   Phe Lys Pro Ala Asn Asp Leu Leu Val Asp Gln Pro Gly Asp Glu Lys
       450                 455                 460
   Phe Asp Gln Phe Val Ser Val Val Ser Asn Glu Glu Lys Ser Lys Ile
   465                 470                 475                 480
   Leu Ile Tyr Ser Pro Val Lys Gln Pro Phe Lys Leu Phe Asn Ile Asn
                   485                 490                 495
   Gly Asn Thr Tyr Gln Leu Arg Trp Phe Asn Pro Ile Asp Asn Thr Tyr
               500                 505                 510
   Met Glu Gly Asn Leu Ser Pro Lys Asp Pro Ile Leu Glu Phe Glu Asn
           515                 520                 525
   Pro Leu Asp Gln Asp Met Val Leu Val Leu Glu Arg Lys Glu
       530                 535                 540
<210> 36
   <211> 559
   <212> PRT
   <213> Artificial Sequence
<220>
   <223> ZP_03628444.1
<400> 36
   Met Ile Pro Asp His Tyr Met Lys Arg Thr Ser Trp Arg Leu Trp Arg
    1               5                  10                  15
   Phe Leu Ser Ile Ile Cys Phe Phe Leu Ser Asn Thr Cys Phe Cys Gln
               20                  25                  30
   Ala Lys Val Ile Pro Trp Pro Val Ile Ala Lys Trp Gly Val Phe Glu
           35                  40                  45
   Lys Glu Phe Arg Ser Ser Val Thr Tyr Ala Asn Pro Leu Gln Asp Ile
       50                  55                  60
   Thr Leu Ser Val Glu Phe Thr Ser Pro Gln Gly Glu Lys Ile Lys Thr
   65                  70                  75                  80
   Tyr Gly Phe Trp Asp Gly Ala Arg Ile Trp Lys Phe Arg Phe Ser Pro
                   85                  90                  95
   Asp Gln Ala Gly Lys Trp Thr Tyr Gln Thr Ser Cys Ser Asp Ala Ala
               100                 105                 110
   Asn Thr Asn Leu Asn Lys Val Ile Gly Glu Phe Leu Cys Val Lys Thr
           115                 120                 125
   Thr Arg Lys Ser Arg Phe Asp Leu His Gly Pro Ile Gln Leu Ser His
       130                 135                 140
   Asp Gln Val Cys Leu Glu His Gln Asp His Thr Pro Phe Leu Trp Leu
   145                 150                 155                 160
   Ala Asp Ile Ala Trp Ser Gly Ala Leu Leu Ser Lys Ser Thr Glu Trp
                   165                 170                 175
   Glu Thr Tyr Ala Gly Gln Arg Ala Lys Gln Ser Phe Thr Ala Val Gln
               180                 185                 190
   Trp Val Ala Ala Pro Gly Arg Asn Ser Arg Asn Gln Ser Val Phe Glu
           195                 200                 205
   Ser Thr Ser Thr Thr Leu Thr Ile Asn Leu Asn Leu Phe Gln Gln Leu
       210                 215                 220
   Asp Ser Arg Ile Glu Thr Leu Asn Arg Ala Gly Leu Leu Ser Ala Ile
   225                 230                 235                 240
   Leu Pro Leu Arg Asp Leu Glu Ala Gln Ala Pro Gly Leu Glu Thr Ile
                   245                 250                 255
   Pro Glu Glu Gln Ala Val Gln Leu Leu Arg Tyr Met Val Ala Arg Trp
               260                 265                 270
   Gly Ala Tyr Asn Val Ala Trp Ile Ile Met Cys Glu Gly Asp Asn Leu
           275                 280                 285
   Ser Thr Gln Val Ala Arg Trp Lys Arg Val Gly Arg Ser Val Phe Ala
       290                 295                 300
   Asn Gln His His Ala Pro Val Ile Leu His Pro Gly Thr Thr Tyr Trp
   305                 310                 315                 320
   Val Leu Asp Glu Phe Arg Ala Glu Pro Trp Val Asp Val Leu Ser Tyr
                   325                 330                 335
   Gln Ser Gly Gln Glu Ala Asp Asp Asn Ala Leu Gln Trp Leu Leu Ala
               340                 345                 350
   Gly Pro Leu Ala Ile Asp Trp Gln Arg Val Pro Leu Lys Pro Phe Ile
           355                 360                 365
   Asn Leu Ala Pro Tyr Tyr Glu Asn Ser Ser Ala Pro Ala Ser Val Thr
       370                 375                 380
   Gln Thr Ser Met Val Arg Arg Leu Ile Tyr Trp Ser Leu Leu Asn Ala
   385                 390                 395                 400
   Pro Thr Ala Gly Val Ser Tyr Gly Ala Asp Gly Val Trp Asn Trp Gln
                   405                 410                 415
   Lys Glu Ala Ala Asn Lys Arg Pro Asn Ser Asn Ala Gly Ser Pro Val
               420                 425                 430
   Trp Gln Lys Asn Leu Thr Met Pro Val Ala Glu Gln Met Lys Ile Val
           435                 440                 445
   Gly Gln Ile Phe Asn Ser Ile Glu Phe Trp Arg Leu Arg Pro Ala Pro
       450                 455                 460
   Gly Met Leu Val Val Gln Pro Gly Asp Glu His Pro Ala Arg His Ile
   465                 470                 475                 480
   Ser Ala Thr Gln Ser Ser Leu Lys Asp Leu Ser Val Ile Tyr Ile Pro
                   485                 490                 495
   Glu Glu Arg Ser Val Asp Leu Lys Ala Thr Thr Ile Pro Thr Pro Leu
               500                 505                 510
   Lys Ala Thr Trp Ile Asn Ala Arg Thr Gly Asp Arg Met Asp Ala Val
           515                 520                 525
   Gly Tyr Val Ala Gly Pro Ile Cys Arg Phe Ile Thr Pro Gln Pro Gly
       530                 535                 540
   Asp Trp Leu Leu Leu Ile Gln Pro Asp Pro Leu Pro Ser Ala His
   545                 550                 555
<210> 37
   <211> 531
   <212> PRT
   <213> Artificial Sequence
<220>
   <223> YP_003585990.1
<400> 37
   Met Lys Leu Lys Thr Ala Gln Pro Met Leu Tyr Glu Arg Thr Asp Phe
   1               5                   10                  15
   Asn Ile Ser Leu Asn Glu Gln Trp Glu Asn Pro Tyr Leu Ala Glu Asp
               20                  25                  30
   Ile Ala Val Asp Ile Lys Met Gln Ser Pro Ser Gly Lys Glu Ile Leu
           35                  40                  45
   Leu Pro Cys Phe Tyr Val Ser Gly Glu Ser Gly Glu Ala Ser Glu Trp
       50                  55                  60
   Glu Ala Arg Phe Ala Pro Gln Glu Ile Gly Arg Tyr Thr Tyr His Leu
   65                  70                  75                  80
   Val Val Ser Lys Lys Gly Glu Glu Met Val Phe Asp Glu Asn Glu Phe
                   85                  90                  95
   Thr Val Glu Asn Ser Glu Asn Lys Gly Phe Leu His Ala Glu Asn Asn
               100                 105                 110
   Trp Ile Leu Arg Tyr Asp Asn Gly Asp Pro Phe Arg Gly Ile Gly Glu
           115                 120                 125
   Asn Ile Gly Trp Glu Ser Arg Thr Asn Asp Asp Ser Lys Phe Phe His
       130                 135                 140
   Glu Leu His Glu Lys Lys Lys Tyr Asn Tyr Asn Tyr Leu Leu Gly Glu
   145                 150                 155                 160
   Leu Ser Arg Asn Gly Gly Asn Phe Phe Arg Thr Trp Ile Cys Ser Trp
                   165                 170                 175
   Asn Leu Pro Leu Asp Trp Lys Asp Asn Phe Asn Asn Ser Arg Tyr Thr
               180                 185                 190
   Ala Ser Asp Ala Tyr Tyr Asn Pro Ser Ala Val Ala Lys Leu Asp Ser
           195                 200                 205
   Leu Val Asp Leu Ser Lys Lys Leu Asp Leu His Met Met Leu Thr Leu
       210                 215                 220
   Gly Pro Gly Asn Tyr Ser Lys Glu Asp Gly Gly Phe Ala Glu Ser Thr
   225                 230                 235                 240
   Ala Asp Phe Phe Val Asn Pro Lys Ser Arg Gln Arg Tyr Lys Asn Arg
                   245                 250                 255
   Leu Arg Tyr Ile Ile Ala Arg Trp Gly Tyr Ser Thr Ser Ile Ala Ala
               260                 265                 270
   Trp Glu Leu Phe Asn Glu Ile Asp Asn Val Gln Tyr Arg Asn Arg Asp
           275                 280                 285
   Asn Pro Ile Asp Ala Lys Thr Ile Val Asp Trp His Glu Glu Met Ser
       290                 295                 300
   Asn Tyr Ile Asp Lys Ile Asp Pro Tyr Asn His Ile Ile Thr Thr Ser
   305                 310                 315                 320
   Ile Ser His Arg Asp Leu Glu Gly Leu Asn Ser Leu Pro Ala Ile Asp
                   325                 330                 335
   Ile Asn Gln Lys His Ile Tyr Asn Arg Thr Lys Asp Ile Pro Gly Glu
               340                 345                 350
   Ile Ile Asp Tyr Glu Lys Arg Phe Gly Lys Pro Tyr Val Ile Gly Glu
           355                 360                 365
   Phe Ser Tyr Glu Trp Asp Trp Ser Lys Asn Phe Asp Glu Phe Pro Glu
       370                 375                 380
   Glu Met Asp Ser Asp Phe Lys Arg Gly Leu Trp Tyr Gly Met Phe Thr
   385                 390                 395                 400
   Ser Thr Pro Ile Leu Pro Leu Ser Trp Trp Trp Glu Tyr Phe Asp Glu
                   405                 410                 415
   Arg Gly Met Thr Ser Tyr Phe Arg Gly Val Ser Lys Ile Asn Asp Met
               420                 425                 430
   Met Leu Lys Ala Gly Asn Gly Lys Phe Glu Lys Val Ser Phe Lys Thr
           435                 440                 445
   Gly Asn Gln Leu Glu Ser Phe Gly Val Lys Cys Gly Asn Lys Ile Phe
       450                 455                 460
   Ile Tyr Leu Tyr Asn Pro Leu Asp Lys Ala Leu Asn Tyr His Val Glu
   465                 470                 475                 480
   Val Pro Glu Leu Arg Asp Ala Ser Tyr Glu Val Gln Ser Phe His Pro
                   485                 490                 495
   Leu Asn Leu Glu Phe Ser Gln Glu Lys Thr Arg Glu Gly Ile Glu Val
               500                 505                 510
   Ala Ala Gly Lys Ser Leu Asp Ala Gly Lys Glu Ile Leu Leu Ile Ile
           515                 520                 525
   Asp Phe Asp
       530

## Description

### CROSS-REFERENCE TO RELATED APPLICATION

This application claims the benefit of U.S. Provisional Application No. 61/369,388, filed July 30, 2010.

### SUBMISSION OF SEQUENCE LISTING AS ASCII TEXT FILE

The content of the following submission on ASCII text file is incorporated herein by reference in its entirety: a computer readable form (CRF) of the Sequence Listing (file name: 677792000940SEQLIST.txt, date recorded: June 30, 2011, size: 206 KB).

### FIELD

The present disclosure relates to hydrolysis of cellulose-containing polysaccharides and degradation of biomass using polypeptides having cellulase activity, including hyperthermophilic polypeptides. In particular the present disclosure relates to archaeal polynucleotides encoding the polypeptides, the polypeptides themselves, and compositions, methods and uses thereof.

### BACKGROUND

Cellulose, the major component of plant biomass, is considered the most abundant biopolymer. Bayer, E.A., Chanzy, H., Lamed, R., Shoham, Y. (1998) Cellulose, cellulases and cellulosomes. Curr. Opin. Struct. Biol. 8, 548-557. Certain microorganisms are able to convert the monomer of cellulose, glucose, into various products useful in the production of biofuels and other methods. Cellulose is highly stable, has a high storage potential, low cost, and plentiful supply. Based on these and other properties, cellulose and enzymes capable of degrading and hydrolyzing it are useful in the sequestration, storage, and production of bioenergy. Lynd LR, Weimer PJ, van Zyl WH, Pretorius IS (2002), "Microbial cellulose utilization: fundamentals and biotechnology," Microbiol Mol Biol Rev 66: 506-577.

Crystalline cellulose is composed of linear polymers of β1-4 linked glucose, held in a tightly crosslinked crystalline lattice by a high degree of intermolecular hydrogen bonding. This structure confers stability but also hinders efficient deconstruction of cellulose. Strategies for commercial depolymerization of cellulose typically combine pretreatment to disrupt the crystalline structure, followed by enzymatic hydrolysis. Hilden L, Johansson G (2004), "Recent developments on cellulases and carbohydrate-binding modules with cellulose affinity," Biotechnol Lett, 26: 1683-1693. Disruption of the crystalline structure and chemical hydrolysis typically requires high temperatures and low pH. *See* Kim JS, Lee YY, Torget, RW. (2001) "Cellulose hydrolysis under extremely low sulfuric acid and high-temperature conditions, Appl. Biochem. Biotechnol. 91-93 331-340. Enzymatic hydrolysis generally occurs under milder conditions. The degree of pretreatment required and the expense of subsequent cleanup steps are affected by properties of the enzymes used.

Bacteria capable of degrading cellulose include those belonging to the genera *Aquifex, Rhodothermus, Thermobifida*, *Anaerocellum,* and *Caldicellulosiruptor.* A recombinant thermostable endoglucanase of *Aquifex aeolicus* produced in *E. coli* showed maximal activity at 80°C and pH 7.0 with a half-life of 2 h at 100°C (Kim JS, Lee YY, Torget, RW (2001). Cellulose hydrolysis under extremely low sulfuric acid and high-temperature conditions. Appl. Biochem. Biotechnol. 91-93. 331-340)). The endoglucanases produced by *Anaerocellum thermophilum* and *Caldicellulosiruptor saccharolyticus* are multidomain enzymes composed of two catalytic domains, linked to carbohydrate binding domains by proline-threonine-rich regions (Zverlov V, Mahr S, Riedel K, Bronnenmeier K (1998a), "Properties and gene structure of a bifunctional cellulolytic enzyme (CelA) from the extreme thermophile 'Anaerocellum thermophilum' with separate glycosyl hydrolase family 9 and 48 catalytic domains," Microbiology 144 ( Pt 2): 457-465; Te'o VS, Saul DJ, Bergquist PL (1995), "celA, another gene coding for a multidomain cellulase from the extreme thermophile Caldocellum saccharolyticum," Appl Microbiol Biotechnol 43: 291-296; Saul et al. 1990. The recombinant endoglucanase of *Rhodothermus marinus* has a pH optimum of 6.0-7.0 and a temperature optimum at 100°C (Halldórsdóttir S, Thórólfsdóttir ET, Spilliaert R, Johansson M, Thorbjarnardóttir SH, Palsdottir A, Hreggvidsson GO, Kristjánsson JK, Holst O, Eggertsson G. (1998), "Cloning, sequencing and overexpression of a Rhodothermus marinus gene encoding a thermostable cellulase of glycosyl hydrolase family 12," Appl Microbiol Biotechnol 49: 277-284). The aerobic thermophilic bacterium *Thermus caldophilus* also produces an endoglucanase which exhibits high activity on CMC with cellobiose and cellotriose as products (Kim D, Park BH, Jung B-W, Kim M-K, Hong SI, Lee, DS (2006) Identification and molecular modeling of a family 5 endocellulase from Thermus caldophilus GK24, a cellulolytic strain of Thermus thermophilus. Int J Mol Sci 7: 571-589). In contrast, high-temperature, crystalline deconstructing cellulases from hyperthermophilic Archaea are few in number, despite efforts to identify such enzymes. Hyperthermophilic enzymes that act on cellulose typically lack identifiable cellulose binding domains.

Thus there is a need for improved cellulases, including cellulases encoded by hyperthermophilic archaea, and cellulases having high stability and tolerance to a range of chemical and physical parameters, including cellulases with activity at high temperatures and over a broad range of temperatures and pH, cellulases with higher catalytic activity and rate of conversion, activity in the presence of salts, ionic detergents, sulfhydryl reagents, and ionic liquids. Provided are polypeptides, compositions and methods that meet this need.

### BRIEF SUMMARY

The present disclosure relates to isolated polypeptides (proteins), and in particular cellulases, including cellulases encoded by hyperthermophilic archaea, and cellulases having high stability and tolerance to a range of chemical and physical parameters, including cellulases with activity at high temperatures and over a broad range of temperatures and pH, cellulases with higher catalytic activity and rate of conversion, activity in the presence of salts, ionic detergents, sulfhydryl reagents, and ionic liquids. For example, provided are polypeptides, such as EBI244, having cellulase activity, *e.g.*, endoglucanase, exoglucanase and/or β-Glucosidase or β-Glucosidaseglucohydrolase activity, such as cellulases produced by archaea. Certain aspects of the present disclosure relate to an isolated EBI244 protein having the amino acid sequence of SEQ ID NO: 1, and variants and fragments thereof. The present disclosure also relates to isolated polynucleotides encoding the polypeptides, as well as vectors and genetically modified host cells containing such isolated polynucleotides.

The present disclosure further relates to compositions comprising the isolated polypeptides or enriched in such polypeptides. Moreover the present disclosure relates to methods for the identification and production of the polypeptides, and methods for their use in the degradation and hydrolysis of poly- and oligo-saccharides, such as biomass, e.g., hemicellulose, for example, in the conversion of biomass, such as lignocellulocytic biomass, including pretreated lignocellulocytic biomass, into soluble sugars, including for use in the fermentive production of biofuels, polishing of cotton fabrics, production of laundry detergents, production of polished crystalline cellulose, assays of cellulases, expansins, and cellulose binding proteins, and in pulping cellulolytic materials.

In some embodiments, the provided polypeptides are isolated proteins that include a domain having an amino acid sequence at least at or about 30 %, 40 %, 50 %, 60 %, typically at least at or about 70 %, 75 %, 80 %, 85 %, 90%, 91%, 92%, 93%, 94%, 95%, 96%, 97%, 98%, or 99% identical or at or about 100% identical to amino acids 250-580 of SEQ ID NO: 1, further including a domain at least 70 %, 75 %, 80 %, 85 %, 90%, 91%, 92%, 93%, 94%, 95%, 96%, 97%, 98%, or 99% identical or at or about 100% identical to amino acids 750-842 of SEQ ID NO: 1, further including a domain at least 70 %, 75 %, 80 %, 85 %, 90%, 91%, 92%, 93%, 94%, 95%, 96%, 97%, 98%, or 99% identical or at or about 100% identical to amino acids 580-750 of SEQ ID NO: 1, where the protein is a cellulase. In some embodiments, the protein includes or further includes a domain at least at about 70 %, 75 %, 80 %, 85 %, 90%, 91%, 92%, 93%, 94%, 95%, 96%, 97%, 98%, or 99% identical or at or about 100% identical to amino acids 130-250 of SEQ ID NO: 1. In one aspect, the protein contains a domain having an amino acid sequence at least 70 %, 75 %, 80 %, 85 %, 90%, 91%, 92%, 93%, 94%, 95%, 96%, 97%, 98%, or 99% identical or 100% identical to amino acids 250 through 580 of SEQ ID NO: 1, amino acids 130-250 of SEQ ID NO: 1, amino acids 750-842 of SEQ ID NO: 1, or amino acids 580-750 of SEQ ID NO: 1, where the protein is a cellulase.

In one embodiment, the isolated protein has an amino acid sequence that is at least 70 %, 75 %, 80 %, 85 %, 90%, 91%, 92%, 93%, 94%, 95%, 96%, 97%, 98%, or 99% identical or 100% identical to SEQ ID NO: 1In some embodiments, the protein exhibits cellulase activity, for example, one or more of endoglucanase activity, exoglucanase activity, and β-Glucosidase activity. In some embodiments, the protein exhibits such activity over a range of physical and chemical conditions, such as at a high temperature or over a broad temperature range, such as at a temperature greater than 105°C, 95°C to 110°C, or at a temperature exceeding 90, 91, 92, 93, 94, 95, 96, 07, 98, 99, or 100°C, or over a broad temperature range, such as between at or about 60°C and 110°C or between 65°C and 110°C, such as between 90 and 110°C, between 65 and 70°C, between 85 and 105°C, or between 95 and 105°C.

Also provided are compositions containing the isolated proteins, and nucleic acids encoding the proteins, such as polynucleotides encoding any of the proteins,.

Also provided are expression vectors containing the isolated nucleic acids, operably linked to a regulatory sequence, and host cells containing the expression vectors, and methods for producing a protein by culturing the host cell in a culture medium, under suitable conditions to produce a protein encoded by the expression vector. Also provided are methods for converting a biomass to sugars, hydrolyzing or degrading a biomass, by contacting the biomass with the provided compositions and/or proteins. Also provided are methods for producing a fermentation product by contacting biomass with the compositions or proteins to form a first product, and then culturing the first product with one or more fermentive microorganisms under conditions sufficient to produce a fermentation product, or incubating the first product with a chemical solution, under conditions sufficient to produce a fermentation product by a chemical process. Also provided are methods for producing a fermentation product, by hydrolyzing or degrading biomass with the provided compositions and proteins to form a first product and then culturing the first product with one or more fermentive microorganisms under conditions sufficient to produce a fermentation product, or incubating the first product with a chemical solution, under conditions sufficient to produce a fermentation product by a chemical process. In some aspects, the fermentation product is a biofuel.

Also provided are methods for textile cleaning by contacting a soiled textile with the composition or protein, to yield a clean textile. Also provided are methods for paper pulp bleaching by contacting paper pulp with the composition or protein to yield bleached paper pulp.

Also provided are laundry detergent compositions, containing the provided proteins and detergent, and methods for use of such compositions in cleaning, anti-deposition, or color care, by contacting the laundry detergent composition with a textile.

In some aspects, the methods, e.g., the contacting, are conducted at a pH between 4.5 and 8.5, such as a pH of at least 5.5 or at least 6.5, for example, at least 7,at least 7.5,at least 8, at least 8.5. In some aspects, the methods or contacting are performed at a temperature between 90 and 110 °C, between 60 and 70 °C, between 95 and 105 °C, or at least 100 °C. In some aspects, the method or contacting is performed in a solution containing KCl or NaCl, for example, at a concentration of at least 1 M, 2 M, 3 M, or 4 M, or at a saturating condition. In one aspect, the method or contacting is performed in a solution containing at least 10 %, at least 20 %, at least 30 % or at least 40 % ionic liquid.

In some aspects, the biomass is a lignocellulose. In some embodiments, the biomass is pretreated prior to contacting.

### BRIEF DESCRIPTION OF THE DRAWINGS

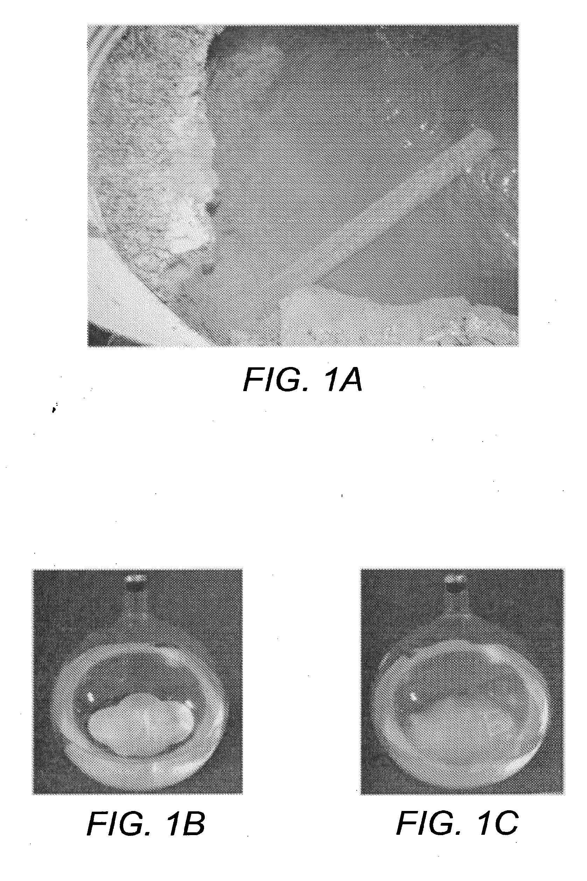

**FIG. 1A** shows the source of lignocellulose-degrading consortium of hyperthermophilic Archea enrichment, and degradation of filter paper. A circumneutral geothermal pool at 94°C, with a level-maintaining syphon. Sediment from the floor of this site was enriched on pulverized Miscanthus at 90°C and subsequently transferred to filter paper enriched media. **FIG. 1B** depicts the degradation of filter paper by the enrichment culture in a spherical 2L culture flask. Circular discs of Whatman^{®} #3 filter paper were shredded and partially dissolved after incubation for 30 days at 90°C. **FIG. 1C** depicts control Whatman^{®} #3 filter paper discs. Incubation as in panel B.

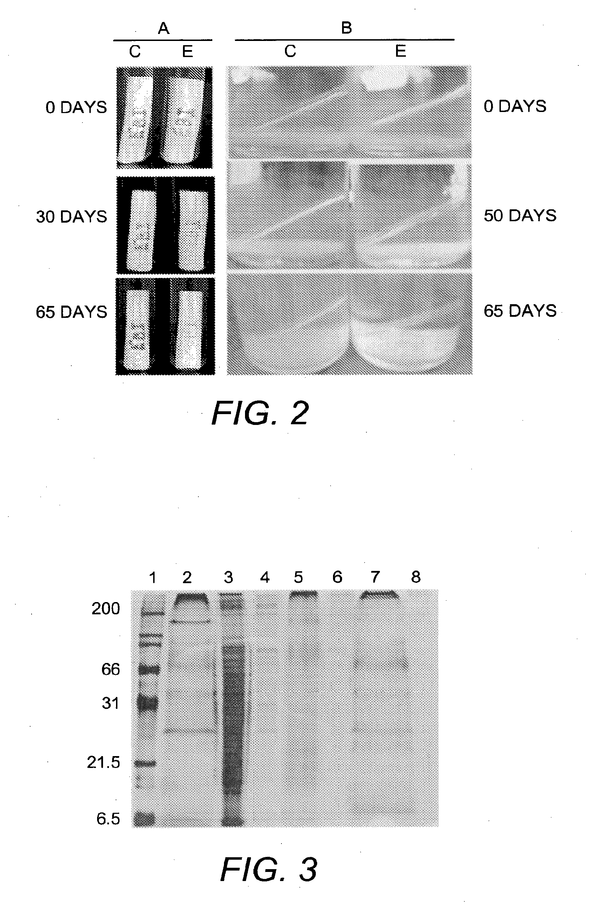

**FIG. 2** depicts results of additional experiments showing filter degradation by the enrichment. **FIG. 2A** shows Whatman^{®} #1 filter paper in media without (C) and with (E) inoculation with the enrichment at 90°C. Lettering was applied with a number 2 graphite pencil. **FIG. 2B** shows Whatman^{®} #3 filter paper strip (2 mm x 40 mm) in growth media supported by a glass tube, without (C) and with (E) inoculation with the enrichment at 90°C. The inoculated sampled showed complete dissolution of the filter paper strip.
**FIG. 3** shows endoglucanase activity of protein in the three-organism hyperthermophilic Archaea consortium enriched on Avicel^{®} as described in Example 1 A, measured by zymograms on SDS-PAGE fractions from detergent wash of Avicel^{®} from the enrichment culture. The lanes are labeled as follows: 1 (Marker), (2) 1 % SDS wash (experiment 1); (3) whole cell extract; (4) Avicel^{®}; (5) CHAPS fraction; (6) Pellet after CHAPS wash, (7) 1 % SDS wash (experiment 2).

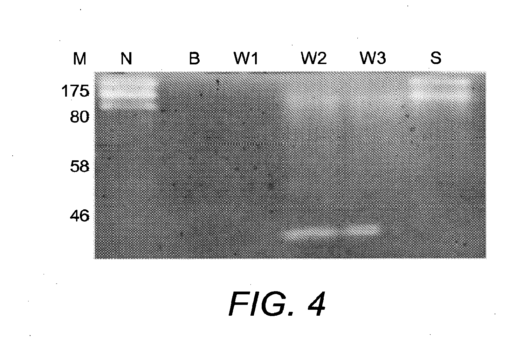

**FIG. 4** shows protein extraction and detection of CMCase activity. Protein extraction and detection of CMCase activity from proteins eluted from Avicel^{®} particles after deconstruction by enrichment at 90°C for 8 days. Image shows SDSPAGE gradient zymogram, 10%-15% acrylimide, with 0.2% CMC embedded in gel. Lanes: M-marker, N-native whole SDS extract, B-buffer only soluble extract, W1-0.6% CHAPS extract, W2-1% CHAPS 5% Cellobiose extract #1 (1 hr incubation at 90°C), W-3 1% CHAPS 5% Cellobiose extract #2 (1 hr incubation at 90°C), S-1% SDS extract final wash (15 minute incubation 100°C). For lanes B through S, the Avicel^{®} pellet was sonicated continuously for 2 minutes in the wash solution.

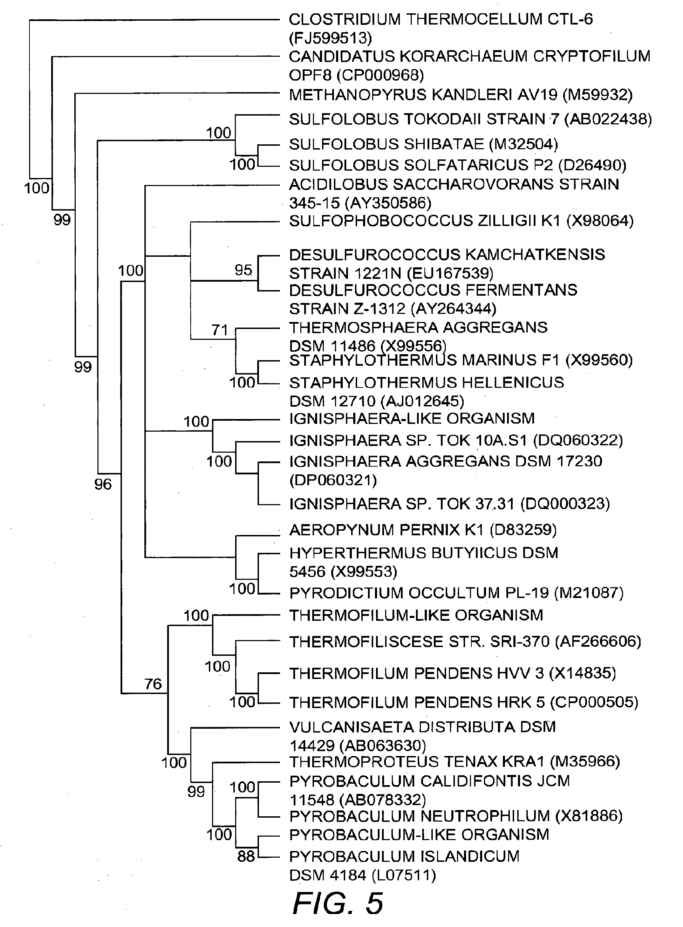

**FIG. 5** depicts a maximum likelihood phylogenetic tree. Maximum likelihood 16S rRNA phylogenetic tree, showing the relationship of full-length 16S rRNAs from the three component organisms of the assembled metagenome. Branches in bold and labeled with larger type represent the three sequences from the metagenome.

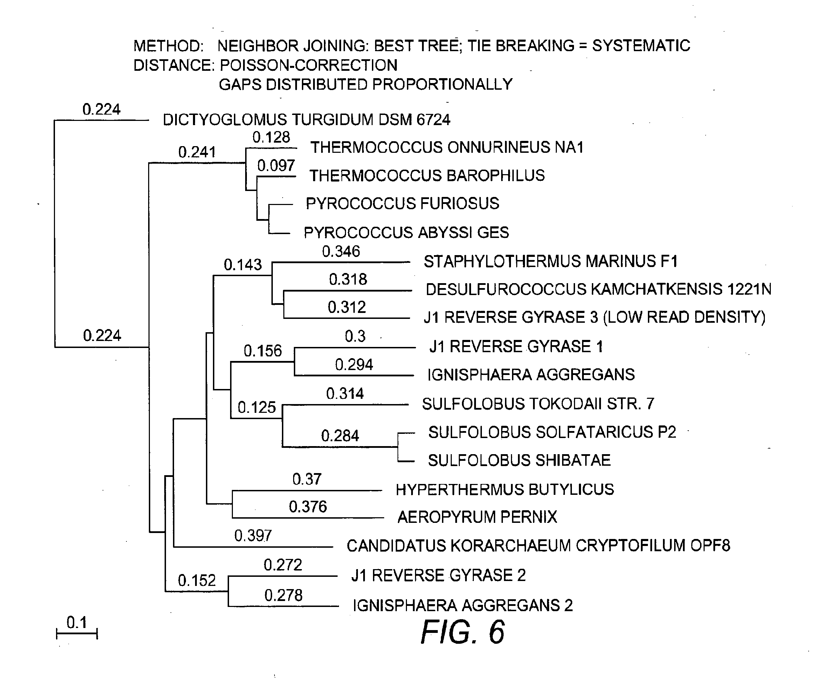

**FIG. 6** displays a phylogentic tree, showing relationship of three reverse gyrases, from the metagenome described in Example 1A, to other archaeal reverse gyases. Reverse gyrase 1 and 2, found on high-read density contigs, appear closely allied with the two reverse gyrases encoded by *Ignisphaera aggregans.* The reverse gyrase of the bacterium *Dictyoglomus turgidum* was set as the root.

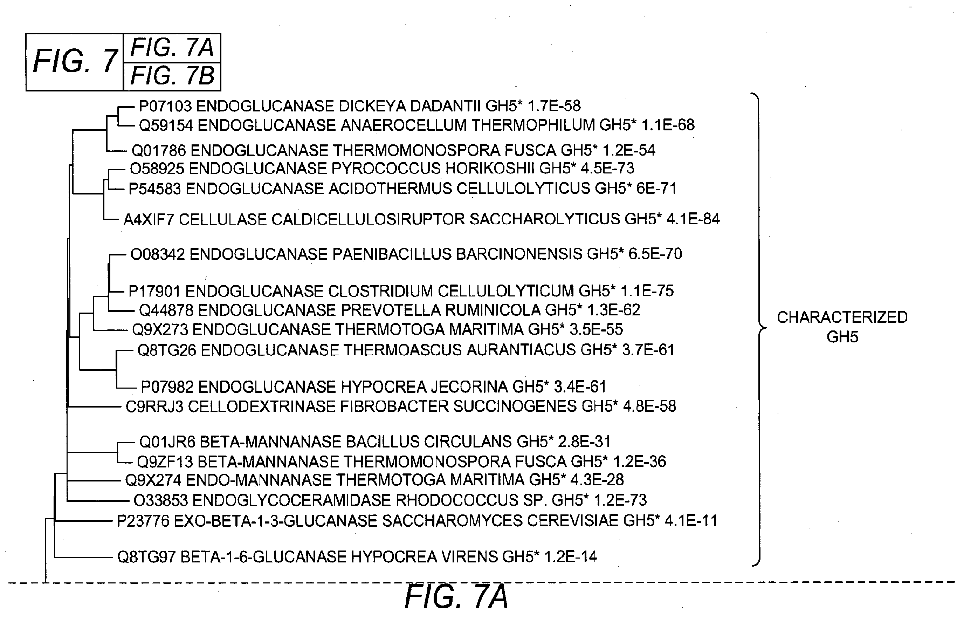

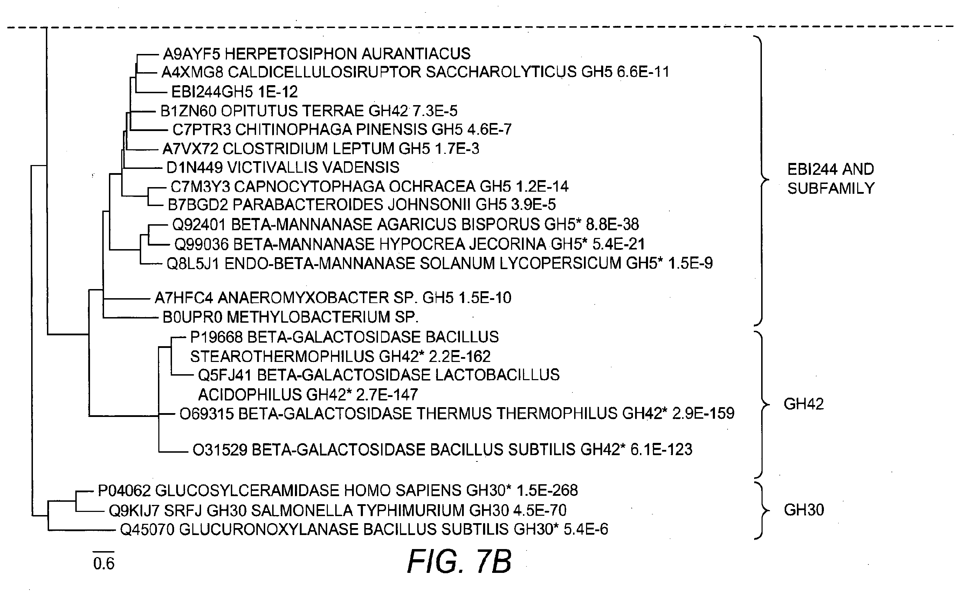

**FIG. 7** depicts the phylogeny of the EBI244 protein putative catalytic domain. A phylogenetic tree was produced showing the relationship of EBI244's catalytic domain to the closest characterized GH families. Tree entry information: Uniprot identifier; enzyme function (if known); organism name; Pfam hit GH family (asterisk indicated characterized enzyme in CAZY database); and E-value (no GH listed indicates no Pfam hits).

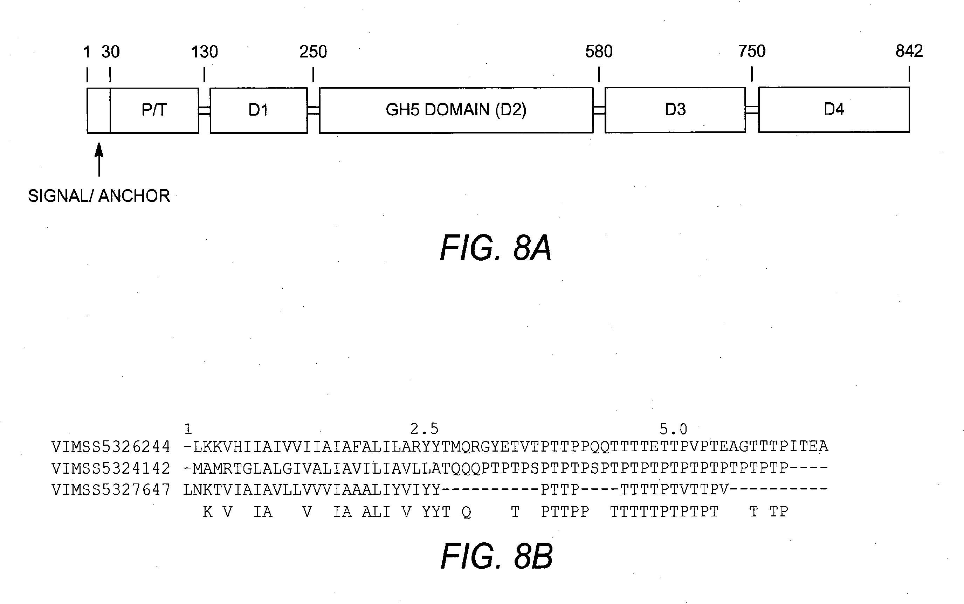

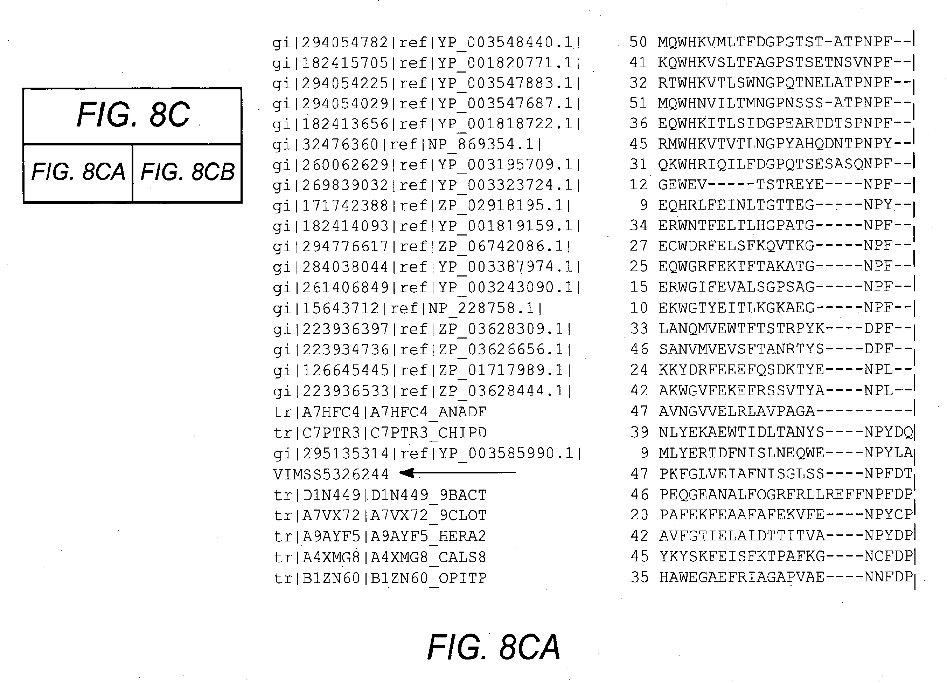

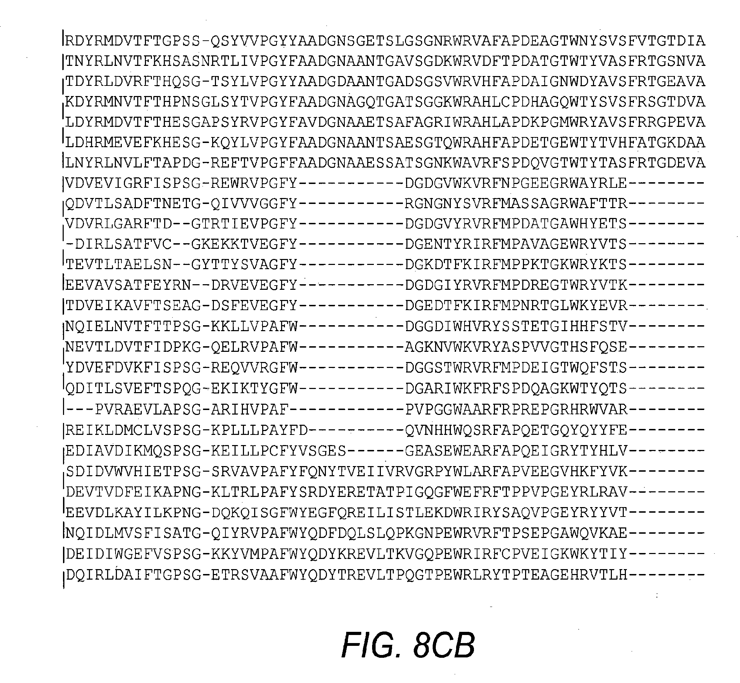

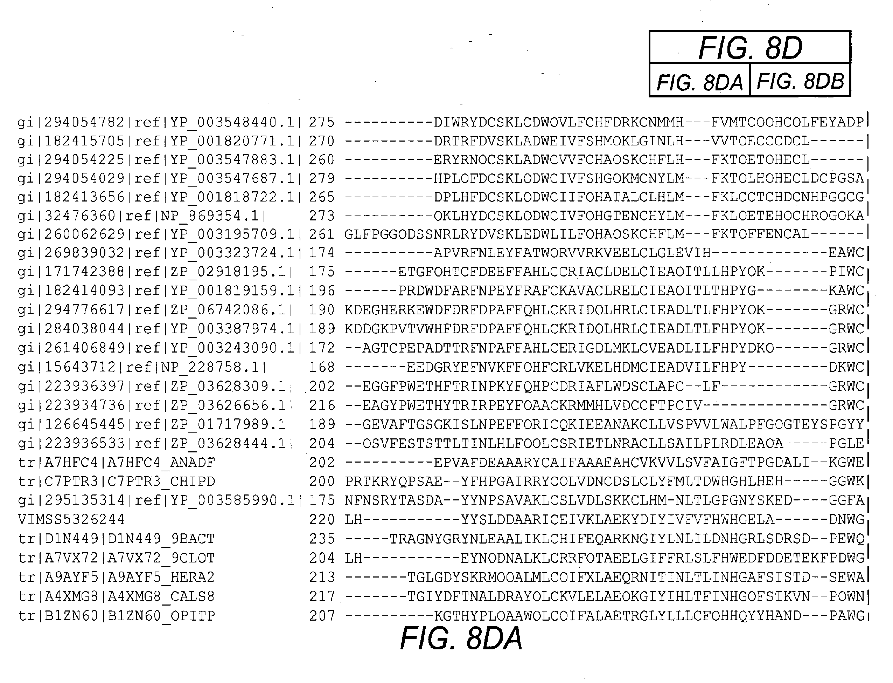

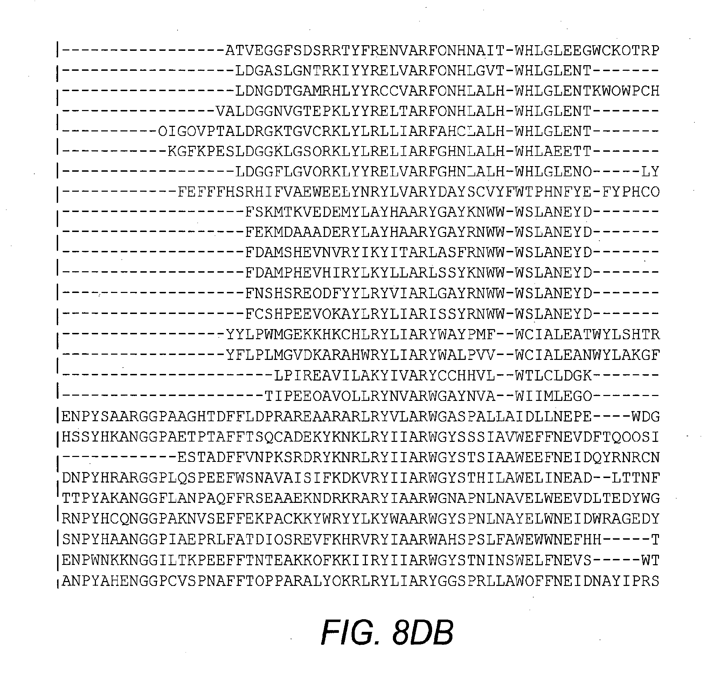

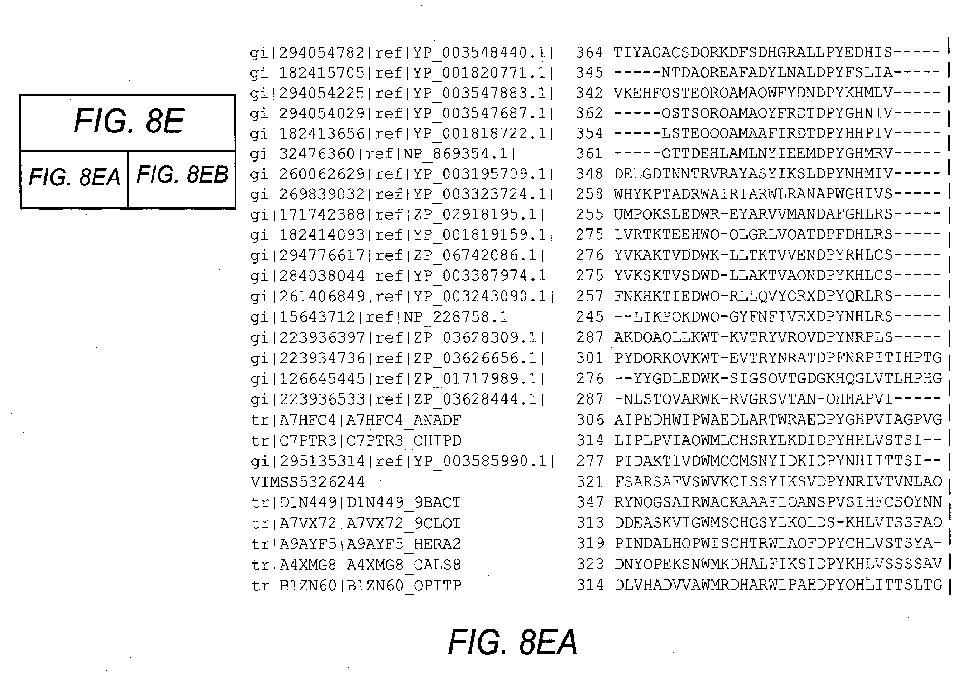

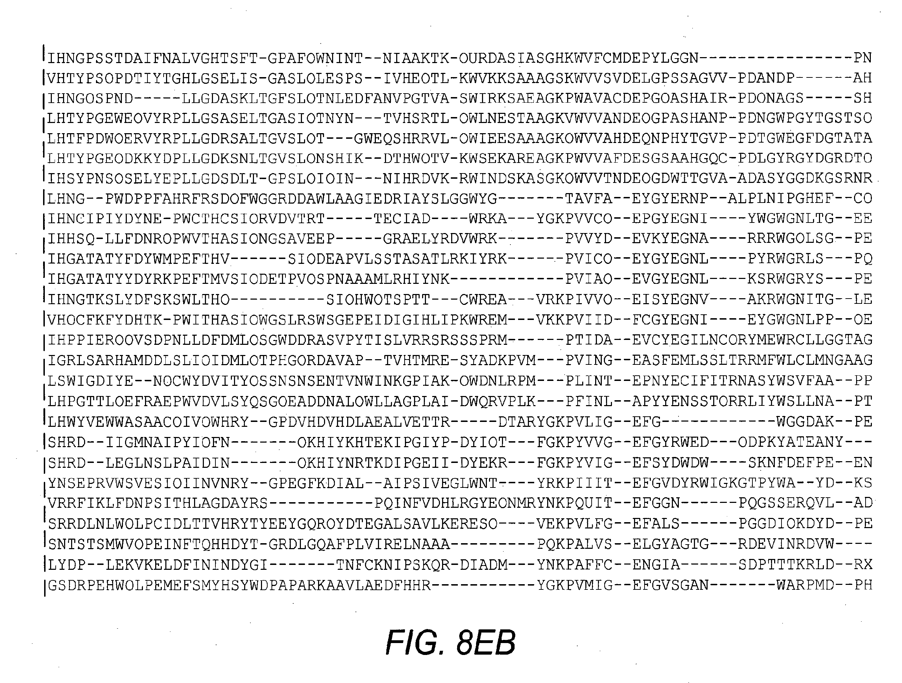

**FIG. 8A** displays schematically the predicted domain architecture of EBI244 protein sequence, with approximate amino acid positions of domain boundaries labeled. **FIG. 8B** displays similar N-terminal protein regions among genes identified in the hyperthermophilic Archaea consortium metagenome in which EBI244 was discovered, as described in Example 1. The top sequence is EBI244. **FIG. 8C** shows a multiple sequence alignment of a non-redundant sample of the thirty-eight sequences identified using Hidden Markov Model (HMM) searching and analysis based on domain 1 of EBI244, as described in Example 1. **FIG. 8D** shows a multiple sequence alignment of EBI244 domain 2 with sequences identified in the domain 1 HMM search/analysis (see FIG. 8C). Catalytic residues of EBI244 predicted from Pfam analysis (glutamates 413 and 506) both glutamates) are highlighted in yellow. **FIG. 8E** shows a Multiple sequence alignment of all hits to domain 4 HMM searching. Domain 4 search area is highlighted in orange. All sequences were globally aligned using the MUSCLE program.

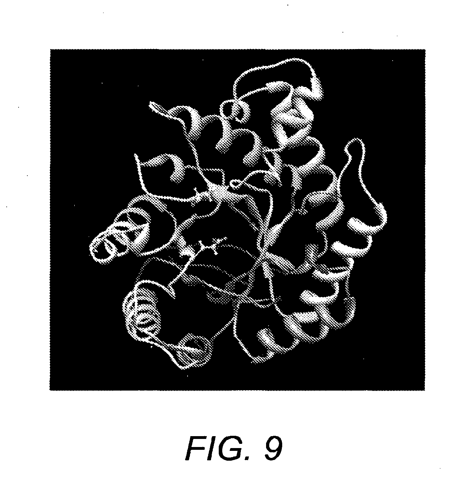

**FIG. 9** shows a homology structural model of EBI244 domain 2, constructed by the I-TASSER server, built from multiple GH5 domain structures in the PDB database, showing the common TIM-barrel architecture with 8 beta sheets inside 8 alpha-helices.

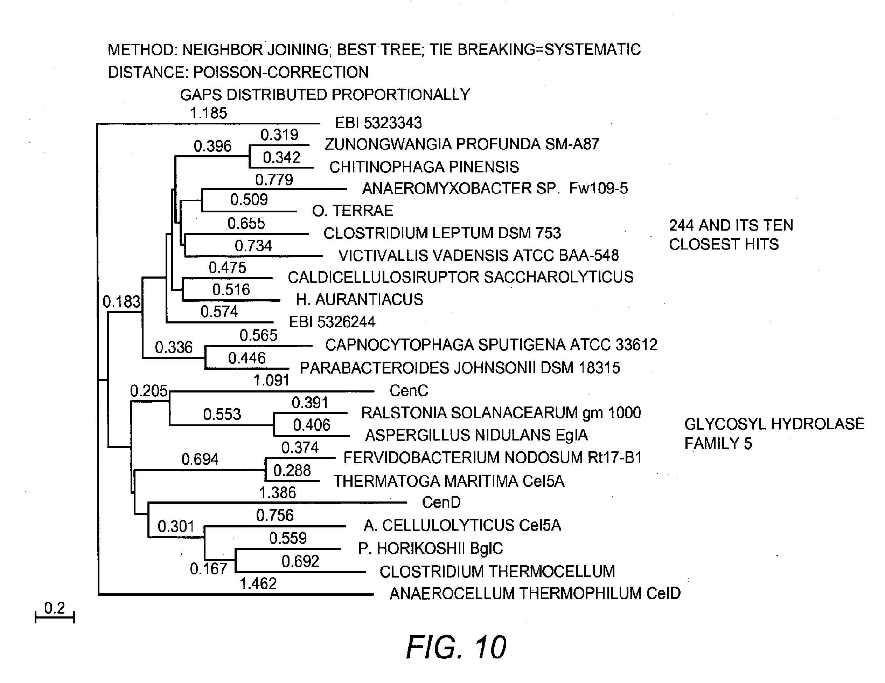

**FIG. 10** shows schematically a relationship of the glycolytic domain of EBI244 to known glycosyl hydrolase family 5 proteins.

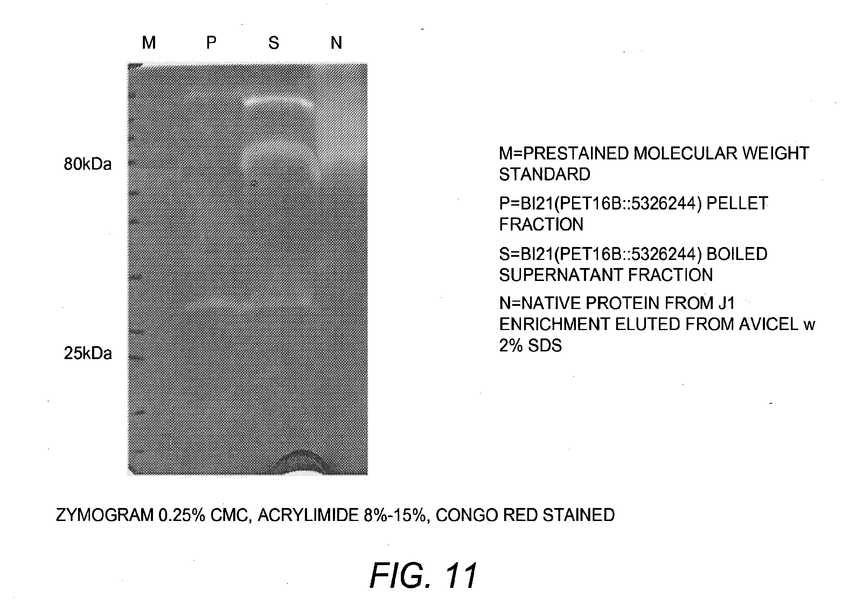

**FIG. 11** shows zymogram activity of recombinant protein fractions, compared to native protein fraction. M=prestained molecular weight standard; P=B121 (pet16b::5326244 (His-tagged EBI244 protein)), pellet fraction; S= B121 (pet16b::5326244 (His-tagged EBI244 protein)), boiled fraction; N=native protein from J1 enrichment eluted from Avicel^{®} with 2 % SDS. Cleared areas (white) represent activity, while dark areas represent intact carboxymethylcellulose. Recombinant protein fractions (P and S) were insoluble or soluble portions of the *E. coli* extract. Native fraction was eluted from Avicel^{®} with boiling SDS. The lower band represents an internal control, *E. coli* endoglucanase.

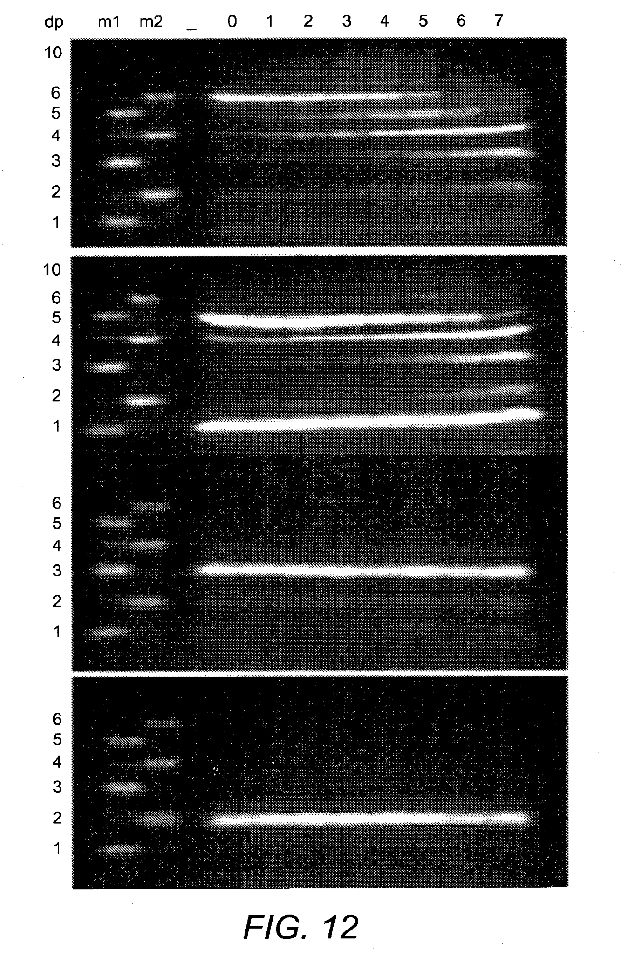

**FIG. 12** shows the Fluorophore Assisted Carbohydrate Electropheresis (FACE) results of time course of EBI244 on cellohexaose. Reaction condition was 10 µg enzyme, 0.33 mM cellohexaose in 25mM HEPPS ph 6.8, 95°C in 100 µL volume. The experiment tracked degree of polymerization (dp) over time. FIG. 12A depicts cellohexaose (0.33mM) substrate. FIG. 12B depicts cellopentaose (0.4 mM) and glucose (0.4 mM) substrates. FIG. 12C depicts cellotriose (0.67 mM) substrates. FIG. 12D depicts cellobiose (1 mM) substrate. Standards were a mixture of glucose, cellotriose and cellopentaose (ml) and mixture of cellobiose, cellotetraose and cellopentaose (m2). Time points (minutes, label) were (0,0), (1:20,1), (2:40, 2), (6:20, 3), (12:40, 4), (25:20, 5), (50:40, 6), (120:00, 7). Oligomers higher than cellohexaose up to dp ~11 were rapidly formed then degraded over time.

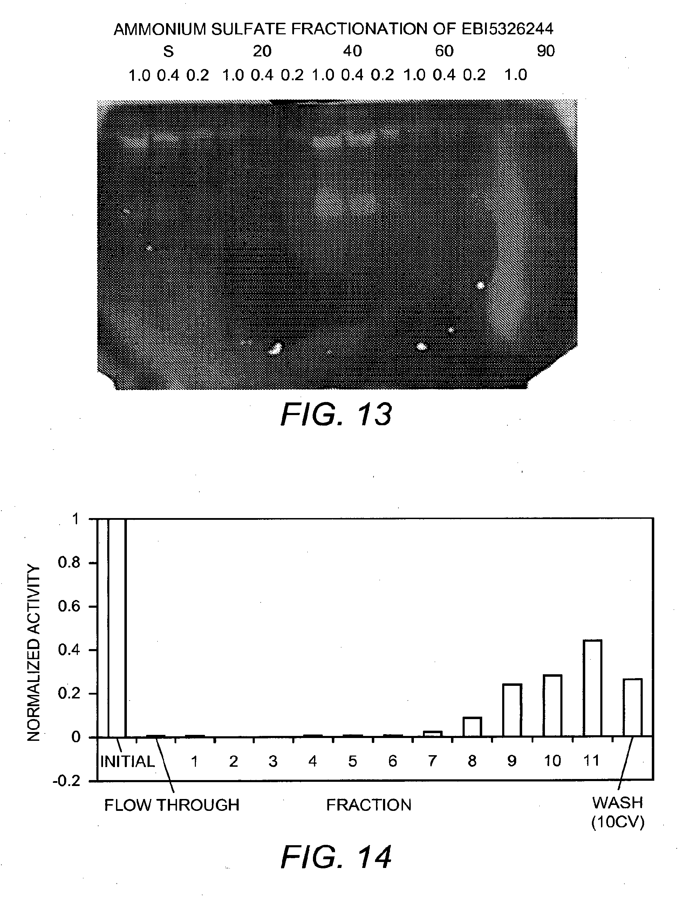

**FIG. 13** shows results of a zymogram assay, showing EBI244 activity distributed among 20-40% saturating ammonium sulfate fractions. Each fraction is represented by three lanes: undiluted (1.0), dilution 2 in 5 (0.4), and dilution 1 in 5 (0.2). Initial sample was soluble recombinant protein after pretreatment at 80 C for 30 minutes. Protein was precipitated using 20, 40, 60, and 90 % saturating ammonium sulfate.
**FIG. 14** shows a graph of endoglucanase activity, measured by DNS assay, with 1% low-viscosity carboxymethylcellulose as the substrate. Fractions 1-11 represent a linear gradient from 1 M to 0 M ammonium sulfate in potassium phosphate buffer, pH 7.0.

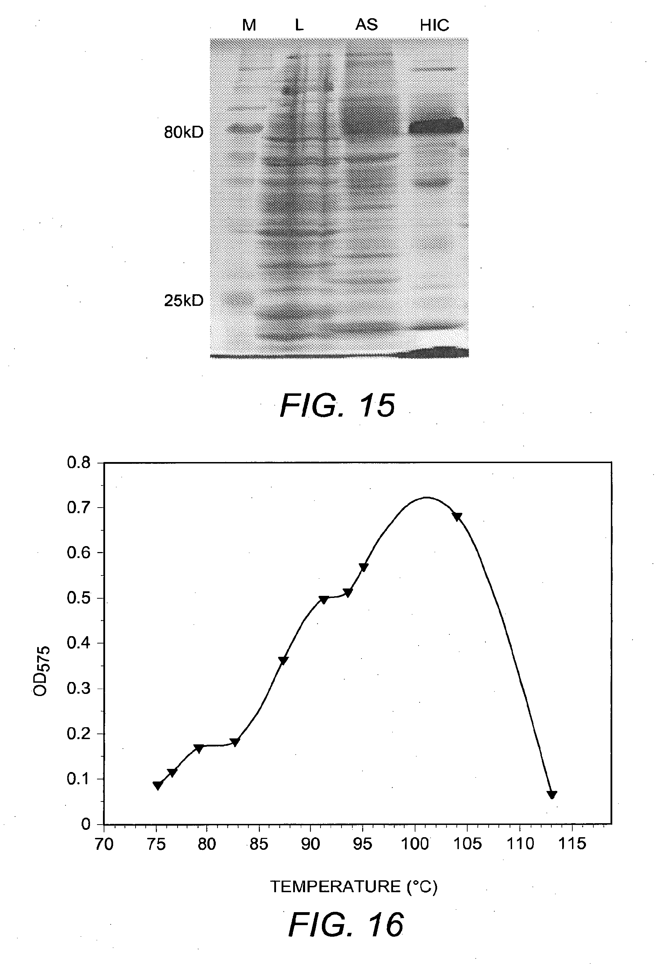

**FIG. 15** shows a picture of a comassie-stained SDS-PAGE gel, demonstrating stepwise purification of EBI244 to ~60% purity. M=marker; L=whole cell lysate; AS=20-40 % ammonium sulfate fraction; HIC=pooled active fraction, purified using Macro-Prep t-butyl hydrophobic interaction chromatography (HIC) support (methacrylate-based, 50 µm beads) (butyl HIC). The sample was heated to 80 °C prior to ammonium sulfate fractionation.
**FIG. 16** shows an activity-temperature profile of EBI244 on 1 % CMC (carboxymethyl cellulose) (DNS assay).

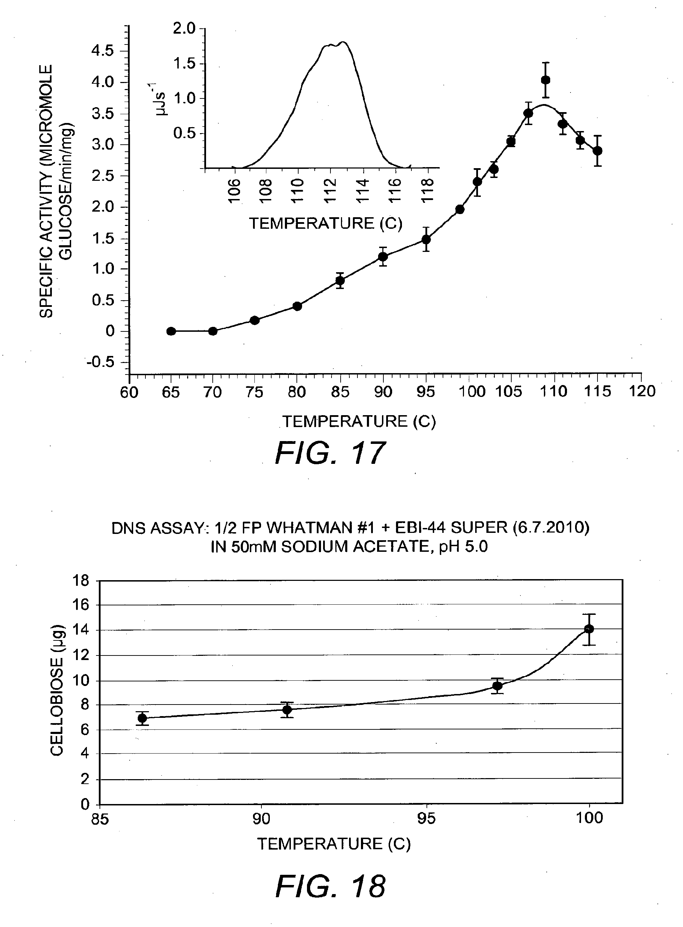

**FIG. 17** shows the temperature profile of EBI244. The temperature vs. activity profile was measured by 20-min assay in 1% CMC in 25 mM sodium acetate buffer, pH 6.0. The products were detected by DNS reducing sugar assay and normalized to a cellobiose standard. Error for this experiment was below 15%. Inset: Differential scanning calorimetry results of enzyme from 102-116°C. A dual Tm was observed at 111.5°C and 113°C.
**FIG. 18** shows results of a DNS assay using Whatman^{®} #1 filter paper in 10 mM Sodium Acetate pH 5.0 curve, demonstrating enzyme activity on filter paper over a range of temperatures.

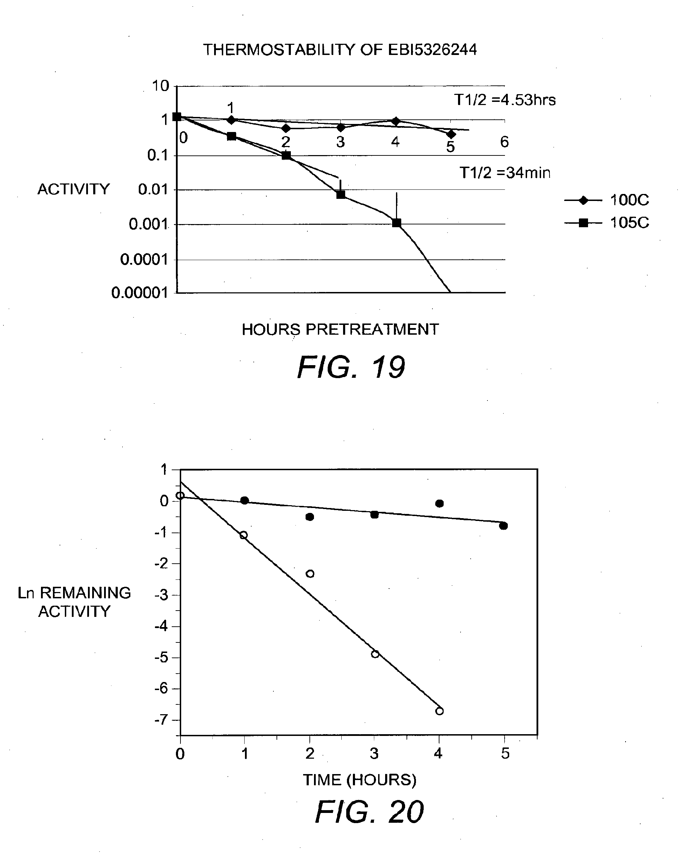

**FIG. 19** shows thermostability of EBI244 activity, preincubated at 100°C or 105°C in buffer, then assayed for activity on 1% CMC at 95°C.
**FIG. 20** shows the thermostability of EBI244 at 100°C (●) and 105°C (○) in 50 mM HEPPS buffer, pH 6.8. Data points represent the mean of four assays. Enzyme was incubated at the appropriate temperature, samples were collected at 1 hour intervals, and activity was measured using the DNS assay with cellobiose as a standard.

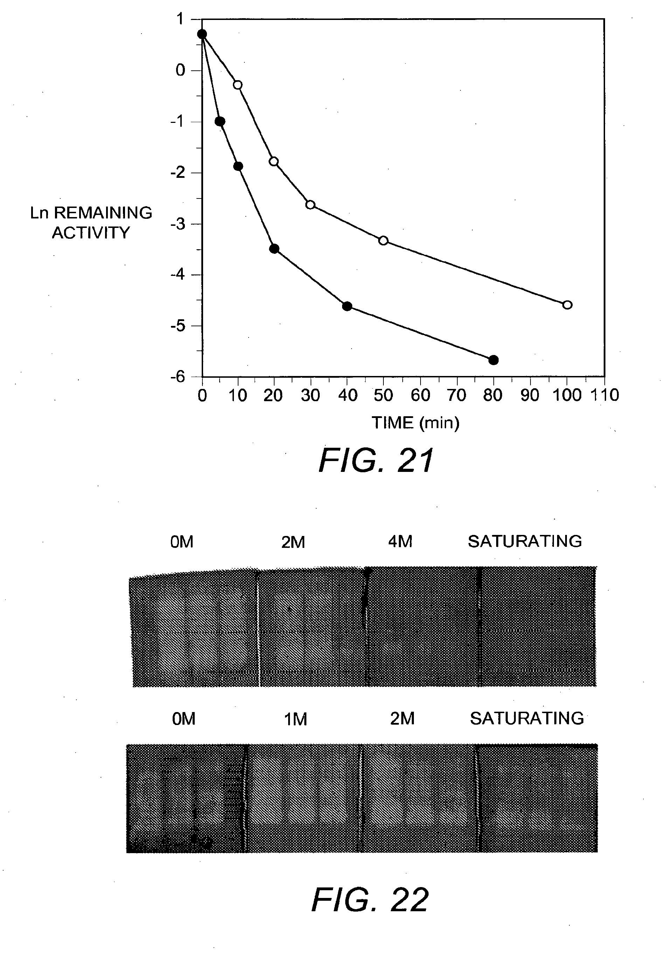

**FIG. 21** shows thermostability ofEBI244 at 108°C with (○) and without (●) 0.5% w/v Avicel^{®} in 25 mM sodium acetate buffer, pH 6.0. Enzyme was pretreated for 30 min at 90°C prior to incubation at 108°C to allow for interaction with the cellulose. Samples were removed at time intervals and activity was measured in triplicate using the DNS assay using cellobiose as a standard.
**FIG. 22** shows zymogram assay results following incubation of recombinant EBI244 enzyme at 90°C in phosphate buffer, at various salt concentrations. Upper panel: NaCl; lower panel: KCl.

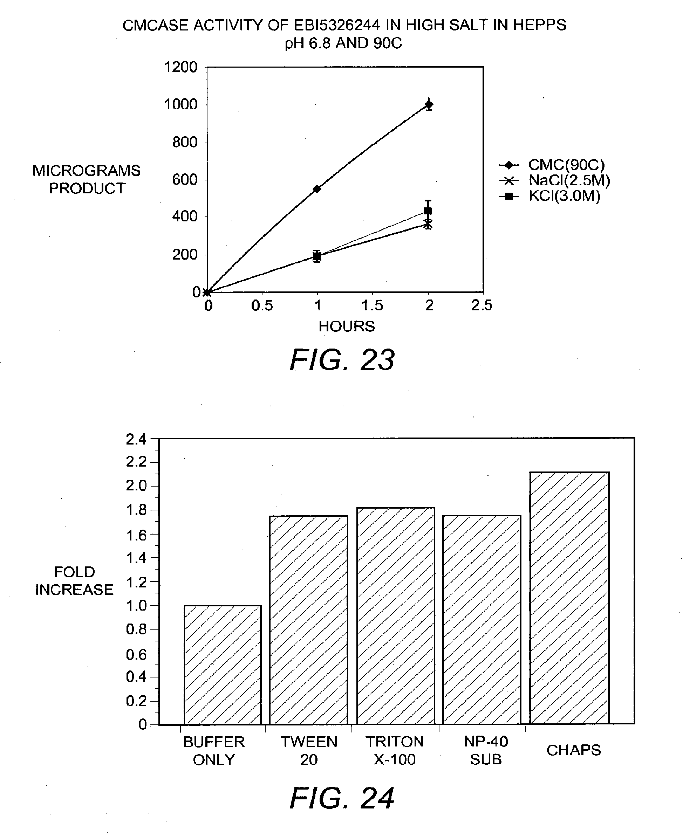

**FIG. 23** shows DNS assay results showing product formation for EBI244 with 1% CMC in HEPPS buffer with no added salt, 2.5 M NaCl, or 3.0 M KCl.
**FIG. 24** depicts activity of EBI244 against PNP-cellobioside at 95°C in the presence of various detergents. Conditions tested were 25 mM potassium phosphate buffer, pH 6.8 alone or buffer plus 0.1% of either Tween^{®} 20, Triton^{®} x-100, NP-40 substitute or CHAPS. After a 20 min incubation, sodium hydroxide was added to 50 mM and absorbance was measured at 410 nm. Values were calculated via paranitrophenol standard in the same buffer. Ratios were calculated based on activity in buffer alone.

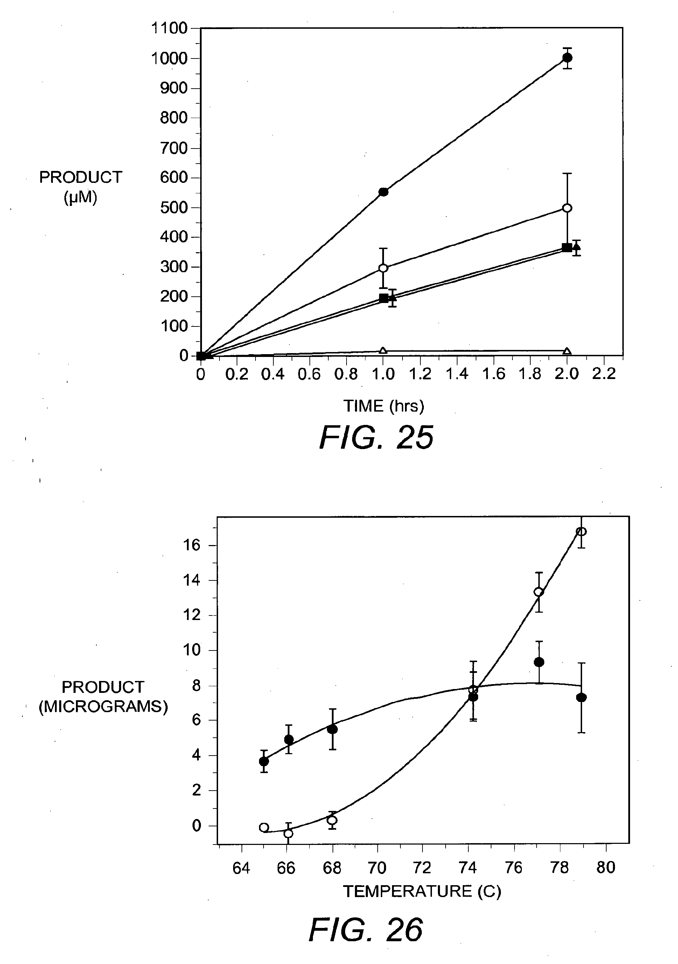

**FIG. 25** depicts a time course of EBI244 activity against 1% CMC while in the presence of salts or ionic liquids. All assays were done in HEPPS buffer, pH 6.8 at 90°C (shown with ●) and either 2.5 M sodium chloride (▲), 3.0 M potassium chloride (■), 25% (v/v) [DMM]DMP (○), or 25% (v/v) [EMM]Acetate (Δ). Activity was measured using DNS assay after each time point using cellobiose as a standard. Error bars represent the standard error of the mean of four assays.
**FIG. 26** depicts a temperature profiles showing CMC activity of EBI244 in 50% ionic liquid. Enzyme activity was measured in 50% (v/v) [DMIM]DMP in 25 mM phosphate, pH 6.8 (●) and 25 mM potassium phosphate buffer, pH 6.8 alone (○). Activity was measured using DNS assay after 2 hours using cellobiose as a standard.

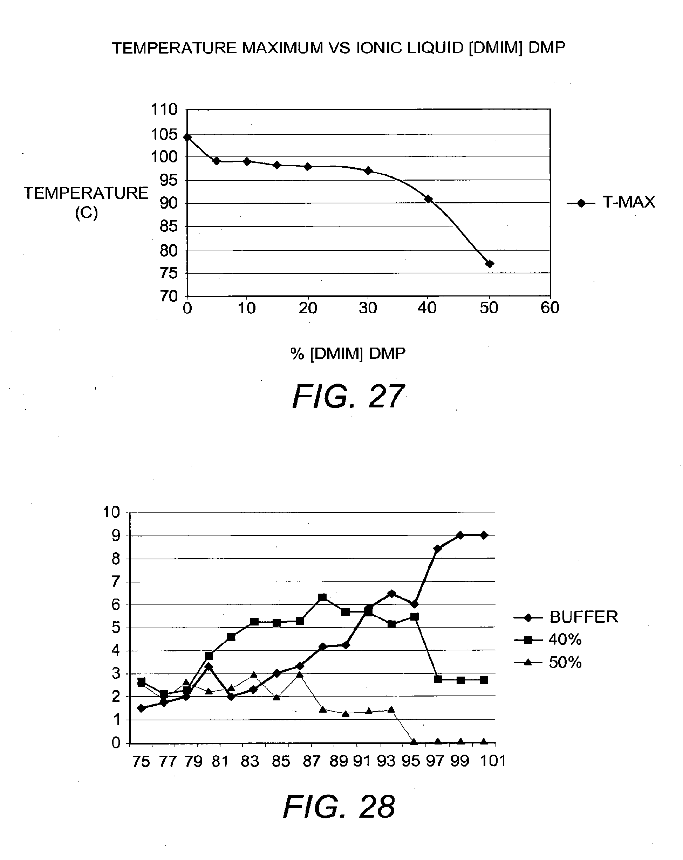

**FIG. 27** shows results of a DNS assay, representing temperature optima compiled from activity-temperature profiles of EBI244 in increasing amounts of the ionic liquid [DMIM]DMP.
**FIG. 28** shows results of a DNS assay, showing activity of EBI244 on 1% CMC in buffer alone, and in the presence of 40% and 50% [DMIM]DMP.

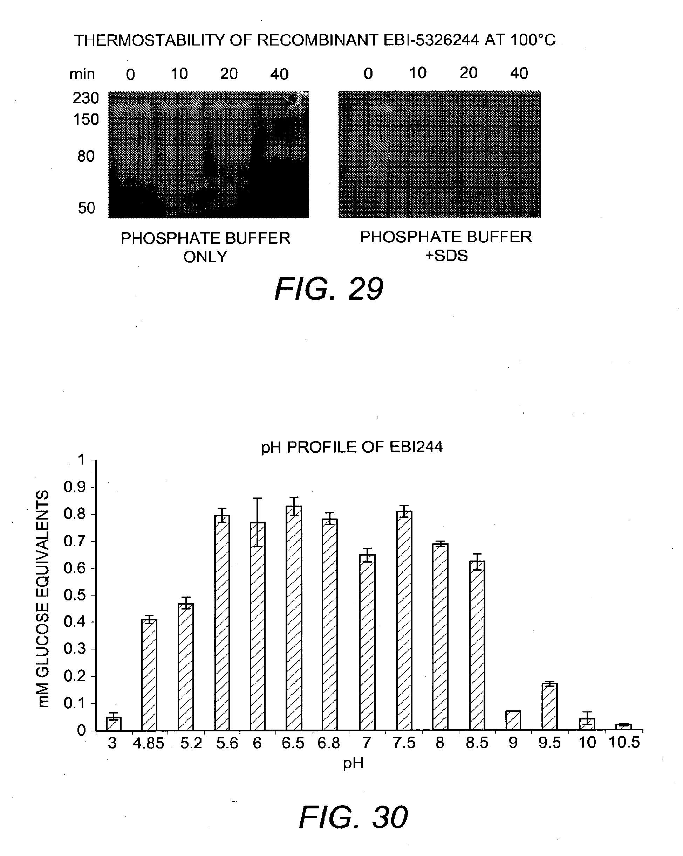

**FIG. 29** shows the results of a zymogram assay of EBI244, after pretreatment in phosphate buffer or phosphate buffer plus 0.1% sodium dodecyl sulfate at 100 C, demonstrating the thermostability of recombinant EBI244.
**FIG. 30** shows a pH-profile of EBI244 activity, based on DNS assays of CMC hydrolysis.
**FIG, 31** shows a pH profile of EBI244 activity measured against PNP-cellobioside at 95°C. Buffers used were sodium acetate/ acetic acid (pH 2.5-5.5), MED (pH 6.5), HEPPS (pH 7.5-8.5), and CAPS (pH 9.5-10.5). After 20 min incubation, sodium hydroxide to a final concentration of 50 mM and absorbance was measured at 410 nm. Values were calculated by a paranitrophenol standard in the same buffer. Error bars are standard deviations of the mean of four duplicate addays.

### Definitions

The term "catalytic activity" or "activity" describes quantitatively the conversion of a given substrate under defined reaction conditions. The term "residual activity" is defined as the ratio of the catalytic activity of the enzyme under a certain set of conditions to the catalytic activity under a different set of conditions. The term "specific activity" describes quantitatively the catalytic activity per amount of enzyme under defined reaction conditions.

The term "thermostability" describes the property of a protein to withstand a limited exposure to certain temperatures, such as high temperatures, without losing the activity it possesses at temperatures where its activity is measurable or is optimal. The term "thermoactive" describes a property of a protein which retains activity at high temperatures.

The term "pH-stability" describes the property of a protein to withstand a limited exposure to pH-values significantly deviating from the pH where its stability is optimal (e.g., more than one pH-unit above or below the pH-optimum, without losing its activity under conditions where its activity is measurable). The term "pH active" describes a property of a protein which retains activity at a pH value deviating significantly from pH values typically optimal for such activities.

The term "cellulase" refers to an enzyme (or enzymatic activity thereof) that catalyzes an enzymatic reaction in which cellulose is hydrolyzed into glucose, cellobiose, or cellooligotose, including enzymes having endoglucanase, exoglucanase, e.g., glucanohydrolase or cellobiohydrolase, β-Glucosidase or β-Glucosidaseglucohydrolase activity, and the corresponding enzymatic activity of such enzymes.

The term "lignocellulose" refers to any material primarily consisting of cellulose, hemicellulose, and lignin.

The term "hemicellulose" refers to a polymer of short, highly-branched chains of mostly five-carbon pentose sugars (e.g., xylose and arabinose) and to a lesser extent six-carbon hexose sugars (e.g., galactose, glucose and mannose).

The term "renewable resources" refers to biomass substrates that are grown and harvested, like crops, straw, wood and wood products. The term "biological fuels" refers to solid, liquid, or gas fuel including or derived from biomass, such as biodiesel, biogas, vegetable oil, bioethanol, and biohydrogen.

As used herein, when it is generally stated that a polypeptide or nucleic acid molecule or region thereof contains or has "identity" or "homology," per se (without specifying a particular percent identity), to another polypeptide or nucleic acid molecule or region thereof, the two molecules and/or regions share at least at or about 40%, and typically at least at or about 50 %, 60 % or 70 % sequence identity, such as at least at or about 60%, 65 %, 70%, 75 %, 80%, 85%, 90%, 95%, 96 %, 97 %, 98 %, 99 % or 100 % sequence identity. The precise percentage of identity can be specified.

Sequence "identity" has an art-recognized meaning. The percentage of sequence identity between two nucleic acid or polypeptide molecules and/or regions can be calculated using well-known and published techniques, such as those described below. In general, for determination of the percentage sequence identity, sequences are aligned so that the highest order match is obtained (see, *e.g.:* Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48:1073). For sequence identity, the number of conserved amino acids or nucleotides is determined by standard alignment algorithms programs, and can be used with default gap penalties established by each supplier. Substantially homologous nucleic acid molecules specifically hybridize typically at moderate stringency or at high stringency all along the length of the nucleic acid of interest.

The term "identity," when associated with a particular number, represents a comparison between the sequences of a first and a second polypeptide or polynucleotide or regions thereof. As used herein, the term at least "90% identical to" refers to percent identities from 90 to 99.99 of one nucleotide or amino acid sequence to the other. Identity of 90% or more is indicative of the fact that, assuming for exemplification purposes, the full length of a first and second polypeptide, each 100 amino acids in length, are compared, no more than 10% (*i*.*e*., 10 out of 100) of the amino acids in the first polypeptide differs from that of the second polypeptide. Similar comparisons can be made between first and second polynucleotides. Such differences among the first and second sequences can be represented as point mutations randomly distributed over the entire length of a polypeptide or they can be clustered in one or more locations of varying length up to the maximum allowable, e.g. 10/100 amino acid difference (approximately 90% identity). Differences are defined as nucleotide or amino acid residue substitutions, insertions, additions or deletions. At the level of homologies or identities above about 85-90%, the result should be independent of the program and gap parameters set; such high levels of identity can be assessed readily, often by manual alignment without relying on software.

Sequence identity can be measured along the full length of a polynucleotide or polypeptide or along a region thereof. Sequence identity compared along the full length of two polynucleotides or polypeptides refers to the percentage of identical nucleotide or amino acid residues along the full-length of the molecule. Alternatively, sequence identity can be compared along the length of a molecule, compared to a region of another molecule.

### DETAILED DESCRIPTION

Crystalline cellulose is composed of linear polymers of β1-4 linked glucose, held in the crystalline lattice by a high degree of intermolecular hydrogen bonding. The tightly crosslinked structure is primarily responsible for the inherent stability of cellulose, but also can hinder efficient deconstruction. The conversion of cellulose to glucose is generally accomplished by chemical hydrolysis (typically using a single step of acid treatment) or enzymatic hydrolysis (generally involving acid pretreatment followed by hydrolysis with cellulase enzymes). High temperatures combined with low pH are generally required for the disruption of the crystalline structure and chemical hydrolysis. *See* Kim JS, Lee YY, Torget, RW. (2001) "Cellulose hydrolysis under extremely low sulfuric acid and high-temperature conditions, Appl. Biochem. Biotechnol. 91-93. 331-340. Enzymatic hydrolysis generally occurs under milder conditions. Strategies for commercial depolymerization of cellulose typically combine pretreatment and enzymatic hydrolysis. Hilden L, Johansson G (2004), "Recent developments on cellulases and carbohydrate-binding modules with cellulose affinity," Biotechnol Lett, 26: 1683-1693. The degree of pretreatment required and the expense of subsequent cleanup steps required depend upon the properties of the enzymes that will be used.

### Embodiments

The present disclosure relates to isolated polypeptides, including cellulases and other polypeptides, for example, cellulases having endoglucanase, exoglucanase and/or β-Glucosidase or β-Glucosidaseglucohydrolase activity, activity, including those produced by archaea, such as an EBI244 polypeptide (SEQ ID NO: 1) and variants and fragments thereof. The present disclosure also relates to isolated polynucleotides encoding the polypeptides, as well as vectors and genetically modified host cells containing such isolated polynucleotides. The present disclosure further relates to compositions comprising the isolated polypeptides or enriched in such polypeptides. Moreover the present disclosure relates to methods for the identification and production of the polypeptides, and methods for their use in the degradation and hydrolysis of poly- and oligo-saccharides, such as biomass, e.g., hemicellulose, for example, in the conversion of biomass, such as lignocellulocytic biomass, including pretreated lignocellulocytic biomass, into soluble sugars, including for use in the fermentive production of biofuels, polishing of cotton fabrics, production of laundry detergents, production of polished crystalline cellulose, assays of cellulases, expansins, and cellulose binding proteins, and in pulping cellulolytic materials. Also provided herein are hyperthermophilic organisms and polypeptides encoded by the organisms, capable of utilizing crystalline cellulose, and methods for their identification and production.

### Polypeptides

The present disclosure relates to isolated polypeptides having cellulase activity and fragments thereof. In particular, the present disclosure provides polypeptides of SEQ ID NO: 1, SEQ ID NO: 5 (background art), SEQ ID NO: 16 (background art), and fragments and variants thereof. In some embodiments, the polypeptide includes a sequence having at least 70 %, 75 %, 80 %, 85 %, 90 %, 91 %, 92 %, 93 %, 94 %, 95 %, 96 %, 97 %, 98 %, 99 % or 100 % sequence identity to SEQ ID NO: 1, or to one or more regions or domains thereof, including amino acid residues 1-25 of SEQ ID NO: 1, amino acid residues 30-130 of SEQ ID NO: 1, amino acid residues 250 through 580 of SEQ ID NO: 1 (Domain 2), amino acids 130-250 of SEQ ID NO: 1 (domain 1), amino acids 750-842 of SEQ ID NO: 1 (Domain 4), or amino acids 580-750 proline-threonine rich region, Domain 1, Domain 2, Domain 3, or Domain 4 of SEQ ID NO: 1, where the polypeptide is a cellulase.

In some embodiments, the polypeptide is a variant or fragment of SEQ ID NO: 1, SEQ ID NO: 5 (background art), or SEQ ID NO: 16 (background art), with one or more amino acid deletions, insertions, modifications, or substitutions, such as a polypeptide having at least 70 %, 75 %, 80 %, 85 %, 90 %, 91 %, 92 %, 93 %, 94 %, 95 %, 96 %, 97 %, 98 %, 99 % or 100 % sequence identity to SEQ ID NO: 1, SEQ ID NO: 5 (background art), or SEQ ID NO: 16 (background art), or containing a domain, such as a catalytic domain or carbohydrate binding motif (CBM) that is at least 30 %, 40 %, 50 %, 60 %, typically at least 30%, 40 %, 50%, 60 %, 70 %, 75 %, 80 %, 85 %, 90 %, 91 %, 92 %, 93 %, 94 %, 95 %, 96 %, 97 %, 98 %, 99 % or 100 % identical to a domain thereof. Typically, the variant or fragment retains a substantial amount of the cellulase or other enzymatic activity or cellulose binding capability of the wild-type protein. For example, in some embodiments, the variant or fragment retains one, typically both, of the wild type active site residues at E413 and E506. In some embodiments (for purposes of background art), the variants include a protein comprising the sequence of a protein listed in any of Tables 1, 2, 3, and 4, such as a polypeptide having a sequence at least 30 %, 40 %, 50 %, 60 %, typically at least 70 %, 75 %, 80 %, 85 %, 90 %, 91 %, 92 %, 93 %, 94 %, 95 %, 96 %, 97 %, 98 %, 99 % or 100 % identical to SEQ ID NO: 6, 7, 8, 9, 10, 11, 12, or 13.

Whether any two nucleic acid or polypeptide molecules have sequences that contain, or contain at least, a certain percent (e.g. 60%, 70%, 80%, 85%, 90%, 95%, 96%, 97%, 98% or 99%) sequence identity can be determined using known computer algorithms such as the "FASTA" program, using for example, the default parameters as in Pearson et al. (1988) Proc. Natl. Acad. Sci. USA 85:2444 (other programs include the GCG program package (Devereux, J., et al., Nucleic Acids Research 12(I):387 (1984)), BLASTP, BLASTN, FASTA (Altschul, S.F., et al., J Molec Biol 215:403 (1990); Guide to Huge Computers, Martin J. Bishop, ed., Academic Press, San Diego, 1994, and Carrillo et al. (1988) SIAM J Applied Math 48:1073). For example, the BLAST function of the National Center for Biotechnology Information database can be used to determine identity. Other commercially or publicly available programs include, DNAStar "MegAlign" program (Madison, WI) and the University of Wisconsin Genetics Computer Group (UWG) "Gap" program (Madison WI)). The extent of sequence identity (homology) and complementarity may be determined using any computer program and associated parameters, including those described herein, such as BLAST 2.2.2. or FASTA version 3.0t78, with the default parameters. It is understood that for the purposes of determining sequence identity among DNA and RNA sequences thymidine nucleotide is equivalent to (represents identity with) a uracil nucleotide. Percent identity further can be determined, for example, by comparing sequence information using a GAP computer program (*e.g.,* Needleman et al. (1970) J. Mol. Biol. 48:443, as revised by Smith and Waterman ((1981) Adv. Appl. Math. 2:482). Briefly, the GAP program defines similarity as the number of aligned symbols (*i.e.,* nucleotides or amino acids), which are similar, divided by the total number of symbols in the shorter of the two sequences. Default parameters for the GAP program can include: (1) a unary comparison matrix (containing a value of 1 for identities and 0 for non-identities) and the weighted comparison matrix of Gribskov et al. (1986) Nucl. Acids Res. 14:6745, as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps. Various programs and methods for assessing identity are known to those of skill in the art. High levels of identity, such as 90% or 95% identity, readily can be determined without software.

In some embodiments, the polypeptides are produced recombinantly, while in others the polypeptides are produced synthetically, or are purified from a native source, such as an archaea, such as one described herein.

The provided polypeptides generally have cellulase activity, for example, endoglucanase, exoglucanase, *e.g*., glucanohydrolase or cellobiohydrolase, β-Glucosidase or β-Glucosidaseglucohydrolase activity, and/or cellulose binding ability. In one aspect, the provided polypeptides exhibit the cellulase activity or binding ability, for example, an activity or binding ability of at least 40 %, 50 %, 60 %, 70 %, 75 %, or more of maximum (or with a half-life of activity or binding ability of at least 10 minutes, 15 minutes, 20 minutes, 25 minutes, 30 minutes, 35 minutes, 40 minutes, 45 minutes, 50 minutes, 55 minutes, 1 hour, 1.25 hours, 1.5 hours, 1.75 hours, 2 hours, 3 hours, 4 hours, 5 hours, or more hours) over a broad range of conditions, for example, over range of conditions that is broader than that observed for one or more known cellulases, such as bacterial cellulases, including those produced by *Anaerocellum thermophilum, Caldicellulosiruptor saccharolyticus, Rhodothermus marinus,* or *Thermus caldophilus*. For example, in some aspects, the polypeptides exhibit activity or binding ability in the presence of high salt solution, such as in the presence of a saturating concentration of salt, such as in a solution containing sodium chloride (NaCl) at a concentration of at least at or about 0.5 M, 1 M, 1.5 M, 2 M, 2.5 M, 3 M, 3.5 M, or 4 M sodium chloride, or potassium chloride (KCl), at a concentration at or about 0.5 M, 1 M, 1.5 M, 2 M, 2.5 M 3.0 M or 3.2 M KCl and/or ionic liquids, such as 1,3-dimethylimidazolium dimethyl phosphate ([DMIM]DMP) or [EMIM]OAc, or in the presence of one or more detergents, such as ionic detergents (e.g., SDS, CHAPS), sulfhydryl reagents, such as in saturating ammonium sulfate or ammonium sulfate between at or about 0 and 1 M.

In some aspects, the polypeptides exhibit the activity or binding ability at high temperatures, such as a temperature exceeding 105°C, 106°C, 107°C, 108°C, 109°C, or 110°C, or over a broad temperature range, such as between 95°C and 110°C. In some aspects, the polypeptides exhibit the activity or binding ability over a broad pH range, for example, at a pH of between about 4.5 and 8.75, at a pH of greater than 7 or at a pH of 8.5, or at a pH of at least 5.0, 5.5, 6.0, 6.5, 7.0, 7.5, 83.0, or 8.5.

Cellulase activity and binding capabilities can be measured by a number of well-known methods, including zymograms, reducing sugar assays (*e.g*., DNS Micro or Macro, Nelson-Somogyi Micro or Macro, Nelson Semi-Micro, Ferricyanide-1, Ferricyanide-2, PAHBAH Micro or Macro, BCA, and Modified BCA), assays using paranitrophenol-labeled glycosides, product analysis, total sugar assays, such as Phenol-H₂SO₄ or Anthrone H₂SO₄, enzymatic glucose assays, and cellulose binding assays, for example, using the methods described herein.

Substrates for cellulase activity and binding assays include soluble and insoluble substrates. Soluble substrates include, for example, cellodextrins and their derivatives, including radiolabelled versions thereof, short chain cellulase, β-methylumbelliferyl-oligosaccharides, p-nitrophenol-oligosaccharides, Long chain cellulose derivatives, Carboxymethyl cellulose (CMC), hydroxyethyl cellulose (HEC), dyed CMC. Insoluble substrates, include, for example, cotton, Whatman No. 1 filter paper, pulp (e.g., Solka Floc), crystalline cellulose, such as cotton, microcrystalline cellulose (*e.g*., Avicel^{®}), valonia cellulose, bacterial cellulose, amorphous cellulose (*e.g*., PASC, alkali-swollen cellulose), dyed cellulose, fluorescent cellulose, chromogenic and fluorephoric derivatives, such as trinitrophenyl-carboxymethylcellulose (TNP-CMC) and fluram-cellulose, practical cellulose-containing substrates, α-cellulose, and pretreated lignocellulosic biomass.

In some embodiments, the polypeptides are produced as N- and/or C-terminal fusion proteins, for example to aid in extraction, detection and/or purification and/or to add functional properties to the cellulases. Examples of fusion protein partners include, but are not limited to, glutathione-S-transferase (GST), 6XHis, GAL4 (DNA binding and/or transcriptional activation domains), FLAG-, MYC-tags or other tags well known to anyone skilled in the art. In some embodiments, a proteolytic cleavage site is provided between the fusion protein partner and the protein sequence of interest to allow removal of fusion protein sequences. Preferably, the fusion protein does not hinder the cellulase activity of the polypeptides.

In some embodiments, the polypeptide is fused to one or more domains, for example, of other proteins, such as other cellulases or sugar-reducing enzyme, including a bacterial, archaeal, and/or hyperthermophilic cellulase or enzyme, for example, cellulases and enzymes belonging to glycosyl hydrolase family GH5 or GH12 or CBM family 1 or 2, such as those encoded by mesophiles, such as *B. fibrisolvens,* and cellulases encoded by thermophiles such as *S. solfataricus, R. marinus, A. cellulolyticus, P. furiosus, P. horikoshii, P. abyssi*, or *A*. *cellulolyticus*, *S. lividans, B. fibrisolvens,* or *T. reesei.*

Such domains can include a leader peptide, propeptide, binding domain and/or catalytic domain. Suitable binding domains include, but are not limited to, carbohydrate-binding domains (e.g., CBM) of various specificities, providing increased affinity to carbohydrate components present during the application of the cellulase. Suitable enzymatically active domains possess an activity that supports the action of the polypeptide in producing the desired product. Non-limiting examples of catalytic domains include: cellulases, hemicellulases such as xylanase, mannanases, exo-mannanases, glucanases, arabinases, galactosidases, pectinases, and/or other activities such as proteases, lipases, acid phosphatases and/or others or functional fragments thereof.

In some embodiments, the fusion proteins contain the catalytic or enzymatically active domain of another cellulase or sugar-reducing enzyme, such as fused to one or more domains of the provided polypeptides, such as a CMB domain, for example, to Domain 1, Domain 4, or Domain 3 of SEQ ID NO: 1 or a variant thereof. In another embodiment, the fusion protein contains a catalytic domain of one of the provided peptides, such as a domain having a certain percent identity to domain 2 of SEQ ID NO: 1, amino acid residues 250 through 580. Typically, the fusion proteins exhibit improved stability, cellulase activity, tolerance for various conditions, and/or cellulose binding compared to the other enzyme, e.g., cellulase, alone.

For example, the tight binding of the provided polypeptides to crystalline cellulose as described herein, makes it useful in methods for identifying and producing new hyperstable cellulases. In one embodiment, the hyperstable cellulases are produced using well-known engineering methods, which have been used to engineer thermophilic and hyperthermophilic cellulases to improve the activity on crystalline substrates. In one example, the methods involve the addition of a thermostable cellulose binding domain provided herein to a catalytic domain, for example, as carried out to introduce chitin binding domains to increase binding and activity toward crystalline cellulose.

Domains of the fusion proteins are optionally linked to the polypeptides through a linker sequence that simply joins the provided cellulose polypeptide or fragment thereof and the fusion domain without significantly affecting the properties of either component, or the linker optionally has a functional importance for the intended application.

In some embodiments, the provided polypeptides are used in conjunction with one or more additional proteins of interest. Non-limiting examples of proteins of interest include: hemicellulases, alpha-galactosidases, beta-galactosidases, lactases, beta-glucanases, endo-beta-1,4-glucanases, cellulases, xylosidases, xylanases, xyloglucanases, xylan acetyl-esterases, galactanases, exo-mannanases, pectinases, pectin lyases, pectinesterases, mannanases, polygalacturonases, arabinases, rhamnogalacturonases, laccases, reductases, oxidases, phenoloxidases, ligninases, proteases, amylases, phosphatases, lipolytic enzymes, cutinases and/or other enzymes.

### Polynucleotides

Also provided are isolated and/or purified nucleic acid molecules, e.g., polynucleotides, encoding the provided polypeptides, e.g., cellulases. In some embodiments, the isolated polynucleotide encodes SEQ ID NO: 1, SEQ ID NO: 5 (background art), SEQ ID NO: 15 (background art), or a fragment or variant thereof, such as fragments thereof including amino acid residues 1-25 of SEQ ID NO: 1, amino acid residues 30-130 of SEQ ID NO: 1, amino acid residues 250 through 580 of SEQ ID NO: 1 (Domain 2), amino acids 130-250 of SEQ ID NO: 1 (domain 1), amino acids 750-842 of SEQ ID NO: 1 (Domain 4), or amino acids 580-750 proline-threonine rich region, Domain 1, Domain 2, Domain 3, or Domain 4 of SEQ ID NO: 1, or containing a sequence of SEQ ID NO: 1, or a sequence that is at least 70 %, 75 %, 80 %, 85 %, 90 %, 91 %, 92 %, 93 %, 94 %, 95 %, 96 %, 97 %, 98 %, 99 % or 100 % identical to such a protein or region thereof. For example, provided are polynucleotides encoding polypeptides containing a domain of the provided polypeptide, such as a catalytic domain or carbohydrate binding motif (CBM) that is at least 70 %, 75 %, 80 %, 85 %, 90 %, 91 %, 92 %, 93 %, 94 %, 95 %, 96 %, 97 %, 98 %, 99 % or 100 % identical to such a domain thereof, where the encoded polypeptide is a cellulase. In one embodiment (for purposes of background art), provided are polynucleotides containing a nucleic acid sequence having at least 50 %, 60 %, typically at least 70 %, 75 %, 80 %, 85 %, 90 %, 91 %, 92 %, 93 %, 94 %, 95 %, 96 %, 97 %, 98 %, 99 % or 100 % sequence identity to SEQ ID NO: 3, SEQ ID NO: 4, SEQ ID NO: 15, or to one or more regions or domains thereof. Also provided are polynucleotides encoding the polypeptides listed in Tables 1-4.

Typically, the variant or fragment encodes a protein retaining a substantial amount of the cellulase or other enzymatic activity or cellulose binding capability of the wild-type protein. For example, in some embodiments, the variant or fragment retains one, typically both, of the wild type active site residues at E413 and E506. In some embodiments, the variants include polypeptides encoding a protein comprising the sequence of a protein listed in any of Tables 1, 2, 3, and 4.

### Methods for the identification of hyperthermophiles and hyperthermophilic cellulases and characterization assays

There is an absence of known archaeal hyperthermophiles subsisting on plant biomass as exclusive carbon sources. Despite the discovery of multiple endo and exocellulases in thermophiles, the upper temperature limit for organisms known to grow on crystalline cellulose has risen slowly. The compositions provided here are based in part on the discovery that a known method for identifying thermophilic cellulases is the isolate-centric nature of these studies. Thus, provided are high throughput metagenomic, transcriptomic, and proteomic methods for identification of cellulases, including for the identification of hyperthermophilic cellulases. For example, provided is a metagenomic approach for identification of cellulases, such as stable and thermoactive endoglucanase from a lignocellulose-degrading consortium of hyperthermophilic Archaea.

In one embodiment, such methods are carried out by cultivating archaea growing on a cellulose-containing carbon source, such as crystalline cellulose, at above a certain temperature, such as at or about at least 90°C, 94°C, or 100°C, and selection of organisms capable of utilizing cellulose under these conditions. In one aspect, the method allows for selection of a minimal consortium, rather than a single isolates. An exemplary method is the isolation described herein in Example 1.

Also provided are methods for identifying and producing new hyperstable cellulases by mutating known enzymes to include one or more domains, such as the cellulose binding domain, for example, any of domains 1, 3, and/or 4, of the provided polypeptides, for example, to improve the activity on crystalline substrates. In one example, the methods involve the addition of a thermostable cellulose binding domain provided herein to a catalytic domain, for example, as carried out to introduce chitin binding domains to increase binding and activity toward crystalline cellulose.

Also provided are methods using the provide polypeptides for the characterization of cellulose degradation and production of polished crystalline cellulose for assays of cellulases, expansins, and cellulose binding proteins.

### Vectors and Host Cells

Also provided are vectors, host cells, and methods for the production of the provided polypeptides and polynucleotides. In some embodiments, DNA encoding the polypeptide is chemically synthesized based on the provided sequences or obtained directly from host cells harboring the gene (e.g., by cDNA library screening or PCR amplification). In some embodiments, the provided polynucleotide is included in an expression cassette and/or cloned into a suitable expression vector by standard molecular cloning techniques. Such expression cassettes or vectors contain sequences that assist initiation and termination of transcription (e.g., promoters and terminators), and generally contain a selectable marker.

Expression vector/host cell combinations are well known and can be used in the provided methods. Typically, the expression cassette or vector is introduced in a suitable expression host cell, which then expresses the corresponding polypeptide. Particularly suitable expression hosts are bacterial expression host genera including *Escherichia (e.g., Escherichia coli), Pseudomonas (e.g., P. fluorescens or P. stutzerei), Proteus (e.g., Proteus mirabilis), Ralstonia (e.g., Ralstonia eutropha), Streptomyces, Staphylococcus (e.g., S. carnosus), Lactococcus (e.g., L. lactis), or Bacillus (subtilis, megaterium, licheniformis, etc.).* Also particularly suitable are yeast expression hosts such as *Saccharomyces cerevisiae, Schizosaccharomyces pombe, Yarrowia lipolytica, Hansenula polymorpha, Kluyveromyces lactis or Pichia pastoris*, and fungal expression hosts such as *Aspergillus niger, Chrysosporium lucknowense, Aspergillus (e.g., A. oryzae, A. niger, A. nidulans, etc.)* or *Trichoderma reesei*. Also suited are mammalian expression hosts such as mouse (e.g., NS0), Chinese Hamster Ovary (CHO) or Baby Hamster Kidney (BHK) cell lines. Other eukaryotic hosts such as insect cells or viral expression systems (e.g., bacteriophages such as M13, T7 phage or Lambda, or viruses such as Baculovirus) are also suitable.

Promoters and/or signal sequences associated with secreted proteins in a particular host of interest are candidates for use in the heterologous production and secretion of the provided polypeptides in that host or in other hosts. Such sequences are well known. In some embodiments, the provided polynucleotide is recombinantly associated with a polynucleotide encoding a suitable homologous or heterologous signal sequence that leads to secretion of the enzyme into the extracellular (or periplasmic) space, thereby allowing direct detection of enzyme activity in the cell supernatant (or periplasmic space or lysate). Particularly suitable signal sequences for *Escherichia coli,* other Gram negative bacteria and other organisms known in the art include those that drive expression of the HlyA, DsbA, Pbp, PhoA, PelB, OmpA, OmpT or M13 phage Gill genes. For *Bacillus subtilis,* Gram-positive organisms and other organisms known in the art, particularly suitable signal sequences further include those that drive expression of the AprE, NprB, Mpr, AmyA, AmyE, Blac, SacB, and for *S. cerevisiae* or other yeast, include the killer toxin, Bar1, Suc2, Mating factor alpha, InulA or Ggplp signal sequence. Signal sequences can be cleaved by a number of signal peptidases, thus removing them from the rest of the expressed protein. In some embodiments, the provided polypeptide is expressed alone or as a fusion with other peptides, tags or proteins located at the N- or C-terminus (e.g., 6XHis, HA or FLAG tags). Suitable fusions include tags, peptides or proteins that facilitate affinity purification or detection (e.g., 6XHis, HA, chitin binding protein, thioredoxin or FLAG tags), as well as those that facilitate expression, secretion or processing of the provided polypeptide. Suitable processing sites include enterokinase, STE13, Kex2 or other protease cleavage sites for cleavage in vivo or in vitro.

In some embodiments, the provided polynucleotides are introduced into expression host cells by any of a number of transformation methods including, but not limited to, electroporation, lipid-assisted transformation or transfection ("lipofection"), chemically mediated transfection (e.g., CaCl and/or CaP), lithium acetate-mediated transformation (e.g., of host-cell protoplasts), biolistic "gene gun" transformation, PEG-mediated transformation (e.g., of host-cell protoplasts), protoplast fusion (e.g., using bacterial or eukaryotic protoplasts), liposome-mediated transformation, *Agrobacterium tumefaciens,* adenovirus or other viral or phage transformation or transduction.

Alternatively, the polypeptides are expressed intracellularly. Optionally, after intracellular expression of the polypeptides, or secretion into the periplasmic space using signal sequences such as those mentioned above, a permeabilisation or lysis step can be used to release the cellulase into the supernatant. The disruption of the membrane barrier is effected by the use of mechanical means such as ultrasonic waves, pressure treatment (French press), cavitation or the use of membrane-digesting enzymes such as lysozyme or enzyme mixtures. As a further alternative, the polynucleotides encoding the polypeptides are expressed by use of a suitable cell-free expression system. In cell-free systems, the polynucleotide of interest is typically transcribed with the assistance of a promoter, but ligation to form a circular expression vector is optional. In other embodiments, RNA is exogenously added or generated without transcription and translated in cell free systems.

### Reduction of the Viscosity of Pretreated Biomass Mixtures (for background art purposes)

The provided polypeptides and compositions containing the polypeptides find use in a variety of industrial applications, including in the reduction of the viscosity of pretreated biomass mixtures prior to their degradation into monosaccharides and oligosaccharides, for example, in biofuel production.

Biomass that is used for as a feedstock, for example, in biofuel production generally contains high levels of lignin, which can block hydrolysis of the cellulosic component of the biomass. Typically, biomass is pretreated with, for example, high temperature and/or high pressure to increase the accessibility of the cellulosic component to hydrolysis. However, pretreatment generally results in a biomass mixture that is highly viscous. The high viscosity of the pretreated biomass mixture can also interfere with effective hydrolysis of the pretreated biomass. Advantageously, the polypeptides and compositions of the present disclosure can be used to reduce the viscosity of pretreated biomass mixtures prior to further degradation of the biomass.

Accordingly, certain embodiments of the present disclosure relate to methods of reducing the viscosity of a pretreated biomass mixture, by contacting a pretreated biomass mixture having an initial viscosity with any of the polypeptides or compositions of the present disclosure; and incubating the contacted biomass mixture under conditions sufficient to reduce the initial viscosity of the pretreated biomass mixture.

In some embodiments, the disclosed methods are carried out as part of a pretreatment process. The pretreatment process may include the additional step of adding any of the polypeptides or compositions of the present disclosure to pretreated biomass mixtures after the step of pretreating the biomass under high temperature, and incubating the pretreated biomass with the polypeptides or compositions under conditions sufficient to reduce the viscosity of the mixture. The polypeptides or compositions may be added to the pretreated biomass mixture while the temperature of the mixture is high, or after the temperature of the mixture has decreased. In some embodiments, the methods are carried out in the same vessel or container where the heat pretreatment was performed. In other embodiments, the methods are carried out in a separate vessel or container where the heat pretreatment was performed.

In some embodiments, the methods are carried out in the presence of high salt, such as solutions containing saturating concentrations of salts, solutions containing sodium chloride (NaCl) at a concentration of at least at or about 0.5 M, 1 M, 1.5 M, 2 M, 2.5 M, 3 M, 3.5 M, or 4 M sodium chloride, or potassium chloride (KCl), at a concentration at or about 0.5 M, 1 M, 1.5 M, 2 M, 2.5 M 3.0 M or 3.2 M KCl and/or ionic liquids, such as 1,3-dimethylimidazolium dimethyl phosphate ([DMIM]DMP) or [EMIM]OAc, or in the presence of one or more detergents, such as ionic detergents (e.g., SDS, CHAPS), sulfydryl reagents, such as in saturating ammonium sulfate or ammonium sulfate between at or about 0 and 1 M. In other embodiments, the polypeptides or compositions of the present disclosure are contacted with the pretreated biomass mixture at a temperature exceeding 90°C, 91°C, 92°C, 93°C, 94°C, 95°C, 96°C, 97°C, 98°C, 99°C, 100°C, 101 °C, 102°C, 103°C, 104°C, 105°C, 106°C, 107°C, 108°C, 109°C, or 110°C, or over a broad temperature range, such as between at or about 60°C and 110°C or between 65°C and 110°C, such as between 90°C and 110°C, between 65°C and 70° C, between 85°C and 105°C, between 85°C and 110°C, between 95°C and 105°C, or between 95°C and 110°C. In some aspects, the polypeptides exhibit the activity or binding ability over a broad pH range, for example, at a pH of between about 4.5 and 8.75, at a pH of greater than 7 or at a pH of 8.5, or at a pH of at least 5.0, 5.5, 6.0, 6.5, 7.0, 7.5, 83.0, or 8.5.

Biomass includes, but is not limited to, plant material, municipal solid waste, and wastepaper, including lignocellulosic feedstocks, *e*.*g*., agricultural residues such as corn stover, wheat straw, barley straw, oat straw, rice straw, canola straw, and soybean stover, grasses such as switch grass, miscanthus, cord grass, and reed canary grass, fiber process residues such as corn fiber, beet pulp, pulp mill fines and rejects and sugar cane bagasse, forestry wastes such as aspen wood, other hardwoods, softwood and sawdust, and post-consumer waste paper products; palm kernel, coconut, konjac, locust bean gum, gum guar, soy beans. Suitable crop residue for production of biomass includes but is not limited to palm kernel meal, palm kernel expellers, copra meal, copra pellets and soy bean hulls.

### Degradation of Biomass to Mono- and Oligosaccharides

The polypeptides, polynucleotides, vectors, and host cells of the present disclosure find use in a variety of industrial applications, including in the degradation of biomass, e.g., cellulase and lignocellulose, into monosaccharides and oligosaccharides, for example, in biofuel production, textile methods, including cleaning, cotton softening, and denim finishing, in production and uses of detergents, for example, for color care, cleaning, and anti-deposition; for food-based methods, including food processing and mashing; for pulp and paper methods, such as paper pulp bleaching, deinking, drainage improvement, and fiber modification. Thus, also provided are methods and uses of the provided polypeptides, polynucleotides, and compositions for such purposes, for example, in degrading or hydrolyzing cellulose-containing compositions to produce soluble sugars, for example, followed by enzymatic or chemical fermentation.

In some embodiments, the methods are carried out in the presence of high salt, such as solutions containing saturating concentrations of salts, solutions containing sodium chloride (NaCl) at a concentration of at least at or about 0.5 M, 1 M, 1.5 M, 2 M, 2.5 M, 3 M, 3.5 M, or 4 M sodium chloride, or potassium chloride (KCl), at a concentration at or about 0.5 M, 1 M, 1.5 M, 2 M, 2.5 M 3.0 M or 3.2 M KCl and/or ionic liquids, such as 1,3-dimethylimidazolium dimethyl phosphate ([DMIM]DMP) or [EMIM]OAc, or in the presence of one or more detergents, such as ionic detergents (e.g., SDS, CHAPS), sulfydryl reagents, such as in saturating ammonium sulfate or ammonium sulfate between at or about 0 and 1 M. In some embodiments, the conversion occurs at a temperature exceeding 105°C, 106°C, 107°C, 108°C, 109°C, or 110°C, or over a broad temperature range, such as between 95°C and 110°C. In some aspects, the polypeptides exhibit the activity or binding ability over a broad pH range, for example, at a pH of between about 4.5 and 8.75, at a pH of greater than 7 or at a pH of 8.5, or at a pH of at least 5.0, 5.5, 6.0, 6.5, 7.0, 7.5, 83.0, or 8.5.

Bioenergy feedstocks consist primarily of the plant cell wall components cellulose and hemicellulose. Hydrolysis of these polysaccharides to their monomeric sugars involves a set of enzymes acting synergistically to cleave the different chemical linkages (Dodd and Cann, GCB Bioenergy, 1:2, 2009). Cellulose is the predominant polysaccharide in biomass (with others including hemicellulose, lignin, and pectin). Cellulose is a homopolymer of anhydrocellobiose (a linear beta-(1-4)-D-glucan), and includes glucose units linked together in β-1,4-glycosidic linkages. The hemicellulosic component can vary in chemical composition. Hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains.

The provided polypeptides may be used to degrade various types of cellulosic biomass, which are well-known in the art, including plant biomass, microbial biomass, purified cellulose, and lignocellulosic feedstocks. Cellulosic biomass includes lignocellulose biomass, containing cellulose, hemicellulose, and lignin. Purified celluloses include holocellulases, such as Solka Flok, microcrystalline celluloses, such as Avicel^{®} and Sigmacell^{®}, and the highly soluble cellulose ether, carboxymethylcellulose (CMC). Cellulose-containing substrates include soluble and substrates, such as cellodextrins and their derivatives, short chain cellulase, β-methylumbelliferyl-oligosaccharides, p-nitrophenol-oligosaccharides, long chain cellulose derivatives, carboxymethyl cellulose (CMC), hydroxyethyl cellulose (HEC), and insoluble substrates, including cotton, Whatman No. 1 filter paper, Pulp (e.g., Solka Floc), Crystalline cellulose, such as cotton, microcrystalline cellulose (*e.g.,* Avicel^{®}), Valonia cellulose, bacterial cellulose, Amorphous cellulose (*e.g.,* PASC, alkali-swollen cellulose), dyed cellulose, fluorescent cellulose, chromogenic and fluorephoric derivatives, such as trinitrophenyl-carboxymethylcellulose (TNP-CMC) and Fluram-cellulose, practical cellulose-containing substrates, α-cellulose, and pretreated lignocellulosic biomass.

### Biofuel Production (for purposes of background art)

The provided polypeptides and compositions containing the polypeptides find use in the degradation and hydrolysis of cellulase and cellulase-containing biomass and feedstocks, for example, for the production of monosaccharides, disaccharides, and oligosaccharides from biomass, such as chemical or fermentation feedstocks, for the production of biofuel, such as ethanol, butanol, other products, and intermediates. Provided are methods and compositions for such uses of the provided polypeptides, such as conversion of lignocellulocytic biomass into soluble sugars for fermentative production of biofuels, conversion of pretreated lignocelluose into soluble sugars, conversion of lignocellulose into soluble sugars in the presence of high salt or ionic liquids, conversion of crystalline cellulose into soluble sugars at high temperatures, such as those exceeding 90°C, 91°C, 92°C, 93°C, 94°C, 95°C, 96°C, 97°C, 98°C, 99°C, 100 °C, 101°C, 102°C, 103°C, 104°C, 105°C, 106°C, 107°C, 108°C, 109°C, 110°C, or over a broad temperature range, such as between at or about 60°C and 110 °C or between 65°C and 110°C, such as between 90°C and 110°C, between 65°C and 70°C, between 85°C and 105°C, between 85°C and 1110°C, between 95°C and 105°C, or between 95°C and 110°C, or under other conditions as described herein above.

In one embodiment, the provided composition includes the peptide in a composition of crude fermentation broth, with or without the cells removed, or in the form of a semi-purified or purified enzyme preparation. In another embodiment, the provided host cells are used as a source of the polypeptide in a fermentation process with the biomass.

In one embodiment, the polypeptides of the present disclosure find use in the degradation of cellulose to aid in the degradation of biomass, to form biofuels, such as ethanol. Ethanol is produced by enzymatic degradation of biomass and conversion of the released saccharides to ethanol (often referred to as bioethanol or biofuel, used as a fuel additive or extender in blends of from less than 1% and up to 100% (a fuel substitute)). In one embodiment, for the production of biofuels from biomass, the provided polypeptides, compositions, and methods are used in the conversion of cellulose to its monomer (glucose) or other soluble sugar, for subsequent conversion to biofuel (e.g., ethanol) by fermentation, such as by microbial or chemical fermentation. For example, the provided polypeptides and methods may be used for such conversion by enzymatic hydrolysis, optionally including acid pretreatment, typically carried out at high temperatures, followed by hydrolysis with the provided polypeptides.

In one embodiment, the polypeptides are used in combination with other carbohydrases (e.g., mannanases, glucanase, xylanase, alpha-galactosidase and/or cellulase) for more extensive hydrolysis of the plant material.

### Food Processing(for purposes of background art)

Compositions comprising the polypeptides of the present disclosure also find use in the processing and manufacturing of food or animal feed, such as in mashing. Provided are methods employing the provided compositions in such uses. Several anti-nutritional factors limit the use of specific plant material in the preparation of animal feed and food for humans. Plant material containing oligosaccharides can reduce the digestibility and absorption of nutritional compounds such as minerals, vitamins, sugars and fats by the animals. Provided are methods for food processing using the provided compositions. In one embodiment, the polypeptides and compositions are used to degrade or hydrolyze polymers into simpler sugars, which can be more readily assimilated to provide additional energy.

Polypeptides of the present disclosure also are useful as additives to feed for monogastric animals such as poultry and swine, as well as for human food. In some embodiments, the polypeptides are used to pretreat the feed instead of as a feed additive. In some embodiments, the polypeptides are added to or used to pretreat feed for weanling pigs, nursery pigs, piglets, fattening pigs, growing pigs, finishing pigs, laying hens, broiler chicks, turkeys, for example, added to or used to pretreat feed from plant material such as palm kernel, coconut, konjac, locust bean gum, gum guar, soy beans, barley, oats, flax, wheat, corn, linseed, citrus pulp, cottonseed, groundnut, rapeseed, sunflower, peas, and lupines.

Because of their stability, *e*.*g*., thermostability, they find used in processes of producing pelleted feed in which heat is applied to the feed mixture before the pelleting step, as it is the case in most commercial pellet mills. In one example, the polypeptides are added to the other feed ingredients in advance of the pelleting step or after the pelleting step to the already formed feed pellets.

In some embodiments, the provided compositions containing the provided polypeptide for use in food processing or as a feed supplement contain other substituents, such as coloring agents, aroma compounds, stabilizers, vitamins, minerals, other feed or food enhancing enzymes and the like. This applies in particular to the so-called pre-mixes. Food additives according to this present disclosure may be combined with other food components to produce processed food products. The resulting, combined food additive is mixed in an appropriate amount with other food components such as cereal or plant proteins to form a processed food product.

### Textile Cleaning and laundry detergents

The provided polypeptides, methods, and compositions also find use in textile methods, including cleaning, cotton softening, and denim finishing, the polishing of cotton fabrics under high temperature treatments, and in production and uses of detergents, for example, for color care, cleaning, and anti-deposition. For example, the provided polypeptides find use in detergent compositions to facilitate the removal of cellulose-containing stains and soils. In one embodiment, the polypeptides are used in detergent compositions; provided are such detergent compositions and methods for their use. In one embodiment, the detergent compositions contain the polypeptides in combination with other enzymes from the group of amylases, mannases, cellulases, lipases, pectinases, proteases, endoglucanases, and exoglucanases.

The detergent compositions include those in any convenient form, including in a bar, a tablet, a powder, a granule, a paste or a liquid. A liquid detergent is generally aqueous, typically containing up to 70% water and 0-30% organic solvent(s), or non-aqueous component(s). Typically, the detergent composition comprises one or more surfactants (e.g., non-ionic including semi-polar, anionic, cationic and/or zwitterionic). The surfactants are typically present at a level of from 0.1 % to 60% by weight. When included, detergents typically contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid, or soap. When included, detergents typically contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine (glucamides).

Detergent compositions optionally comprise 0-65% of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic add, soluble silicates, or layered silicates. Detergent compositions optionally comprise one or more polymers such as carboxymethylcellulose (CMC), poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers, and lauryl methacrylate/acrylic acid copolymers. The detergent optionally comprises a bleaching system (e.g., hydrogen peroxide source) such as perborate or percarbonate, which may be combined with a peracid-forming bleach activator such as tetraacetylethylenediamine or nonanoyloxybenzenesulfonate. Alternatively, the bleaching system comprise peroxyacids of the amide, imide, or sulfone type.

In one embodiment, the provided polypeptides are added to the detergent composition in an amount corresponding to 0.01-100 mg of enzyme protein per liter of wash liquor, preferably 0.05-5 mg of enzyme protein per liter of wash liquor, in particular 0.1-1 mg of enzyme protein per liter of wash liquor.

### Paper Pulp processes

In another embodiment, the provided compositions and polypeptides find use in pulp and paper methods, such as in paper pulp bleaching, deinking, drainage improvement, and fiber modification, for example, in high temperature applications for the pulping of cellulolytic materials. Provided are methods and compositions for use of the provided polypeptides for such purposes. For example, in some embodiments, the polypeptides find use in the enzyme aided bleaching of paper pulps such as chemical pulps, semi-chemical pulps, kraft pulps, mechanical pulps or pulps prepared by the sulfite method. In some embodiments, the pulps are chlorine free pulps bleached with oxygen, ozone, peroxide or peroxyacids. In some embodiments, the provided polypeptides are used in enzyme aided bleaching of pulps produced by modified or continuous pulping methods that exhibit low lignin contents. In some embodiments, the provided polypeptides are applied alone; in other embodiments, they are provided in combination with other enzymes, such as xylanase and/or endoglucanase and/or alpha-galactosidase and/or cellobiohydrolase enzymes.

The following examples are offered to illustrate provided embodiments and are not intended to limit the scope of the invention.

### EXAMPLES

The following examples describe the results of a metagenomic approach to identify extremely stable and thermoactive endoglucanases from a lignocellulose-degrading consortium of hyperthermophilic Archaea, including the endoglucanase EBI244, with a capacity to tightly bind microcrystalline cellulose (Avicel^{®} PH-101).

### Example 1: Enrichment of hyperthermophilic Archaea and metagenomic sequencing

Hyperthermophilic Archaea were enriched on pulverized plant biomass (microcrystalline cellulose). For this process, a sample of sediment collected from a continental volcanic hot spring at 94°C and neutral pH was selectively enriched to obtain a consortium of hyperthermophilic Archaea growing on lignocellulose as sole carbon source. A secondary minimal enrichment of three hyperthermophilic Archaea was isolated on minimal salts medium containing microcrystalline cellulose (Avicel^{®}) as the major carbon source.

### Source material

Sediment was sampled from great boiling springs near Gerlach Nevada, from a pool having a temperature of 94°C, known to maintain temperatures around 90°C (FIG. 1A). A small glass jar (4 oz) was filled with sediment, topped off with spring water, closed, and sealed with Parafilm^{®} M. Samples were transported on ice; long-term storage was carried out in anaerobic jars at 4°C.

### Enrichment of hyperthermophilic Archaea

Approximately 3 mL of sediment was used as inoculum to generate an anaerobic microbial enrichment on minimal salts medium (90 mL). The medium was similar to DSMZ medium #516 (ANAEROCELLUM MEDIUM), except that pulverized lignocellulosic feedstock *Miscanthus gigas,* ground to 80uM particle size, was used as the carbon source feedstock, and yeast extract was reduced to 0.2 g/L. Specifically, the medium contained NH4Cl (0.33 g), KH2PO4 (0.33 g), KCl (0.33 g), MgCl2 x 6 H2O (0.33 g), CaCl2 x 2 H2O (0.33 g), Trace element solution (Nitrilotriacetic acid 1.500 g, MgSO4 x 7 H2O 3.000 g, MnSO4 x H2O 0.500 g, NaCl 1.000 g, FeSO4 x 7 H2O 0.100 g, CoSO4 x 7 H2O 0.180 g, CaCl2 x 2 H2O 0.100 g, ZnSO4 x 7 H2O 0.180 g, CuSO4 x 5 H2O 0.010 g, KAl(SO4)2 x 12 H2O 0.020 g, H3BO3 0.010 g, Na2MoO4 x 2 H2O 0.010 g, NiCl2 x 6 H2O 0.025 g, Na2SeO3 x 5 H2O 0.300 mg, Distilled water 1000.000 ml, made by first dissolving nitrilotriacetic acid and adjusting pH to 6.5 with KOH, then adding minerals, adjusting pH to 7.0 with KOH), Distilled water 1000.000 ml) (1.00 ml), Yeast extract (0.2 g), Resazurin (0.50 mg), Vitamin solution (Biotin 2.000 mg, Folic acid 2.000 mg, Pyridoxine-HCl 10.000 mg, Thiamine-HCl x 2 H2O 5.000 mg, Riboflavin 5.000 mg, Nicotinic acid 5.000 mg, D-Ca-pantothenate 5.000 mg, Vitamin B12 0.100 mg, p-Aminobenzoic acid 5.000 mg, Lipoic acid 5.000 mg) (10.00 ml), NaHCO3 (1.50 g), pulverized lignocellulosic feedstock Miscanthus gigas, ground to 80uM particle size for use as the carbon source (5.00 g), Na2S x 9 H2O 0.50 g, Distilled water 1000.00 ml, with ingredients (except vitamins, bicarbonate, cellobiose and sulfide) dissolved, boiled for 1 min., then cooled to room temperature under 80% N2 and 20% CO2 gas atmosphere, adding vitamins, feedstock solutions and bicarbonate from a sterile stock solution, prior to inoculation, adjusted to a pH of 7.1 - 7.3.

After incubation for three weeks at 90°C, a secondary enrichment was performed by innoculating with microcrystalline cellulose, with ~50 µm particle size (Avicel^{®} pH101 Fluka, Ireland), as the carbon source. The minimal enrichment obtained on microcrystalline cellulose (Avicel^{®}) was transferred to the same salts medium described above, with Whatman^{®} #3 (Qualitative Grade 3) Filter Paper as a carbon source, (FIG. 1B and 1C). Enrichment on Avicel^{®} was chosen for scaled up production of the consortium because this finely divided crystalline substrate resulted in more rapid growth.

This enrichment strategy yielded a three-organism consortium, capable of deconstructing crystalline filter paper at 90°C, as demonstrated by pitting, shredding or complete dissolution of strips of Whatman^{®} #1 (Qualitative Grade 1) or Whatman^{®} # 3 (Qualitative Grade 3) filter paper (FIG. 2). Specifically, the consortium degraded a strip of Whatman #1 filter paper supported by glass tubing, a circular piece of Whatman^{®} #3 filter paper (confirmed by visible pits). Pits were more often seen with the thicker Whatman^{®} #3 filter paper (FIG. 2B), while shredding/dissolution was more often seen with the thinner Whatman^{®} #1 filter paper (FIG. 2A).

Repeated efforts to separate the three species of the consortium failed.

### Extraction, purification, and analysis of native protein

Avicel^{®} from a 17.5L enrichment, grown on Avicel^{®} PH 101 in a 20 L specialized fermentor, was washed and extracted with CHAPS detergent and SDS as follows. The enrichment was harvested by centrifugation and the pellet, principally Avicel^{®}, was washed 3 times with Tris buffer (100mM sodium chloride and 0.05% Tween^{®} 20) to remove soluble proteins. The remaining pellet was washed with 0.6% CHAPS detergent in TE (Tris-EDTA) buffer, then twice with 2% CHAPS in TE buffer, 20 minutes each, at 90°C, then boiled in 1% SDS for 20 minutes, and in 2% SDS for 20 minutes. The 1% SDS and 2% SDS fractions contained proteins determined to have been transferred to Avicel^{®} during growth, and tightly bound to partly digested cellulose fibrils.

### Preliminary assay of endoglucanase function using zymograms

Zymograms were used as a preliminary assay to screen the fractions for endoglucanase activity. As shown in FIG. 3, Zymograms performed on the protein extractions from the Avicel^{®} enrichment demonstrated detectable activity in a split band at apparent molecular weights ranging from 80 to 250 kDa for the 2% CHAPS fractions. As shown in FIG. 3, subsequent washes with 1-2% SDS yielded the most activity, localized in a small number of distinct protein bands.

The 1% CHAPS/5% cellobiose fraction showed detectable CMCase activity on zymograms. Active cellulases with apparent molecular weights of about 40 kDa and 80 kDa were detected (FIG. 4). Subsequent washes with 1% SDS at 100°C yielded the release of additional hyperstable, high molecular weight enzymes with CMCase activity as indicated by the activity in a smaller number of more distinct bands with apparent molecular weights of about 80 kDa and 180 kDa (FIG. 4). It was apparent that this consortium was producing cellulases that could bind to Avicel^{®} particles, and were able to withstand boiling in 1% SDS, abilities not yet observed in well-characterized cellulases from hyperthermophilic archaea. Therefore, metagenomics was employed to identify potential cellulases from this consortium.

### Extraction of high molecular weight DNA from Avicel^{®} enrichment

Standard protocols were used to extract high molecular weight DNA from the Avicel^{®} enrichment using the CTAB method (Ausubel et al., Current Protocols in Molecular Biology. Vol. 2 (John Wiley & Sons Inc., 1994) with volumes increased 4-fold. Using this method, approximately 20 µg of high molecular weight DNA was obtained from a 1.5 L enrichment grown on 5 g Avicel^{®}/L. The average size of the DNA was determined by pulsed-field electrophoresis to be about 50 kDa.

### Sequencing and sequence analysis

Metagenomic analysis was performed on the minimal enrichment identified multiple endoglucanase homologs in the metagenome.

Metagenomic sequencing was performed on DNA from the consortium. Library preparation and sequencing was performed at the University of Illinois, W. M. Keck Center for Comparative and Functional Genomics. Sequencing was done via Roche 454 Titanium Shotgun Sequencing. Initial automated assembly was by done at the Center by Newbler Assembly program (Newbler Assembler software, 454 Sequencing / Roche). Automated annotation was done using a local MANATEE database and the nr BLAST database, available through NCBI. In addition, further annotation was conducted through the MicrobesOnline Comparative Genomics Database (VIMSS funded by DOE Genomics:GTL), which includes protein coding prediction using CRITICA and Glimmer3, followed by annotation using the VIMSS genome pipeline composed of all publicly available sequence databases.

The consortium of three Archaea contained a dominant organism related to *Ignisphaera aggregans,* but sufficiently distinct to be assigned to a different genus, as well as two Archaea related to *Pyrobaculum islandicum* and *Thermofilum pendens.* The major organism is designated *Pyrosphaera cellulolytica* Candidatus Nov Gen Nov Sp (*P. cellulolytic*). The incomplete genome of this hyperthermophilic Archaeon shares several features of the genome of *I. aggregans,* including a pair of homologous but somewhat distantly related genes encoding reverse gyrase. The genome of *P*. *cellulolytica* indicates that the strain is specialized for heterotrophic utilization of a variety of carbohydrates. The draft genome has significant coding capacity for glycolytic enzymes including putative endo and exocellulases, glucosidases and hemicellulases.

Metagenomic sequencing yielded 1,283,902 reads, with a total of 497,707,575 bases. Assembly yielded 4206 contigs representing 6,954,058 bases. One complete 16S RNA and two fragmented 16S RNAs were identified, which matched most closely to characterized organisms *Ignisphaera aggregans* DSM 17320 (95%), *Pyrobaculum islandicum* DSM 4184 (98%), and *Thermofilum pendens* Hrk (93%), respectively. A maximum likelihood 16S rRNA gene phylogenetic tree is shown in FIG. 5.

Proteomics analysis was done by tandem mass spectrometry conducted at the California Institute for Quantitative Biosciences Proteomics/Mass Spectrometry Core Facility. Briefly, gel slices were prepared by vortexing with 25mM ammonium bicarbonate 1:1 acetonitrile/water for 10min and discarding the supernatant. This step was repeated three times. Slices were vacuum-dried, then reduced by incubation with 10mM DTT in 25mM ammonium bicarbonate with 10% acetonitrile and alkylated with 55mM iodoacetamide in 25mM ammonium bicarbonate. Proteins were then digested with one volume of trypsin for 6h at 37°C. After digestion, the slices were washed with water and the supernatant saved. Gel slices were then washed twice with a solution of 45% water, 50% acetonitrile, and 5% formic acid; all supernatants were saved. Supernatants containing the peptides were reduced to a volume of 10µL and then analyzed with tandem mass spectrometry. Peptide sequences were annotated using the annotated genome created by MicrobesOnline.

Similar topology and bootstrap supported was obtained for the Neighbor-joining method (results not shown) The 16S rRNA gene from the Ignisphaera-like organism was 99% identical to 16S rRNA clones from uncultured archaea from geothermal systems in both Nevada (accession number HM448083.1) and Montana (accession number EU635921.1). The *Ignisphaera*-like 16S RNA was 94% identical to the type species and represented the dominant organism in the enrichment, based on large number of reads per kilobase of sequence (~300) for 16S RNA and the hyperthermophilic housekeeping gene reverse gyrase, compared to read densities (<20) for 16 RNA fragments and reverse gyrases from the other organisms. Like *Ignisphaera aggregans,* the *Ignisphaera*-like organism appeared to have two reverse gyrase genes, as shown in FIG. 6. The sum of the high read density contigs represented about 1.8 Mb, or most of the expected coding sequence of a single hyperthermophile (~2.0 Mb). Sequence analysis found a large number a glycosyl hydrolases (>40) and 21 contigs containing potential cellulases, based on automated annotation.

### Example 2: Identification of carbohydrate active enzymes

Annotation analysis found a large number of GHs (37) and included 4 potential GH family 5 endoglucanases, based on automated annotation. Twelve of these GHs were encoded by the closed genome of the dominant strain. One predicted GH, designated EBI244 (accession number JF509452), was chosen for further study because it was a potential multi-domain cellulase, 842 amino acids in length, and a member of the TIM barrel glycosyl hydrolase superfamily (β/α)8. Large multidomain cellulases are ubiquitous amongst cellulolytic organisms but have not been previously found in hyperthermophilic archaea. The central domain of this enzyme (AA250-580) had a Pfam match (E-value 1X e⁻¹²) to the GH family 5 (GH5). The gene encoding EBI244 was found on the chromosome of the dominant organism and at 94 kDa EBI244 was the largest of three proteins encoded on the chromosome with Pfam hits to GH family 5 (GH5); the others were a 43 kDa Pfam match (E-value 6.3 E⁻⁶⁷) and a 44 kDa Pfam match (E-value 8 E⁻⁵²).

Potential homologs were gathered with PSI-BLAST (Johnson, M et al., Nucleic Acids Res. 36, W5-9, 2008) using each putative domain of EBI244 as the query sequence against the nr protein sequence database. The SAM software package (Karplus et al., Bioinformatics 14, 846-856, 1998) was used to build hidden Markov models (HMM's), score the potential homolog sequences, and create alignments for building new models. This method was used iteratively with each putative domain to build more general models in order to detect distant homologs. Jalview (Waterhouse, A.M. et al., Bioinformatics 25, 1189-1191, 2009) was used to view and edit multiple sequence alignments. The resulting alignments allowed for approximate domain boundary determination.

According to BLASTp searches EBI244 is a weak match to its closest apparent homolog, an uncharacterized hypothetical protein from *Caldicellulosiruptor saccharolyticus* (35% identity). The conserved central domain (AA250-580) had only 9 significant hits (NCBI nonredundant protein database) with BLAST E-values less then 1E-20, including proteins from *Herpetosiphon aurantiacus* ATCC 23779, *Spirochaeta thermophila* DSM 6578, *Spirochaeta thermophila* DSM 6192, *Opitutus terrae* PB90-1, *Chitinophaga pinensis* DSM 2588, *Zunongwangia profunda* SM-A87, *Clostridium leptum* DSM 753, *Victivallis vadensis* ATCC BAA-548; with % identities ranging from 25-35%.

### Example 3: Analysis of a hyperthermophilic cellulase-encoding gene (ebi244) and polypeptide encoded thereby (EBI244 Protein)

Based on sequencing and analysis, one gene and polypeptide encoded thereby were chosen for further study, based on the gene's homology to the cellulase superfamily/ glycosylhydrolase family 5/ EC 3.2.1.4. The gene/protein was designated *ebi244*/EBI244. The EBI244 protein had apparent but distant similarity to type 5 glycosyl hydrolases (cellulase superfamily). The gene mapped to a high-read density contig embedded in a sequence flanked by other assembled genes. The contig did not display synteny or detectable homology to the draft genome sequence of *I*. *aggregans* web site genome.ornl.gov/microbial/iag17230/.

Sequence analysis revealed that *ebi244* was a putative cellulase-encoding gene, isolated from a hyperthermophilic archaeal consortium metagenome, having no global identity to any previously characterized protein or enzyme. The predicted open reading frame (ORF) encodes a protein having a deduced sequence 842 amino acids in length, set forth as SEQ ID NO: 1. The recombinant forms generally add a terminal methionine (Met) bringing the total to 843 amino acids (SEQ ID NO:14.) Achea proteins sometimes start with amino acids other than Met, such as leucine (Leu).

Sequence comparison revealed that the protein contained no close global identity to any previously characterized protein or enzyme. A central region of the protein (Domain 2) showed similarity to the known glycosyl-hydrolase family 5 (GH5) domain, present in a family of glycosyl hydrolases, which was evidence of cellulase or similar sugar hydrolase activity. Aside from this glycosyl hydrolase domain, none of the remainder of the amino acid sequence shows any similarity to any known domain or protein in the major databases.

Phylogenetic analysis of EBI244 was carried out using the sequence of domain 2 (GH5 match) in order to determine its evolutionary relationship to characterized enzymes (FIG. 7). The phylogenetic tree was built using the SATCHMO-JS server (Hagopian, R et al., Nucleic Acids Res. 38, W29-34, 2010). All sequences were aligned with the Expresso server (Armougom, F. et al., Nucleic Acids Res. 34, W604-608, 2006) in order to trim sequences down to only the structurally related GH domain. All characterized GH family 5 and GH family 42 sequences in the CAZy database (Cantarel, B. et al., Nucleic Acids Res. 37, D233-238, 2009) were used initially to compare to EBI244 and its closest homologs. The size of the tree was reduced by using Jalview's remove-redundancy function, thereby also preserving the diversity of each family. The Pfam web server (Finn, RD et al., Nucleic Acids Res. 38, D211-222, 2010) was used to score the sequences against Pfam HMM models of the GH families.

The catalytic domain of EBI244 clustered with a unique subset of TIM barrel sequences that show distant relationships to both GH families 5 and 42 in the calculated phylogenetic tree. In this analysis, three members of Family 30 formed a distant out-group although they are assigned to the Clan A structural clade that includes the families GH5 and GH12. EBI244 clusters with three characterized mannanases that have been classified in the GH5 family. The eight closest homologs of the EBI244 catalytic domain include six that have a GH Pfam match (five from GH5, one from GH42), and two with no predictive matches (E-values shown in FIG. 7). Given this uncertain association, the unique architecture, and the diversity of the GH5 family, it is unclear whether the sequence cluster containing the EBI244 catalytic domain is a divergent subfamily of the GH5 family or the nucleus of a new family of glycoside hydrolases.

### EBI244 Domain Architecture:

Protein database searches and bioinformatic server predictors indicated that EBI244 contains four structural domains, one unstructured region, and an N-terminal signal or lipid-anchor sequence. The domains and regions are shown schematically in FIG. 8A, with approximate amino acid positions indicated for each.

### N-terminal sequence:

The analysis revealed that the first approximately 25 amino acids of the native EBI244 enzyme are highly hydrophobic and likely represent a signal peptide (for directing protein localization with eventual cleavage) or membrane/lipid anchor (to hold the protein on the cell surface). While signal sequence and transmembrane (TM) region prediction servers are not built with archaeal sequences, they can be useful for some guidance. Thus, various servers were used to analyze this region of EBI244, given mixed results, with some predicting a TM-region (e.g. Phobius: TM region a.a. 6-25. TMHMM: TM region a.a. 5-27), some predicting a signal peptide (e.g. SignalP 3.0: predicted cleavage between a.a. 22 and 23), and others giving inconclusive predictions (e.g. SIG-Pred: Eukaryote predicted signal sequence with cleavage between a.a. 18 and 19, but no prokaryotic signal sequence predicted).

Given the varied results using server predictions, further studies were carried out to identify similar N-terminal protein regions among genes found in the metagenome (FIG. 8B). Two representative sequences are shown in illustration 2, VIMSS5327647 (Pfam hit: Extracellular solute-binding protein family 5) and VIMSS5324142 (Pfam hit: Extracellular solute-binding protein family 1). This type of proteins (according to Pfam's description) is known in gram(+) bacteria (containing no outer membrane) to be bound in the membrane via N-terminal lipid-anchors, indicating that EBI244 may also be attached to the extracellular side of the lipid membrane with its N-terminal hydrophobic amino-acid region.

Well-known methods, such as those employing software (free and commercially available services) may be used to predict signal sequences (see, for example, the Transmembrane helix and signal peptide prediction list available on the World Wide Web, at the URL cmgm.stanford.edu/WWW/www_predict.html, and the program "SignalP 3.0 Server," available on the World Wide Web at www.cbs.dtu.dk/services/SignalP. The SignalP 3.0 program was used to predict the location of a signal sequence for the polypeptide of SEQ ID NO: 1. Using this method, a cleavage site was predicted between amino acids 23 and 24. Thus, the predicted mature protein is 24-842 of SEQ ID NO: 1.

### Proline/threonine-rich region

The analysis revealed that the N-terminal putative signal peptide is followed by a one hundred (100) amino acid region, rich in threonine and proline. Threonine/proline rich regions are generally highly unstructured, often serving as flexible linkers in cellulases. Such sequences are known to be found in many types of proteins, including cellulases. The size of the threonine/proline-rich region in EBI244, as well as the degree of enrichment for threonine (44% - for the region 33-126) and proline (24% for the region 33-126) are highly unusual. In many cellulases, threonine/proline rich regions serve as linker domains, connecting different domains (*e.g.,* a catalytic domain connected to a cellulose-binding domain). In EBI244, however, this region is positioned too close to the N-terminus to be positioned between functional domains. Other deduced carbohydrate enzymes from the metagenome also showed threonine rich motifs at N- or C termini. None was as dramatic as the region from EBI244.

### Domains 1-4

Based on Hidden Markov Modeling (HMM), the remainder of the protein was predicted to encode up to four structural domains (Domains 1-4).

Hidden Markov Model (HMM) searching and analysis was carried out on the domain 1 region of EBI244. This searching and analysis identified sequences of thirty-eight (38) proteins, a non-redundant sample of which is shown in FIG. 8C. Table 1 lists the ID (GenBank Accession number or UniProt ID), start and stop amino acid positions for domain with identity to domain 1, e-value, protein length, and organism for each hit. The same information also is provided for EBI244 (with VIMSS5326244 listed as the ID. VIMSS5326244 is electronically designated by the sequence analysis software (microbes on line) for specific open reading frames (orfs). Prior to this work, none of the identified proteins had been experimentally characterized; almost all had only electronically-inferred annotations. Annotations varied among sequences, with a good number of glycoside hydrolases; many had no annotations.

Global alignment of sequences identified by domain 1 HMM revealed that the next domain in the carboxy direction (domain 2 in EBI244) was related among all these sequences. Thus, based on the HMM multiple sequence analysis, Domain 1 appeared always to be accompanied by Domain 2.

**Table 1: Protein sequence hits and e-values from domain 1 HMM searching.**

| **Protein ID** | **start** | **stop** | **e-value** | **length** | **Organism** |
|---|---|---|---|---|---|
| A9AYF5_HERA2 | 60 | 168 | 7.52E-29 | 591 | Herpetosiphon aurantiacus (strain ATCC 23779 / DSM 785) |
| VIMSS5326244 (EB144) | 157 | 273 | 1.56E-27 | 842 | 94C Metagenome |
| A4XMG8_CALS8 | 62 | 203 | 7.04E-27 | 611 | Caldicellulosiruptor saccharolyticus (strain ATCC 43494 / DSM 8903) |
| YP_003585990.1 | 26 | 127 | 4.45E-26 | 531 | Zunongwangia profunda SM-A87 |
| C7PTR3_CHIPD | 56 | 153 | 3.35E-25 | 557 | Chitinophaga pinensis (strain ATCC 43595 / DSM 2588 / NCIB 11800 / UQM 2034) |
| B1ZN60_OPITP | 52 | 169 | 2.46E-24 | 749 | Opitutus terrae (strain DSM 11246 / PB90-1) |
| DIN449_9BACT | 214 | 330 | 4.36E-24 | 777 | Victivallis vadensis ATCC BAA-548 |
| ZP_03628444.1 | 63 | 157 | 2.32E-23 | 559 | bacterium Ellin514 |
| NP_870950.1 | 148 | 240 | 2.84E-23 | 634 | Rhodopirellula baltica SH 1 |
| ZP 03626656.1 | 66 | 170 | 4.66E-23 | 1596 | bacterium Ellin514 |
| ZP_01717989.1 | 53 | 140 | 1.13E-22 | 542 | Algoriphagus sp. PR1 |
| YP_003323724.1 | 24 | 121 | 2.17E-22 | 528 | Thermobaculum terrenum ATCC BAA-798 |
| A7VX72 9CLOT | 37 | 157 | 1.49E-21 | 787 | Clostridium leptum DSM 753 |
| YP_001297703.1 | | | 2.91E-21 | 534 | Bacteroides vulgatus ATCC 8482 |
| ZP_05256313.1 | | | 2.94E-21 | 534 | Bacteroides sp. 4 3 47FAA |
| ZP 06742086.1 | | | 2.94E-21 | 534 | Bacteroides vulgatus PC510 |
| NP 228758.1 | | | 3.68E-21 | 509 | Thermotoga maritima MSB8 |
| ZP_04540112.1 | | | 1.28E-20 | 518 | Bacteroides sp. 9_1_42FAA |
| ZP_03298724.1 | | | 1.30E-20 | 534 | Bacteroides dorei DSM 17855 |
| ZP 04555706.1 | | | 1.30E-20 | 534 | Bacteroides sp. D4 |
| YP_003548440.1 | 382 | 466 | 5.86E-20 | 1258 | Coraliomargarita akajimensis DSM 45221 |
| YP 001819159.1 | | | 1.26E-19 | 536 | Opitutus terrae PB90-1 |
| YP 003195709.1 | 52 | 137 | 4.31E-19 | 1160 | Robiginitalea biformata HTCC2501 |
| ZP 03628309.1 | 55 | 148 | 6.44E-19 | 725 | bacterium Ellin514 |
| YP 003243090.1 | | | 1.18E-18 | 481 | Geobacillus sp. Y412MC10 |
| YP_764889.1 | | | 2.53E-18 | 506 | |
| YP_001819827.1 | | | 3.02E-17 | 570 | |
| YP 002278657.1 | | | 3.46E-17 | 506 | |
| NP 869354.1 | 472 | 574 | 4.06E-17 | 1043 | Rhodopirellula baltica SH 1 |
| YP_001818722.1 | | | 4.95E-16 | 648 | Opitutus terrae PB90-1 |
| YP_826861.1 | | | 1.18E-15 | 604 | |
| YP_001820771.1 | 62 | 148 | 1.35E-15 | 859 | Opitutus terrae PB90-1 |
| YP_003547883.1 | | | 4.64E-15 | 606 | Coraliomargarita akajimensis DSM 45221 |
| A7HFC4 ANADF | 6C | 150 | 9.41E-15 | 566 | Anaeromyxobacter sp. (strain Fw109-5) |
| ZP_04488111.1 | | | 1.60E-14 | 526 | |
| YP_003387974.1 | | | 1.61E-14 | 534 | Spirosoma linguale DSM 74 |
| ZP_02918195.1 | | | 2.30E-14 | 529 | Bifidobacterium dentiumATCC 27678 |
| YP_003547687.1 | 718 | 821 | 1.10E-12 | 1853 | Coraliomargarita akajimensis DSM 45221 |
| YP_003011267.1 | | | 3.54E-09 | 554 | |
| YNP18_461130 | | | | 311 | Microbial community from Yellowstone Hot Springs (Washburn Springs #1) |
| BISONR 127760 | | | | 597 | Bison Hot Spring Pool, Yellowstone 597(11FEB08 BISONR) |
| BISONS_6715 | | | | 777 | Bison Hot Spring Pool, Yellowstone (14JAN08 BISONS) |

Domain 2 represents the largest predicted domain of EBI244, and is the region having similarity to the known glycosyl-hydrolase family 5 (GH5) domain family glycosyl hydrolases, evidencing the protein's cellulase or similar sugar hydrolase activity. The sequence of the GH5 domain was determined to be highly divergent (Pfam server analysis; e-value = 1e-12) compared to previously characterized GH5 proteins. FIG. 8D shows a number of highly conserved residues across all sequences in the domain 2 region, including the two predicted catalytic residues of EBI244 (highlighted in yellow; glutamates 413 and 506).

Despite low sequence identity in this region across all sequences, the conservation of key residues, including the predicted catalytic residues glutamate 413 and glutamate 506, suggests a similar fold in this region. Other structural predictions revealed that the protein is a member of the glycosidase superfamily, within the TIM-barrel fold (InterProScan; e-values∼1e-27; see FIG. 9). FIG. 10 shows a schematic representation of the relationship of domain 2 of EBI244 to other glycosylhydrolases in this superfamily. Many of the known glycoside hydrolase families are within the TIM-barrel fold (the CAZY database shows at least 18), which includes GH5 (see Illustration 5). HMM analysis/searching carried out for domain 2 of EBI244 identified a very large number of significant hits.

Table 2 lists the ID (GenBank Accession number, UniProt ID), e-value, protein length, and organism for each hit, with the same information provided for EBI244 (listing VIMSS5326244 as the ID). As shown in Tables 1 and 2, many of the top hits (eight top hits) were the same protein sequences identified as top hits in the domain 1 searching. However, beyond those first eight, most of the hits were not identified in other domain searches indicating that they do not have very similar domains outside of domain 2.

Even though the sequence identity is very low in this region across all sequences, the high conservation of a number of residues, especially the predicted catalytic residues of EBI244, indicates that all of these sequences have the possibility of a similar fold in this region. The observation that the domain 1 region did not appear to be present in any protein not having this similar domain 2 region indicates that the function of the domain 1 region may be dependent on or affect the function of the domain 2 region.

**Table 2: Protein sequence hits and E-values from domain 2 HMM searching.**

| **Protein ID** | **e-val** | **length** |
|---|---|---|
| VIMSS5326244 (EBI244) | 7.01E-143 | 842 |
| A4XMG8_CALS8 | 1.93E-134 | 611 |
| A9AYF5 HERA2 | 3.55E-125 | 591 |
| B1ZN60_OPITP | 2.46E-123 | 749 |
| A7VX72_9CLOT | 2.26E-118 | 787 |
| B7BGD2 9PORP | 1.02E-116 | 470 |
| D1N449_9BACT | 1.39E-110 | 777 |
| C7PTR3_CHIPD | 3.82E-69 | 557 |
| YP_003585990.1 | 1.01E-61 | 531 |
| ZP_04378853.1 | 1.14E-56 | 446 |
| C7M3Y3_CAPOD | 1.16E-56 | 470 |
| ZP_03390557.1 | 6.80E-54 | 466 |
| A7HFC4_ANADF | 9.39E-46 | 566 |
| B0UPR0_METS4 | 3.32E-32 | 504 |
| B9RN03 RICCO | 1.67E-28 | 404 |
| C6T835_SOYBN | 2.75E-28 | 418 |
| XP_002264115.1 | 2.44E-25 | 433 |
| C7A7X8_MALDO | 2.58E-25 | 429 |
| VIMSS9423033 | 9.82E-25 | 431 |
| MAN7_ARATH | 9.86E-25 | 431 |
| XP_002281804.1 | 1.92E-24 | 433 |
| C7A7X6 9ERIC | 2.98E-24 | 433 |
| B2BMP9_PRUPE | 3.27E-24 | 431 |
| B9H4D6_POPTR | 4.59E-24 | 420 |
| XP_002272344.1 | 4.61E-24 | 402 |
| C7A7X7_MALDO | 4.84E-24 | 428 |
| Q9FT03_COFAR | 5.55E-24 | 416 |
| Q9P893_AGABI | 5.91E-24 | 439 |
| B9GRV2_POPTR | 7.04E-24 | 415 |
| C6TAY0_SOYBN | 7.20E-24 | 431 |
| XP_002270023.1 | 1.72E-23 | 403 |
| B9R7X5 RICCO | 1.90E-23 | 432 |
| VIMSS9886800 | 2.25E-23 | 379 |
| B2BMQ0_PRUPE | 2.91E-23 | 433 |
| Q2I011_HORVD | 2.92E-23 | 380 |
| B0FPH4_9ROSA | 3.26E-23 | 433 |
| Q0ZR47_THEHA | 3.56E-23 | 431 |

HMM searching on Domain 3 revealed only one significant hit (B1zn60_OPITP), which was also a hit in searching the other three domains. This hit appears co-linear with EBI244 except for the threonine rich N-terminus. Secondary structure predictions show mostly beta-sheets. Table 3 lists the ID (GenBank Accession number or UniProt ID) for each hit. The same information also is provided for EBI244 (with VIMSS5326244 listed as the ID). The start and stop positions of domain 3 in EB244 also are listed. 03379646.1 was unlikely a true domain hit because of low sequence identity

**Table 3: Protein sequence hits from domain 3 HMM searching.**

| **Domain 3** | **start** | **stop** |
|---|---|---|
| VIMSS5326244 | 605 | 734 |
| B1ZN60_OPITP | | |
| YP_003379646.1 | | |

Domain 4 is the C-terminal domain of EBI244. HMM search analysis of Domain 4 returned seven significant hit. As shown in FIG. 8E, all seven of these sequences aligned globally with EBI244, except over the domain 3 and T/P rich regions. This result indicates that Domain 4 is related in some way to domains 1 and 2. Given that only one other sequence aligned in the domain 3 region, domain 3 may have been added to EBI244 at some point in evolution or it was removed from an ancestor to the other proteins. Each of these seven sequences were top hits in the domain 2 searching; 6 of them showed up in the hits of domain 1 searching, providing further evidence of the link between domain 4 and the rest of the protein. Many of the domain 1 hits do not have a related region to domain 4.

Table 4 lists the ID (GenBank Accession number or UniProt ID), start and stop amino acid positions for domain with identity to domain 4, e-value, and organism for each hit. The same information also is provided for EBI244 (with VIMSS5326244 listed as the ID).

**Table 4: Protein sequence hits and E-values from domain 4 HMM searching.**

| **Domain 4** | **start** | **stop** | **e-val** | **length** | |
|---|---|---|---|---|---|
| A4XMG8_CALS8 | 534/557 | 604 | 5.64E-23 | 611 | Caldicellulosiruptor saccharolyticus (strain ATCC 43494 / DSM 8903) |
| VIMSS5326244 | 759/785 | 838 | 1.87E-21 | 842 | 94C Metagenome |
| A7VX72_9CLOT | 519/544 | 597 | 4.65E-20 | 787 | Clostridium leptumDSM 753 |
| B1ZN60_OPITP | 667/692 | 745 | 1.68E-18 | 749 | Opitutus terrae (strain DSM 11246 / PB90-1) |
| B0UPR0_METS4 | 423/447 | 501 | 1.10E-16 | 504 | Methylobacterium sp. (strain4-46) |
| D1N449_9BACT | 698/718 | 771 | 4.54E-16 | 777 | Victivallis vadensis ATCC BAA-548 |
| A7HFC4_ANADF | 493/510 | 562 | 1.59E-15 | 566 | Anaeromyxobacter sp. (strainn Fw109-5) |
| A9AYF5_HERA2 | 537 | 588 | 1.46E-14 | 591 | Herpetosiphon aurantiacus (strain ATCC 23779 / DSM 785) |

In summary, no highly similar BLAST hits resulted during searches with EBI244, implying that no known and sequenced Archaea or other hyperthermophiles in the NCBI non-redundant protein database have cellulase-encoding genes with the same domain structure as this enzyme. This enzyme occupies a highly divergent sequence space with less than 30% identity to the catalytic domain of the nearest characterized endoglucanase. Consideration of the weak homologs identified established that none are biochemically characterized, and the conserved glycosyl hydrolase family 5 catalytic domains of the hyperthermophilic cellulase is extremely divergent from characterized proteins of the family, with its nearest blast hits separated from known members of this family. Thus, this enzyme may represent the first characterized member of a highly divergent branch of the glycosyl hydrolase family 5 catalytic motif, or alternatively should be classified as the prototype a new glycosyl hydrolase family.

Thus, the EBI244 cellulase appears to represent a highly unusual type of glycosidase, based on structural alignments and sequence-based homolog searches. For example, the enzyme contains a highly divergent core catalytic domain and unusual domains flanking the catalytic domain. The few distant homologs of EBI244 in the public databases are distributed in organisms that occupy a broad swath of habitats, from rice paddies to mammalian intestines.

### Example 4: Expression and analysis of synthetic protein

An *ebi244* protein-coding region, having the nucleic acid sequence set forth in SEQ ID NO: 2 (original sequence with hyperthermophilic codon usage) was synthesized de novo by GenScript, ltd (Piscataway, NJ). A second version of the coding region, which was codon-optimized for expression in *E. coli* (SEQ ID NO: 3) also was synthesized by DNA 2.0 (Menlo Park, CA).

### Protein expression and purification

The 94kDa protein was expressed by autoinduction in *E. coli* and purified. Expression of the recombinant EBI244 protein in *E. coli* was carried out by the auto-induction (Studier, FW, Protein Expres. Purif. 41, 207-234, 2005).

Using this method, EBI244 was successfully expressed in two *E. coli* strains, BL21 (de3) and Rosetta cells (Invitrogen, Carlsbad, CA), as an N-terminally His tagged protein, from the plasmid pET16b, in shaking flasks or in a 17.5 L fermenter. For expression, each strain was transformed with plasmid and plated on YT media supplemented with 0.8% glucose at 35 °C . The pET16b N-terminal His-tagged gene appeared to be toxic, producing variable colony size. Only smaller colonies picked from freshly transformed plates resulted in significant expression. These were picked into a small volume of ZYP-0.8G media, 5 mL-50mL and incubated at 25°C until cells reached an optical density at 550nm of ~0.4. Then about 2.5mL was inoculated per liter of ZYP-5052 rich media for auto-induction.

Cells were then incubated with shaking at 20°C or 25°C for 48 hours or 36 hours respectively. Expression was optimized in 1 liter shake flask cultures, and subsequently scaled up to 17.5 L in a specialized New Brunswick Bioflow IV fermentor. Cells were grown to an OD 55 0nm of approximately 2.5-3.0 then harvested by centrifugation at 6,000 x g. Expression in the fermentor yielded 3-5 times higher levels of cellulose activity as compared with shake flasks. Cells were lysed by French Pressure Cell in 50mM Na phosphate buffer or 50mM HEPPS buffer and incubated for 30 min at 90°C. Denatured host proteins were removed by centrifugation at 8,000 x g for 15 minutes followed by 100,000 x g for 30 minutes and the cleared supernatant, representing a partially purified soluble fraction was used for immediate and downstream assays or purification

Expression levels were low (50 micrograms per g cells) but the protein was readily obtained in soluble form after heating whole cell extracts to 90°C.

C-terminal poly his-tagged codon optimized gene is expressed by a similar process, using well-known methods and plasmids. Recombinant protein was purified as follows: Clarified supernatants were fractionated by ammonium sulfate precipitation. The initial supernatant was brought to 20% saturating ammonium sulfate, centrifuged at 10,000 x g, and decanted. The supernatant was then brought to 40% saturating ammonium sulfate and centrifuged at 10,000 x g. The pellet fraction was resuspended in 50 mM phosphate buffer. The buffer was exchanged twice on a PES membrane centrifugal concentrator (Sartorius). Ammonium sulfate was added to a concentration of 500mM (sans potassium chloride and the protein was loaded on a hi-trap butyl-hydrophobic interaction column (GE Healthcare, Piscataway, NJ) and eluted with a linear gradient from 1M KCl to 0M KCl in 50 mM phosphate pH 7.0. The most active fractions were then pooled, buffer exchanged in 50mM borate (pH 9.5) and loaded on a Q sepharose fast flow column (GE Healthcare, Piscataway, NJ) and eluted with a potassium chloride gradient from 0M to 500 mM.

Additionally, an *ebi244* gene construct was generated by replacing the native signal peptide sequence of *ebi244* with the ompA signal peptide sequence from *E. coli.* The construct was generated by two rounds of amplification by PCR with primers that collectively reconstruct the signal peptide sequence from ompA in place of the native signal peptide sequence. The construct was subcloned into pet16b and expressed in *E. coli* Rosetta cells by standard IPTG induction at 25°C or autoinduction at 25°C. The replacement of the archaeal signal peptide with the ompA signal peptide resulted in increased expression of the new construct ebi 244-OA in *E*. *coli* as compared to the unmodified sequence ebi 244.

The nucleotide sequence ebi 244-OA is set forth as SEQ ID NO: 15. The amino acid sequence of the EBI244 encoded by ebi 244-OA is set forth as SEQ ID NO: 16.

The results of a comparison of the expression levels of EBI244 and EBI244-OA expression when induced with IPTG is shown in Table 5. Expression via auto-induction resulted in a 5-fold increase in the expression of EBI244-OA as compared to EBI244.

### Expression Results:

**Table 5: EBI244-OA Expression**

| **Method (25°C)** | **EBI244** | **EBI244-OA** |
|---|---|---|
| IPTG | N.D. | 18 µg/g cell pellet* |
| Auto-induction | 20 µg/g cell pellet | 100 µg/g cell pellet* |

### Analysis of purified protein

Activity of the recombinant protein was analyzed by a number of methods, as follows.

### Zymograms

Zymograms were performed as described above, with gels made as standard 8% SDS-PAGE gels, with 0.25% medium viscosity carboxymethyl cellulose incorporated into the gel. In the case of gradient gels the gels were 10% to 15% acrylamide and contained 0.20% CMC. Standard SDS-PAGE protocols were used, with standard loading buffer, with the exception that samples were kept at 20 °C and were not boiled prior to loading. Gels were gently agitated for 30 minutes in 50mM tris buffer pH 6.8 with 2% triton X-100, and then for 30 minutes in 50mM tris buffer, pH 6.8, to reactivate cellulases. Gels were then incubated in 50mM potassium phosphate, pH 6.8, or 50 mM HEPPS buffer, pH 6.8, for 3 hours at 90 °C. After incubation, the gels were cooled to 20 °C and stained with 0.5 % Congo Red (sodium salt of benzidinediazo-bis-1-naphthylamine-4-sulfonic acid (formula: C₃₂H₂₂N₆Na₂O₆S₂; molecular weight: 696.66 g/mol), for 40 minutes, then destained with 1M Tris Buffer, pH 6.8, for approximately 15 minutes. The dye then was set in 1 M MgCl₂.

### Reducing sugar assays

Reducing sugar assays were performed to detect the presence of reducing sugars. Dinitrosalicylic acid (DNS) reagent was made according to International Union of Pure and Applied Chemistry (IUPAC) guidelines. Results were calibrated to standard solutions of calaboose. Assays on CMC (carboxymethyl cellulose), Avicel^{®}, ionic liquid pretreated Avicel^{®} and Whatman^{®} #1 filter paper were carried out in 50 mM potassium phosphate pH 6.8 or 50 mM sodium acetate pH 5.0. Assays with high concentrations of salts or ionic liquids were carried out in Phosphate buffer. To compare activity at various pH levels, the following buffers were used 50mM sodium acetate/acetic acid pH 3.5, 4, 4.5, 5, 5.6; 50mM sodium phosphate buffer: pH 6, 6.5; 50mM MOPS: pH 7, 7.5; 50mM EPPS: pH 6.8, 8, 8.5, 9; 50mM CAPS: pH 9.5-11.1. Assays were generally conducted in 100 µL of buffer, in dome-capped PCR tubes, for a temperature of less than 99 °C, incubated in a bio-rad mycycler thermocycler with heated lid. Screw cap 1.5 mL polypropylene tubes in a silicone oil bath were used for temperature range from 99-114°C. Alternatively, assays from 100-130°C were conducted in 10 ml sealed serum stoppered Hungate tubes over-pressured with 30 psi of N₂ then incubated in a Binder oven. In the case of the Hungate tubes, controls were removed from the oven at the calculated time of temperature equilibration (equilibration times were calculated using standard equations for unsteady-state heat conduction, see for example, J.R. Welty, C.E. Wicks, and R.E. Wilson, Fundamentals of Momentum, Heat, and Mass Transfer, 3rd Edition, John Wiley & Sons, 1984, pp. 297-304) and stopped with the addition of an equal volume of cold 0.1 M sodium hydroxide.

Assays on alternative substrates described in Table 6 were done as follows: Pretreated substrates were treated as preciously described (Kim, T et al., Biotechnol. Bioeng, 2010). All cellulolytic assays for insoluble substrates were carried out in quadruplicate in a final volume of 70 µL containing 1%(w/v) substrate (glucan loading), 0.2 µM of the EBI244 and 100 mM sodium acetate buffer, pH 5.5 at 90°C in a thermal cycler (Applied Biosystems). Cellulase activities were measured for Avicel^{®}, Lichenan, AFEX pretreated corn stover, ionic-liquid pretreated Avicel^{®} (IL-Avicel^{®}), Miscanthus (IL-Miscanthus), and corn stover (IL-corn stover). The mixtures were incubated at 90°C for 15 h after which they were cooled to 4°C prior to measuring the amount of soluble reducing sugar released using the glucose oxidase-peroxidase assay as previously described (Kim, T et al., Biotechnol. Bioeng, 2010).

### Paranitrophenol-labeled glycosides

The chromogenic substrate 4-nitrophenyl-beta-D-glucopyranoside was utilized at 2.5 mM in sodium acetate buffer pH 5.0. Alternatively the chromogenic substrate 4-nitrophenyl-beta-D-cellobioside was utilized as a substrate in 100 mM sodium acetate buffer. Sodium acetate buffer containing 4-nitrophenol was used as a standard and reagent blank during assays at 95°C. Absorbance was measured at 410 nm. To compare activity at various pH levels, the following buffers were used at a buffer strength of 50mM: pH 2.5-5.5 acetate/acetic acid, pH 6.5 MES, pH 7.5 -8.5 HEPPS, pH 9.5-10.5 CAPS. All assays on PNP-substrates and standards were adjusted with an equal volume of 100 mM sodium hydroxide before recording the absorbance at 410 nm.

### Dionex product analysis

For Dionex product analysis, assay conditions were the same as those utilized for the DNS assay. Reactions were stopped with the addition of an equal volume of 0.1 M sodium hydroxide.

### Cellulose binding assay

Cellulose binding assays were carried out as follows. Soluble extract was adjusted to 50 mL in 25 mM HEPPS buffer pH 6.8 with 1 g of Avicel^{®}, then incubated at 80°C for 30 minutes with shaking. The suspension was centrifuged at 8,000 x g, the supernatant removed and the Avicel^{®} resuspended in 5mL of HEPPS buffer with 0.6% CHAPS detergent added. The suspension was centrifuged at 8,000 x g, the supernatant removed, and the Avicel^{®} resuspended in 5mL 0.6% CHAPS buffer, heated to 80°C, for 15 min with shaking. The suspension was centrifuged at 8,000 x g, the supernatant removed, and the Avicel^{®} re-suspended in 5 ml of 2.0% CHAPS at 25°C and shaken. The suspension then was centrifuged at 8,000 x g, the supernatant removed and the Avicel^{®} re-suspended in 5mL of 2% CHAPS and incubated at 80°C for 15 minutes. The suspension was centrifuged at 8,000 x g, the supernatant removed, and the Avicel^{®} re-suspended in 5mL of 2% CHAPS and incubated at 90°C for 30 minutes. The suspension was centrifuged at 8,000 x g and the supernatant removed.

### Endoglucanase activity of recombinant EBI244 on a wide range of high molecular weight carbohydrate substrates containing β1-4 linked glucose.

Zymograms performed on recombinantly expressed EBI244 proteins revealed endoglucanase activity of recombinant EBI244, both with and without a refolding step. As shown in FIG. 11, the behavior of the protein on zymogram gels was similar to that observed for active endoglucanase fractions from the archaeal enrichment. The enzyme was active on carboxymethyl cellulose in liquid assays as well.

The enzyme also showed activity on a range of high molecular weight carbohydrate substrates that contained β1-4 linked glucose (Table 6). Product analysis by fluorophore-assisted carbohydrate electrophoresis (FACE) revealed release of oligomers from Avicel^{®} (FIG. 12). Purified EBI244 was supplied with various cellulose oligomers at 95°C and the reaction was monitored over two hours. The reactions show the conversion of higher order oligomers into mixtures of cellobiose, cellotriose and cellotetraose. The reactions show a dramatic pattern of trans-glycosylation resulting in transient formation of oligomers up to dp (degree of polymerization) of eleven when starting with cellohexaose (FIG. 12A). The transglycosylation activity was not greatly enhanced by the presence of glucose (FIG. 12B) and the enzyme showed no significant activity on cellotriose or cellobiose (FIGs. 12C and 12D).

**Table 6: The specific activity of EBI 244 endoglucanase on different substrates.**

| **Substrate** | **Activity** | **Error (%)** |
|---|---|---|
| pNP-cellobioside | 178^{a} | 1 |
| CMC | 138^{a} | 5 |
| Barley Glucan | 518^{a} | 7 |
| Lichenan | 6296^{b} | 5 |
| Avicel | 1241^{b} | 3 |
| IL-Avicel | 8261^{b} | 2 |
| IL-Miscanthus | 1002^{b} | 4 |
| IL-Cornstover | 1318^{b} | 2 |
| AFEX Cornstover | 89^{b} | 5 |
| Xylan | NA | - |
| Mannan | NA | - |

In Table 6 above, "a" represents µmol GE/µmol Enzyme/min, "b" represents µmol GE/µmol Enzyme/15hr, and "GE" represents glucose equivalents. Substrates pretreated with Ionic Liquid (IL) and Ammonia Fiber Expansion (AFEX) are indicated. "NA" indicates no measurable activity.

Truncated versions of the EBI244 protein were analyzed for activity on PNP-cellobiose, CMC, and Avicel^{®} to determine potential functions for each domain. A truncation variant (EBI244 Δ1-127 V128M-hereafter EBI244ΔN) lacking the Thr/Pro rich region, maintained similar activity as the full length version on the PNP-cellbioside and CMC (data not shown). This result is expected because the threonine/proline rich region is predicted to be a highly flexible low complexity region. Domains 3 and 4 do not align to experimentally characterized domains, thus it is possible that these domains act as a cellulose binding domain (CBD) or function is protein-protein interactions. Truncations removing both domains 3 and 4, or just domain 4 alone, were constructed and expressed at higher levels than the full length protein, but were inactive against all substrates. This result indicates that domain 3, and possibly 4 as well, is required for the enzyme to remain active, possibly due to a stabilizing effect on the enzyme. Treatment of the recombinant enzyme with proteinase K at 50°C for 30 minutes, resulted in a uniform N-terminal truncation to threonine-121, determined by N-terminal Edman degradation. The proteinase treated enzyme showed similar mobility and activity to the EBI244ΔN variant, suggesting that the remainder of the protein forms an integrated structure that is inaccessible to proteinase K at 50°C.

### Amenability of the enzyme to ammonium sulfate fractionation and purification

The EBI244 enzyme also proved amenable to ammonium sulfate fractionation (see FIG. 13, showing results of a zymogram assay showing activity distributed among the 20-40 % saturating ammonium sulfate fractions, each represented by three lanes (undiluted (1.0), dilution 2 in 5 (0.4), and 1 in 5 (0.2); initial sample was soluble recombinant protein after pretreatment at 80 °C for 30 minutes; protein was precipitated using 20, 40, 60, and 90 % saturating ammonium sulfate), hydrophobic interaction chromatography (see FIG. 14, showing results of a DNS assay using 1 % low-viscosity carboxymethylcellulose as the substrate, with fractions 1-11 representing a linear gradient from 1 M to 0 M ammonium sulfate in potassium phosphate buffer, pH 7), and anion exchange chromatography.

The N-terminal histidine tagged enzyme, however, did not interact with a nickel or cobalt affinity column, presumably because the threonine rich N-terminal region occluded the tag. FIG. 15 shows a comassie stained SDS-PAGE gel demonstrating stepwise purification to 60 % purity, with the sample heated prior to ammonium sulfate fractionation. While this figure shows EBI244 that is approximately 60% pure, purities over 95% have been obtained.

### Thermostability

When assayed on 1% CMC (carboxymethyl cellulose) (DNS assay), 50 mM HEPPS buffer, the enzyme demonstrated almost no activity at 75°C , 50% maximal activity at ~92°C, and maximal activity at about 109°C. The results are shown in FIGs. 16 and 17, showing activity-temperature profiles of EBI244 on 1 % CMC.

The temperature profile of the enzyme on Whatman^{®} #1paper showed a similar trend, with overall activity decreasing with the increasing crystalline nature of the substrate (FIG. 18).

To assess thermostability, the enzyme was preincubated at 100 °C or 105 °C in HEPPS buffer, then assayed for activity on 1 % CMC at 90°C. The results, shown in FIGs. 19 and 20, demonstrate that the enzyme had a half-life of about 4.5 hours at 100°C, and about 34 minutes at 105°C. Additionally, the enzyme had a half-life of 10 min in HEPPS buffer, pH 6.8, at 108°C in the presence of microcrystalline cellulose (0.5% Avicel^{®}) (FIG. 21). Differential scanning calorimetry of the enzyme (FIG. 17, inset) showed a bifurcated transition with two Tm's of 111°C and 113°C.

### Stability and activity in high ionic strength,

Zymogram assays also revealed that the recombinant enzyme is active in solutions of high ionic strength. For this study, zymogram gels were made as described, then equilibrated to various salt concentrations at room temperature prior to incubation at 90°C. The results are presented in FIG. 22, showing the enzyme exhibited zymogram activity in up to 4 M sodium chloride and up to saturating potassium chloride.

A DNS assay was used to measure product formation for EBI244 with 1 % CMC in HEPES buffer with no salt added, 2.5 M sodium chloride, and 3.0 M KCl. The results, shown in FIG. 23, revealed that the initial reaction kinetics of the enzyme were linear in up to 2.5 M sodium chloride and 3.0 M potassium chloride, at rates about 40 % of that of buffer alone. These results indicate that the enzyme is very halotolerant but functions better at lower salt concentrations. Moreover, ionic detergents, including SDS, had little effect on enzyme activity or stability and both non-ionic and non-denaturing ionic detergents such as CHAPS stimulated activity (FIG. 24).

Given that EBI244 remained active under high (NaCl) to near-saturating (KCl) salt conditions (FIG. 25), its activity was measured in the presence of the ionic liquids 1,3-dimethylimidazolium dimethyl phosphate ([DMIM]DMP) and 1-ethyl-3-methylimidazole acetate ([EMIM]OAc), which could potentially be used to pretreat substrates like Miscanthus17. The concentrations tested, 25% and 50% (v/v), are well above the expected residual ionic liquid of 10-15% that may be carried over after pretreatment (18). CMCase activity was demonstrated in zymograms incubated at 90°C in 25% (v/v) of either ionic liquid (pH 6.8). EBI244 remained stable and active at 90°C in 25% [DMIM]DMP (FIG. 23). Interestingly, in these assays, the enzyme's Topt decreased in the presence of ionic liquids (FIG. 26), suggesting that denaturing effects of the ionic liquids may stimulate activity at lower temperatures at which the enzyme would otherwise be inactive.

The enzyme was also equilibrated in buffer with ionic liquid added in both zymogram assays and liquid DNS assays, with carboxymethylcellulose as the substrate. The enzyme was tested in two different ionic liquids, [DMIM]DMP and [EMIM]OAc. Zymogram activity was detected in gels incubated in 25% of either ionic liquid at 90°C in 50 mM phosphate buffer at pH 6.8. The enzyme was shown to be active in up to 50% 1,3-dimethylimidazolium dimethyl phosphate. The temperature of maximum activity was determined for different concentrations of this ionic liquid. FIG. 27 shows results from a DNS assay, representing temperature optima compiled from activity-temperature profiles of EBI244 in increasing amounts of the ionic liquid (DMIM) DMP. While the maximum active temperature declined with increasing ionic liquid, purified EBI244 was demonstrated to be active in liquid assays at high concentrations of ionic liquids through a wide range of temperatures.

FIG. 28 shows the results of a DNS assay measuring activity ofEBI244 on 1 % CMC in buffer alone, and in the presence of 40 % and 50 % [DIMM] DMP. As shown, the highest activities in the low temperature range from 50-80°C were recorded in the presence of ionic liquid, implying that the enzyme is activated at low temperature by the addition of ionic liquids.

### Tolerance for various detergents

All detergents tested, including SDS at 100 °C, had little effect on enzyme stability. No loss of activity was observed in non-ionic detergents, Triton x-100, NP-40, Tween 20. The enzyme was stable in up to 2% CHAPS (ionic non-denaturing detergent). Zymogram activity was retained after SDS-PAGE without the customary wash and refold steps, indicating a tolerance for 0.1-1% SDS at room temperature. The recombinant enzyme was pretreated at 100 °C with and without the addition of 0.1% SDS, then assayed by zymography at 90 °C, showing thermostability at 100 °C in the presence of 0.1% SDS (FIG. 29).

### Activity over a broad pH range

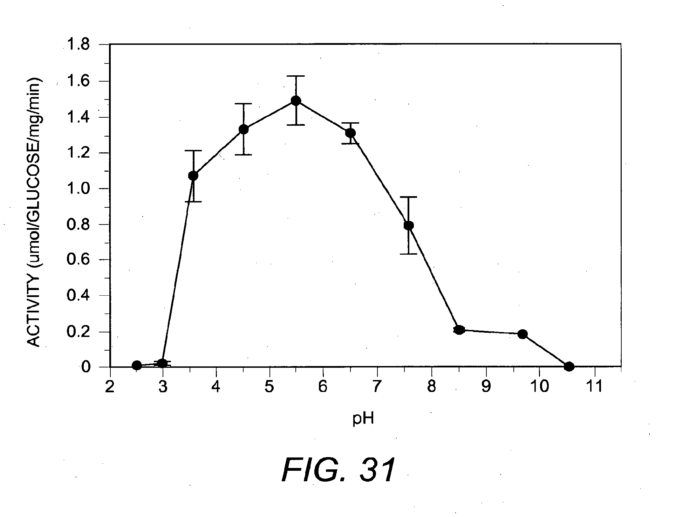

The enzyme retained activity over a very broad pH range with significant activity up to pH 8.5, as shown in FIG. 30 (showing results of a DNS assay of CMC hydrolysis over a broad pH range). Moreover, the enzyme had an optimum of about pH 5.5 (FIG. 31).

The results of this study demonstrate that the recombinant enzyme has cellulolytic activity, releasing reducing sugars from carboxymethyl-cellulose, microcrystalline cellulose (Avicel^{®}) and Whatman^{®} #1 filter paper, at reaction temperatures exceeding 105°C, with an optimal temperature range from 95-110°C. The results further demonstrate that the enzyme has a half-life of greater than five hours at 100°C and tolerates sodium chloride in near saturating concentrations (4M) at 90°C and potassium chloride at saturating concentration (∼3.2 M) at 90°C. The results further show that the enzyme is active toward carboxymethylcellulose in the presence of the ionic detergents CHAPS (2%) and sodium dodecyl sulfate (0.1 %) and to function in up 50% ionic liquids (i.e., 1,3-dimethylimidazolium dimethyl phosphate) at 90°C, and functions over an unusually broad range of pH, with greater than 50% of the maximum activity exhibited from pH 4.5-8.75.

The results demonstrate that the EBI244 enzyme is an extremely thermostable, thermoactive cellulose-binding endoglucanase, with a unique sequence composition. Because the enzyme maintains a high proportion of its activity over an exceptionally broad range of salinities, ionic strength, detergents, and pH, the enzyme is useful in providing cellulase activity suitable for long-term use under the broad and variable range of conditions encountered in industrial conditions. Furthermore, given the ability of EBI244 to bind tightly to crystalline cellulose, the enzyme will be useful in engineering hyperstable endocellulases for greater activity on crystalline substrates, for example, by the addition of thermostable cellulose binding domain, *e.g.,* the N-terminal and/or C-terminal domain(s) of EBI244 to catalytic domains.

Throughout this application, various website data content, publications, patent applications and patents are referenced. (Websites are referenced by their Uniform Resource Locator, or URL, addresses on the World Wide Web.)

The present invention is not to be limited in scope by the embodiments disclosed herein, which are intended as single illustrations of individual aspects of the invention, and any that are functionally equivalent are within the scope of the invention. Various modifications to the compositions and methods of the invention, in addition to those described herein, will become apparent to those skilled in the art from the foregoing description and teachings, and are similarly intended to fall within the scope of the invention. Such modifications or other embodiments can be practiced without departing from the true scope of the invention.

## Claims

1. An isolated protein, comprising a domain having an amino acid sequence at least 70 %, 75 %, 80 %, 85 %, 90%, 91%, 92%, 93%, 94%, 95%, 96%, 97%, 98%, or 99% identical or 100% identical to amino acids 250-580 of SEQ ID NO: 1,
further comprising a domain having an amino acid sequence at least 70 %, 75 %, 80 %, 85 %, 90%, 91%, 92%, 93%, 94%, 95%, 96%, 97%, 98%, or 99% identical or 100% identical to amino acids 580-750 of SEQ ID NO: 1,
further comprising a domain having an amino acid sequence at least 70 %, 75 %, 80 %, 85 %, 90%, 91%, 92%, 93%, 94%, 95%, 96%, 97%, 98%, or 99% identical or 100% identical to amino acids 750-842 of SEQ ID NO: 1
wherein the protein is a cellulase.

2. The isolated protein of claim 1, further comprising a domain having an amino acid sequence at least 70 %, 75 %, 80 %, 85 %, 90%, 91%, 92%, 93%, 94%, 95%, 96%, 97%, 98%, or 99% identical or 100% identical to amino acids 130-250 of SEQ ID NO: 1.

3. The isolated protein of any of claims 1-2, wherein the protein has an amino acid sequence at least 70 %, 75 %, 80 %, 85 %, 90%, 91%, 92%, 93%, 94%, 95%, 96%, 97%, 98%, or 99% identical or 100% identical to SEQ ID NO: 1.

4. The isolated protein of claims 1-3, wherein the protein exhibits cellulase activity at a temperature greater than 105°C.

5. The isolated protein of claims 1-3, wherein the protein exhibits cellulase activity at 95°C to 110°C.

6. An isolated nucleic acid encoding a protein of any of claims 1-3.

7. An expression vector, comprising the isolated nucleic acid of claim 6, operably linked to a regulatory sequence.

8. A host cell, comprising the expression vector of claim 7.

9. A composition comprising the isolated protein of claims 1-3.

10. A method of hydrolyzing or degrading biomass, comprising contacting said biomass with the composition of claim 9.

11. A method of textile cleaning comprising:
contacting a soiled textile with the composition of claim 9, to yield a clean textile.

12. A method of paper pulp bleaching comprising:
contacting paper pulp with the composition of claim 9, to yield bleached paper pulp.

13. A laundry detergent composition, comprising the protein of any of claims 1-3 and a detergent.

## Patentansprüche

1. Isoliertes Protein umfassend eine Domäne mit einer Aminosäuresequenz, die zumindest um 70 %, 75 %, 80 %, 85 %, 90 %, 91 %, 92 %, 93 %, 94 %, 95 %, 96 %, 97 %, 98 % oder 99 % oder 100 % mit Aminosäuren 250-580 gemäß SEQ ID NO: 1 identisch ist,
ferner umfassend eine Domäne mit einer Aminosäuresequenz, die zumindest um 70%, 75%, 80%, 85%, 90%, 91%, 92 %, 93 %, 94 %, 95 %, 96 %, 97 %, 98 % oder 99 % oder 100 % mit Aminosäuren 580-750 gemäß SEQ ID NO: 1 identisch ist,
ferner umfassend eine Domäne mit einer Aminosäuresequenz, die zumindest um 70%, 75%, 80%, 85%, 90%, 91%, 92 %, 93 %, 94 %, 95 %, 96 %, 97 %, 98 % oder 99 % oder 100 % mit Aminosäuren 750-842 gemäß SEQ ID NO: 1 identisch ist,
wobei das Protein eine Cellulase ist.

2. Isoliertes Protein nach Anspruch 1, ferner umfassend eine Domäne mit einer Aminosäuresequenz, die zumindest um 70 %, 75 %, 80 %, 85 %, 90 %, 91 %, 92 %, 93 %, 94 %, 95 %, 96 %, 97 %, 98 % oder 99 % oder 100 % mit Aminosäuren 130-250 gemäß SEQ ID NO: 1 identisch ist.

3. Isoliertes Protein nach einem der Ansprüche 1-2, wobei das Protein eine Aminosäuresequenz aufweist, die zumindest um 70 %, 75 %, 80 %, 85 %, 90 %, 91 %, 92 %, 93 %, 94 %, 95 %, 96 %, 97 %, 98 % oder 99 % oder 100 % mit SEQ ID NO: 1 identisch ist.

4. Isoliertes Protein nach Anspruch 1-3, wobei das Protein eine Cellulase-aktivität bei einer Temperatur höher als 105 °C aufweist.

5. Isoliertes Protein nach Anspruch 1-3, wobei das Protein eine Cellulase-aktivität bei 95 °C bis 110 °C aufweist.

6. Isolierte Nukleinsäure, die für ein Protein nach einem der Ansprüche 1-3 kodiert.

7. Expressionsvektor umfassend die isolierte Nukleinsäure nach Anspruch 6, die in operativer Weise mit einer regulatorischen Sequenz verbunden ist.

8. Wirtszelle umfassend den Expressionsvektor nach Anspruch 7.

9. Zusammensetzung umfassend das isolierte Protein nach Anspruch 1-3.

10. Verfahren für das Hydrolysieren oder den Abbau von Biomasse, umfassend das Inkontaktbringen der Biomasse mit der Zusammensetzung nach Anspruch 9.

11. Verfahren zur Textilreinigung umfassend:
Inkontaktbringen eines verschmutzten Textils mit der Zusammensetzung nach Anspruch 9, um ein sauberes Textil zu gewinnen.

12. Verfahren zum Bleichen von Papierzellstoff umfassend:
Inkontaktbringen von Papierzellstoff mit der Zusammensetzung nach Anspruch 9, um gebleichten Papierzellstoff zu gewinnen.

13. Waschmittelzusammensetzung umfassend das Protein nach einem der Ansprüche 1-3 und ein Reinigungsmittel.

## Revendications

1. Protéine isolée, comprenant un domaine ayant une séquence d'acides aminés au moins 70%, 75%, 80%, 85%, 90%, 91%, 92%, 93%, 94%, 95%, 96%, 97%, 98% ou 99% identiques ou 100% identiques aux acides aminés 250-580 de SEQ ID NO: 1;
comprenant en outre un domaine ayant une séquence d'acides aminés au moins 70%, 75%, 80%, 85%, 90%, 91%, 92%, 93%, 94%, 95%, 96%, 97%, 98% ou 99% identiques ou 100% identiques aux acides aminés 580-750 de SEQ ID NO: 1;
comprenant en outre un domaine ayant une séquence d'acides aminés au moins 70%, 75%, 80%, 85%, 90%, 91%, 92%, 93%, 94%, 95%, 96%, 97%, 98% ou 99% identiques ou 100% identiques aux acides aminés 750-842 de SEQ ID NO: 1
dans lequel la protéine est une cellulase.

2. Protéine isolée selon la revendication 1, comprenant en outre un domaine ayant une séquence d'acides aminés au moins 70%, 75%, 80%, 85%, 90%, 91%, 92%, 93%, 94%, 95%, 96%, 97%, 98% ou 99% identiques ou 100% identiques aux acides aminés 130-250 de SEQ ID NO: 1.

3. Protéine isolée selon l'une quelconque des revendications 1 à 2, dans lequel la protéine a une séquence d'acides aminés au moins 70%, 75%, 80%, 85%, 90%, 91%, 92%, 93%, 94%, 95%, 96%, 97%, 98% ou 99% identiques ou 100% identiques à SEQ ID NO: 1.

4. Protéine isolée selon les revendications 1 à 3, dans laquelle la protéine présente une activité cellulase à une température supérieure à 105°C.

5. Protéine isolée selon les revendications 1 à 3, dans laquelle la protéine présente une activité cellulase à une température comprise entre 95°C et 110°C.

6. Acide nucléique isolé codant pour une protéine selon l'une quelconque des revendications 1 à 3.

7. Vecteur d'expression comprenant l'acide nucléique isolé selon la revendication 6, lié de manière fonctionnelle à une séquence régulatrice.

8. Cellule hôte comprenant le vecteur d'expression selon la revendication 7.

9. Composition comprenant la protéine isolée selon les revendications 1 à 3.

10. Procédé d'hydrolyse ou de dégradation d'une biomasse, comprenant la mise en contact de ladite biomasse avec la composition selon la revendication 9.

11. Procédé de nettoyage des textiles comprenant:
la mise en contact d'un textile sali avec la composition selon la revendication 9, pour obtenir un textile propre.

12. Procédé de blanchiment de pâte à papier, comprenant:
la mise en contact de pâte à papier avec la composition selon la revendication 9, pour obtenir la pâte à papier blanchie.

13. Composition de détergent de blanchisserie, comprenant la protéine selon l'une quelconque des revendications 1 à 3 et un détergent
